(19) **Europäisches Patentamt**
**European Patent Office**
**Office européen des brevets**

(11) **EP 4 501 956 A2**

(12) **EUROPEAN PATENT APPLICATION**

(43) Date of publication:
05.02.2025  Bulletin 2025/06

(21) Application number: 24198835.1

(22) Date of filing: 30.03.2011

(51) International Patent Classification (IPC):
**C07K 16/00** (2006.01)

(52) Cooperative Patent Classification (CPC):
**A61P 43/00; C07K 14/70535; C07K 16/248;
C07K 16/28; C07K 16/2866;** A61K 2039/545;
C07K 16/00; C07K 2317/21; C07K 2317/24;
C07K 2317/52; C07K 2317/71; C07K 2317/72;
C07K 2317/92; C07K 2317/94; C07K 2319/30

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 30.03.2010   JP 2010079667
09.11.2010   JP 2010250830

(62) Document number(s) of the earlier application(s) in
accordance with Art. 76 EPC:
**17154576.7 / 3 181 581**
**11714860.1 / 2 552 955**

(71) Applicant: **Chugai Seiyaku Kabushiki Kaisha
Kita-ku
Tokyo 115-8543 (JP)**

(72) Inventors:
• **IGAWA, Tomoyuki**
  **Shizuoka, 412-8513 (JP)**
• **ISHII, Shinya**
  **Shizuoka, 412-8513 (JP)**
• **MAEDA, Atsuhiko**
  **Shizuoka, 412-8513 (JP)**
• **NAKAI, Takashi**
  **Shizuoka, 412-8513 (JP)**

(74) Representative: **Vossius & Partner
Patentanwälte Rechtsanwälte mbB
Siebertstrasse 3
81675 München (DE)**

Remarks:
•The complete document including Reference
Table(s) and the Sequence Listing(s) can be
downloaded from the EPO website
•This application was filed on 06.09.2024 as a
divisional application to the application mentioned
under INID code 62.
•Claims filed after the date of receipt of the divisional
application (Rule 68(4) EPC)

(54) **ANTIGEN-BINDING MOLECULES THAT PROMOTE ANTIGEN CLEARANCE**

(57)   An objective of the present invention is to provide methods for facilitating antigen-binding molecule-mediated antigen uptake into cells, methods for facilitating the reduction of antigen concentration in plasma, methods for increasing the number of antigens to which a single antigen-binding molecule can bind, methods for improving pharmacokinetics of antigen-binding molecules, antigen-binding molecules improved for facilitated antigen uptake into cells, antigen-binding molecules capable of facilitating the reduction of antigen concentration in plasma, antigen-binding molecules capable of repeatedly binding to antigens, antigen-binding molecules with improved pharmacokinetics, pharmaceutical composi-
tions comprising such an antigen-binding molecule, and methods for producing those described above. The present inventors discovered that antigen uptake into cells is facilitated by an antibody having human FcRn-binding activity at the plasma pH and a lower antigen-binding activity at the early endosomal pH than at the plasma pH; such antibodies can increase the number of antigens to which a single antibody molecule can bind; the reduction of antigen in plasma can be facilitated by administering such an antibody; and antibody pharmacokinetics can be improved by using such antibodies.

**EP 4 501 956 A2**

## Description

## Technical Field

The present invention relates to:

methods for facilitating antigen-binding molecule-mediated antigen uptake into cells;
methods for increasing the number of antigens to which a single antigen-binding molecule can bind;
methods for enhancing the reduction of plasma antigen concentration by administering antigen-binding molecules;
methods for improving pharmacokinetics of antigen-binding molecules;
methods for reducing total or free antigen concentration in plasma;
antigen-binding molecules that improve antigen uptake into cells;
antigen-binding molecules that have an increased number of binding antigens;
antigen-binding molecules capable of enhancing the reduction of plasma antigen concentration by administration of the molecules;
antigen-binding molecules with improved pharmacokinetics;
pharmaceutical compositions comprising the antigen-binding molecules;
methods for producing those described above; and the like.

Priority

The present invention claims the benefit of Japanese Patent Application No. 2010-079667, filed on March 30, 2010, and Japanese Patent Application No. 2010-250830, filed on November 9, 2010, the entire contents of which are incorporated be reference herein.

## Background Art

Antibodies are drawing attention as pharmaceuticals as they are highly stable in plasma and have few side effects. At present, a number of IgG-type antibody pharmaceuticals are available on the market and many antibody pharmaceuticals are currently under development (NPLs 1 and 2). Meanwhile, various technologies applicable to second-generation antibody pharmaceuticals have been reported, including those that enhance effector function, antigen-binding ability, pharmacokinetics, and stability, and those that reduce the risk of immunogenicity (NPL 3). In general, the requisite dose of an antibody pharmaceutical is very high. This, in turn, has led to problems, such as high production cost, as well as the difficulty in producing subcutaneous formulations. In theory, the dose of an antibody pharmaceutical may be reduced by improving antibody pharmacokinetics or improving the affinity between antibodies and antigens.

The literature has reported methods for improving antibody pharmacokinetics using artificial substitution of amino acids in constant regions (NPLs 4 and 5). Similarly, affinity maturation has been reported as a technology for enhancing antigen-binding ability or antigen-neutralizing activity (NPL 6). This technology enables enhancement of antigen-binding activity by introduction of amino acid mutations into the CDR region of a variable region or such. The enhancement of antigen-binding ability enables improvement of in vitro biological activity or reduction of dosage, and further enables improvement of in vivo efficacy (NPL 7).

The antigen-neutralizing capacity of a single antibody molecule depends on its affinity. By increasing the affinity, an antigen can be neutralized by smaller amount of an antibody. Various methods can be used to enhance the antibody affinity (NPL 6). Furthermore, if the affinity could be made infinite by covalently binding the antibody to the antigen, a single antibody molecule could neutralize one antigen molecule (a divalent antibody can neutralize two antigen molecules). However, the stoichiometric neutralization of one antibody against one antigen (one divalent antibody against two antigens) is the limit of pre-existing methods, and thus it is impossible to completely neutralize antigen with the smaller amount of antibody than the amount of antigen. In other words, the affinity enhancing effect has a limit (NPL 9). To prolong the neutralization effect of a neutralizing antibody for a certain period, the antibody must be administered at a dose higher than the amount of antigen produced in the body during the same period. With the improvement of antibody pharmacokinetics or affinity maturation technology alone described above, there is thus a limitation in the reduction of the required antibody dose. Accordingly, in order to sustain antibody's antigen-neutralizing effect for a target period with smaller amount of the antibody than the amount of antigen, a single antibody must neutralize multiple antigens. An antibody that binds to an antigen in a pH-dependent manner has recently been reported as a novel method for achieving the above objective (PTL 1). The pH-dependent antigen-binding antibodies, which strongly bind to an antigen under the neutral conditions in plasma and dissociate from the antigen under acidic conditions in the endosome, can dissociate from the antigen in the endosome. When a pH-dependent antigen-binding antibody dissociates from the antigen is recycled to the plasma by FcRn, it can bind to another antigen again. Thus, a single pH-dependent antigen-binding antibody can bind to a number of antigens repeatedly.

In addition, plasma retention of an antigen is very short as compared to antibodies recycled via FcRn binding. When an antibody with such long plasma retention binds to the antigen, the plasma retention time of the antigen-antibody complex is prolonged to the same as that of the antibody. Thus, the plasma retention of the antigen is prolonged by binding to the antibody, and thus the plasma antigen concentration is increased.

IgG antibody has longer plasma retention time as a result of FcRn binding. The binding between IgG and FcRn is only observed under an acidic condition (pH 6.0). By contrast, the binding is almost undetectable under a neutral condition (pH 7.4). IgG antibody is taken up into cells in a nonspecific manner. The antibody returns to the cell surface by binding to endosomal FcRn under the endosomal acidic condition, and then is dissociated from FcRn under the plasma neutral condition. When the FcRn binding under the acidic condition is lost by introducing mutations into the IgG Fc domain, absence of antibody recycling to the plasma from the endosome markedly impairs the antibody retention time in plasma. A reported method for improving the plasma retention of IgG antibody is to enhance the FcRn binding under acidic conditions. Amino acid mutations are introduced into the Fc domain of IgG antibody to improve the FcRn binding under acidic conditions. This increases the efficiency of recycling to the plasma from the endosome, resulting in improvement of the plasma retention. An important requirement in the amino acid substitution is not to augment the FcRn binding under neutral conditions. If an IgG antibody binds to FcRn under neutral conditions, the antibody returns to the cell surface by binding to FcRn under the endosomal acidic condition is not dissociated from FcRn under the plasma neutral condition. In this case, the plasma retention is rather lost because the IgG antibody is not recycled to the plasma. For example, as described in J Immunol. (2002) 169(9): 5171-80, an IgG1 antibody modified by introducing amino acid substations so that the resulting antibody is capable of binding to mouse FcRn under a neutral condition (pH 7.4) was reported to exhibit very poor plasma retention when administered to mice. Furthermore, as described in J Immunol. (2009) 182(12): 7663-71; J Biol Chem. 2007 Jan. 19; 282(3): 1709-17; and J Immunol. 2002 Nov. 1; 169(9): 5171-80, an IgG1 antibody has been modified by introducing amino acid substitutions so that the resulting antibody exhibits improved human FcRn binding under an acidic condition (pH 6.0) and at the same time becomes capable of binding to human FcRn under a neutral condition (pH 7.4). The resulting antibody was reported to show neither improvement nor alteration in the plasma retention when administered to cynomolgus monkeys. Thus, the antibody engineering technology for improving antibody functions has only focused on the improvement of antibody plasma retention by enhancing the human FcRn binding under acidic conditions without enhancing it under a neutral condition (pH 7.4). To date, there is no report describing the advantage of improving the human FcRn binding under a neutral condition (pH 7.4) by introducing amino acid substitutions into the Fc domain of an IgG antibody. Even if the antigen affinity of the antibody is improved, antigen elimination from the plasma cannot be enhanced. The above-described pH-dependent antigen-binding antibodies have been reported to be more effective as a method for enhancing antigen elimination from the plasma as compared to typical antibodies (PTL 1).

Thus, a single pH-dependent antigen-binding antibody binds to a number of antigens and is capable of facilitating antigen elimination from the plasma as compared to typical antibodies. Accordingly, the pH-dependent antigen-binding antibodies have effects not achieved by typical antibodies. However, to date, there is no report on antibody engineering methods for further improving the ability of pH-dependent antigen-binding antibodies to repeatedly bind to antigens and the effect of enhancing antigen elimination from the plasma.

Prior art documents related to the present invention are shown below:

**Citation List**

**Patent Literature**

[PTL 1] WO 2009/125825, ANTIGEN-BINDING MOLECULE CAPABLE OF BINDING TO TWO OR MORE ANTIGEN MOLECULES REPEATEDLY

**Non-Patent Literature**

[NPL 1] Monoclonal antibody successes in the clinic, Janice M Reichert, Clark J Rosensweig, Laura B Faden & Matthew C Dewitz, Nature Biotechnology 23, 1073 - 1078 (2005)
[NPL 2] Pavlou AK, Belsey MJ., The therapeutic antibodies market to 2008., Eur J Pharm Biopharm. 2005 Apr; 59(3): 389-96
[NPL 3] Kim SJ, Park Y, Hong HJ., Antibody engineering for the development of therapeutic antibodies., Mol Cells. 2005 Aug 31; 20(1): 17-29. Review
[NPL 4] Hinton PR, Xiong JM, Johlfs MG, Tang MT, Keller S, Tsurushita N., An engineered human IgG1 antibody with longer serum half-life., J Immunol. 2006 Jan 1; 176(1): 346-56

[NPL 5] Ghetie V, Popov S, Borvak J, Radu C, Matesoi D, Medesan C, Ober RJ, Ward ES., Increasing the serum persistence of an IgG fragment by random mutagenesis., Nat Biotechnol. 1997 Jul; 15(7): 637-40

[NPL 6] Proc Natl Acad Sci USA. 2005 Jun 14; 102(24): 8466-71. Epub 2005 Jun 6. A general method for greatly improving the affinity of antibodies by using combinatorial libraries. Rajpal A, Beyaz N, Haber L, Cappuccilli G, Yee H, Bhatt RR, Takeuchi T, Lerner RA, Crea R

[NPL 7] Wu H, Pfarr DS, Johnson S, Brewah YA, Woods RM, Patel NK, White WI, Young JF, Kiener PA. Development of Motavizumab, an Ultra-potent Antibody for the Prevention of Respiratory Syncytial Virus Infection in the Upper and Lower Respiratory Tract. J Mol Biol. (2007) 368: 652-665

[NPL 8] Hanson CV, Nishiyama Y, Paul S. Catalytic antibodies and their applications. Curr Opin Biotechnol. 2005 Dec; 16(6): 631-6

[NPL 9] Rathanaswami P, Roalstad S, Roskos L, Su QJ, Lackie S, Babcook J. Demonstration of an in vivo generated sub-picomolar affinity fully human monoclonal antibody to interleukin-8. Biochem Biophys Res Commun. 2005 Sep 9; 334(4): 1004-13

## Summary of Invention

### Technical Problem

The present invention was achieved in view of the circumstances described above. An objective of the present invention is to provide methods for facilitating antigen uptake into cells by using antigen-binding molecules, methods for increasing the number of antigens to which a single antigen-binding molecule can bind, methods for enhancing the reduction of plasma antigen concentration by administering antigen-binding molecules, methods for improving pharmacokinetics of antigen-binding molecules, antigen-binding molecules that facilitate antigen uptake into cells, antigen-binding molecules that have an increased number of binding antigens, antigen-binding molecules capable of enhancing the reduction of plasma antigen concentration by administration, antigen-binding molecules with improved pharmacokinetics, pharmaceutical compositions comprising the antigen-binding molecules, and methods for producing those described above.

### Solution to Problem

The present inventors conducted dedicated studies on methods for facilitating antigen uptake into cells via antigen-binding molecules (molecules, such as polypeptides, that have antigen-binding ability), methods for allowing antigen-binding molecules to repeatedly bind to antigens, methods for enhancing the reduction of plasma antigen concentration by administering antigen-binding molecules, and methods for improving plasma retention of antigen-binding molecules. As a result, the present inventors discovered that antigen-binding molecules that have human FcRn-binding ability at the early endosomal pH and higher human FcRn-binding activity than that of the intact human IgG-type immunoglobulin at the plasma pH could facilitate antigen uptake into cells. The present inventors also discovered that the antigen-binding molecule-mediated antigen uptake into cells could be further enhanced, and the number of antigens to which a single antigen-binding molecule can bind could be increased by using an antigen-binding molecule that has a weaker antigen-binding activity at the early endosomal pH than at the plasma pH; the reduction of plasma antigen concentration could be enhanced by administering such antigen-binding molecule; and the pharmacokinetics of an antigen-binding molecule could be improved.

Specifically, the present invention relates to:

methods for facilitating antigen-binding molecule-mediated antigen uptake into cells;
methods for increasing the number of antigens to which a single antigen-binding molecule can bind;
methods for enhancing the reduction of plasma antigen concentration by administering antigen-binding molecules;
methods for improving pharmacokinetics of antigen-binding molecules;
methods for reducing total or free antigen concentration in plasma;
antigen-binding molecules that improve antigen uptake into cells;
antigen-binding molecules that have an increased number of binding antigens;
antigen-binding molecules capable of enhancing the reduction of plasma antigen concentration by administration of the molecules;
antigen-binding molecules with improved pharmacokinetics;
pharmaceutical compositions comprising the antigen-binding molecules;
methods for producing those described above; and the like. More specifically, the present invention provides:

[1] an antigen-binding molecule comprising an antigen-binding domain and a human FcRn-binding domain, which has a human FcRn-binding activity in the acidic and neutral pH ranges, wherein the human FcRn-binding activity in the neutral pH range is stronger than 3.2 micromolar;

[2] an antigen-binding molecule comprising an antigen-binding domain and a human FcRn-binding domain, which has a human FcRn-binding activity in the neutral pH range, wherein the human FcRn-binding activity in the neutral pH range is 28 fold stronger than an intact human IgG;

[3] an antigen-binding molecule comprising an antigen-binding domain and a human FcRn-binding domain, which has a human FcRn-binding activity in the neutral pH range, wherein the human FcRn-binding activity in the neutral pH ranges is stronger than 2.3 micromolar;

[4] an antigen-binding molecule comprising an antigen-binding domain and a human FcRn-binding domain, which has a human FcRn-binding activity in the neutral pH range, wherein the human FcRn-binding activity in the neutral pH range is 38-fold stronger than an intact human IgG;

[5] the antigen-binding molecule of any one of [1] to [4], wherein the neutral pH range is pH 7.0 to 8.0;

[6] an antigen-binding molecule comprising an antigen-binding domain and a human FcRn-binding domain in which a total antigen concentration in plasma after administration of the antigen-binding molecule to non-human animal is lower than a total antigen concentration in plasma after administration of a reference antigen-binding molecule to non-human animal comprising the same antigen-binding domain and intact human IgG Fc domain as a human FcRn-binding domain;

[7] an antigen-binding molecule in which a plasma antigen concentration after administration of the antigen-binding molecule to non-human animal is lower than a total antigen concentration in plasma obtained from the non-human animal to which the antigen-binding molecule is not administered;

[8] an antigen-binding molecule comprising an antigen-binding domain and a human FcRn-binding domain wherein a molar antigen/antigen-binding molecule ratio (C) of the antigen-binding molecule calculated as follows;

$$C = A/B,$$

is lower than a molar antigen/antigen-binding molecule ratio (C') of an antigen-binding molecule comprising the same antigen-binding domain and intact human IgG Fc domain as a human FcRn-binding domain calculated as follows;

$$C'=A'/B',$$

wherein;

A is a total antigen concentration in plasma after administration of the antigen-binding molecule to non-human animal,
B is a plasma concentration of an antigen-binding molecule after administration of the antigen-binding molecule to non-human animal,
A' is a total antigen concentration in plasma after administration of a reference antigen-binding molecule to non-human animal,
B' is a plasma concentration of an antigen-binding molecule after administration of a reference antigen-binding molecule to non-human animal;

[9] the antigen-binding molecule of any one of [6] to [8], wherein the non-human animal is a human FcRn transgenic mouse;

[10] the antigen-binding molecule of any one of [6] to [9], wherein the antigen concentration in plasma is a long-term total antigen concentration in plasma;

[11] the antigen-binding molecule of any one of [6] to [9], wherein the antigen concentration in plasma is a short-term total antigen concentration in plasma;

[12] an antigen-binding molecule comprising an antigen-binding domain and a human FcRn-binding domain, which has a human FcRn-binding activity in the acidic and neutral pH ranges, wherein the human FcRn-binding activity in the neutral pH range is stronger than that of an intact human IgG;

[13] the antigen-binding molecule of any one of [1] to [11], wherein an antigen-binding activity of the antigen-binding domain in the acidic pH range is lower than that in the neutral pH range;

[14] the antigen-binding molecule of [12] or [13], wherein the ratio of antigen-binding activity in the acidic pH range and neutral pH range is at least 2 in the value of KD (in the acidic pH range) /KD (in the neutral pH range);

[15] the antigen-binding molecule of any one of [12] to [14], which comprises an amino acid mutation of the antigen-binding domain, which comprises a substitution of histidine for at least one amino acid of the antigen-binding domain or the insertion of at least one histidine;

[16] the antigen-binding molecule of any one of [12] to [14], wherein the antigen-binding domain is obtained from antigen-binding domain library;

[17] the antigen-binding molecule of any one of [1] to [16], which comprises as the human FcRn-binding domain an Fc domain resulting from substituting a different amino acid for at least one amino acid in the Fc domain of parent IgG;

[18] the antigen-binding molecule of any one of [1] to [17], wherein the human FcRn-binding domain is a human FcRn-binding domain comprising an amino acid sequence with a substitution of a different amino acid for at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the Fc domain of parent IgG;

[19] the antigen-binding molecule of any one of [1] to [18], which comprises a human FcRn-binding domain comprising amino acid substitution in the Fc domain of parent IgG which comprises at least one amino acid substitution selected from:

> an amino acid substitution of Met for Gly at position 237;
> an amino acid substitution of Ala for Pro at position 238;
> an amino acid substitution of Lys for Ser at position 239;
> an amino acid substitution of Ile for Lys at position 248;
> an amino acid substitution of Ala, Phe, Ile, Met, Gln, Ser, Val, Trp, or Tyr for Thr at position 250;
> an amino acid substitution of Phe, Trp, or Tyr for Met at position 252;
> an amino acid substitution of Thr for Ser at position 254;
> an amino acid substitution of Glu for Arg at position 255;
> an amino acid substitution of Asp, Glu, or Gln for Thr at position 256;
> an amino acid substitution of Ala, Gly, Ile, Leu, Met, Asn, Ser, Thr, or Val for Pro at position 257;
> an amino acid substitution of His for Glu at position 258;
> an amino acid substitution of Ala for Asp at position 265;
> an amino acid substitution of Phe for Asp at position 270;
> an amino acid substitution of Ala, or Glu for Asn at position 286;
> an amino acid substitution of His for Thr at position 289;
> an amino acid substitution of Ala for Asn at position 297;
> an amino acid substitution of Gly for Ser at position 298;
> an amino acid substitution of Ala for Val at position 303;
> an amino acid substitution of Ala for Val at position 305;
> an amino acid substitution of Ala, Asp, Phe, Gly, His, Ile, Lys, Leu, Met, Asn, Pro, Gln, Arg, Ser, Val, Trp, or Tyr for Thr at position 307;
> an amino acid substitution of Ala, Phe, Ile, Leu, Met, Pro, Gln, or Thr for Val at position 308;
> an amino acid substitution of Ala, Asp, Glu, Pro, or Arg for Leu or Val at position 309;
> an amino acid substitution of Ala, His, or Ile for Gln at position 311;
> an amino acid substitution of Ala, or His for Asp at position 312;
> an amino acid substitution of Lys, or Arg for Leu at position 314;
> an amino acid substitution of Ala, or His for Asn at position 315;
> an amino acid substitution of Ala for Lys at position 317;
> an amino acid substitution of Gly for Asn at position 325;
> an amino acid substitution of Val for Ile at position 332;
> an amino acid substitution of Leu for Lys at position 334;
> an amino acid substitution of His for Lys at position 360;
> an amino acid substitution of Ala for Asp at position 376;
> an amino acid substitution of Ala for Glu at position 380;
> an amino acid substitution of Ala for Glu at position 382;
> an amino acid substitution of Ala for Asn or Ser at position 384;
> an amino acid substitution of Asp, or His for Gly at position 385;
> an amino acid substitution of Pro for Gln at position 386;
> an amino acid substitution of Glu for Pro at position 387;
> an amino acid substitution of Ala, or Ser for Asn at position 389;
> an amino acid substitution of Ala for Ser at position 424;
> an amino acid substitution of Ala, Asp, Phe, Gly, His, Ile, Lys, Leu, Asn, Pro, Gln, Ser, Thr, Val, Trp, or Tyr for Met at position 428;

an amino acid substitution of Lys for His at position 433;
an amino acid substitution of Ala, Phe, His, Ser, Trp, or Tyr for Asn at position 434;
and an amino acid substitution of His for Tyr or Phe at position 436 in EU numbering;

[20] the antigen-binding molecule of any one of [1] to [18], whose human FcRn-binding domain comprises at least one amino acid selected from:

Met at amino acid position 237;
Ala at amino acid position 238;
Lys at amino acid position 239;
Ile at amino acid position 248;
Ala, Phe, Ile, Met, Gln, Ser, Val, Trp, or Tyr at amino acid position 250;
Phe, Trp, or Tyr at amino acid position 252;
Thr at amino acid position 254;
Glu at amino acid position 255;
Asp, Glu, or Gln at amino acid position 256;
Ala, Gly, Ile, Leu, Met, Asn, Ser, Thr, or Val at amino acid position 257;
His at amino acid position 258;
Ala at amino acid position 265;
Phe at amino acid position 270;
Ala or Glu at amino acid position 286;
His at amino acid position 289;
Ala at amino acid position 297;
Gly at amino acid position 298;
Ala at amino acid position 303;
Ala at amino acid position 305;
Ala, Asp, Phe, Gly, His, Ile, Lys, Leu, Met, Asn, Pro, Gln, Arg, Ser, Val, Trp, or Tyr at amino acid position 307;
Ala, Phe, Ile, Leu, Met, Pro, Gln, or Thr at amino acid position 308;
Ala, Asp, Glu, Pro, or Arg at amino acid position 309;
Ala, His, or Ile at amino acid position 311;
Ala or His at amino acid position 312;
Lys or Arg at amino acid position 314;
Ala or His at amino acid position 315;
Ala at amino acid position 317;
Gly at amino acid position 325;
Val at amino acid position 332;
Leu at amino acid position 334;
His at amino acid position 360;
Ala at amino acid position 376;
Ala at amino acid position 380;
Ala at amino acid position 382;
Ala at amino acid position 384;
Asp or His at amino acid position 385;
Pro at amino acid position 386;
Glu at amino acid position 387;
Ala or Ser at amino acid position 389;
Ala at amino acid position 424;
Ala, Asp, Phe, Gly, His, Ile, Lys, Leu, Asn, Pro, Gln, Ser, Thr, Val, Trp, or Tyr at amino acid position 428;
Lys at amino acid position 433;
Ala, Phe, His, Ser, Trp, or Tyr at amino acid position 434;
and His at amino acid position 436 (EU numbering) in the Fc domain of parent IgG;

[21] the antigen-binding molecule of any one of [18] to [20], wherein the parent IgG is selected from an IgG obtained from a non-human animal;
[22] the antigen-binding molecule of any one of [18] to [20], wherein the parent IgG is a human IgG;
[23] the antigen-binding molecule of any one of [1] to [22], which has an antagonistic activity;
[24] the antigen-binding molecule of [1] to [23], which binds to a membrane antigen or soluble antigen;
[25] the antigen-binding molecule of any one of [1] to [24], wherein the antigen-binding domain comprises an artificial ligand which binds to a receptor;

[26] the antigen-binding molecule of any one of [1] to [24], wherein the antigen-binding domain comprises an artificial receptor which binds to a ligand;

[27] the antigen-binding molecule of any one of [1] to [24], which is an antibody;

[28] the antigen-binding molecule of [27], wherein the antibody is selected from a chimeric antibody, a humanized antibody, or human antibody;

[29] a pharmaceutical composition comprising any one of the antigen-binding molecule of [1] to [28];

[30] a method for facilitating antigen-binding molecule-mediated antigen uptake into a cell by increasing its human FcRn-binding activity in the neutral pH range, wherein the antigen-binding molecule comprises an antigen-binding domain and a human FcRn-binding domain, and has a human FcRn-binding activity in the acidic pH range;

[31] a method for facilitating antigen-binding molecule-mediated antigen uptake into a cell by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range;

[32] a method for increasing the number of antigens to which a single antigen-binding molecule can bind by increasing its human FcRn-binding activity in the neutral pH range, wherein the antigen-binding molecule comprises an antigen-binding domain and a human FcRn-binding domain, and has a human FcRn-binding activity in the acidic pH range;

[33] a method for increasing the number of antigens to which a single antigen-binding molecule can bind by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range;

[34] a method for augmenting the ability of an antigen-binding molecule to eliminate an antigen from plasma by increasing its human FcRn-binding activity in the neutral pH range, wherein the antigen-binding molecule comprises an antigen-binding domain and a human FcRn-binding domain, and has a human FcRn-binding activity in the acidic pH range;

[35] a method for augmenting the ability of an antigen-binding molecule to eliminate an antigen from plasma by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range;

[36] a method for improving pharmacokinetics of an antigen-binding molecule by increasing its human FcRn-binding activity in the neutral pH range, wherein the antigen-binding molecule comprises an antigen-binding domain and a human FcRn-binding domain, and has a human FcRn-binding activity in the acidic pH range;

[37] a method for improving pharmacokinetics of an antigen-binding molecule by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range;

[38] a method for facilitating intracellular dissociation of an antigen bound to an antigen-binding molecule outside the cell from the antigen-binding molecule, by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range;

[39] a method for facilitating extracellular release of the antigen-free form of an antigen-binding molecule taken up into a cell in an antigen-bound form, by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range;

[40] a method for reducing total or free plasma antigen concentration in plasma, by increasing its human FcRn-binding activity in the neutral pH range, wherein the antigen-binding molecule comprises an antigen-binding domain and a human FcRn-binding domain, and has a human FcRn-binding activity in the acidic pH range;

[41] a method for reducing total or free plasma antigen concentration in plasma, by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range;

[42] the method of any one of [30] to [41], wherein the acidic pH range is pH 5.5 to pH 6.5 and the neutral pH range is pH 7.0 to pH 8.0;

[43] the method of any one of [30] to [41], wherein the increase in the human FcRn-binding activity in the neutral pH range is an increase by substituting a different amino acid for at least one amino acid in the parent IgG Fc domain of the human FcRn-binding domain;

[44] the method of any one of [30] to [41], wherein the increase in the human FcRn-binding activity in the neutral pH range is an increase by substituting a different amino acid for at least one amino acid selected from those at positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of the human FcRn-binding domain;

[45] the method of any one of [31], [33], [35], [37] to [39], and [41], wherein the antigen-binding activity of the antigen-binding molecule in the acidic pH range is reduced to less than that in the neutral pH range by substituting histidine for at least one amino acid of the antigen-binding molecule or inserting at least one histidine;

[46] The method of any one of [31], [33], [35], [37] to [39], and [41], wherein the antigen-binding domain is obtained from antigen-binding domain library;

[47] the method of any one of [31], [33], [35], [37] to [39], and [41], wherein the decrease in the antigen-binding activity is represented by an increase in the value of KD (in the acidic pH range) /KD (in the neutral pH range) which is a ratio of antigen-binding activity in the acidic pH range and neutral pH range, relative to before histidine substitution or insertion;

[48] a method for producing an antigen-binding molecule, which comprises the steps of:

(a) selecting an antigen-binding molecule that has stronger human FcRn-binding activity in the neutral pH range than 3.2 micromolar obtained by altering at least one amino acid in the human FcRn-binding domain of an antigen-binding molecule;
(b) obtaining a gene encoding an antigen-binding molecule in which a human FcRn-binding domain and an antigen-binding domain prepared in (a) are linked; and
(c) producing an antigen-binding molecule using the gene prepared in (b);

[49] a method for producing an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;
(b) altering at least one amino acid in the antigen-binding domain of an antigen-binding molecule and selecting an antigen-binding molecule that has stronger antigen-binding activity in the neutral pH range than in the acidic pH range;
(c) obtaining a gene encoding an antigen-binding molecule in which a human FcRn-binding domain and an antigen-binding domain prepared in (a) and (b) are linked; and
(d) producing an antigen-binding molecule using the gene prepared in (c); and

[50] a method for producing an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;
(b) selecting an antigen-binding molecule that has stronger antigen-binding activity in the neutral pH range than in the acidic pH range;
(c) obtaining a gene encoding an antigen-binding molecule in which a human FcRn-binding domain and an antigen-binding domain prepared in (a) and (b) are linked; and
(d) producing an antigen-binding molecule using the gene prepared in (c);

[51] an antigen-binding molecule produced by the production method of any one of [48] to [50];

[52] a method for screening an antigen-binding molecule, which comprises the steps of:

(a) selecting an antigen-binding molecule that has stronger human FcRn-binding activity in the neutral pH range than 3.2 micromolar obtained by altering at least one amino acid in the human FcRn-binding domain of an antigen-binding molecule;
(b) obtaining a gene encoding an antigen-binding molecule in which a human FcRn-binding domain and an antigen-binding domain prepared in (a) are linked; and

(c) producing an antigen-binding molecule using the gene prepared in (b);

[53] a method for screening an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;
(b) altering at least one amino acid in the antigen-binding domain of an antigen-binding molecule and selecting an antigen-binding molecule that has stronger antigen-binding activity in the neutral pH range than in the acidic pH range;
(c) obtaining a gene encoding an antigen-binding molecule in which a human FcRn-binding domain and an antigen-binding domain prepared in (a) and (b) are linked; and
(d) producing an antigen-binding molecule using the gene prepared in (c);

[54] a method for screening an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;
(b) selecting an antigen-binding molecule that has stronger antigen-binding activity in the neutral pH range than in the acidic pH range;
(c) obtaining a gene encoding an antigen-binding molecule in which a human FcRn-binding domain and an antigen-binding domain prepared in (a) and (b) are linked; and
(d) producing an antigen-binding molecule using the gene prepared in (c);

[55] The method of any one of [30] to [54], wherein the antigen-binding domain comprises an artificial ligand which binds to a receptor;
[56] the method of any one of [30] to [54], wherein the antigen-binding domain comprises an artificial receptor which binds to a ligand; and
[57] the method of any one of [30] to [54], wherein the antigen-binding molecule is an antibody.

**Advantageous Effects of Invention**

The present invention provides:
methods for facilitating antigen-binding molecule-mediated antigen uptake into cells; methods for increasing the number of antigens to which a single antigen-binding molecule can bind; and methods for enhancing the reduction of plasma antigen concentration by administering antigen-binding molecules. When the antigen-binding molecule-mediated antigen uptake into cells is facilitated, the reduction of plasma antigen concentration can be enhanced by administering such antigen-binding molecules, and the pharmacokinetics of antigen-binding molecule can be improved to increase the number of antigens to which a single antigen-binding molecule can bind. Thus, the antigen-binding molecules can produce more superior in vivo effects than ordinary antigen-binding molecules.

**Brief Description of Drawings**

[fig.1] Fig. 1 shows in a graph a time course of plasma concentration of the soluble form of human IL-6 receptor after administration of anti-human IL-6 receptor antibody to human FcRn transgenic mice (line 276) in which the plasma concentration of soluble form human IL-6 receptor is constant (steady-state infusion model).
[fig.2]Fig. 2 is a schematic diagram showing that dissociation of IgG antibody molecule from soluble antigen in the endosome results in enhancement of antigen elimination, leading to a new round of binding to another antigen.
[fig.3]Fig. 3 shows in a graph a time course of plasma antibody concentration in human FcRn transgenic mice.
[fig.4]Fig. 4 shows in a graph a time course of plasma concentration of the soluble form of human IL-6 receptor in human FcRn transgenic mice.
[fig.5]Fig. 5 shows in a graph a time course of plasma antibody concentration in normal mice.
[fig.6]Fig. 6 shows in a graph a time course of plasma concentration of the soluble form of human IL-6 receptor in normal mice.
[fig.7]Fig. 7 shows in a graph a time course of plasma concentration of the unbound soluble form of human IL-6 receptor in normal mice.

[fig.8]Fig. 8 shows in a graph a time course of plasma concentration of the soluble form of human IL-6 receptor in human FcRn transgenic mice.

[fig.9]Fig. 9 shows in a graph a time course of plasma concentration of the soluble form of human IL-6 receptor after administration of Fv4-IgG1-F14 at a low dose (0.01 mg/kg) or 1 mg/kg.

[fig.10] Fig. 10 shows in a graph a time course of plasma antibody concentration after administration of Fv4-IgG1-F14 at a low dose (0.01 mg/kg) or 1 mg/kg.

[fig.11] Fig. 11 shows in a graph a time course of plasma concentration of the soluble form of human IL-6 receptor after administration of anti-human IL-6 receptor antibody to normal mice in which the plasma concentration of soluble form human IL-6 receptor is constant.

[fig.12] Fig. 12 shows in a graph a time course of plasma antibody concentration after co-injection of hsIL-6R and anti-human IL-6 receptor antibody to human FcRn transgenic mice (line 276).

[fig.13] Fig. 13 shows in a graph a time course of plasma concentration of the soluble form of human IL-6 receptor after co-injection of hsIL-6R and anti-human IL-6 receptor antibody to human FcRn transgenic mice (line 276).

[fig.14] Fig. 14 describes the relationship between the binding affinity of Fc variants to human FcRn at pH 7.0 and plasma hsIL-6R concentration at day 1 after co-injection of hsIL-6R and anti-human IL-6 receptor antibody to human FcRn transgenic mice (line 276).

[fig.15] Fig. 15 describes the relationship between the binding affinity of Fc variants to human FcRn at pH 7.0 and plasma antibody concentration at day 1 after co-injection of hsIL-6R and anti-human IL-6 receptor antibody to human FcRn transgenic mice (line 276).

[fig.16] Fig 16 describes the time courses of molar antigen/antibody ratio (value C) after co-injection of hsIL-6R and anti-human IL-6 receptor antibody to human FcRn transgenic mice (line 276).

[fig.17] Fig. 17 describes the relationship between the binding affinity of Fc variants to human FcRn at pH 7.0 and molar antigen/antibody ratio (value C) at day 1 after co-injection of hsIL-6R and anti-human IL-6 receptor antibody to human FcRn transgenic mice (line 276).

[fig. 18]Fig. 18 shows in a graph a time course of plasma concentration of hsIL-6R after administration of Fv4-IgG1-F14 at lower doses (0.01 or 0.2 mg/kg) or 1 mg/kg to human FcRn transgenic mice (line 276) in which the plasma concentration of hsIL-6R is constant (steady-state infusion model).

[fig.19] Fig. 19 describes the time course of plasma hsIL-6R concentration in human FcRn transgenic mouse line 276 and line 32 after co-injection of hsIL-6R and anti-human IL-6 receptor antibody to human FcRn transgenic mice (line 276 and 32).

[fig.20]Fig. 20 describes the time course of plasma antibody concentration in human FcRn transgenic mouse line 276 and line 32 after co-injection of hsIL-6R and anti-human IL-6 receptor antibody to human FcRn transgenic mice (line 276 and 32).

[fig.21]Fig. 21 shows in a graph a time course of plasma concentration of hsIL-6R after administration of anti-human IL-6 receptor antibody to human FcRn transgenic mice (line 32) in which the plasma concentration of hsIL-6R is constant (steady-state infusion model).

[fig.22]Fig. 22 shows in a graph a time course of plasma concentration of antibody after administration of anti-human IL-6 receptor antibody to human FcRn transgenic mice (line 32) in which the plasma concentration of hsIL-6R is constant (steady-state infusion model).

[fig.23]Fig 23 describes the time courses of molar antigen/antibody ratio (value C) after administration of anti-human IL-6 receptor antibody to human FcRn transgenic mice (line 32) in which the plasma concentration of hsIL-6R is constant (steady-state infusion model).

[fig.24]Fig. 24 describes the relationship between the binding affinity of Fc variants to human FcRn at pH7.0 and molar antigen/antibody ratio (value C) at day 1 after administration of anti-human IL-6 receptor antibody to human FcRn transgenic mice (line 32) in which the plasma concentration of hsIL-6R is constant (steady-state infusion model).

[fig.25]Fig. 25 shows in a graph a time course of plasma antibody concentration after administration of anti-human IL-6 receptor antibodies having Fc variant of F11, F39, F48, and F264 to human FcRn transgenic mice (line 32) in which the plasma concentration of hsIL-6R is constant (steady-state infusion model).

[fig.26]Fig. 26 shows in a graph a time course of plasma concentration of hsIL-6R after administration of anti-human IL-6 receptor antibodies having Fc variant of F11, F39, F48, and F264 to human FcRn transgenic mice (line 32) in which the plasma concentration of hsIL-6R is constant (steady-state infusion model).

[fig.27]Fig. 27 shows in a graph a time course of plasma antibody concentration after administration of anti-human IL-6 receptor antibodies having Fc variant of F157, F196, and F262 to human FcRn transgenic mice (line 32) in which the plasma concentration of hsIL-6R is constant (steady-state infusion model).

[fig.28]Fig. 28 shows in a graph a time course of plasma concentration of hsIL-6R after administration of anti-human IL-6 receptor antibodies having Fc variant of F157, F196, and F262 to human FcRn transgenic mice (line 32) in which the plasma concentration of hsIL-6R is constant (steady-state infusion model).

[fig.29]Fig. 29 describes a pharmacokinetic model used for in silico study of conventional antibody and antigen eliminating antibody.

[fig.30]Fig. 30 shows in a graph a time course of plasma concentration of the human IL-6 after co-injection of human IL-6 and anti-human IL-6 antibody to normal mouse.

[fig.31]Fig. 31 shows in a graph a time course of plasma concentration of the antibody after co-injection of human IL-6 and anti-human IL-6 antibody to normal mouse.

[fig.32]Fig. 32 shows a sensorgrams of human IgA binding to CD89-Fc fusion protein at pH 7.4 and pH 6.0 using Biacore.

[fig.33]Fig. 33 shows in a graph a time course of plasma concentration of the human IgA after co-injection of human IgA and CD89-Fc fusion protein to normal mouse.

[fig.34]Fig. 34 shows in a graph a time course of plasma concentration of the antibody after co-injection of human IgA and CD89-Fc fusion protein to normal mouse.

[fig.35]Fig. 35 shows in a graph of plasma concentration of the soluble human plexin A1 at 7 hour after co-injection of soluble human plexin A1 and anti-human plexi A1 antibody to normal mouse.

## Description of Embodiments

The present invention provides methods for facilitating antigen-binding molecule-mediated antigen uptake into cells. More specifically, the present invention provides methods for facilitating the antigen uptake into cells by an antigen-binding molecule having human FcRn-binding activity in the acidic pH range, which are based on increasing the human FcRn-binding activity of the antigen-binding molecule in the neutral pH range. The present invention also provides methods for improving antigen uptake into cells by an antigen-binding molecule having human FcRn-binding activity in the acidic pH range, which are based on altering at least one amino acid in the human FcRn-binding domain of the antigen-binding molecule.

The present invention also provides methods for facilitating antigen uptake into cells by an antigen-binding molecule having human FcRn-binding activity in the acidic pH range, which are based on using a human FcRn-binding domain comprising an amino acid sequence with a substitution of a different amino acid for at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of the human FcRn-binding domain comprising the Fc domain of parent IgG.

The present invention also provides methods for facilitating antigen-binding molecule-mediated antigen uptake into cells, by reducing the antigen-binding activity (binding ability) in the acidic pH range of the above-described antigen-binding molecule to less than its antigen-binding activity in the neutral pH range; and this facilitates antigen uptake into cells. The present invention also provides methods for facilitating antigen-binding molecule-mediated antigen uptake into cells, which are based on altering at least one amino acid in the antigen-binding domain of the above-described antigen-binding molecule which facilitates antigen uptake into cells. The present invention also provides methods for facilitating antigen-binding molecule-mediated antigen uptake into cells, which are based on substituting histidine for at least one amino acid or inserting at least one histidine into the antigen-binding domain of the above-described antigen-binding molecule which facilitates antigen uptake into cells.

Herein, "antigen uptake into cells" mediated by an antigen-binding molecule means that antigens are taken up into cells by endocytosis. Meanwhile, herein, "facilitate the uptake into cells" means that the rate of intracellular uptake of antigen-binding molecule bound to an antigen in plasma is enhanced, and/or the quantity of recycling of uptaken antigen to the plasma is reduced. This means that the rate of uptake into cells is facilitated as compared to the antigen-binding molecule before increasing the human FcRn-binding activity of the antigen-binding molecule in the neutral pH range, or before increasing the human FcRn-binding activity and reducing the antigen-binding activity (binding ability) of the antigen-binding molecule in the acidic pH range to less than its antigen-binding activity in the neutral pH range. The rate is improved preferably as compared to intact human IgG, and more preferably as compared to intact human IgG. Thus, in the present invention, whether antigen uptake into cells is facilitated by an antigen-binding molecule can be assessed based on an increase in the rate of antigen uptake into cells. The rate of antigen uptake into cells can be calculated, for example, by monitoring over time reduction in the antigen concentration in the culture medium containing human FcRn-expressing cells after adding the antigen and antigen-binding molecule to the medium, or monitoring over time the amount of antigen uptake into human FcRn-expressing cells. Using methods of the present invention for facilitating the rate of antigen-binding molecule-mediated antigen uptake into cells, for example, the rate of antigen elimination from the plasma can be enhanced by administering antigen-binding molecules. Thus, whether antigen-binding molecule-mediated antigen uptake into cells is facilitated can also be assessed, for example, by testing whether the rate of antigen elimination from the plasma is accelerated or whether the total antigen concentration in plasma is reduced by administering an antigen-binding molecule.

Herein, "total antigen concentration in plasma" means the sum of antigen-binding molecule bound antigen and non-bound antigen concentration, or "free antigen concentration in plasma" which is antigen-binding molecule non-bound antigen concentration. Various methods to measure "total antigen concentration in plasma" or "free antigen concentration in plasma" is well known in the art as described hereinafter.

"Intact human IgG" as used herein is meant an unmodified human IgG and is not limited to a specific class of IgG. This means that human IgG1, IgG2, IgG3 or IgG4 can be used as "intact human IgG" as long as it can bind to the human FcRn in the acidic pH range. Preferably, "intact human IgG" can be human IgG1.

The present invention also provides methods for increasing the number of antigens to which a single antigen-binding molecule can bind. More specifically, the present invention provides methods for increasing the number of antigens to which a single antigen-binding molecule having human FcRn-binding activity in the acidic pH range can bind, by increasing the human FcRn-binding activity of the antigen-binding molecule in the neutral pH range. The present invention also provides methods for increasing the number of antigens to which a single antigen-binding molecule having human FcRn-binding activity in the acidic pH range can bind, by altering at least one amino acid in the human FcRn-binding domain of the antigen-binding molecule.

The present invention also provides methods for increasing the number of antigens to which a single antigen-binding molecule having human FcRn-binding activity in the acidic pH range can bind, by using a human FcRn-binding domain comprising an amino acid sequence in which at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of human FcRn-binding domain comprising an parent IgG Fc domain is substituted with a different amino acid.

"Parent IgG" as used herein means an unmodified IgG that is subsequently modified to generate a variant as long as a modified variant of parent IgG can bind to human FcRn in the acidic pH range (therefore, parent IgG does not necessary requires binding activity to human FcRn in the acidic condition). The parent IgG may be a naturally occurring IgG, or a variant or engineered version of a naturally occurring IgG. Parent IgG may refer to the polypeptide itself, compositions that comprise the parent IgG, or the amino acid sequence that encodes it. It should be noted that "parent IgG" includes known commercial, recombinantly produced IgG as outlined below. The origin of "parent IgG" is not limited and may be obtained from any organisms of non-human animals or human. Preferably, organism is selected from mouse, rat, guinea pig, hamster, gerbil, cat, rabbit, dog, goat, sheep, cow, horse, camel, and non-human primate. In another embodiment, "parent IgG" can also be obtained from cynomologous, marmoset, rhesus, chimpanzee or human. Preferably, "parent IgG" is obtained from human IgG1 but not limited to a specific class of IgG. This means that human IgG1, IgG2, IgG3, or IgG4 can be appropriately used as "parent IgG". In the similar manner, any class or subclass of IgGs from any organisms hereinbefore can be preferably used as "parent IgG". Example of variant or engineered version of a naturally occurring IgG is described in Curr Opin Biotechnol. 2009 Dec; 20(6): 685-91, Curr Opin Immunol. 2008 Aug; 20(4): 460-70, Protein Eng Des Sel. 2010 Apr; 23(4): 195-202, WO 2009/086320, WO 2008/092117, WO 2007/041635 and WO 2006/105338, but not limited thereto.

Furthermore, the present invention provides methods for increasing the number of antigens to which a single antigen-binding molecule can bind, by reducing the antigen-binding activity (binding ability) in the acidic pH range of the above-described antigen-binding molecule which has an increased number of antigen binding event to less than its antigen-binding activity in the neutral pH range. The present invention also provides methods for increasing the number of antigens to which a single antigen-binding molecule can bind, by altering at least one amino acid in the antigen-binding domain of the above-described antigen-binding molecule which has an increased number of antigen binding event. The present invention also provides methods for increasing the number of antigens to which a single antigen-binding molecule can bind, by substituting histidine for at least one amino acid or inserting at least one histidine into the antigen-binding domain of the above-described antigen-binding molecule which has an increased number of antigen binding event.

Herein, the "number of antigens to which a single antigen-binding molecule can bind" means the number of antigens to which a single antigen-binding molecule can bind until the molecule is eliminated due to degradation. Herein, "increasing the number of antigens to which a single antigen-binding molecule can bind" means an increase in the numbers of cycles achieved until the antigen-binding molecule is eliminated due to degradation, where each cycle consists of: binding of an antigen to the antigen-binding molecule in plasma, intracellular uptake of the antigen-binding molecule bound to the antigen, and dissociation from the antigen in the endosome, followed by return of the antigen-binding molecule to the plasma. This means that the number of cycles is increased as compared to the antigen-binding molecule before increasing the human FcRn-binding activity of the antigen-binding molecule in the neutral pH range, or before increasing the human FcRn-binding activity and reducing the antigen-binding activity (binding ability) of the antigen-binding molecule in the acidic pH range to less than its antigen-binding activity in the neutral pH range. Thus, whether the number of cycles is increased can be assessed by testing whether the above-described "intracellular uptake is facilitated" or whether the "pharmacokinetics is improved" as described below.

The present invention also provides methods for facilitating the intracellular dissociation of antigen from an antigen-binding molecule that binds to the antigen outside of the cell. More specifically, the present invention provides methods for facilitating the intracellular dissociation of antigen from an antigen-binding molecule that binds to the antigen outside of the cell, by increasing the human FcRn-binding activity in the neutral pH range of the antigen-binding molecule which has human FcRn-binding activity in the acidic pH range, and reducing its antigen-binding activity in the acidic pH range to less than that in the neutral pH range. The present invention also provides methods for facilitating the intracellular dissociation of antigen from an antigen-binding molecule that binds to the antigen outside of the cell, which are based on altering at least one amino acid in the antigen-binding domain of the antigen-binding molecule and simultaneously altering at least one amino acid in the human FcRn-binding domain of the antigen-binding molecule having human FcRn-binding activity in the acidic pH range. The present invention also provides methods for facilitating the intracellular dissociation of antigen from an antigen-binding molecule that binds to the antigen outside of the cell, by substituting for histidine at least one amino acid or inserting at least one histidine into the antigen-binding domain of the antigen-binding molecule and simultaneously substituting at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of the human FcRn-binding domain with a different amino acid.

In the present invention, antigens may be dissociated from the antigen-binding molecule anywhere inside the cell; however, a preferred dissociation site is early endosome. Herein, "intracellular dissociation of an antigen bound to an antigen-binding molecule outside of the cell from the antigen-binding molecule" does not necessarily mean that all of the antigens taken up into cells via binding to the antigen-binding molecule are dissociated from the antigen-binding molecule within the cell. Thus, it is acceptable that the proportion of antigens dissociated from the antigen-binding molecule within the cell is increased as compared to before reducing the antigen-binding activity of the antigen-binding molecule in the acidic pH range to less than that in the neutral pH range and simultaneously increasing the human FcRn-binding activity in the neutral pH range. Such method for facilitating intracellular dissociation of antigen from an antigen-binding molecule bound to the antigen outside of the cell is synonymous to a method for conferring on an antigen-binding molecule a property to facilitate intracellular dissociation of antigen from the antigen-binding molecule by facilitating the uptake of antigen-binding molecule bound to the antigen.

The present invention also provides methods for facilitating the extracellular release of antigen-free antigen-binding molecule taken up into cells in an antigen-bound form. More specifically, the present invention provides methods for facilitating the extracellular release of antigen-free antigen-binding molecule taken up into cells in an antigen-bound form, by increasing the human FcRn-binding activity in the neutral pH range of the antigen-binding molecule which has human FcRn-binding activity in the acidic pH range and reducing its antigen-binding activity in the acidic pH range to less than that in the neutral pH range. The present invention also provides methods for facilitating the extracellular release of antigen-free antigen-binding molecule taken up into cells in an antigen-bound form, which are based on altering at least one amino acid in an antigen-binding molecule and simultaneously altering at least one amino acid in the human FcRn-binding domain. The present invention also provides methods for facilitating the extracellular release of antigen-free antigen-binding molecule taken up into cells in an antigen-bound form, by substituting histidine for at least one amino acid or inserting at least one histidine into an antigen-binding molecule, and simultaneously substituting at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of the human FcRn-binding domain with a different amino acid.

Herein, the "extracellular release of antigen-free antigen-binding molecule taken up into cells in an antigen-bound form" does not necessarily mean that all of the antigen-binding molecules bound to antigen taken up into cells are released in an antigen-free form outside of the cell. It is acceptable that the proportion of antigen-binding molecules released in an antigen-free form to the outside of the cell is increased as compared to before reducing the antigen-binding activity of the antigen-binding molecule in the acidic pH range to less than that in the neutral pH range and increasing the human FcRn-binding activity in the neutral pH range. The antigen-binding molecule released to the outside of the cell preferably retains the antigen-binding activity. Such method for facilitating the extracellular release of antigen-free antigen-binding molecule taken up into cells in an antigen-bound form is synonymous to a method for conferring on an antigen-binding molecule a property to facilitate extracellular release of antigen-free antigen-binding molecule taken up into cells in an antigen-bound form by facilitating the uptake of antigen-binding molecules bound to antigen into cells.

The present invention also provides methods for increasing the ability to eliminate plasma antigen by administering antigen-binding molecules. In the present invention, "methods for increasing the ability to eliminate plasma antigen" is synonymous to "methods for augmenting the ability of an antigen-binding molecule to eliminate antigen from plasma". More specifically, the present invention provides methods for increasing the ability to eliminate plasma antigen by an antigen-binding molecule having human FcRn-binding activity in the acidic pH range, by increasing the human FcRn-binding activity of the antigen-binding molecule in the neutral pH range. The present invention also provides methods for increasing the ability to eliminate plasma antigen by an antigen-binding molecule having human FcRn-binding activity in the acidic pH range, which are based on altering at least one amino acid in the human FcRn-binding domain of the antigen-binding molecule.

The present invention also provides methods for increasing the ability to eliminate plasma antigen by an antigen-binding molecule having human FcRn-binding activity in the acidic pH range, by using a human FcRn-binding domain comprising an amino acid sequence with a substitution of at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of the human FcRn-binding domain comprising the Fc domain of parent IgG with a different amino acid.

The present invention also provides methods for increasing the ability to eliminate plasma antigen by an antigen-binding molecule, by reducing the antigen-binding activity in the acidic pH range of the above-described antigen-binding molecule with improved ability to eliminate plasma antigen as compared to the antigen-binding activity in the neutral pH range. The present invention also provides methods for increasing the ability to eliminate plasma antigen by an antigen-binding molecule, by altering at least one amino acid in the antigen-binding domain of the above-described antigen-binding molecule with improved ability to eliminate plasma antigen. The present invention also provides methods for increasing the ability to eliminate plasma antigen by administering an antigen-binding molecule, by substituting histidine for at least one amino acid or inserting at least one histidine into the antigen-binding domain of the above-described antigen-binding molecule with improved ability to eliminate plasma antigen.

Herein, the "ability to eliminate plasma antigen" means the ability to eliminate antigen from the plasma when antigen-binding molecules are administered or secreted in vivo. Thus, "increase in the ability of antigen-binding molecule to eliminate plasma antigen" herein means that the rate of antigen elimination from the plasma is accelerated upon administration of the antigen-binding molecule as compared to before increasing the human FcRn-binding activity of the antigen-binding molecule in the neutral pH range or before increasing the human FcRn-binding activity and simultaneously reducing its antigen-binding activity in the acidic pH range to less than that in the neutral pH range. Increase in the activity of an antigen-binding molecule to eliminate antigen from the plasma can be assessed, for example, by administering a soluble antigen and an antigen-binding molecule in vivo, and measuring the concentration of the soluble antigen in plasma after administration. When the concentration of soluble antigen in plasma after administration of the soluble antigen and antigen-binding molecule is reduced by increasing the human FcRn-binding activity of the antigen-binding molecule in the neutral pH range, or by increasing its human FcRn-binding activity and simultaneously reducing its antigen-binding activity in the acidic pH range to less than that in the neutral pH range, the ability of antigen-binding molecule to eliminate plasma antigen can be judged to be increased. A form of soluble antigen can be antigen-binding molecule bound antigen or antigen-binding molecule non-bound antigen whose concentration can be determined as "antigen-binding molecule bound antigen concentration in plasma" and "antigen-binding molecule non-bound antigen concentration in plasma" respectively (The latter is synonymous to "free antigen concentration in plasma". Since "total antigen concentration in plasma" means the sum of antigen-binding molecule bound antigen and non-bound antigen concentration, or "free antigen concentration in plasma" which is antigen-binding molecule non-bound antigen concentration, the concentration of soluble antigen can be determined as "total antigen concentration in plasma". Various methods for measuring "total antigen concentration in plasma" or "free antigen concentration in plasma" are well known in the art as described hereinafter.

The present invention also provides methods for improving the pharmacokinetics of antigen-binding molecules. More specifically, the present invention provides methods for improving the pharmacokinetics of the antigen-binding molecule having human FcRn-binding activity in the acidic pH range by increasing the human FcRn-binding activity of the antigen-binding molecule in the neutral pH range. Furthermore, the present invention provides methods for improving the pharmacokinetics of an antigen-binding molecule having human FcRn-binding activity in the acidic pH range by altering at least one amino acid in the human FcRn-binding domain of the antigen-binding molecule.

The present invention also provides methods for improving the pharmacokinetics of an antigen-binding molecule having human FcRn-binding activity in the acidic pH range by using a human FcRn-binding domain comprising an amino acid sequence with a substitution of different amino acid for at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of the human FcRn-binding domain comprising the Fc domain of IgG.

Furthermore, the present invention provides methods for improving the pharmacokinetics of an antigen-binding molecule, by reducing the antigen-binding activity in the acidic pH range of the above-described antigen-binding molecule with improved pharmacokinetics to less than its antigen-binding activity in the neutral pH range. The present invention also provides methods for improving the pharmacokinetics of an antigen-binding molecule having human FcRn-binding activity in the acidic pH range, by altering at least one amino acid in the antigen-binding domain of the above-described antigen-binding molecule with improved pharmacokinetics. The present invention also provides methods for improving the pharmacokinetics by substituting histidine for at least one amino acid or inserting at least one histidine into the antigen-binding domain of the above-described antigen-binding molecule with improved pharmacokinetics.

Herein, "enhancement of pharmacokinetics", "improvement of pharmacokinetics", and "superior pharmacokinetics" can be restated as "enhancement of plasma (blood) retention", "improvement of plasma (blood) retention", "superior plasma (blood) retention", and "prolonged plasma (blood) retention". These terms are synonymous.

Herein, "improvement of pharmacokinetics" means not only prolongation of the period until elimination from the plasma (for example, until the antigen-binding molecule is degraded intracellularly or the like and cannot return to the plasma) after administration of the antigen-binding molecule to humans, or non-human animals such as mice, rats, monkeys, rabbits, and dogs, but also prolongation of the plasma retention of the antigen-binding molecule in a form that allows antigen binding (for example, in an antigen-free form of the antigen-binding molecule) during the period of administration to elimination due to degradation. Intact human IgG can bind to FcRn from non-human animals. For example, mouse can be preferably used to be administered in order to confirm the property of the antigen-binding molecule of the invention since intact human IgG can bind to mouse FcRn stronger than to human FcRn (Int Immunol. 2001 Dec; 13(12): 1551-9). As another example, mouse in which its native FcRn genes are disrupted and a transgene for human FcRn gene is harbored to be expressed (Methods Mol Biol. 2010; 602: 93-104) can also be preferably used to be administered in order to confirm the property of the antigen-binding molecule of the invention described hereinafter. Specifically, "improvement of pharmacokinetics" also includes prolongation of the period until elimination due to degradation of the antigen-binding molecule not bound to antigens (the antigen-free form of antigen-binding molecule). The antigen-binding molecule in plasma cannot bind to a new antigen if the antigen-binding molecule has already bound to an antigen. Thus, the longer the period that the antigen-binding molecule is not bound to an antigen, the longer the period that it can bind to a new antigen (the higher the chance of binding to another antigen). This enables reduction of the time period that an antigen is free of the antigen-binding molecule in vivo and prolongation of the period that an antigen is bound to the antigen-binding molecule. The plasma concentration of the antigen-free form of antigen-binding molecule can be increased and the period that the antigen is bound to the antigen-binding molecule can be prolonged by accelerating the antigen elimination from the plasma by administration of the antigen-binding molecule. Specifically, herein "improvement of the pharmacokinetics of antigen-binding molecule" includes the improvement of a pharmacokinetic parameter of the antigen-free form of the antigen-binding molecule (any of prolongation of the half-life in plasma, prolongation of mean retention time in plasma, and impairment of plasma clearance), prolongation of the period that the antigen is bound to the antigen-binding molecule after administration of the antigen-binding molecule, and acceleration of antigen-binding molecule-mediated antigen elimination from the plasma. The improvement of pharmacokinetics of antigen-binding molecule can be assessed by determining any one of the parameters, half-life in plasma, mean plasma retention time, and plasma clearance for the antigen-binding molecule or the antigen-free form thereof ("Pharmacokinetics: Enshu-niyoru Rikai (Understanding through practice)" Nanzando). For example, the plasma concentration of the antigen-binding molecule or antigen-free form thereof is determined after administration of the antigen-binding molecule to mice, rats, monkeys, rabbits, dogs, or humans. Then, each parameter is determined. When the plasma half-life or mean plasma retention time is prolonged, the pharmacokinetics of the antigen-binding molecule can be judged to be improved. The parameters can be determined by methods known to those skilled in the art. The parameters can be appropriately assessed, for example, by noncompartmental analysis using the pharmacokinetics analysis software WinNonlin (Pharsight) according to the appended instruction manual. The plasma concentration of antigen-free antigen-binding molecule can be determined by methods known to those skilled in the art, for example, using the assay method described in Clin Pharmacol. 2008 Apr; 48(4): 406-17.

Herein, "improvement of pharmacokinetics" also includes prolongation of the period that an antigen is bound to an antigen-binding molecule after administration of the antigen-binding molecule. Whether the period that an antigen is bound to the antigen-binding molecule after administration of the antigen-binding molecule is prolonged can be assessed by determining the plasma concentration of free antigen. The prolongation can be judged based on the determined plasma concentration of free antigen or the time period required for an increase in the ratio of free antigen concentration to the total antigen concentration.

The plasma concentration of free antigen not bound to the antigen-binding molecule or the ratio of free antigen concentration to the total concentration can be determined by methods known to those skilled in the art, for example, by the method described in Pharm Res. 2006 Jan; 23 (1): 95-103. Alternatively, when an antigen exhibits a particular function in vivo, whether the antigen is bound to an antigen-binding molecule that neutralizes the antigen function (antagonistic molecule) can be assessed by testing whether the antigen function is neutralized. Whether the antigen function is neutralized can be assessed by assaying an in vivo marker that reflects the antigen function. Whether the antigen is bound to an antigen-binding molecule that activates the antigen function (agonistic molecule) can be assessed by assaying an in vivo marker that reflects the antigen function.

Determination of the plasma concentration of free antigen and ratio of the amount of free antigen in plasma to the amount of total antigen in plasma, in vivo marker assay, and such measurements are not particularly limited; however, the assays are preferably carried out after a certain period of time has passed after administration of the antigen-binding molecule. In the present invention, the period after administration of the antigen-binding molecule is not particularly limited; those skilled in the art can determine the appropriate period depending on the properties and the like of the administered antigen-binding molecule. Such periods include, for example, one day after administration of the antigen-binding molecule, three days after administration of the antigen-binding molecule, seven days after administration of the antigen-binding molecule, 14 days after administration of the antigen-binding molecule, and 28 days after administration of the antigen-binding molecule. Herein, "plasma antigen concentration" means either "total antigen concentration in plasma" which is the sum of antigen-binding molecule bound antigen and non-bound antigen concentration or "free antigen concentration in plasma" which is antigen-binding molecule non-bound antigen concentration.

Total antigen concentration in plasma can be lowered by administration of antigen-binding molecule of the present invention by 2-fold, 5-fold, 10-fold, 20-fold, 50-fold, 100-fold, 200-fold, 500-fold, 1,000-fold, or even higher compared to the administration of a reference antigen-binding molecule comprising the intact human IgG Fc domain as a human FcRn-binding domain or compared to when antigen-binding domain molecule of the present invention is not administered.

Molar antigen/antigen-binding molecule ratio can be calculated as shown below;

value A: Molar antigen concentration at each time point
value B: Molar antigen-binding molecule concentration at each time point
value C: Molar antigen concentration per molar antigen-binding molecule concentration (molar antigen/antigen-binding molecule ratio) at each time point

$$C = A/B.$$

Smaller value C indicates higher efficiency of antigen elimination per antigen-binding molecule whereas higher value C indicates lower efficiency of antigen elimination per antigen-binding molecule.

Molar antigen/antigen-binding molecule ratio can be calculated as described above.

Molar antigen/antigen-binding molecule ratio can be lowered by administration of antigen-binding molecule of present invention by 2-fold, 5-fold, 10-fold, 20-fold, 50-fold, 100-fold, 200-fold, 500-fold, 1,000-fold, or even higher as compared to the administration of a reference antigen-binding molecule comprising the intact human IgG Fc domain as a human FcRn-binding domain.

Herein, an intact human IgG1, IgG2, IgG3 or IgG4 is preferably used as the intact human IgG for a purpose of a reference intact human IgG to be compared with the antigen-binding molecules for their human FcRn binding activity or in vivo activity. Preferably, a reference antigen-binding molecule comprising the same antigen-binding domain as an antigen-binding molecule of the interest and intact human IgG Fc domain as a human FcRn-binding domain can be appropriately used. More preferably, an intact human IgG1 is used for a purpose of a reference intact human IgG to be compared with the antigen-binding molecules for their human FcRn binding activity or in vivo activity.

Reduction of total antigen concentration in plasma or molar antigen/antibody ratio can be assessed as described in Examples 6, 8, and 13. More specifically, using human FcRn transgenic mouse line 32 or line 276 (Jackson Laboratories, Methods Mol Biol. 2010; 602: 93-104), they can be assessed by either antigen-antibody co-injection model or steady-state antigen infusion model when antigen-binding molecule do not cross-react to the mouse counterpart antigen. When an antigen-binding molecule cross-react with mouse counterpart, they can be assessed by simply injecting antigen-binding molecule to human FcRn transgenic mouse line 32 or line 276 (Jackson Laboratories). In co-injection model, mixture of antigen-binding molecule and antigen is administered to the mouse. In steady-state antigen infusion model, infusion pump containing antigen solution is implanted to the mouse to achieve constant plasma antigen concentration, and then antigen-binding molecule is injected to the mouse. Test antigen-binding molecule is administered at same dosage. Total antigen concentration in plasma, free antigen concentration in plasma and plasma antigen-binding molecule concentration is measured at appropriate time point using method known to those skilled in the art.

Total or free antigen concentration in plasma and molar antigen/antigen-binding molecule ratio can be measured at 2, 4, 7, 14, 28, 56, or 84 days after administration to evaluate the long-term effect of the present invention. In other words, a long term plasma antigen concentration is determined by measuring total or free antigen concentration in plasma and molar antigen/ antigen-binding molecule ratio at 2, 4, 7, 14, 28, 56, or 84 days after administration of an antigen-binding molecule in order to evaluate the property of the antigen-binding molecule of the present invention. Whether the reduction of plasma antigen concentration or molar antigen/ antigen-binding molecule ratio is achieved by antigen-binding molecule described in the present invention can be determined by the evaluation of the reduction at any one or more of the time points described above.

Total or free antigen concentration in plasma and molar antigen/antigen-binding molecule ratio can be measured at 15 min, 1, 2, 4, 8, 12, or 24hours after administration to evaluate the short-term effect of the present invention. In other words, a short term plasma antigen concentration is determined by measuring total or free antigen concentration in plasma and molar antigen/antigen-binding molecule ratio at 15 min, 1, 2, 4, 8, 12, or 24 hours after administration of an antigen-binding molecule in order to evaluate the property of the antigen-binding molecule of the present invention.

Route of administration of an antigen-binding molecule of the present invention can be selected from intradermal, intravenous, intravitreal, subcutaneous, intraperitoneal, parenteral and intramuscular injection.

In the present invention, improvement of pharmacokinetics in human is preferred. When the plasma retention in human is difficult to determine, it may be predicted based on the plasma retention in mice (for example, normal mice, human antigen-expressing transgenic mice, human FcRn-expressing transgenic mice) or monkeys (for example, cynomolgus monkeys).

Herein, the acidic pH range typically refers to pH 4.0 to pH 6.5. The acidic pH range is preferably a range indicated by any pH value within pH 5.5 to pH 6.5, preferably selected from 5.5, 5.6, 5.7, 5.8, 5.9, 6.0, 6.1, 6.2, 6.3, 6.4, and 6.5, particularly preferably pH 5.8 to pH 6.0, which is close to the pH in early endosome in vivo. Meanwhile, herein the neutral pH range typically refers to pH 6.7 to pH 10.0. The neutral pH range is preferably a range indicated by any pH value within pH 7.0 to pH 8.0, preferably selected from pH 7.0, 7.1, 7.2, 7.3, 7.4, 7.5, 7.6, 7.7, 7.8, 7.9, and 8.0, particularly preferably pH 7.4, which is close to in vivo plasma (blood) pH. pH 7.0 can be used as an alternative to pH 7.4 when it is difficult to assess the binding affinity between human FcRn-binding domain and human FcRn due its low affinity at pH 7.4. As a temperature employed in the assay condition, a binding affinity between human FcRn-binding domain and human FcRn may be assessed at any temperature from 10 degrees C to 50 degrees C. Preferably, a temperature at from 15 degrees C to 40 degrees C is employed in order to determine the binding affinity between human FcRn-binding domain and human FcRn. More preferably, any temperature at from 20 degrees C to 35 degrees C, like any one of 20, 21, 22, 23, 24, 25, 26, 27, 28, 29, 30, 31, 32, 33, 34, and 35 degrees C is also employed in order to determine the binding affinity between human FcRn-binding domain and human FcRn. A temperature at 25 degrees C described in Example 5 is one of example for the embodiment of this invention.

Thus, herein "reducing the antigen-binding activity of an antigen-binding molecule in the acidic pH range to less than that in the neutral pH range" means that the antigen-binding activity of the antigen-binding molecule at pH 4.0 to pH 6.5 is impaired as compared to its antigen-binding activity at pH 6.7 to pH 10.0. Preferably, the above phrase means that the antigen-binding activity of an antigen-binding molecule at pH 5.5 to pH 6.5 is impaired as compared to that at pH 7.0 to pH 8.0, more preferably means that its antigen-binding activity at the early endosomal pH is impaired as compared to its antigen-binding activity at the plasma pH in vivo. Specifically, the antigen-binding activity of an antigen-binding molecule at pH 5.8 to pH 6.0 is impaired as compared to the antigen-binding activity of the antigen-binding molecule at pH 7.4.

Meanwhile, herein the expression "reducing the antigen-binding activity of an antigen-binding molecule in the acidic pH range to less than that in the neutral pH range" is also expressed as "increasing the antigen-binding activity of an antigen-binding molecule in the neutral pH range to more than that in the acidic pH range". Specifically, in the present invention, it is possible to increase the ratio of antigen binding activity of an antigen-binding molecule between acidic and neutral pH ranges. For example, the value of KD (pH 5.8)/KD (pH 7.4) is increased in an embodiment described below. The ratio of antigen-binding activity of an antigen-binding molecule between acidic and neutral pH ranges can be increased, for example, by reducing its antigen-binding activity in the acidic pH range, increasing its antigen-binding activity in the neutral pH range, or both.

Herein, the expression "impairing the antigen-binding activity in the acidic pH range as compared to that in the neutral pH range" is sometimes used instead of "reducing the antigen-binding activity in the acidic pH range to less than that in the neutral pH range".

Herein, the human FcRn-binding activity in the acidic pH range means the human FcRn-binding activity at pH 4.0 to pH 6.5, preferably the human FcRn-binding activity at pH 5.5 to pH 6.5, and particularly preferably the human FcRn-binding activity at pH 5.8 to pH 6.0, which is comparable to the in vivo early endosomal pH. Meanwhile, herein the human FcRn-binding activity in the neutral pH range means the human FcRn-binding activity at pH 6.7 to pH 10.0, preferably the human FcRn-binding activity at pH 7.0 to pH 8.0, and particularly preferably the human FcRn-binding activity at pH 7.4, which is comparable to the in vivo plasma pH.

The antigen-binding molecules of the present invention have a human FcRn-binding domain. The human FcRn-binding domain is not particularly limited, as long as the antigen-binding molecules exhibit the human FcRn-binding activity in the acidic and neutral pH ranges. Alternatively, the domain may have a direct or indirect human FcRn-binding activity. Such domains include, for example, the Fc domain of IgG-type immunoglobulin, albumin, albumin domain 3, anti-human FcRn antibodies, anti-human FcRn peptides, and anti-human FcRn scaffold molecules, all of which have the activity to directly bind to human FcRn; and molecules that bind to IgG or albumin, which have the activity to indirectly bind to human FcRn. Such preferred domains of the present invention have human FcRn-binding activity in the acidic and neutral pH ranges. It is possible to use the domains without any alteration as long as they already have human FcRn-binding activity in the acidic and neutral pH ranges. When the domains have only weak or no human FcRn-binding activity in the acidic and/or neutral pH ranges, the human FcRn-binding activity may be conferred by altering amino acids in the antigen-binding molecules. However, it is preferred that human FcRn-binding activity in the acidic and/or neutral pH ranges is conferred by altering amino acids in the human FcRn-binding domain. Alternatively, amino acids in the domains that already have human FcRn-binding activity in the acidic and/or neutral pH ranges may be altered to increase the human FcRn-binding activity. Desired amino acid alterations in the human FcRn-binding domain can be selected by comparing the human FcRn-binding activity in the acidic and/or neutral pH ranges before and after amino acid alteration.

The preferred human FcRn-binding domain is a region that directly binds to human FcRn. Such preferred human FcRn-binding regions include, for example, antibody Fc domains. Meanwhile, regions capable of binding to a polypeptide such as albumin or IgG, which has human FcRn-binding activity, can indirectly bind to human FcRn via albumin, IgG, or such. Thus, such a human FcRn-binding region of the present invention may be a region that binds to a polypeptide having human FcRn-binding activity.

The antigen-binding molecules of the present invention are not particularly limited, as long as they include an antigen-binding domain having a binding activity specific to a target antigen. Such preferred antigen-binding domains comprise, for example, domains having an antigen-binding region of an antibody. The antigen-binding region of an antibody comprises, for example, CDRs and variable regions. When the antigen-binding region of an antibody is CDR, it may contain all six CDRs from the whole antibody, or one, two, or more CDRs. When CDRs are contained as a binding region of antibody, they may comprise amino acid deletions, substitutions, additions, and/or insertions, or may be a portion of CDR.

On the other hand, antigen-binding molecules to be used in the methods of the present invention comprise antigen-binding molecules that have an antagonistic activity (antagonistic antigen-binding molecules), antigen-binding molecules that have an agonistic activity (agonistic antigen-binding molecule), and molecules having cytotoxicity. In a preferred embodiment, the antigen-binding molecules comprise antagonistic antigen-binding molecules, in particular, antagonistic antigen-binding molecules that recognize an antigen such as a receptor or cytokine.

In the present invention, the antigen-binding molecule of interest is not particularly limited, and may be any antigen-binding molecules. The antigen-binding molecule of the present invention preferably includes both antigen-binding activity (antigen-binding domain) and human FcRn-binding domain. In particular, preferred antigen-binding molecule of the present invention includes a domain that binds to human FcRn. The antigen-binding molecule including both antigen-binding domain and human FcRn-binding domain includes, for example, antibodies. The antibodies preferred in the context of the present invention include, for example, IgG antibodies. When the antibody to be used is an IgG antibody, the type of IgG is not limited; the IgG belonging to any isotype (subclass) such as IgG1, IgG2, IgG3, or IgG4 can be used. Furthermore, the antigen-binding molecules of the present invention may include antibody constant region, and amino acid mutations may be introduced into the constant region. Amino acid mutations to be introduced include, for example, those potentiate or impair the binding to Fcgamma receptor (Proc Natl Acad Sci USA. 2006 Mar 14; 103(11): 4005-10), but are not limited to these examples. Alternatively, it is also possible to alter the pH-dependent binding by selecting an appropriate constant region such as of IgG2.

When the antigen-binding molecule of interest of the present invention is an antibody, it may be an antibody derived from any animal, such as a mouse antibody, human antibody, rat antibody, rabbit antibody, goat antibody, or camel antibody. Furthermore, the antibody may be an altered antibody, for example, a chimeric antibody, and in particular, an altered antibody including amino acid substitution in the sequence of a humanized antibody, etc. The antibodies also include bispecific antibodies, antibody modification products linked with various molecules, and polypeptides including antibody fragments.

"Chimeric antibodies" are antibodies prepared by combining sequences derived from different animals. Specifically, the chimeric antibody includes, for example, antibodies having heavy and light chain variable (V) regions from a mouse antibody and heavy and light chain constant (C) regions from a human antibody.

"Humanized antibodies", also referred to as reshaped human antibodies, are antibodies in which complementarity determining regions (CDRs) of an antibody derived from a nonhuman mammal, for example, a mouse, are transplanted into the CDRs of a human antibody. Methods for identifying CDRs are known (Kabat et al., Sequence of Proteins of Immunological Interest (1987), National Institute of Health, Bethesda, Md.; Chothia et al., Nature (1989) 342: 877). General genetic recombination technologies suitable for this purpose are also known (see European Patent Application EP 125023; and WO 96/02576).

Bispecific antibody refers to an antibody that has, in the same antibody molecule, variable regions that recognize different epitopes. A bispecific antibody may be an antibody that recognizes two or more different antigens, or an antibody that recognizes two or more different epitopes on a same antigen.

Furthermore, polypeptides including antibody fragments include, for example, Fab fragments, F(ab')2 fragments, scFvs (Nat Biotechnol. 2005 Sep; 23(9): 1126-36), domain antibodies (dAbs) (WO 2004/058821, WO 2003/002609), scFv-Fc (WO 2005/037989), dAb-Fc, and Fc fusion proteins. Fc domain can be used as a human FcRn-binding domain when a molecule includes an Fc domain. Alternatively, an FcRn-binding domain may be fused to these molecules.

Further, the antigen-binding molecules that are applicable to the present invention may be antibody-like molecules. An antibody-like molecule (scaffold molecule, peptide molecule) is a molecule that can exhibit functions by binding to a target molecule (Current Opinion in Biotechnology (2006) 17: 653-658; Current Opinion in Biotechnology (2007) 18: 1-10; Current Opinion in Structural Biology (1997) 7: 463-469; Protein Science (2006) 15: 14-27), and includes, for example, DARPins (WO 2002/020565), Affibody (WO 1995/001937), Avimer (WO 2004/044011; WO 2005/040229), and Adnectin (WO 2002/032925). If these antibody-like molecules can bind to target molecules in a pH-dependent manner and/or have human FcRn-binding activity in the neutral pH range, it is possible to facilitate antigen uptake into cells by antigen-binding molecules, facilitate the reduction of plasma antigen concentration by administering antigen-binding molecules, and improve pharmacokinetics of the antigen-binding molecules, and increase the number of antigens to which a single antigen-binding molecule can bind.

Furthermore, the antigen-binding molecule may be a protein resulting from fusion between a human FcRn-binding domain and a receptor protein that binds to a target including a ligand, and includes, for example, TNFR-Fc fusion proteins, IL1R-Fc fusion proteins, VEGFR-Fc fusion proteins, and CTLA4-Fc fusion proteins (Nat Med. 2003, Jan; 9(1): 47-52; BioDrugs. (2006) 20(3): 151-60). If these receptor-human FcRn-binding domain fusion proteins bind to a target molecule including a ligand in a pH-dependent manner and/or have human FcRn-binding activity in the neutral pH range, it is possible to facilitate antigen uptake into cells by antigen-binding molecules, facilitate the reduction of plasma antigen concentration by administering antigen-binding molecules, and improve pharmacokinetics of the antigen-binding molecules, and increase the number of antigens to which a single antigen-binding molecule can bind. A receptor protein is appropriately designed and modified so as to include a binding domain of the receptor protein to a target including a ligand. As referred to the example hereinbefore including TNFR-Fc fusion proteins, IL1R-Fc fusion proteins, VEGFR-Fc fusion proteins and CTLA4-Fc fusion proteins, a soluble receptor molecule comprising an extracellular domain of those receptor proteins which is required for binding to those targets including ligands is a preferable used in the present invention. Those designed and modified receptor molecule is referred to as an artificial receptor in the present invention. A method employed to design and modify a receptor molecule to construct an artificial receptor molecule is known in the art.

Moreover, the antigen-binding molecule may be a fusion protein in which artificial ligand protein that binds to a target and has the neutralizing effect is fused with a human FcRn-binding domain, and an artificial ligand protein includes, for example, mutant IL-6 (EMBO J. 1994 Dec 15; 13(24): 5863-70). If such artificial ligand fusion proteins can bind to target molecules in a pH-dependent manner and/or have human FcRn-binding activity in the neutral pH range, it is possible to facilitate antigen uptake into cells by antigen-binding molecules, facilitate the reduction of plasma antigen concentration by administering antigen-binding molecules, and improve pharmacokinetics of the antigen-binding molecules, and increase the number of antigens to which a single antigen-binding molecule can bind.

Furthermore, the antibodies of the present invention may include modified sugar chains. Antibodies with modified sugar chains include, for example, antibodies with modified glycosylation (WO 99/54342), antibodies that are deficient in fucose that is added to the sugar chain (WO 00/61739; WO 02/31140; WO 2006/067847; WO2 006/067913), and antibodies having sugar chains with bisecting GlcNAc (WO 02/79255).

Conditions used in the assay for the antigen-binding or human FcRn-binding activity other than pH can be appropriately selected by those skilled in the art, and the conditions are not particularly limited. For example, the conditions of using MES buffer at 37 degrees C as described in WO 2009/125825 may be used to determine the activity. In another embodiment, Na-phosphate buffer at 25 degrees C as described in Examples 4 or 5 may be used to determine the activity. Meanwhile, the antigen-binding activity and human FcRn-binding activity of antigen-binding molecule can be determined by methods known to those skilled in the art, for example, using Biacore (GE Healthcare) or such. When the antigen is a soluble antigen, the activity of an antigen-binding molecule to bind to the soluble antigen can be determined by loading the antigen as an analyte onto a chip immobilized with the antigen-binding molecule. Alternatively, when the antigen is a membrane-type antigen, the activity of the antigen-binding molecule to bind to the membrane-type antigen can be determined by loading the antigen-binding molecule as an analyte onto an antigen-immobilized chip. The human FcRn-binding activity of an antigen-binding molecule can be determined by loading human FcRn or the antigen-binding molecule as an analyte onto a chip immobilized with the antigen-binding molecule or human FcRn, respectively.

In the present invention, the ratio between the antigen-binding activity in the acidic pH range and that in neutral pH range is not particularly limited as long as the antigen-binding activity in the acidic pH range is lower than that in the neutral pH range. However, the value of KD (pH 5.8)/KD (pH 7.4), which is a ratio of dissociation constant (KD) against an antigen at pH 5.8 and pH 7.4, is preferably 2 or greater, more preferably 10 or greater, and still more preferably 40 or greater. The upper limit of the KD (pH 5.8)/KD (pH 7.4) value is not particularly limited, and may be any value, for example, 400, 1,000, or 10,000, as long as production is possible using the technologies of those skilled in the art.

When the antigen is a soluble antigen, the value of antigen-binding activity can be presented in terms of the dissociation constant (KD). On the other hand, when the antigen is a membrane-type antigen, the activity can be presented in terms of apparent dissociation constant (apparent KD). The dissociation constant (KD) and apparent dissociation constant (apparent KD) can be determined by methods known to those skilled in the art, for example, using Biacore (GE Healthcare), Scatchard plot, flow cytometer, or such.

In the present invention, other parameters that are representative of the ratio of antigen-binding activity between the acidic and neutral pH ranges include, for example, dissociation rate constant $k_d$. When the dissociation rate constant ($k_d$) is used instead of the dissociation constant (KD) as a parameter representative of the binding activity ratio, the value of $k_d$ (in the acidic pH range)/$k_d$ (in the neutral pH range), which is a ratio of $k_d$ (dissociation rate constant) against an antigen in the acidic pH range and neutral pH range, is preferably 2 or greater, more preferably 5 or greater, even more preferably 10 or greater, and still more preferably 30 or greater. The upper limit of the $k_d$ (in the acidic pH range)/$k_d$ (in the neutral pH range) value is not particularly limited, and may be any value, for example, 50, 100, or 200, as long as production is possible using the technologies of those skilled in the art.

When the antigen is a soluble antigen, the value of antigen-binding activity can be presented using the dissociation rate constant ($k_d$). Alternatively, when the antigen is a membrane-type antigen, the value can be presented in terms of apparent $k_d$ (apparent dissociation rate constant). The dissociation rate constant ($k_d$) and apparent dissociation rate constant (apparent $k_d$) can be determined by methods known to those skilled in the art, for example, using Biacore (GE Healthcare), flow cytometer, or the like.

In the present invention, when the antigen-binding activity of an antigen-binding molecule is determined at different pHs, it is preferred that the measurement conditions except pH are constant.

The methods for reducing (impairing) the antigen-binding activity of an antigen-binding molecule in the acidic pH range to less than that in the neutral pH range (methods for conferring the pH-dependent binding ability) are not particularly limited and may be achieved by any methods. Specifically, as described in WO 2009/125825, the methods include, for example, methods for reducing (impairing) the antigen-binding activity in the acidic pH range to less than that in the neutral pH range by substituting histidine for an amino acid in the antigen-binding molecule or inserting histidine into the antigen-binding molecule. It is already known that an antibody can be conferred with a pH-dependent antigen-binding activity by substituting histidine for an amino acid in the antibody (FEBS Letter (1992) 309(1): 85-88). Such histidine mutation (substitution) or insertion sites are not particularly limited; and histidine may be substituted for an amino acid at any site or inserted at any site. Preferred sites for histidine mutation (substitution) or insertion include, for example, regions where the mutation or insertion has an impact on the antigen-binding activity of an antigen-binding molecule. Such regions include sites where the mutation or insertion reduces (impairs) the antigen-binding activity in the acidic pH range to less than that in the neutral pH range (the KD (in the acidic pH range)/KD (in the neutral pH range) value is increased) as compared to before mutation or insertion. When the antigen-binding molecule is an antibody, such regions include, for example, antibody variable regions and CDRs. The number of histidine mutations or insertions to be introduced (achieved) can be appropriately determined by those skilled in the art. Histidine substitution may be introduced at only a single site, or at two or more sites. Alternatively, histidine may be inserted at only a single site, or at two or more sites. Furthermore, mutations other than histidine mutation (mutation (deletion, addition, insertion, and/or substitution) with an amino acid other than histidine) may be introduced in addition to histidine mutation. Alternatively, histidine mutation may be combined with histidine insertion. Such histidine substitution or insertion may be carried out by a random method such as histidine scanning, which is conducted by using histidine instead of alanine in alanine scanning known to those skilled in the art. Then, antigen-binding molecules having a greater KD (in the acidic pH range) /KD (in the neutral pH range) value than before introduction of the mutation may be selected from a library of antigen-binding molecules introduced with random histidine mutation or insertion.

When histidine is substituted for an amino acid in the antigen-binding molecule or histidine is inserted into the antigen-binding molecule, the antigen-binding activity of the antigen-binding molecule in the neutral pH range after histidine substitution or insertion is preferably equivalent to the antigen-binding activity of the antigen-binding molecule in the neutral pH range before histidine substitution or insertion, but is not particularly limited thereto. Herein, "the antigen-binding activity of an antigen-binding molecule in the neutral pH range after histidine substitution or insertion is equivalent to the antigen-binding activity of the antigen-binding molecule in the neutral pH range before histidine substitution or insertion" means that the antigen-binding molecule after histidine substitution or insertion retains 10% or more, preferably 50% or more, more preferably 80% or more, and still more preferably 90% or more of the antigen-binding activity of the antigen-binding molecule before histidine substitution or insertion. When the antigen-binding activity of an antigen-binding molecule is reduced due to histidine substitution or insertion, the antigen-binding activity may be adjusted by substituting, deleting, adding, and/or inserting one or more amino acids into the antigen-binding molecule so that the antigen-binding activity becomes equivalent to that before histidine substitution or insertion. The present invention also comprises such antigen-binding molecules whose binding activity has been made equivalent as a result of substitution, deletion, addition, and/or insertion of one or more amino acids into the antigen-binding molecule after histidine substitution or insertion.

Other methods for reducing (impairing) the antigen-binding activity of an antigen-binding molecule in the acidic pH range to less than that in the neutral pH range include methods for substituting non-natural amino acids for amino acids in the antigen-binding molecule or inserting non-natural amino acids into the antigen-binding molecule. It is known that pKa can be artificially adjusted by using non-natural amino acids (Angew. Chem. Int. Ed. 2005, 44, 34; Chem Soc Rev. 2004 Sep 10, 33 (7): 422-30; Amino Acids. (1999) 16(3-4): 345-79). Thus, in the present invention, it is possible to use non-natural amino acids instead of histidine described above. The sites where non-natural amino acids are introduced are not particularly limited, and non-natural amino acids may be substituted or inserted at any site. Preferred sites of non-natural amino acid substitution or insertion include, for example, regions where the substitution or insertion has an impact on the antigen-binding activity of an antigen-binding molecule. For example, when the antigen-binding molecule is an antibody, such regions include antibody variable regions and complementarity determining regions (CDRs). Meanwhile, the number of non-natural amino acids to be introduced is not particularly limited; and it is possible to substitute non-natural amino acids at only a single site, or two or more sites. Alternatively, non-natural amino acids may be inserted at only a single site, or two or more sites. Furthermore, other amino acids may be deleted, added, inserted, and/or substituted in addition to substitution or insertion of non-natural amino acids. Furthermore, non-natural amino acids may be substituted and/or inserted in combination with the above-described histidine substitution and/or insertion. Any non-natural amino acid may be used in the present invention. It is possible to use non-natural amino acids known to those skilled in the art.

In the present invention, when the antigen-binding molecule is an antibody, possible sites of histidine or non-natural amino acid substitution include, for example, CDR sequences and sequences responsible for the CDR structure of an antibody, including, for example, the sites described in WO 2009/125825. The amino acid positions are indicated according to the Kabat numbering (Kabat EA et al. (1991) Sequences of Proteins of Immunological Interest, NIH).

The Kabat numbering system is generally used when referring to a residue in the variable domain (approximately residues 1-107 of the light chain and residues 1-113 of the heavy chain) (e.g., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., supra). The "EU index as in Kabat" refers to the residue numbering of the human $IgG_1$ EU antibody. Unless stated otherwise herein, references to residue numbers in the variable domain of antibodies means residue numbering by the Kabat numbering system. Unless stated otherwise herein, references to residue numbers in the constant domain of antibodies means residue numbering by the EU numbering system (see e.g., WO 2006073941).

Heavy chain: H27, H31, H32, H33, H35, H50, H58, H59, H61, H62, H63, H64, H65, H99, H100b, and H102

Light chain: L24, L27, L28, L32, L53, L54, L56, L90, L92, and L94

Of these alteration sites, H32, H61, L53, L90, and L94 are assumed to be highly general alteration sites.

When the antigen is an IL-6 receptor (for example, human IL-6 receptor), the preferred alteration sites include the following. However, the alteration sites are not particularly limited thereto.

Heavy chain: H27, H31, H32, H35, H50, H58, H61, H62, H63, H64, H65, H100b, and H102

Light chain: L24, L27, L28, L32, L53, L56, L90, L92, and L94

Specifically, preferred combinations of sites for histidine or non-natural amino acid substitution include, for example, the combination of H27, H31, and H35; the combination of H27, H31, H32, H35, H58, H62, and H102; the combination of L32 and L53; and the combination of L28, L32, and L53. Furthermore, preferred combinations of substitution sites in the heavy and light chains include, for example, the combination of H27, H31, L32, and L53.

Of these sites, histidine or non-natural amino acids are substituted at only a single site or more sites.

Meanwhile, when the antigen-binding molecule is a substance having an antibody constant region, methods for reducing (impairing) the antigen-binding activity of an antigen-binding molecule in the acidic pH range to less than that in the neutral pH range include, for example, methods for altering amino acids in the antibody constant region. Specifically, such methods comprise, for example, methods for substituting a constant region described in WO 2009/125825 (SEQ ID NOs: 11, 12, 13, and 14). Meanwhile, methods for altering an antibody constant region comprise, for example, methods for assessing various constant region isotypes (IgG1, IgG2, IgG3, and IgG4) and selecting isotypes that reduce the antigen-binding activity in the acidic pH range (increase the dissociation rate in the acidic pH range). Such methods also include methods for reducing the antigen-binding activity in the acidic pH range (increasing the dissociation rate in the acidic pH range) by introducing amino acid substitutions into the amino acid sequences of wild-type isotypes (amino acid sequences of wild type IgG1, IgG2, IgG3, or IgG4). The sequence of hinge region in the antibody constant region is considerably different among isotypes (IgG1, IgG2, IgG3, and IgG4), and the difference in the hinge region amino acid sequence has a great impact on the antigen-binding activity. Thus, it is possible to select an appropriate isotype to reduce the antigen-binding activity in the acidic pH range (increase the dissociation rate in the acidic pH range) depending on the type of antigen or epitope. Furthermore, since the difference in the hinge region amino acid sequence has a great impact on the antigen-binding activity, preferred amino acid substitution sites in the amino acid sequences of wild type isotypes are assumed to be within the hinge region.

When the antigen-binding activity of an antigen-binding molecule in the acidic pH range is reduced (weakened) to less than that in the neutral pH range (when the value of KD (in the acidic pH range)/KD (in the neutral pH range) is increased) by the above-described method and the like, it is generally preferable that the KD (in the acidic pH range)/KD (in the neutral pH range) value is twice or more, preferably five times or more, and more preferably ten times or more as compared to the original antibody, but it is not particularly limited thereto.

The above-described methods can be used to produce antigen-binding molecules whose antigen-binding activity in the acidic pH range is reduced (weakened) to less than that in the neutral pH range (antigen-binding molecules that bind in a pH-dependent manner) by amino acid substitution or insertion from antigen-binding molecules that do not have such property. Other methods include methods for directly obtaining antigen-binding molecules having the above-described property. For example, antibodies having a desired property of interest may be directly selected by screening using the pH-dependent antigen binding as an indicator from antibodies obtained by immunizing animals (mice, rats, hamsters, rabbits, human immunoglobulin-transgenic mice, human immunoglobulin-transgenic rats, human immunoglobulin-transgenic rabbits, llamas, camels, etc.) with an antigen. Antibodies can be generated by hybridoma technology or B-cell cloning technology (Proc Natl Acad Sci USA. 1996 Jul 23; 93(15): 7843-8; J Immunol Methods. 2006 Oct 20; 316(1-2): 133-43; Journal of the Association for Laboratory Automation; WO 2004/106377; WO 2008/045140; and WO 2009/113742) which are methods known to those skilled in the art, but not limited thereto. Alternatively, antibodies that have the property of interest may be directly selected by screening using the pH-dependent antigen binding as an indicator from a library of presenting antigen-binding domain in vitro. Such library includes human naive library, immunized library from non-human animal and human, semi-synthetic library and synthetic library which are libraries known to those skilled in the art (Methods Mol Biol. 2002; 178: 87-100; J Immunol Methods. 2004 Jun; 289(1-2): 65-80; and Expert Opin Biol Ther. 2007 May; 7(5): 763-79), but not limited thereto. However, the methods are not particularly limited to these examples.

Present invention utilized a difference of pH as an environmental difference between plasma and endosome for differential binding affinity of an antigen binding molecule to an antigen at plasma and endosome (strong binding at plasma and weak binding at endosome). Since environmental difference between plasma and endosome is not limited to a difference of pH, pH dependent binding property on binding of an antigen-binding molecule to an antigen can be substituted by utilizing other factors whose concentration is different within the plasma and the endosome. Such factor may also be used to generate an antibody that binds to the antigen within plasma but dissociates the antigen within endosome. Therefore, present invention also includes an antigen-binding molecule comprising an antigen-binding domain and a human FcRn-binding domain, which has a human FcRn-binding activity in the acidic and neutral pH ranges, and a lower antigen-binding activity in the endosome than in the plasma, wherein the human FcRn-binding activity in the plasma is stronger than that of intact human IgG.

Methods for increasing the human FcRn-binding activity of the human FcRn-binding domain in an antigen-binding molecule of the present invention in the neutral pH range are not particularly limited and may be increased by any methods. Specifically, when the Fc domain of an IgG-type immunoglobulin is used as human FcRn-binding domain, the human FcRn-binding activity in the neutral pH range can be increased by altering its amino acids. Such a preferred Fc domain of IgG-type immunoglobulin to be altered includes, for example, the Fc domain of a parent human IgG (IgG1, IgG2, IgG3, or IgG4 and their engineered variants). Amino acids at any sites may be altered to other amino acids as long as the human FcRn-binding activity is conferred or increased in the neutral pH range. When the antigen-binding molecule has a human IgG1 Fc domain as the human FcRn-binding domain, it is preferred that the molecule has alterations that potentiate the binding to human FcRn in the neutral pH range as compared to that of the parent human IgG1. Amino acids where such alteration can be achieved include, for example, amino acids of positions 221 to 225, 227, 228, 230, 232, 233 to 241, 243 to 252, 254 to 260, 262 to 272, 274, 276, 278 to 289, 291 to 312, 315 to 320, 324, 325, 327 to 339, 341, 343, 345, 360, 362, 370, 375 to 378, 380, 382, 385 to 387, 389, 396, 414, 416, 423, 424, 426 to 438, 440, and 442 (EU numbering). More specifically, such amino acid alterations include, for example, those listed in Table 1. The human FcRn binding of the Fc domain of an IgG-type immunoglobulin in the neutral pH range can be potentiated by using the alterations described above.

Furthermore, alterations that can potentiate the binding to human FcRn in the acidic pH range as compared to the parent human IgG are shown as an example in Table 2. When appropriate alterations that can also potentiate the binding to human FcRn in the neutral pH range are selected from the above-described alterations, they are applicable to the present invention. Meanwhile, combinations of alterations that can potentiate the binding of Fv4- IgG 1 to human FcRn under acidic conditions are shown in Tables 6-1 and 6-2. Particularly preferred amino acids to be altered in the parent human IgG Fc domain include, for example, amino acids of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering). The human FcRn-binding activity of an antigen-binding molecule can be increased in the neutral pH range by substituting a different amino acid for at least one amino acid selected from the above-described amino acids.

Particularly preferred alterations include, for example,

> an amino acid substitution of Met for Gly at position 237;
> an amino acid substitution of Ala for Pro at position 238;
> an amino acid substitution of Lys for Ser at position 239;
> an amino acid substitution of Ile for Lys at position 248;
> an amino acid substitution of Ala, Phe, Ile, Met, Gln, Ser, Val, Trp, or Tyr for Thr at position 250;
> an amino acid substitution of Phe, Trp, or Tyr for Met at position 252;
> an amino acid substitution of Thr for Ser at position 254;
> an amino acid substitution of Glu for Arg at position 255;
> an amino acid substitution of Asp, Glu, or Gln for Thr at position 256;
> an amino acid substitution of Ala, Gly, Ile, Leu, Met, Asn, Ser, Thr, or Val for Pro at position 257;
> an amino acid substitution of His for Glu at position 258;
> an amino acid substitution of Ala for Asp at position 265;
> an amino acid substitution of Phe for Asp at position 270;

an amino acid substitution of Ala, or Glu for Asn at position 286;
an amino acid substitution of His for Thr at position 289;
an amino acid substitution of Ala for Asn at position 297;
an amino acid substitution of Gly for Ser at position 298;
an amino acid substitution of Ala for Val at position 303;
an amino acid substitution of Ala for Val at position 305;
an amino acid substitution of Ala, Asp, Phe, Gly, His, Ile, Lys, Leu, Met, Asn, Pro, Gln, Arg, Ser, Val, Trp, or Tyr for Thr at position 307;
an amino acid substitution of Ala, Phe, Ile, Leu, Met, Pro, Gln, or Thr for Val at position 308;
an amino acid substitution of Ala, Asp, Glu, Pro, or Arg for Leu or Val at position 309;
an amino acid substitution of Ala, His, or Ile for Gln at position 311;
an amino acid substitution of Ala, or His for Asp at position 312;
an amino acid substitution of Lys, or Arg for Leu at position 314;
an amino acid substitution of Ala, or His for Asn at position 315;
an amino acid substitution of Ala for Lys at position 317;
an amino acid substitution of Gly for Asn at position 325;
an amino acid substitution of Val for Ile at position 332;
an amino acid substitution of Leu for Lys at position 334;
an amino acid substitution of His for Lys at position 360;
an amino acid substitution of Ala for Asp at position 376;
an amino acid substitution of Ala for Glu at position 380;
an amino acid substitution of Ala for Glu at position 382;
an amino acid substitution of Ala for Asn or Ser at position 384;
an amino acid substitution of Asp, or His for Gly at position 385;
an amino acid substitution of Pro for Gln at position 386;
an amino acid substitution of Glu for Pro at position 387;
an amino acid substitution of Ala, or Ser for Asn at position 389;
an amino acid substitution of Ala for Ser at position 424;
an amino acid substitution of Ala, Asp, Phe, Gly, His, Ile, Lys, Leu, Asn, Pro, Gln, Ser, Thr, Val, Trp, or Tyr for Met at position 428;
an amino acid substitution of Lys for His at position 433;
an amino acid substitution of Ala, Phe, His, Ser, Trp, or Tyr for Asn at position 434;
and an amino acid substitution of His for Tyr or Phe at position 436 (EU numbering) in the parent IgG Fc domain. Meanwhile, the number of amino acids to be altered is not particularly limited; and it is possible to alter amino acids at only a single site or at two or more sites. Combinations of two or more amino acid alterations include, for example, those shown in Table 3. Meanwhile, combinations of alterations that can potentiate the binding to human FcRn in the acidic pH range as compared to the parent human IgG are shown in Tables 4-1 to 4-5. When appropriate combinations of alterations that can also potentiate the binding to human FcRn in the neutral pH range are selected from the above-described alterations, they are applicable to the present invention. Furthermore, combinations of alterations that can potentiate the binding of Fv4-IgG1 to human FcRn under neutral conditions are shown in Tables 6-1 and 6-2.

The symbol "^" in the Tables shows an amino acid insertion after the indicated number in EU numbering. For example, ^281S means that S is inserted between positions 281 and 282 in EU numbering.

[Table 1]

| POSITION | AMINO ACID ALTERATION |
|---|---|
| 256 | P |
| 280 | K |
| 339 | T |
| 385 | H |
| 428 | L |
| 434 | W, Y, F, A, H |

[Table 2]

| POSITION | AMINO ACID ALTERATION |
|----------|----------------------|
| 221 | Y, K |
| 222 | Y |
| 223 | E, K |
| 224 | Y, E |
| 225 | E, K, W |
| 227 | K, E, G |
| 228 | Y, K, G |
| 230 | E, G |
| 232 | K |
| 233 | R, S, M, T, W, Y, G |
| 234 | H, R, E, I, V, F, D, Y, G |
| 235 | Y, V, N, S, T, Q, D |
| 236 | I, V, K, P, E, Q. H, W, Y, D, T, M, A, F, S, N, R |
| 237 | I, W, S, T, E, R, N, Q, K, H, D, P, L, M |
| 238 | A, L, D, S, T, H, W, V, I, G, M, F, E, K |
| 239 | M, R, T, G, V, E, D, L, A |
| 240 | I, M, T |
| 241 | E, W, L |
| 243 | E, W |
| 244 | L |
| 245 | R |
| 246 | Y, H |
| 247 | D |
| 248 | Y |
| 249 | P, Q, Y, H |
| 250 | I, E, Q |
| 251 | T, D |
| 252 | Y, W, Q |
| 254 | H |
| 255 | E, Y, H |
| 256 | A |
| 257 | A, I, M, N, S, V, T, L, Y, C |
| 258 | D, Y, H, A |
| 259 | I, F, N |
| 260 | S, D, E, H, Y |
| 262 | L, E |
| 263 | I |
| 264 | F, A, I, T, N, S, D |
| 265 | R, P, G, A |

(continued)

| POSITION | AMINO ACID ALTERATION |
|----------|----------------------|
| 266 | I |
| 267 | K, E, A |
| 268 | E, M |
| 269 | M, W, K, P, I, S, G, V, F, Y, A |
| 270 | K, S, I, A |
| 271 | A, V, S, Y, I, T |
| 272 | A, L, R, I, D, H, V, W, Y, P, T |
| 274 | M, F, G, E, I, T, N |
| 276 | D, F, H, R, L, V, W, A |
| 278 | R, S, V, M, N, I, L, D |
| 279 | A, D, G, H, M, N, Q, R, S, T, W, Y, C, I |
| 281 | D, Y |
| 282 | G, K, E, Y |
| 283 | A, D, F, G, H, I, K, L, N, P, Q, R, S, T, W, Y |
| 284 | T, L, Q, E |
| 285 | N, Y, W, Q, K, E, D, Y |
| 286 | F, L, Y, E, P, D, K, A |
| 287 | S, H |
| 288 | N, P, Y, H, D, I, V, C, E, G, L, Q, R |
| 289 | H |
| 291 | Q, H |
| 292 | Y, E, D |
| 293 | V |
| 294 | I, K, G |
| 295 | V, T |
| 296 | E, I, L |
| 298 | F, E, T, H |
| 299 | W, F, H, Y |
| 300 | K, A, G, V, M, Q, N, E |
| 301 | E |
| 302 | I |
| 303 | Y, E, A |
| 304 | N, T |
| 305 | A, H |
| 306 | Y |
| 307 | A, E, M, G, W, H |
| 308 | A, R, F, C, Y, W, N, H |
| 311 | A, I, K, L, M, V, W, T, H |
| 312 | A, P, H |

(continued)

| POSITION | AMINO ACID ALTERATION |
|---|---|
| 315 | T, H |
| 316 | K |
| 317 | A, P, H |
| 318 | N, T, R, L, Y |
| 319 | L, I, W, H, M, V, A |
| 320 | L, W, H, N |
| 324 | T, D |
| 325 | F, M, D |
| 326 | A |
| 327 | D, K, M, Y, H, L |
| 328 | G, A, W, R, F |
| 329 | K, R, W |
| 330 | G, W, V, P, H, F |
| 331 | L, F, Y |
| 332 | F, H, K, L, M, R, S, W, T, Q, E, Y, D, N, Y |
| 333 | L, F, M, A |
| 334 | A |
| 335 | H, F, N, V, M, W, I, S, P, L |
| 336 | E, K |
| 337 | A |
| 338 | A |
| 339 | N, W |
| 341 | P |
| 343 | E, H, K, Q, R, T, Y |
| 360 | H, A |
| 362 | A |
| 375 | R |
| 376 | A, G, I, M, P, T, Y |
| 377 | K |
| 378 | Q, D, N, W |
| 380 | A, N, S, T, Q, R, H |
| 382 | A, F, H, I, K, L, M, N, Q, R, S, T, V, W, Y |
| 385 | N, E |
| 386 | H |
| 387 | H, Q |
| 414 | A |
| 423 | N |
| 424 | A |
| 426 | H, L, V, R |

(continued)

| POSITION | AMINO ACID ALTERATION |
|----------|----------------------|
| 427 | N |
| 428 | F |
| 429 | Q |
| 430 | A, F, G, H, I, K, L, M, N, Q, R, S, T, V, Y |
| 431 | H, K |
| 432 | H |
| 433 | P |
| 434 | G, T, M, S, |
| 435 | K |
| 436 | I, L, T |
| 437 | H |
| 438 | K, L, T, W |
| 440 | K |
| 442 | K |

[Table 3]

| COMBINATION OF AMINO ACID ALTERATION |
|--------------------------------------|
| M252Y/S254T/T256E |
| M252Y/S254T/T256E/H433K/N434F/Y436H |
| H433K/N434F/Y436H |
| T307A/E380A |
| T307A/E380A/N434H |
| T307A/E380A/N434A |
| N434H/N315H |
| N434H/T289H |
| N434H/T370A/E380A |
| T250Q/M428L |
| T250Q/N434A |
| M252W/N434A |
| M252Y/N434A |
| T256A/N434A |
| T256D/N434A |
| T256E/N434A |
| T256S/N434A |
| P257I/Q311I |
| T307A/N434A |
| T307E/N434A |
| T307Q/N434A |
| V308P/N434A |

(continued)

| COMBINATION OF AMINO ACID ALTERATION |
| --- |
| L309G/N434A |
| Q311H/N434A |
| Q311R/N434A |
| N315D/N434A |
| A378V/N434A |
| E380S/N434A |
| E382V/N434A |
| S424E/N434A |
| M428L/N434A |
| N434A/Y436I |
| T437Q/N434A |
| T437R/N434A |

[Table 4-1]

| COMBINATION OF AMINO ACID ALTERATION |
| --- |
| L234I/L235D |
| G236A/V308F/I332E |
| G236R/L328R |
| G236A/I332E/N434S |
| S239E/V264I/A330Y/I332E |
| S239E/V264I/I332E |
| S239E/V264I/S298A/A330Y/I332E |
| S239D/D265H/N297D/I332E |
| S239D/E272Y/I332E |
| S239D/E272S/I332E |
| S239D/E272I/I332E |
| S239D/N297D/I332E |
| S239D/K326T/I332E |
| S239Q/I332Q |
| S239Q/I332N |
| S239D/I332D |
| S239D/I332E |
| S239Q/I332E |
| S239E/I332E |
| F241W/F243W |
| F241Y/F243Y/V262T/V264T |
| F241W/F243W/V262A/V264A |
| F241L/V262I |
| F243L/V262I/V264W |

(continued)

| COMBINATION OF AMINO ACID ALTERATION |
|---|
| F243L/K288D/R292P/Y300L/V305I/P396L/H435K |
| F243L/K288D/R292P/Y300L/H435K |
| F243L/R292P/Y300L/V305I/P396L/H435K |
| P245G/V308F |
| T250I/V259I/V308F |
| T250I/V308F |
| T2501/V308F/N434S |
| T250Q/V308F/M428L |
| T250Q/M428L |
| L251I/N434S |
| L251N/N434S |
| L251F/N434S |
| L251V/N434S |
| L251M/N434S |
| T252L/T254S/T256F |
| M252Y/S254T/T256E/N434M |
| M252Y/S254T/T256E/M428L/N434S |
| M252Y/S254T/T256E |
| M252Y/S254T/T256E/V308F |
| M252Y/S254T/T256E/N434S |
| M252Y/S254T/T256E/N434A |
| M252Y/S254T/T256E/M428L |
| M252Y/S254T/T256E/T3070 |
| M252F/T256D |
| M252Y/T256Q |
| M252Y/P257L |
| M252Y/P257N |
| M252Y/V259I |
| M252Y/V279Q |
| M252Y/V308P/N434Y |
| M252Q/V308F |
| M252Y/V308F |

Table 4-2 is a continuation of Table 4-1.

[Table 4-2]

| M252Q/V308F/N434S |
|---|
| M252Y/V308F/M428L |
| M252Y/V308F/N434M |
| M252Y/V308F/N434S |

(continued)

| |
|---|
| M252Y/Y319I |
| M252Q/M428L/N434S |
| M252Y/M428L |
| M252Y/N434M |
| M252Y/N434S |
| M252Y/N434A |
| M252Y/N434Y |
| S254T/V308F |
| R255H/N434A |
| R255Q/N434S |
| R255H/N434S |
| T256V/V308F |
| T256P/Q311I |
| T256P/I332E |
| T256P/I332E/S440Y |
| T256P/E430Q |
| T256P/N434H |
| T256E/N434Y |
| T256P/S440Y |
| P257Y/V279Q |
| P257L/V279E |
| P257N/V279Q |
| P257N/V279E |
| P257N/V279Y |
| P257L/V279Q |
| P257N/^281S |
| P257L/^281S |
| P257N/V284E |
| P257N/L306Y |
| P257L/V308Y |
| P257L/V308F |
| P257N/V308Y |
| P257I/Q311I/N434H |
| P257L/Q311V |
| P257L/G385N |
| P257L/M428L |
| P257I/E430Q |
| P257I/N434H |
| P257L/N434Y |
| E258H/N434A |

(continued)

| |
|---|
| E258H/N434H |
| V259I/T307Q/V308F |
| V259I/V308F |
| V259I/V308F/Y319L |
| V259I/V308F/Y319I |
| V259A/V308F |
| V259I/V308F/N434M |
| V259I/V308F/N434S |
| V259I/V308F/M428L/N434S |
| V259I/V308F/M428L |
| V259I/Y319I |
| V259I/Y319I/N434S |
| V259I/M428L |
| V259I/M428L/N434S |
| V259I/N434S |

Table 4-3 is a continuation of Table 4-2.

[Table 4-3]

| |
|---|
| V259I/N434Y |
| V264I/A330L/I332E |
| V264I/I332E |
| D265F/N297E/I332E |
| S267L/A327S |
| E272R/V279L |
| V279E/V284E |
| V279Q/L306Y |
| V279Y/V308F |
| V279Q/V308F |
| V279Q/G385H |
| ^281S/V308Y |
| ^281S/V308F |
| ^281S/N434Y |
| E283F/V284E |
| V284E/V308F |
| V284E/G385H |
| K288A/N434A |
| K288D/H435K |
| K288V/H435D |
| T289H/N434A |
| T289H/N434H |

(continued)

| |
|---|
| L3061/V308F |
| T307P/V308F |
| T307Q/V308F/N434S |
| T307Q/V308F/Y319L |
| T307S/V308F |
| T307Q/V308F |
| T307A/E310A/N434A |
| T307Q/E380A/N434A |
| T307Q/M428L |
| T307Q/N434M |
| T307I/N434S |
| T307V/N434S |
| T307Q/N434S |
| T307Q/N434Y |
| V308T/L309P/Q311S |
| V308F/L309Y |
| V308F/Q311V |
| V308F/Y319F |
| V308F/Y319I/N434M |
| V308F/Y319I |
| V308F/Y319L |
| V308F/Y319I/M428L |
| V308F/Y319I/M428L/N434S |
| V308F/Y319L/N434S |
| V308F/I332E |
| V308F/G385H |
| V308F/M428L/N434M |
| V308F/M428L |
| V308F/M428L/N434S |
| V308P/N434Y |
| V308F/N434M |
| V308F/N434S |
| V308F/N434Y |
| Q311G/N434S |
| Q311D/N434S |
| Q311E/N434S |
| Q311N/N434S |

Table 4-4 is a continuation of Table 4-3.

[Table 4-4]

| |
|---|
| Q311Y/N434S |
| Q311F/N434S |
| Q311W/N434S |
| Q311A/N434S |
| Q311K/N434S |
| Q311T/N434S |
| Q311R/N434S |
| Q311L/N434S |
| Q311M/N434S |
| Q311V/N434S |
| Q311I/N434S |
| Q311A/N434Y |
| D312H/N434A |
| D312H/N434H |
| L314Q/N434S |
| L314V/N434S |
| L314M/N434S |
| L314F/N434S |
| L314I/N434S |
| N315H/N434A |
| N315H/N434H |
| Y319I/V308F |
| Y319I/M428L |
| Y319I/M428L/N434S |
| Y319I/N434M |
| Y319I/N434S |
| L328H/I332E |
| L328N/I332E |
| L328E/I332E |
| L328I/I332E |
| L3284/I332E |
| L328D/I332E |
| L328R/M428L/N434S |
| A330L/I332E |
| A330Y/I332E |
| I332E/D376V |
| I332E/N434S |
| P343R/E345D |
| D376V/E430Q |
| D376V/E430R |

(continued)

| |
|---|
| D376V/N434H |
| E380A/N434A |
| G385R/Q386T/P387R/N389P |
| G385D/Q386P/N389S |
| N414F/Y416H |
| M428L/N434M |
| M428L/N434S |
| M428L/N434A |
| M428L/N434Y |
| H429N/N434S |
| E430D/N434S |
| E430T/N434S |
| E430S/N434S |
| E430A/N434S |
| E430F/N434S |
| E430Q/N434S |
| E430L/N434S |
| E430I/N434S |
| A431T/N434S |

Table 4-5 is a continuation of Table 4-4.

[Table 4-5]

| |
|---|
| A431S/N434S |
| A431G/N434S |
| A431V/N434S |
| A431N/N434S |
| A431F/N434S |
| A431H/N434S |
| L432F/N434S |
| L432N/N434S |
| L432Q/N434S |
| L432H/N434S |
| L432G/N434S |
| L432I/N434S |
| L432V/N434S |
| L432A/N434S |
| H433K/N434F |
| H433L/N434S |
| H433M/N434S |
| H433A/N434S |

(continued)

| |
|---|
| H433V/N434S |
| H433K/N434S |
| H433S/N434S |
| H433P/N434S |
| N434S/M428L |
| N434S/Y436D |
| N434S/Y436Q |
| N434S/Y436M |
| N434S/Y436G |
| N434S/Y436E |
| N434S/Y436F |
| N434S/Y436T |
| N434S/Y436R |
| N434S/Y436S |
| N434S/Y436H |
| N434S/Y436K |
| N434S/Y436L |
| N434S/Y436V |
| N434S/Y436W |
| N434S/Y436I |
| N434S/T437I |

Such amino acid alterations can be appropriately introduced using known methods. For example, alterations in the Fc domain of intact human IgG1 are described in Drug Metab Dispos. 2007 Jan. 35(1): 86-94; Int Immunol. 2006 Dec. 18, (12): 1759-69; J Biol Chem. 2001 Mar. 2, 276(9): 6591-604; J Biol Chem. (2007) 282(3): 1709-17; J Immunol. (2002) 169(9): 5171-80; J Immunol. (2009) 182(12): 7663-71; Molecular Cell, Vol. 7, 867-877, April, 2001; Nat Biotechnol. 1997 Jul. 15, (7): 637-40; Nat Biotechnol. 2005 Oct. 23, (10): 1283-8; Proc Natl Acad Sci USA. 2006 Dec. 5, 103(49): 18709-14; EP 2154157; US 20070141052; WO 2000/042072; WO 2002/060919; WO 2006/020114; WO 2006/031370; WO 2010/033279; WO 2006/053301; and WO 2009/086320.

According to the Journal of Immunology (2009) 182: 7663-7671, the human FcRn-binding activity of intact human IgG1 in the acidic pH range (pH 6.0) is KD 1.7 micromolar (microM), while in the neutral pH range the activity is almost undetectable. Thus, in a preferred embodiment, the antigen-binding molecule to be used in the methods of the present invention includes antigen-binding molecules whose human FcRn-binding activity in the acidic pH range is KD 20 micromolar or stronger and is identical or stronger in the neutral pH range than that of intact human IgG. In a more preferred embodiment, the antigen-binding molecule includes antigen-binding molecules whose human FcRn-binding activity is KD 2.0 micromolar or stronger in the acidic pH range and KD 40 micromolar or stronger in the neutral pH range. In a still more preferred embodiment, the antigen-binding molecule includes antigen-binding molecules whose human FcRn-binding activity is KD 0.5 micromolar or stronger in the acidic pH range and KD 15 micromolar or stronger in the neutral pH range. The above KD values are determined by the method described in the Journal of Immunology (2009) 182: 7663-7671 (by immobilizing the antigen-binding molecule onto a chip and loading human FcRn as an analyte).

Dissociation constant (KD) can be used as a value of human FcRn-binding activity. However, the human FcRn-binding activity of intact human IgG has little human FcRn-binding activity in the neutral pH range (pH 7.4), and therefore it is difficult to calculate the activity as KD. Methods for assessing whether the human FcRn-binding activity is higher than that of intact human IgG at pH 7.4 include assessment methods by comparing the intensities of Biacore response after loading analytes at the same concentration. Specifically, when the response after loading a human FcRn chip immobilized with an antigen-binding molecule at pH 7.4 is stronger than the response after loading human FcRn onto a chip immobilized with intact human IgG at pH 7.4, the human FcRn-binding activity of the antigen-binding molecule is judged to be higher than that of intact human IgG at pH 7.4.

pH 7.0 can also be used as a neutral pH range. Using pH 7.0 as a neutral pH can facilitate weak interaction between human FcRn and FcRn-binding domain. As a temperature employed in the assay condition, a binding affinity may be assessed at any temperature from 10 degrees C to 50 degrees C. Preferably, a temperature at from 15 degrees C to 40 degrees C is employed in order to determine the binding affinity between human FcRn-binding domain and human FcRn. More preferably, any temperature at from 20 degrees C to 35 degrees C, like any one of 20, 21, 22, 23, 24, 25, 26, 27, 28, 29, 30, 31, 32, 33, 34, and 35 degrees C is also employed in order to determine the binding affinity between human FcRn-binding domain and human FcRn. A temperature at 25 degrees C described in Example 5 is one of example for the embodiment of this invention. In a preferred embodiment, an interaction between human FcRn and FcRn-binding domain can be measured at pH 7.0 and at 25 degrees C as described in Example 5. Binding affinity of antigen-binding molecule to human FcRn can be measured by Biacore as described in Example 5.

In a more preferred embodiment, the antigen-binding molecules of the present invention have human FcRn-binding activity at pH 7.0 and at 25 degrees C which is stronger than intact human IgG. In a more preferred embodiment, human FcRn-binding activity at pH 7.0 and at 25 degrees C is 28-fold stronger than intact human IgG or stronger than KD 3.2 micromolar. In a more preferred embodiment, human FcRn-binding activity at pH 7.0 and at 25 degrees C is 38-fold stronger than intact human IgG or stronger than KD 2.3 micromolar.

An intact human IgG1, IgG2, IgG3 or IgG4 is preferably used as the intact human IgG for a purpose of a reference intact human IgG to be compared with the antigen-binding molecules for their human FcRn binding activity or in vivo activity. Preferably, a reference antigen-binding molecule comprising the same antigen-binding domain as an antigen-binding molecule of the interest and intact human IgG Fc domain as a human FcRn-binding domain can be appropriately used. More preferably, an intact human IgG1 is used for a purpose of a reference intact human IgG to be compared with the antigen-binding molecules for their human FcRn binding activity or in vivo activity.

More specifically, the antigen-binding molecules with long term effect on activity for eliminating antigen in plasma described in the present invention have human FcRn-binding activity at pH 7.0 and at 25 degrees C within a range of 28-fold to 440-fold stronger than intact human IgG1 or KD within a range of 3.0 micromolar to 0.2 micromolar. A long term plasma antigen concentration is determined by measuring total or free antigen concentration in plasma and molar antigen/antigen-binding molecule ratio at 2, 4, 7, 14, 28, 56, or 84 days after administration of an antigen-binding molecule in order to evaluate the long term effect of the antigen-binding molecule of the present invention on activity for eliminating antigen in plasma. Whether the reduction of plasma antigen concentration or molar antigen/antigen-binding molecule ratio is achieved by antigen-binding molecule described in the present invention can be determined by the evaluation of the reduction at any one or more of the time points described above.

Still more specifically, the antigen-binding molecules with short term effect on activity for eliminating antigen in plasma described in the present invention have human FcRn-binding activity at pH 7.0 and at 25 degrees C 440-fold stronger than intact human IgG or KD stronger than 0.2 micromolar. A short term plasma antigen concentration is determined by measuring total or free antigen concentration in plasma and molar antigen/ antigen-binding molecule ratio at 15 min, 1, 2, 4, 8, 12, or 24 hours after administration of an antigen-binding molecule in order to evaluate the short term effect of the antigen-binding molecule of the present invention on activity for eliminating antigen in plasma.

The methods of the present invention are applicable to any antigen-binding molecules regardless of the type of target antigen.

Antigens that are recognized by antigen-binding molecules such as antibodies of interest in the methods of the present invention are not particularly limited. Such antibodies of interest may recognize any antigen. Antibodies whose pharmacokinetics is improved by the methods of the present invention include, for example, receptor proteins (membrane-bound receptors and soluble receptors), antibodies that recognize a membrane antigen such as cell surface markers, and antibodies that recognize a soluble antigen such as cytokines. Specific examples of an antigen that is recognized by the antibody whose pharmacokinetics has been improved by the methods of the present invention include, for example: 17-IA, 4-1 BB, 4Dc, 6-keto-PGF1a, 8-iso-PGF2a, 8-oxo-dG, A1 Adenosine Receptor, A33, ACE, ACE-2, Activin, Activin A, Activin AB, Activin B, Activin C, Activin RIA, Activin RIA ALK-2, Activin RIB ALK-4, Activin RIIA, Activin RIIB, ADAM, ADAM10, ADAM12, ADAM15, ADAM17/TACE, ADAM8, ADAM9, ADAMTS, ADAMTS4, ADAMTS5, Addressins, adiponectin, ADP ribosyl cyclase-1, aFGF, AGE, ALCAM, ALK, ALK-1, ALK-7, allergen, alpha 1-antichemotrypsin, alpha1-antitrypsin, alpha-synuclein, alpha-V/beta-1 antagonist, aminin, amylin, amyloid beta, amyloid immunoglobulin heavy chain variable region. amyloid immunoglobulin light chain variable region, Androgen, ANG, angiotensinogen, Angiopoietin ligand-2, anti-Id, antithrombinIII, Anthrax, APAF-1, APE, APJ, apo A1, apo serum amyloid A, Apo-SAA, APP, APRIL, AR, ARC, ART, Artemin, ASPARTIC, Atrial natriuretic factor, Atrial natriuretic peptide, atrial natriuretic peptides A, atrial natriuretic peptides B, atrial natriuretic peptides C, av/b3 integrin, Axl, B7-1, B7-2, B7-H, BACE, BACE-1, Bacillus anthracis protective antigen, Bad, BAFF, BAFF-R, Bag-1, BAK, Bax, BCA-1, BCAM, Bcl, BCMA, BDNF, b-ECGF, beta-2-microglobulin, betalactamase, bFGF, BID, Bik, BIM, BLC, BL-CAM, BLK, B-lymphocyte Stimulator (BlyS), BMP, BMP-2 (BMP-2a), BMP-3 (Osteogenin), BMP-4 (BMP-2b), BMP-5, BMP-6 (Vgr-1), BMP-7 (OP-1), BMP-8 (BMP-8a), BMPR, BMPR-IA (ALK-3), BMPR-IB (ALK-6), BMPR-II (BRK-3), BMPs, BOK, Bombesin, Bone-derived neurotrophic factor, bovine growth hormone, BPDE, BPDE-DNA, BRK-2, BTC, B-lymphocyte cell adhesion molecule, C10, C1-inhibitor, C1q, C3, C3a, C4, C5, C5a(complement 5a), CA125, CAD-8, Cadherin-3, Calcitonin, cAMP, Carbonic anhydrase-IX, carcinoembryonic antigen (CEA), carcinoma-associated antigen, Cardiotrophin-1, Cathepsin A, Cathepsin B, Cathepsin C/DPPI, Cathepsin D, Cathepsin E, Cathepsin H, Cathepsin L, Cathepsin O, Cathepsin S, Cathepsin V, Cathepsin X/Z/P, CBL, CCI, CCK2, CCL, CCL1/I-309, CCL11/Eotaxin, CCL12/MCP-5, CCL13/MCP-4, CCL14/HCC-1, CCL15/HCC-2, CCL16/HCC-4, CCL17/TARC, CCL18/PARC, CCL19/ELC, CCL2/MCP-1, CCL20/MIP-3-alpha, CCL21/SLC, CCL22/MDC, CCL23/MPIF-1, CCL24/Eotaxin-2, CCL25/TECK, CCL26/Eotaxin-3, CCL27/CTACK, CCL28/MEC, CCL3/M1P-1-alpha, CCL3LI/LD-78-beta, CCL4/MIP-l-beta, CCL5/RANTES, CCL6/C10, CCL7/MCP-3, CCL8/MCP-2, CCL9/10/MTP-1-gamma, CCR, CCR1, CCR10, CCR2, CCR3, CCR4, CCR5, CCR6, CCR7, CCR8, CCR9, CD1, CD10, CD105, CD11a, CD11b, CD11c, CD123, CD13, CD137, CD138, CD14, CD140a, CD146, CD147, CD148, CD15, CD152, CD16, CD164, CD18, CD19, CD2, CD20, CD21, CD22, CD23, CD25, CD26, CD27L, CD28, CD29, CD3, CD30, CD30L, CD32, CD33 (p67 proteins), CD34, CD37, CD38, CD3E, CD4, CD40, CD40L, CD44, CD45, CD46, CD49a, CD49b, CD5, CD51, CD52, CD54, CD55, CD56, CD6, CD61, CD64, CD66e, CD7, CD70, CD74, CD8, CD80 (B7-1), CD89, CD95, CD105, CD158a, CEA, CEACAM5, CFTR, cGMP, CGRP receptor, CINC, CKb8-1, Claudin18, CLC, Clostridium botulinum toxin, Clostridium difficile toxin, Clostridium perfringens toxin, c-Met, CMV, CMV UL, CNTF, CNTN-1, complement factor 3 (C3), complement factor D, corticosteroid-binding globulin, Colony stimulating factor-1 receptor, COX, C-Ret, CRG-2, CRTH2, CT-1, CTACK, CTGF, CTLA-4, CX3CL1/Fractalkine, CX3CR1, CXCL, CXCL1/Gro-alpha, CXCL10, CXCL11/I-TAC, CXCL12/SDF-1-alpha/beta, CXCL13/BCA-1, CXCL14/BRAK, CXCL15/Lungkine. CXCL16, CXCL16, CXCL2/Gro-beta CXCL3/Gro-gamma, CXCL3, CXCL4/PF4, CXCL5/ENA-78, CXCL6/GCP-2, CXCL7/NAP-2, CXCL8/IL-8, CXCL9/Mig, CXCL10/IP-10, CXCR, CXCR1, CXCR2, CXCR3, CXCR4, CXCR5, CXCR6, cystatin C, cytokeratin tumor-associated antigen, DAN, DCC, DcR3, DC-SIGN, Decay accelerating factor, Delta-like protein ligand 4, des(1-3)-IGF-I (brain IGF-1), Dhh, DHICA oxidase, Dickkopf-1, digoxin, Dipeptidyl peptidase IV, DKI, DNAM-1, Dnase, Dpp, DPPIV/CD26, Dtk, ECAD, EDA, EDA-A1, EDA-A2, EDAR, EGF, EGFR (ErbB-1), EGF like domain containing protein 7, Elastase, elastin, EMA, EMMPRIN, ENA, ENA-78, Endosialin, endothelin receptor, endotoxin, Enkephalinase, eNOS, Eot, Eotaxin, Eotaxin-2, eotaxini, EpCAM, Ephrin B2/EphB4, Epha2 tyrosine kinase receptor, epidermal growth factor receptor (EGFR), ErbB2 receptor, ErbB3 tyrosine kinase receptor, ERCC, erythropoietin (EPO), Erythropoietin receptor, E-selectin, ET-1, Exodus-2, F protein of RSV, F10, F11, F12, F13, F5, F9, Factor Ia, Factor IX, Factor Xa, Factor VII, factor VIII, Factor VIIIc, Fas, FcalphaR, FcepsilonRI, FcgammaIIb, FcgammaRI, FcgammaRIIa, FcgammaRIIIa, FcgammaRIIIb, FcRn, FEN-1, Ferritin, FGF, FGF-19, FGF-2, FGF-2 receptor, FGF-3, FGF-8, FGF-acidic, FGF-basic, FGFR, FGFR-3, Fibrin, fibroblast activation protein (FAP), fibroblast growth factor, fibroblast growth factor-10, fibronectin, FL, FLIP, Flt-3, FLT3 ligand, Folate receptor, follicle stimulating hormone (FSH), Fractalkine (CX3C), free heavy chain, free light chain, FZD1, FZD10, FZD2, FZD3, FZD4, FZD5, FZD6, FZD7, FZD8, FZD9, G250, Gas 6, GCP-2, GCSF, G-CSF, G-CSF receptor, GD2, GD3, GDF, GDF-1, GDF-15 (MIC-1), GDF-3 (Vgr-2), GDF-5 (BMP-14/CDMP-1), GDF-6 (BMP-13/CDMP-2), GDF-7 (BMP-12/CDMP-3), GDF-8 (Myostatin), GDF-9, GDNF, Gelsolin, GFAP, GF-CSF, GFR-alpha1, GFR-alpha2, GFR-alpha3, GF-beta1, gH envelope glycoprotein, GITR, Glucagon, Glucagon receptor, Glucagon-like peptide 1 receptor, Glut 4, Glutamate carboxypeptidase II, glycoprotein hormone receptors, glycoprotein IIb/IIIa (GP IIb/IIIa), Glypican-3, GM-CSF, GM-CSF receptor, gp130, gp140, gp72, granulocyte-CSF (G-CSF), GRO/MGSA, Growth hormone releasing factor, GRO-beta, GRO-gamma, H. pylori, Hapten (NP-cap or NIP-cap), HB-EGF, HCC, HCC 1, HCMV gB envelope glycoprotein, HCMV UL, Hemopoietic growth factor (HGF), Hep B gp120, heparanase, heparin cofactor II, hepatic growth factor, Bacillus anthracis protective antigen, Hepatitis C virus E2 glycoprotein, Hepatitis E, Hepcidin, Her1, Her2/neu (ErbB-2), Her3 (ErbB-3), Her4 (ErbB-4), herpes simplex virus (HSV) gB glycoprotein, HGF, HGFA, High molecular weight melanoma-associated antigen (HMW-MAA), HIV envelope proteins such as GP120, HIV MIB gp 120 V3 loop, HLA, HLA-DR, HM1.24, HMFG PEM, HMGB-1, HRG, Hrk, HSP47, Hsp90, HSV gD glycoprotein, human cardiac myosin, human cytomegalovirus (HCMV), human growth hormone (hGH), human serum albumin, human tissue-type plasminogen activator (t-PA), Huntingtin, HVEM, IAP, ICAM, ICAM-1, ICAM-3, ICE, ICOS, IFN-alpha, IFN-beta, IFN-gamma, IgA, IgA receptor, IgE, IGF, IGF binding proteins, IGF-1, IGF-1 R, IGF-2, IGFBP, IGFR, IL, IL-1, IL-10, IL-10 receptors, IL-11, IL-11 receptors, IL-12, IL-12 receptors, IL-13, IL-13 receptors, IL-15, IL-15 receptors, IL-16, IL-16 receptors, IL-17, IL-17 receptors, IL-18 (IGIF), IL-18 receptors, IL-1alpha, IL-1beta, IL-1 receptors, IL-2, IL-2 receptors, IL-20, IL-20 receptors, IL-21, IL-21 receptors, IL-23, IL-23 receptors, IL-2 receptors, IL-3, IL-3 receptors, IL-31, IL-31 receptors, IL-3 receptors, IL-4, IL-4 receptors IL-5, IL-5 receptors, IL-6, IL-6 receptors, IL-7, IL-7 receptors, IL-8, IL-8 receptors, IL-9, IL-9 receptors, immunoglobulin immune complex, immunoglobulins, INF-alpha, INF-alpha receptors, INF-beta, INF-beta receptors, INF-gamma, INF-gamma receptors, IFN type-I , IFN type-I receptor, influenza, inhibin, Inhibin alpha, Inhibin beta, iNOS, insulin, Insulin A-chain, Insulin B-chain, Insulin-like growth factor 1, insulin-like growth factor 2, insulin-like growth factor binding proteins, integrin, integrin alpha2, integrin alpha3, integrin alpha4, integrin alpha4/beta1, integrin alpha-V/beta-3, integrin alpha-V/beta-6, integrin alpha4/beta7, integrin alpha5/beta1, integrin alpha5/beta3, integrin alpha5/beta6, integrin alpha-delta (alphaV), integrin alpha-theta, integrin beta1, integrin beta2, integrin beta3(GPIIb-IIIa), IP-10, I-TAC, JE, kalliklein, Kallikrein 11, Kallikrein 12, Kallikrein 14, Kallikrein 15, Kallikrein 2, Kallikrein 5, Kallikrein 6, Kallikrein L1, Kallikrein L2, Kallikrein L3, Kallikrein L4, kallistatin, KC, KDR, Keratinocyte Growth Factor (KGF), Keratinocyte Growth Factor-2 (KGF-2), KGF, killer immunoglobulin-like receptor, kit ligand (KL), Kit tyrosine kinase, laminin 5, LAMP, LAPP (Amylin, islet-amyloid polypeptide), LAP (TGF- 1), latency associated peptide, Latent TGF-1, Latent TGF-1 bp1, LBP, LDGF, LDL, LDL receptor, LECT2, Lefty, Leptin, leutinizing hormone (LH), Lewis-Y antigen, Lewis-Y related antigen, LFA-1, LFA-3, LFA-3 receptors, Lfo, LIF, LIGHT, lipoproteins, LIX, LKN, Lptn, L-Selectin, LT-a, LT-b, LTB4, LTBP-1, Lung surfactant, Luteinizing hormone, Lymphotactin, Lymphotoxin Beta Receptor, Lysosphingolipid receptor, Mac-1, macrophage-CSF (M-CSF), MAdCAM, MAG, MAP2, MARC, maspin, MCAM, MCK-2, MCP, MCP-1, MCP-2, MCP-3, MCP-4, MCP-I (MCAF), M-CSF, MDC, MDC (67 a.a.), MDC (69 a.a.), megsin, Mer, MET tyrosine kinase receptor family, METALLOPROTEASES, Membrane glycoprotein OX2, Mesothelin, MGDF receptor, MGMT, MHC (HLA-DR), microbial protein, MIF, MIG, MIP, MIP-1 alpha, MIP-1 beta, MIP-3 alpha, MIP-3 beta, MIP-4, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, monocyte attractant protein, monocyte colony inhibitory factor, mouse gonadotropin-associated peptide, MPIF, Mpo, MSK, MSP, MUC-16, MUC18, mucin (Mud), Muellerian-inhibiting substance, Mug, MuSK, Myelin associated glycoprotein, myeloid progenitor inhibitor factor-1 (MPIF-I), NAIP, Nanobody, NAP, NAP-2, NCA 90, NCAD, N-Cadherin, NCAM, Neprilysin, Neural cell adhesion molecule, neroserpin, Neuronal growth factor (NGF), Neurotrophin-3, Neurotrophin-4, Neurotrophin-6 , Neuropilin 1, Neurturin, NGF-beta, NGFR, NKG20, N-methionyl human growth hormone, nNOS, NO, Nogo-A, Nogo receptor, non-structural protein type 3 (NS3) from the hepatitis C virus, NOS, Npn, NRG-3, NT, NT-3, NT-4, NTN, OB, OGG1, Oncostatin M, OP-2, OPG, OPN, OSM, OSM receptors, osteoinductive factors, osteopontin, OX40L, OX40R, oxidized LDL, p150, p95, PADPr, parathyroid hormone, PARC, PARP, PBR, PBSF, PCAD, P-Cadherin, PCNA, PCSK9, PDGF, PDGF receptor, PDGF-AA, PDGF-AB, PDGF-BB, PDGF-D, PDK-1, PECAM, PEDF, PEM, PF-4, PGE, PGF, PGI2, PGJ2, PlGF, PIN, PLA2, Placenta growth factor, placental alkaline phosphatase (PLAP), placental lactogen, plasminogen activator inhibitor-1, platelet-growth factor, plgR, PLP, poly glycol chains of different size(e.g. PEG-20, PEG-30, PEG40), PP14, prekallikrein, prion protein, procalcitonin, Programmed cell death protein 1, proinsulin, prolactin, Proprotein convertase PC9, prorelaxin, prostate specific membrane antigen (PSMA), Protein A, Protein C, Protein D, Protein S, Protein Z, PS, PSA, PSCA, PsmAr, PTEN, PTHrp, Ptk, PTN, P-selectin glycoprotein ligand-1, R51, RAGE, RANK, RANKL, RANTES, relaxin, Relaxin A-chain, Relaxin B-chain, renin, respiratory syncytial virus (RSV) F, Ret, reticulon 4, Rheumatoid factors, RLI P76, RPA2, RPK-1, RSK, RSV Fgp, S100, RON-8, SCF/KL, SCGF, Sclerostin, SDF-1, SDF1 alpha, SDF1 beta, SERINE, Serum Amyloid P, Serum albumin, sFRP-3, Shh, Shiga like toxin II, SIGIRR, SK-1, SLAM, SLPI, SMAC, SMDF, SMOH, SOD, SPARC, sphingosine 1-phosphate receptor 1, Staphylococcal lipoteichoic acid, Stat, STEAP, STEAP-II, stem cell factor (SCF), streptokinase, superoxide dismutase, syndecan-1, TACE, TACI, TAG-72 (tumor-associated glycoprotein-72), TARC, TB, TCA-3, T-cell receptor alpha/beta, TdT, TECK, TEM1, TEM5, TEM7, TEM8, Tenascin, TERT, testicular PLAP-like alkaline phosphatase, TfR, TGF, TGF-alpha, TGF-beta, TGF-beta Pan Specific, TGF-beta RII, TGF-beta RIIb, TGF-beta RIII, TGF-beta R1 (ALK-5), TGF-beta1, TGF-beta2, TGF-beta3, TGF-beta4, TGF-beta5, TGF-I, Thrombin, thrombopoietin (TPO), Thymic stromal lymphoprotein receptor, Thymus Ck-1, thyroid stimulating hormone (TSH), thyroxine, thyroxine-binding globulin, Tie, TIMP, TIQ, Tissue Factor, tissue factor protease inhibitor, tissue factor protein, TMEFF2, Tmpo, TMPRSS2, TNF receptor I, TNF receptor II, TNF-alpha, TNF-beta, TNF-beta2, TNFc, TNF-RI, TNF-RII, TNFRSF10A (TRAIL R1 Apo-2/DR4), TNFRSF10B (TRAIL R2 DR5/KILLER/TRICK-2A/TRICK-B), TNFRSF10C (TRAIL R3 DcR1/LIT/TRID), TNFRSF10D (TRAIL R4 DcR2/TRUNDD), TNFRSF11A (RANK ODF R/TRANCE R), TNFRSF11B (OPG OCIF/TR1), TNFRSF12 (TWEAK R FN14), TNFRSF12A, TNFRSF13B (TACI), TNFRSF13C (BAFF R), TNFRSF14 (HVEM ATAR/ HveA/LIGHT R/TR2), TNFRSF16 (NGFR p75NTR), TNFRSF17 (BCMA), TNFRSF18 (GITR AITR), TNFRSF19 (TROY TAJ/TRADE), TNFRSF19L (RELT), TNFRSF1A (TNF R1 CD120a/p55-60), TNFRSF1B (TNF RII CD120b/p75-80), TNFRSF21 (DR6), TNFRSF22 (DcTRAIL R2 TNFRH2), TNFRSF25 (DR3 Apo-3/LARD/TR-3/TRAMP/WSL-1), TNFRSF26 (TNFRH3), TNFRSF3 (LTbR TNF RIII/TNFC R), TNFRSF4 (OX40 ACT35/TXGP1 R), TNFRSF5 (CD40 p50), TNFRSF6 (Fas Apo-1/APT1/CD95), TNFRSF6B (DcR3 M68/TR6), TNFRSF7 (CD27), TNFRSF8 (CD30), TNFRSF9 (4-1 BB CD137/ILA), TNFRST23 (DcTRAIL R1 TNFRH1), TNFSF10 (TRAIL Apo-2 Ligand/TL2), TNFSF11 (TRANCE/RANK Ligand ODF/OPG Ligand), TNFSF12 (TWEAK Apo-3 Ligand/DR3 Ligand), TNFSF13 (APRIL TALL2), TNFSF13B (BAFF BLYS/TALL1/THANK/TNFSF20), TNFSF14 (LIGHT HVEM Ligand/LTg), TNFSF15 (TL1A/VEGI), TNFSF18 (GITR Ligand AITR Ligand/TL6), TNFSF1A (TNF-a Conectin/DIF/TNFSF2), TNFSF1B (TNF-b LTa/TNFSF1), TNFSF3 (LTb TNFC/p33), TNFSF4 (OX40 Ligand gp34/TXGP1), TNFSFS (CD40 Ligand CD154/gp39/HIGM1/IMD3/TRAP), TNFSF6 (Fas Ligand Apo-1 Ligand/APT1 Ligand), TNFSF7 (CD27 Ligand CD70), TNFSF8 (CD30 Ligand CD153), TNFSF9 (4-1 BB Ligand CD137 Ligand), TNF-alpha, TNF-beta, TNIL-I, toxic metabolite, TP-1, t-PA, Tpo, TRAIL, TRAIL R, TRAIL-R1, TRAIL-R2, TRANCE, transferrin receptor, transforming growth factors (TGF) such as TGF-alpha and TGF-beta, Transmembrane glycoprotein NMB, Transthyretin, TRF, Trk, TROP-2, Trophoblast glycoprotein, TSG, TSLP, Tumor Necrosis Factor (TNF), tumor-associated antigen CA 125, tumor-associated antigen expressing Lewis Y related carbohydrate, TWEAK, TXB2, Ung, uPAR, uPAR-1, Urokinase, VAP-1, vascular endothelial growth factor (VEGF), vaspin, VCAM, VCAM-1, VECAD, VE-Cadherin, VE-Cadherin-2, VEFGR-1 (flt-1), VEFGR-2, VEGF receptor (VEGFR), VEGFR-3 (flt-4), VEGI, VIM, Viral antigens, VitB12 receptor, Vitronectin receptor, VLA, VLA-1, VLA-4, VNR integrin, von Willebrand Factor (vWF), WIF-1, WNT1, WNT10A, WNT10B, WNT11, WNT16, WNT2, WNT2B/13, WNT3, WNT3A, WNT4, WNT5A, WNT5B, WNT6, WNT7A, WNT7B, WNT8A, WNT8B, WNT9A, WNT9B, XCL1, XCL2/SCM-1-beta,

XCLI/Lymphotactin, XCR1, XEDAR, XIAP, and XPD.

Antigen binding molecules described in present invention are capable of reducing total antigen concentration in plasma of the above-described antigens. Antigen binding molecules described in present invention are also capable of eliminating plasma virus, bacteria, and fungus by binding to structural components of virus, bacteria and fungus. Particularly, F protein of RSV, Staphylococcal lipoteichoic acid, Clostridium difficile toxin, Shiga like toxin II, Bacillus anthracis protective antigen and Hepatitis C virus E2 glycoprotein can be used as a structural components of virus, bacteria and fungus.

Although the methods of the present invention are not limited to any particular theory, the relationship between the reduction (impairment) of the antigen-binding ability of antigen-binding molecule in the acidic pH range to less than that in the neutral pH range and/or the increase (enhancement) of the human FcRn-binding activity in the neutral pH range and the increase in the number of antigens to which a single antigen-binding molecule can bind, due to facilitation of uptake of antigen-binding molecules into cells, and the enhancement of antigen elimination from the plasma can be explained as follows.

For example, when the antigen-binding molecule is an antibody that binds to a membrane antigen, the antibody administered into the body binds to the antigen and then is taken up via internalization into endosomes in the cells together with the antigen while the antibody is kept bound to the antigen. Then, the antibody translocates to lysosomes while the antibody is kept bound to the antigen, and the antibody is degraded by the lysosome together with the antigen. The internalization-mediated elimination from the plasma is called antigen-dependent elimination, and such elimination has been reported with numerous antibody molecules (Drug Discov Today. 2006 Jan; 11(1-2): 81-8). When a single molecule of IgG antibody binds to antigens in a divalent manner, the single antibody molecule is internalized while the antibody is kept bound to the two antigen molecules, and degraded in the lysosome. Accordingly, in the case of typical antibodies, one molecule of IgG antibody cannot bind to three or more molecules of antigen. For example, a single IgG antibody molecule having a neutralizing activity cannot neutralize three or more antigen molecules.

The relatively prolonged retention (slow elimination) of IgG molecules in the plasma is due to the function of human FcRn which is known as a salvage receptor of IgG molecules. When taken up into endosomes via pinocytosis, IgG molecules bind to human FcRn expressed in the endosomes under the acidic condition in the endosomes. While IgG molecules that did not bind to human FcRn transfer to lysosomes where they are degraded, IgG molecules that are bound to human FcRn translocate to the cell surface and return again in the plasma by dissociating from human FcRn under the neutral condition in the plasma.

Alternatively, when the antigen-binding molecule is an antibody that binds to a soluble antigen, the antibody administered into the body binds to the antigen and then is taken up into cells while the antibody is kept bound to the antigen. Many antibodies taken up into cells are released to the outside of the cell via FcRn. However, since the antibodies are released to the outside of the cell, with the antibodies kept bound to antigens, the antibodies cannot bind to antigens again. Thus, similar to antibodies that bind to membrane antigens, in the case of typical antibodies, one molecule of IgG antibody cannot bind to three or more antigen molecules.

pH-dependent antigen-binding antibodies that strongly bind to an antigen under the neutral conditions in plasma but dissociate from the antigen under acidic conditions in the endosome (antibodies that bind under neutral conditions but dissociate under acidic conditions) can dissociate from the antigen in the endosome. Such pH-dependent antigen-binding antibodies can bind to antigens again when they are recycled to the plasma by FcRn after antigen dissociation; thus, each antibody can repeatedly bind to a number of antigens. Furthermore, the antigen bound to the antigen-binding molecule is dissociated in the endosome and not recycled to the plasma. This facilitates the antigen-binding molecule-mediated antigen uptake into cells. Thus, the administration of an antigen-binding molecule can enhance the antigen elimination and thereby reduces the plasma antigen concentration.

The antigen-binding molecule-mediated antigen uptake into cells can be further facilitated by conferring the human FcRn-binding activity under neutral conditions (pH 7.4) to an antibody that binds to an antigen in a pH-dependent manner (binds under neutral conditions but dissociates under acidic conditions). Thus, the administration of an antigen-binding molecule can enhance the antigen elimination and thereby reduces the plasma antigen concentration. Normally, both antibody and antigen-antibody complex are taken up into cells by non-specific endocytosis, and then transported to the cell surface by binding to FcRn under acidic conditions in the endosome. The antibody and antigen-antibody complex are recycled to the plasma via dissociation from FcRn under the neutral condition on cell surface. Thus, when an antibody that exhibits sufficient pH dependency in antigen binding (binds under neutral conditions but dissociates under acidic conditions) binds to the antigen in the plasma and then is dissociated from the bound antigen in the endosome, the antigen elimination rate is assumed to be equal to the rate of antigen uptake into cells via non-specific endocytosis. On the other hand, when the pH dependency is insufficient, the antigen that did not dissociate in the endosome is also recycled to the plasma. Meanwhile, when the pH dependency is sufficient, the rate-determining step in the antigen elimination is the uptake into cells by non-specific endocytosis. Some of FcRn is assumed to be localized on the cell surface because FcRn transports antibodies from the endosome to the cell surface.

The present inventors assumed that IgG-type immunoglobulins, which are one of antigen-binding molecules, typically have little FcRn-binding ability in the neutral pH range, but those that exhibit FcRn-binding ability in the neutral pH range could bind to FcRn on the cell surface and thus are taken up into cells in an FcRn-dependent manner by binding to cell-surface FcRn. The rate of FcRn-mediated uptake into cells is more rapid than the rate of uptake into cells by non-specific endocytosis. Thus, the rate of antigen elimination can be further accelerated by conferring FcRn-binding ability in the neutral pH range. Specifically, an antigen-binding molecule having FcRn-binding ability in the neutral pH range transports an antigen into cells more rapidly than the typical (intact human) IgG-type immunoglobulin, and then the antigen-binding molecule is dissociated from the antigen in the endosome. The antigen-binding molecule is recycled to the cell surface or plasma, and again binds to another antigen and is taken up into cells via FcRn. The rate of this cycle can be accelerated by improving FcRn-binding ability in the neutral pH range, thereby accelerating the rate of antigen elimination from the plasma. Furthermore, the efficiency can be further improved by reducing the antigen-binding activity of an antigen-binding molecule in the acidic pH range to less than that in the neutral pH range. In addition, the number of antigens to which a single antigen-binding molecule can bind is assumed to increase with an increasing number of cycles achieved by a single antigen-binding molecule. The antigen-binding molecule of the present invention comprises an antigen-binding domain and an FcRn-binding domain. Since the FcRn-binding domain does not affect antigen binding, or in view of the mechanism described above, facilitation of the antigen-binding molecule-mediated antigen uptake into cells can be expected regardless of the type of antigen, and as a result increases the antigen elimination rate by reducing the antigen-binding activity of an antigen-binding molecule in the acidic pH range (binding ability) to less than that in the neutral pH range and/or increasing its FcRn-binding activity at the plasma pH.

<Substances that serve as an antigen-binding molecule>

Furthermore, the present invention provides antigen-binding molecules that have human FcRn-binding activity in the acidic and neutral pH ranges and whose antigen-binding activity in the acidic pH range is lower than that in the neutral pH range. Specific examples of antigen-binding molecules include those that have human FcRn-binding activity at pH 5.8 to pH 6.0 and pH 7.4, which are assumed to be the in vivo pH of the early endosome and plasma, respectively, and whose antigen-binding activity is lower at pH 5.8 than at pH 7.4. An antigen-binding molecule whose antigen-binding activity is lower at pH 5.8 than at pH 7.4 can also be referred to as an antigen-binding molecule whose antigen-binding activity is stronger at pH 7.4 than at pH 5.8.

The antigen-binding molecules of the present invention having human FcRn-binding activity in the acidic and neutral pH ranges are preferably antigen-binding molecules that also have human FcRn-binding activity in the acidic pH range and stronger human FcRn-binding activity than intact human IgG in the neutral pH range. The binding activity ratio is not limited, as long as their human FcRn-binding activity is even slightly stronger at pH 7.4.

According to the Journal of Immunology (2009) 182: 7663-7671, the human FcRn-binding activity of intact human IgG1 is KD 1.7 micromolar in the acidic pH range (pH 6.0), while the activity is almost undetectable in the neutral pH range. Thus, in a preferred embodiment, the antigen-binding molecules of the present invention having human FcRn-binding activity in the acidic and neutral pH ranges include antigen-binding molecules that have a human FcRn-binding activity of KD 20 micromolar or stronger in the acidic pH range, which is equal to or stronger than that of intact human IgG in the neutral pH range. In a more preferred embodiment, the antigen-binding molecules of the present invention include antigen-binding molecules whose human FcRn-binding activity is KD 2.0 micromolar or stronger in the acidic pH range and KD 40 micromolar or stronger in the neutral pH range. In a still more preferred embodiment, the antigen-binding molecules of the present invention include antigen-binding molecules whose human FcRn-binding activity is KD 0.5 micromolar or stronger in the acidic pH range and KD 15 micromolar or stronger in the neutral pH range. The above KD values are determined by the method described in the Journal of Immunology (2009) 182: 7663-7671 (by immobilizing the antigen-binding molecule onto a chip and loading human FcRn as an analyte).

The present invention provides an antigen-binding molecule comprising an antigen-binding domain and a human FcRn-binding domain, which has a human FcRn-binding activity in the acidic and neutral pH ranges, wherein a human FcRn and a lower antigen-binding activity in the acidic pH range than in the neutral pH range is stronger than KD 3.2 micromolar. The present invention also provides an antigen-binding molecule comprising an antigen-binding domain and a human FcRn-binding domain, which has a human FcRn-binding activity in the neutral pH ranges, wherein a human FcRn-binding activity in the neutral pH ranges is 28-fold stronger than that of an intact human IgG. The antigen-binding molecules of the present invention have human FcRn-binding activity at pH 7.0 and at 25 degrees C which is stronger than intact human IgG. In a more preferred embodiment, human FcRn-binding activity at pH 7.0 and at 25 degrees C is 28-fold stronger than intact human IgG or stronger than KD 3.2 micromolar.

The present invention provides an antigen-binding molecule comprising an antigen-binding domain and a human FcRn-binding domain, which has a human FcRn-binding activity in the neutral pH range, wherein a human FcRn-binding activity in the neutral pH range is stronger than KD 2.3 micromolar. The present invention also provides an antigen-binding molecule comprising an antigen-binding domain and a human FcRn-binding domain, which has a human FcRn-binding activity in the neutral pH range, wherein a human FcRn-binding activity in the neutral pH range is 38-fold stronger than that of an intact human IgG.

Herein, the acidic pH range typically refers to pH 4.0 to pH 6.5. The acidic pH range is preferably a range indicated by any pH value within pH 5.5 to pH 6.5, preferably selected from 5.5, 5.6, 5.7, 5.8, 5.9, 6.0, 6.1, 6.2, 6.3, 6.4, and 6.5, particularly preferably pH 5.8 to pH 6.0, which is close to the pH in early endosome in vivo. Meanwhile, herein the neutral pH range typically refers to pH 6.7 to pH 10.0. The neutral pH range is preferably a range indicated by any pH value within pH 7.0 to pH 8.0, preferably selected from pH 7.0, 7.1, 7.2, 7.3, 7.4, 7.5, 7.6, 7.7, 7.8, 7.9, and 8.0, particularly preferably pH 7.4, which is close to in vivo plasma (blood) pH. pH 7.0 can be used as an alternative to pH 7.4 when it is difficult to assess the binding affinity between human FcRn-binding domain and human FcRn due its low affinity at pH 7.4. As a temperature employed in the assay condition, a binding affinity between human FcRn-binding domain and human FcRn may be assessed at any temperature from 10 degrees C to 50 degrees C. Preferably, a temperature at from 15 degrees C to 40 degrees C is employed in order to determine the binding affinity between human FcRn-binding domain and human FcRn. More preferably, any temperature at from 20 degrees C to 35 degrees C, like any one of 20, 21, 22, 23, 24, 25, 26, 27, 28, 29, 30, 31, 32, 33, 34, and 35 degrees C is also employed in order to determine the binding affinity between human FcRn-binding domain and human FcRn. A temperature at 25 degrees C described in Example 5 is one of example for the embodiment of this invention.

In a more preferred embodiment, human FcRn-binding activity at pH 7.0 and at 25 degrees C is 38-fold stronger than intact human IgG or stronger than KD 2.3 micromolar. An intact human IgG1, IgG2, IgG3 or IgG4 is used as the intact human IgG for a purpose of a reference intact human IgG to be compared with the antigen-binding molecules for their human FcRn binding activity. More preferably, an intact human IgG1 is used for a purpose of a reference intact human IgG to be compared with the antigen-binding molecules for their human FcRn binding activity.

The present invention provides an antigen-binding molecule comprising an antigen-binding domain and a human FcRn-binding domain wherein a total antigen concentration in plasma after administration of the antigen-binding molecule to non-human animal is lower than a total antigen concentration in plasma after administration of a reference antigen-binding molecule to non-human animal.

The present invention also provides an antigen-binding molecule in which a plasma antigen concentration after administration of the antigen-binding molecule to non-human animal is lower than a total antigen concentration in plasma obtained from the non-human animal to which the antigen-binding molecule is not administered.

Total antigen concentration in plasma can be lowered by administration of antigen-binding molecule of the present invention by 2-fold, 5-fold, 10-fold, 20-fold, 50-fold, 100-fold, 200-fold, 500-fold and 1,000-fold or even higher as compared to the administration of reference antigen-binding molecule comprising the intact human IgG Fc domain as a human FcRn-binding domain or compared to when antigen-binding domain molecule of the present invention is not administered.

In another embodiment, the present invention provides an antigen-binding molecule comprising an antigen-binding domain and a human FcRn-binding domain in which a molar antigen/antigen-binding molecule ratio (C) of the antigen-binding molecule calculated as follows;

$$C = A/B,$$

is lower than a molar antigen/antigen-binding molecule ratio (C') of an antigen-binding molecule comprising the same antigen-binding domain and intact human IgG Fc domain as a human FcRn-binding domain calculated as follows;

$$C' = A'/B',$$

wherein;

A is a total antigen concentration in plasma after administration of the antigen-binding molecule to non-human animal,
B is a plasma concentration of an antigen-binding molecule after administration of the antigen-binding molecule to non-human animal,
A' is a total antigen concentration in plasma after administration of a reference antigen-binding molecule to non-human animal,
B' is a plasma concentration of an antigen-binding molecule after administration of a reference antigen-binding molecule to non-human animal.

Molar antigen/ antigen-binding molecule ratio can be lowered by administration of antigen-binding molecule of present invention by 2-fold, 5-fold, 10-fold, 20-fold, 50-fold, 100-fold, 200-fold, 500-fold and 1,000-fold or even higher as compared to the administration of antigen-binding molecule comprising the intact human IgG Fc domain as a human FcRn-binding domain.

Reduction of total antigen concentration in plasma or molar antigen/antibody ratio can be assessed as described in Examples 6, 8, and 13. More specifically, using human FcRn transgenic mouse line 32 or line 276 (Jackson Laboratories, Methods Mol Biol. (2010) 602: 93-104.), they can be assessed by either antigen-antibody co-injection model or steady-state antigen infusion model when antigen-binding molecule do not cross-react to the mouse counterpart antigen. When antigen-binding molecule cross-react with mouse counterpart, they can be assessed by simply injecting antigen-binding molecule to human FcRn transgenic mouse line 32 or line 276 (Jackson Laboratories). In co-injection model, mixture of antigen-binding molecule and antigen is administered to the mouse. In steady-state antigen infusion model, infusion pump containing antigen solution is implanted to the mouse to achieve constant plasma antigen concentration, and then antigen-binding molecule is injected to the mouse. Test antigen-binding molecule is administered at same dosage. Total antigen concentration in plasma, free antigen concentration in plasma and plasma antigen-binding molecule concentration is measured at an appropriate time point using methods known to those skilled in the art.

Route of administration of an antigen-binding molecule of the present invention can be selected from intradermal, intravenous, intravitreal, subcutaneous, intraperitoneal, parenteral and intramuscular injection.

More specifically, the antigen-binding molecules with long term effect on activity for eliminating antigen in plasma described in the present invention have human FcRn-binding activity at pH 7.0 and 25 degrees C within a range of 28-fold to 440-fold stronger than intact human IgG1 or KD within a range of 3.0 micromolar to 0.2 micromolar. A long term plasma antigen concentration is determined by measuring total or free antigen concentration in plasma and molar antigen/antigen-binding molecule ratio at 2, 4, 7, 14, 28, 56, or 84 days after administration of an antigen-binding molecule in order to evaluate the long term effect of the antigen-binding molecule of the present invention on activity for eliminating antigen in plasma. Whether the reduction of plasma antigen concentration or molar antigen/antigen-binding molecule ratio is achieved by antigen-binding molecule described in the present invention can be determined by the evaluation of the reduction at any one or more of the time points described above.

Still more specifically, the antigen-binding molecules with short term effect on for eliminating antigen in plasma described in the present invention have human FcRn-binding activity at pH 7.0 and at 25 degrees C 440-fold stronger than intact human IgG or KD stronger than 0.2 micromolar. A short term plasma antigen concentration is determined by measuring total or free antigen concentration in plasma and molar antigen/ antigen-binding molecule ratio at 15 min, 1, 2, 4, 8, 12, or 24 hours after administration of an antigen-binding molecule in order to evaluate the short term effect of the antigen-binding molecule of the present invention on activity for eliminating antigen in plasma.

Furthermore, in an antigen-binding molecule of the present invention that has a lower antigen-binding activity in the acidic pH range than in the neutral pH range, the binding activity ratio is not limited, as long as the antigen-binding activity is lower in the acidic pH range than in the neutral pH range. As long as the antigen-binding activity in the acidic pH range is even slightly lower, the antigen-binding molecule is acceptable. In a preferred embodiment, the antigen-binding molecules of the present invention include antigen-binding molecules whose antigen-binding activity at pH 7.4 is twice or higher than that at pH 5.8. In a more preferred embodiment, the antigen-binding molecules of the present invention include antigen-binding molecules whose antigen-binding activity at pH 7.4 is ten times or higher than that at pH 5.8. In a still more preferred embodiment, the antigen-binding molecules of the present invention include antigen-binding molecules whose antigen-binding activity at pH 7.4 is 40 times or higher than that at pH 5.8.

Specifically, the antigen-binding molecules of the present invention include, for example, the embodiments described in WO 2009/125825. More specifically, in a preferred embodiment, the antigen-binding molecule of the present invention has antigen-binding activity at pH 5.8 that is lower than that at pH 7.4, wherein the value of KD (pH5.8)/KD (pH7.4), which is a ratio of KD for the antigen at pH 5.8 and that at pH 7.4, is preferably 2 or greater, more preferably 10 or greater, and still more preferably 40 or greater. The upper limit of the KD (pH5.8)/KD (pH7.4) value is not particularly limited, and may be any value, for example, 400, 1,000, or 10,000, as long as production is possible using the technologies of those skilled in the art.

In another preferred embodiment, the antigen-binding molecule of the present invention whose antigen-binding activity at pH 5.8 is lower than that at pH 7.4, has a value of $k_d$ (pH5.8)/$k_d$ (pH7.4), which is a ratio of the $k_d$ for the antigen at pH 5.8 and the $k_d$ for the antigen at pH 7.4, that is 2 or greater, more preferably 5 or greater, even more preferably 10 or greater, and still more preferably 30 or greater. The upper limit of the $k_d$ (pH5.8)/$k_d$ (pH7.4) value is not particularly limited, and may be any value, for example, 50, 100, or 200, as long as production is possible using the technologies of those skilled in the art.

Conditions other than the pH at which the antigen-binding activity and human FcRn-binding activity is measured can be appropriately selected by those skilled in the art, and the conditions are not particularly limited; however, the measurements can be carried out, for example, under conditions of MES buffer and at 37 degrees C, as described in the Examples. Furthermore, the antigen-binding activity of an antigen-binding molecule can be determined by methods known to those skilled in the art, for example, using Biacore T100 (GE Healthcare) or the like, as described in the Examples.

The antigen-binding molecules of the present invention facilitate antigen uptake into cells. The molecules are easily dissociated from the antigen in the endosome, and then released to the outside of the cell by binding to human FcRn. The antigen-binding molecules of the present invention are assumed to bind easily to an antigen in the plasma again. Thus, for example, when the antigen-binding molecule of the present invention is a neutralizing antigen-binding molecule, reduction of the plasma antigen concentration can be facilitated by administering the molecule. Accordingly, an antigen-binding molecule that has human FcRn-binding activity in the acidic pH range has a lower antigen-binding activity in the acidic pH range than in the neutral pH range; and an antigen-binding molecule that has human FcRn-binding activity in the neutral pH range is likely to be an antigen-binding molecule that has superior pharmacokinetics and can bind to more antigens per molecule.

In a preferred embodiment, such antigen-binding molecules having human FcRn-binding activity in the acidic and neutral pH ranges include those that contain a human FcRn-binding domain having the ability to directly or indirectly bind to human FcRn. When the domain already has a human FcRn-binding ability in the acidic and neutral pH ranges, it may be used as it is. Alternatively, even if the domain has a human FcRn-binding activity in the acidic pH range but exhibits only weak or no human FcRn-binding activity in the neutral pH range, it may be used after altering amino acids in the domain to have human FcRn-binding activity in the neutral pH range. Alternatively, it is possible to enhance the human FcRn-binding activity by altering amino acids in a domain which already has human FcRn-binding ability in the acidic and neutral pH ranges. Such antigen-binding molecules include, for example, those having an amino acid sequence of IgG Fc domain that contains an alteration of at least one amino acid. The amino acid alteration is not particularly limited; and alteration may be performed at any site as long as the human FcRn-binding activity in the neutral pH range is stronger than before alteration.

Specifically, amino acid alterations that yield the human FcRn-binding activity in the acidic and neutral pH ranges include, for example, alterations of amino acids of positions 221 to 225, 227, 228, 230, 232, 233 to 241, 243 to 252, 254 to 260, 262 to 272, 274, 276, 278 to 289, 291 to 312, 315 to 320, 324, 325, 327 to 339, 341, 343, 345, 360, 362, 370, 375 to 378, 380, 382, 385 to 387, 389, 396, 414, 416, 423, 424, 426 to 438, 440, and 442 (EU numbering) in the parent IgG Fc domain described above. More specifically, the amino acid alterations include those at the sites (in EU numbering) shown in Tables, 1, 2, 6-1, 6-2, and 9. Preferred antigen-binding molecules include those comprising an amino acid sequence that results from alteration of at least one amino acid selected from those at positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 in EU numbering.

In a preferred embodiment, such amino acid alterations include:

an amino acid substitution of Met for Gly at position 237;
an amino acid substitution of Ala for Pro at position 238;
an amino acid substitution of Lys for Ser at position 239;
an amino acid substitution of Ile for Lys at position 248;
an amino acid substitution of Ala, Phe, Ile, Met, Gln, Ser, Val, Trp, or Tyr for Thr at position 250;
an amino acid substitution of Phe, Trp, or Tyr for Met at position 252;
an amino acid substitution of Thr for Ser at position 254;
an amino acid substitution of Glu for Arg at position 255;
an amino acid substitution of Asp, Glu, or Gln for Thr at position 256;
an amino acid substitution of Ala, Gly, Ile, Leu, Met, Asn, Ser, Thr, or Val for Pro at position 257;
an amino acid substitution of His for Glu a position 258;
an amino acid substitution of Ala for Asp at position 265;
an amino acid substitution of Phe for Asp at position 270;
an amino acid substitution of Ala, or Glu for Asn at position 286;
an amino acid substitution of His for Thr at position 289;
an amino acid substitution of Ala for Asn at position 297;
an amino acid substitution of Gly for Ser at position 298;
an amino acid substitution of Ala for Val at position 303;
an amino acid substitution of Ala for Val at position 305;
an amino acid substitution of Ala, Asp, Phe, Gly, His, Ile, Lys, Leu, Met, Asn, Pro, Gln, Arg, Ser, Val, Trp, or Tyr for Thr at position 307;
an amino acid substitution of Ala, Phe, Ile, Leu, Met, Pro, Gln, or Thr for Val at position 308;
an amino acid substitution of Ala, Asp, Glu, Pro, or Arg for Leu or Val at position 309;
an amino acid substitution of Ala, His, or Ile for Gln at position 311;
an amino acid substitution of Ala, or His for Asp at position 312;
an amino acid substitution of Lys, or Arg for Leu at position 314;
an amino acid substitution of Ala, or His for Asn at position 315;
an amino acid substitution of Ala for Lys at position 317;

an amino acid substitution of Gly for Asn at position 325;
an amino acid substitution of Val for Ile at position 332;
an amino acid substitution of Leu for Lys at position 334;
an amino acid substitution of His for Lys at position 360;
an amino acid substitution of Ala for Asp at position 376;
an amino acid substitution of Ala for Glu at position 380;
an amino acid substitution of Ala for Glu t position 382;
an amino acid substitution of Ala for Asn or Ser at position 384;
an amino acid substitution of Asp, or His for Gly at position 385;
an amino acid substitution of Pro for Gln at position 386;
an amino acid substitution of Glu for Pro at position 387;
an amino acid substitution of Ala, or Ser for Asn at position 389;
an amino acid substitution of Ala for Ser at position 424;
an amino acid substitution of Ala, Asp, Phe, Gly, His, Ile, Lys, Leu, Asn, Pro, Gln, Ser, Thr, Val, Trp, or Tyr for Met at position 428;
an amino acid substitution of Lys for His at position 433;
an amino acid substitution of Ala, Phe, His, Ser, Trp, or Tyr for Asn at position 434;
and an amino acid substitution of His for Tyr or Phe at position 436 in EU numbering.

The number of amino acids to be altered is not particularly limited; it is possible to alter an amino acid at only a single site or two or more sites. Combinations of two or more amino acid alterations include, for example, those shown in Tables 3, 4-1 to 4-5, 6-1, 6-2, and 9.

Meanwhile, the domains that already have human FcRn-binding ability in the acidic and neutral pH ranges include, for example, human FcRn-binding domains comprising at least one amino acid selected from:

Met at amino acid position 237;
Ala at amino acid position 238;
Lys at amino acid at position 239;
Ile at amino acid position 248;
Ala, Phe, Ile, Met, Gln, Ser, Val, Trp, or Tyr at amino acid position 250;
Phe, Trp, or Tyr at amino acid position 252;
Thr at amino acid position 254;
Glu at amino acid position 255;
Asp, Glu, or Gln at amino acid position 256;
Ala, Gly, Ile, Leu, Met, Asn, Ser, Thr, or Val at amino acid position 257;
His at amino acid position 258;
Ala at amino acid position 265;
Phe t amino acid position 270;
Ala or Glu at amino acid position 286;
His at amino acid position 289;
Ala at amino acid position 297;
Gly at amino acid position 298;
Ala at amino acid position 303;
Ala at amino acid position 305;
Ala, Asp, Phe, Gly, His, Ile, Lys, Leu, Met, Asn, Pro, Gln, Arg, Ser, Val, Trp, or Tyr at amino acid position 307;
Ala, Phe, Ile, Leu, Met, Pro, Gln, or Thr at amino acid position 308;
Ala, Asp, Glu, Pro, or Arg at amino acid position 309;
Ala, His, or Ile at amino acid position 311;
Ala or His at amino acid position 312;
Lys or Arg at amino acid position 314;
Ala or His at amino acid position 315;
Ala at amino acid position 317;
Gly at amino acid position 325;
Val at amino acid position 332;
Leu at amino acid position 334;
His at amino acid position 360;
Ala at amino acid position 376;
Ala at amino acid position 380;

45

Ala at amino acid position 382;

Ala at amino acid position 384;

Asp or His at amino acid position 385;

Pro at amino acid position 386;

Glu at amino acid position 387;

Ala or Ser at amino acid position 389;

Ala at amino acid position 424;

Ala, Asp, Phe, Gly, His, Ile, Lys, Leu, Asn, Pro, Gln, Ser, Thr, Val, Trp, or Tyr at amino acid position 428;

Lys at amino acid position 433;

Ala, Phe, His, Ser, Trp, or Tyr at amino acid position 434;

and His at amino acid position 436 (EU numbering) in the parent IgG Fc domain.

An amino acid at a site or amino acids at two or more sites may have these amino acids. Combinations of amino acids at two or more positions include, for example, those shown in Tables 3, 4-1 to 4-5, 6-1, 6-2, and 9.

Alternatively, in a preferred embodiment, the antigen-binding molecule whose antigen-binding activity in the acidic pH range is lower than that in the neutral pH range includes antigen-binding molecules in which at least one amino acid in the antigen-binding molecule is replaced with histidine or a non-natural amino acid, or in which at least one histidine or a non-natural amino acid has been inserted. The site into which the histidine or non-natural amino acid mutation is introduced is not particularly limited and may be any site, as long as the antigen-binding activity in the acidic pH range is weaker than that in the neutral pH range (the KD (in the acidic pH range)/KD (in the neutral pH range) value is greater or the $k_d$ (in the acidic pH range)/$k_d$ (in the neutral pH range) value is greater) as compared to before substitution. Examples include variable regions and CDRs of an antibody in the case the antigen-binding molecule is an antibody. The number of amino acids to be replaced with histidine or non-natural amino acid and the number of amino acids to be inserted can be appropriately determined by those skilled in the art. One amino acid may be replaced with histidine or non-natural amino acid, or one amino acid may be inserted, or two or more amino acids may be replaced with histidine or non-natural amino acids, or two or more amino acids may be inserted. Moreover, apart from the substitutions of histidine or non-natural amino acid or insertion of histidine or of non-natural amino acid, deletion, addition, insertion, and/or substitution and such of other amino acids may also be simultaneously carried out. Substitutions of histidine or non-natural amino acid or insertion of histidine or of non-natural amino acid may be carried out at random using a method such as histidine scanning, which uses histidine instead of alanine in alanine scanning which is known to those skilled in the art. Antigen-binding molecules whose KD (pH5.8)/KD (pH7.4) or $k_d$ (pH5.8)/$k_d$ (pH7.4) is increased as compared to before mutation can be selected from antigen-binding molecules into which histidine or non-natural amino acid mutation has been introduced at random.

Preferred antigen-binding molecules with mutation to histidine or to non-natural amino acid and whose antigen-binding activity in the acidic pH range is lower than that in the neutral pH range include, for example, antigen-binding molecules whose antigen-binding activity at pH 7.4 after the mutation to histidine or to non-natural amino acid is equivalent to the antigen-binding activity at pH 7.4 before the mutation to histidine or to non-natural amino acid. In the present invention, "an antigen-binding molecule after histidine or non-natural amino acid mutation has an antigen-binding activity that is equivalent to that of the antigen-binding molecule before histidine or non-natural amino acid mutation" means that, when the antigen-binding activity of an antigen-binding molecule before histidine or non-natural amino acid mutation is set as 100%, the antigen-binding activity of the antigen-binding molecule after histidine or non-natural amino acid mutation is at least 10% or more, preferably 50% or more, more preferably 80% or more, and still more preferably 90% or more. The antigen-binding activity at pH 7.4 after histidine or non-natural amino acid mutation may be stronger than the antigen-binding activity at pH 7.4 before histidine or non-natural amino acid mutation. When the antigen-binding activity of the antigen-binding molecule is decreased due to substitution or insertion of histidine or non-natural amino acid, the antigen-binding activity may be adjusted by introducing substitution, deletion, addition, and/or insertion and such of one or more amino acids into the antigen-binding molecule so that the antigen-binding activity becomes equivalent to that before histidine substitution or insertion. The present invention also includes such antigen-binding molecules whose binding activity has been made equivalent as a result of substitution, deletion, addition, and/or insertion of one or more amino acids after histidine substitution or insertion.

Further, when the antigen-binding molecule is a substance including an antibody constant region, in another preferred embodiment of the antigen-binding molecule whose antigen-binding activity at pH 5.8 is lower than that at pH 7.4, the present invention includes methods for altering antibody constant regions contained in the antigen-binding molecules. Specific examples of antibody constant regions after alteration include the constant regions described in the Examples in WO 2009/125825 (SEQ ID NOs: 11, 12, 13, and 14).

When the antigen-binding activity of the antigen-binding substance at pH 5.8 is weakened compared to that at pH 7.4 (when KD (pH5.8)/KD (pH7.4) value is increased) by the above described methods and such, it is generally preferable that the KD (pH5.8)/KD (pH7.4) value is two times or more, more preferably five times or more, and even more preferably ten times or more as compared to that of the original antibody, but is not particularly limited thereto.

Furthermore, the present invention provides antigen-binding molecules having substitution of histidine or a non-natural amino acid for at least one amino acid at one of the sites described below. The amino acid positions are shown according to Kabat numbering (Kabat EA et al. (1991) Sequences of Proteins of Immunological Interest, NIH).
Heavy chain: H27, H31, H32, H33, H35, H50, H58, H59, H61, H62, H63, H64, H65, H99, H100b, and H102

Light chain: L24, L27, L28, L32, L53, L54, L56, L90, L92, and L94
Of these alteration sites, H32, H61, L53, L90, and L94 are assumed to be highly general alteration sites.

Specifically, preferred combinations of sites for histidine or non-natural amino acid substitutions include, for example, the combination of H27, H31, and H35; the combination of H27, H31, H32, H35, H58, H62, and H102; the combination of L32 and L53; and the combination of L28, L32, and L53. Furthermore, preferred combinations of substitutions sites in the heavy and light chains include, for example, the combination of H27, H31, L32, and L53.
An antigen-binding molecule of the present invention may have other properties, and for example may be an agonistic or antagonistic antigen-binding molecule, as long as its antigen-binding activity is lower in the acidic pH range than in the neutral pH range, and it has human FcRn-binding activity in the acidic and neutral pH ranges. Preferred antigen-binding molecules of the present invention include, for example, antagonistic antigen-binding molecules. Such an antagonistic antigen-binding molecule is typically an antigen-binding molecule that inhibits receptor-mediated intracellular signaling by blocking the binding between ligand (agonist) and receptor.
Meanwhile, an antigen-binding molecule of the present invention may recognize any antigen. Specifically, antigens recognized by an antigen-binding molecule of the present invention include, for example, the above-described receptor proteins (membrane-bound receptors and soluble receptors), membrane antigens such as cell-surface markers, and soluble antigens such as cytokines. Such antigens include, for example, the antigens described above.
In a preferred embodiment, the antigen-binding molecules of the present invention include IgG-type immunoglobulins (IgG antibodies) having an antigen-binding domain and a human FcRn-binding domain. When an IgG antibody is used as an antigen-binding molecule, the type is not limited; and it is possible to use IgG1, IgG2, IgG3, IgG4, and such.
The origin of antigen-binding molecule of the present invention is not particularly limited, and may be of any origin. It is possible to use, for example, mouse antibodies, human antibodies, rat antibodies, rabbit antibodies, goat antibodies, camel antibodies, and others. Furthermore, the antibodies may be, for example, the above-described chimeric antibodies, and in particular, altered antibodies with amino acid sequence substitutions, such as humanized antibodies. The antibodies may also be the above-described bispecific antibodies, antibody modification products to which various molecules have been linked, polypeptides including antibody fragments, and antibodies with modified sugar chains.
Bispecific antibody refers to an antibody that has, in the same antibody molecule, variable regions that recognize different epitopes. A bispecific or multispecific antibody may be an antibody that recognizes two or more different antigens, or an antibody that recognizes two or more different epitopes on a same antigen.
Furthermore, polypeptides including antibody fragments include, for example, Fab fragments, F(ab')2 fragments, scFvs (Nat Biotechnol. 2005 Sep; 23(9): 1126-36), domain antibodies (dAbs) (WO 2004/058821, WO 2003/002609), scFv-Fc (WO 2005/037989), dAb-Fc, and Fc fusion proteins. Fc domain can be used as a human FcRn-binding domain when a molecule includes an Fc domain. Alternatively, an FcRn-binding domain may be fused to these molecules.
Further, the antigen-binding molecules that are applicable to the present invention may be antibody-like molecules. An antibody-like molecule (scaffold molecule, peptide molecule) is a molecule that can exhibit functions by binding to a target molecule (Current Opinion in Biotechnology (2006) 17: 653-658; Current Opinion in Biotechnology (2007) 18: 1-10; Current Opinion in Structural Biology (1997) 7: 463-469; Protein Science (2006) 15: 14-27), and includes, for example, DARPins (WO 2002/020565), Affibody (WO 1995/001937), Avimer (WO 2004/044011; WO 2005/040229), and Adnectin (WO 2002/032925). If these antibody-like molecules can bind to target molecules in a pH-dependent manner and/or have human FcRn-binding activity in the neutral pH range, it is possible to facilitate antigen uptake into cells by antigen-binding molecules, facilitate the reduction of plasma antigen concentration by administering antigen-binding molecules, improve pharmacokinetics of the antigen-binding molecules, and increase the number of antigens to which a single antigen-binding molecule can bind.
Furthermore, the antigen-binding molecule may be a protein resulting from fusion between a human FcRn-binding domain and a receptor protein that binds to a target including a ligand, and includes, for example, TNFR-Fc fusion proteins, IL1R-Fc fusion proteins, VEGFR-Fc fusion proteins, and CTLA4-Fc fusion proteins (Nat Med. 2003, Jan; 9(1): 47-52; BioDrugs. (2006) 20(3): 151-60). If these receptor-human FcRn-binding domain fusion proteins bind to a target molecule including a ligand in a pH-dependent manner and/or have human FcRn-binding activity in the neutral pH range, it is possible to facilitate antigen uptake into cells by antigen-binding molecules, facilitate the reduction of plasma antigen concentration by administering antigen-binding molecules, and improve pharmacokinetics of the antigen-binding molecules, and increase the number of antigens to which a single antigen-binding molecule can bind. A receptor protein is appropriately designed and modified so as to include a binding domain of the receptor protein to a target including a ligand.

**EP 4 501 956 A2**

As referred to the example hereinbefore including TNFR-Fc fusion proteins, IL1R-Fc fusion proteins, VEGFR-Fc fusion proteins and CTLA4-Fc fusion proteins, a soluble receptor molecule comprising an extracellular domain of those receptor proteins which is required for binding to those targets including ligands is a preferable used in the present invention. Those designed and modified receptor molecule is referred as an artificial receptor in this application. A method employed to design and modify a receptor molecule to construct an artificial receptor molecule is known in the art.

Moreover, the antigen-binding molecule may be a fusion protein in which artificial ligand protein that binds to a target and has the neutralizing effect is fused with a human FcRn-binding domain, and an artificial ligand protein includes, for example, mutant IL-6 (EMBO J. 1994 Dec 15; 13(24): 5863-70). If such artificial ligand fusion proteins can bind to target molecules in a pH-dependent manner and/or have human FcRn-binding activity in the neutral pH range, it is possible to facilitate antigen uptake into cells by antigen-binding molecules, facilitate the reduction of plasma antigen concentration by administering antigen-binding molecules, improve pharmacokinetics of the antigen-binding molecules, and increase the number of antigens to which a single antigen-binding molecule can bind.

Furthermore, the antibodies of the present invention may include modified sugar chains. Antibodies with modified sugar chains include, for example, antibodies with modified glycosylation (WO 99/54342), antibodies that are deficient in fucose that is added to the sugar chain (WO 00/61739; WO 02/31140; WO 2006/067847; WO2 006/067913), and antibodies having sugar chains with bisecting GlcNAc (WO 02/79255).

Conditions used in the assay for the antigen-binding or human FcRn-binding activity other than pH can be appropriately selected by those skilled in the art, and the conditions are not particularly limited. For example, the conditions of using MES buffer at 37 degrees C as described in WO 2009/125825 may be used to determine the activity. Meanwhile, the antigen-binding activity and human FcRn-binding activity of antigen-binding molecule can be determined by methods known to those skilled in the art, for example, using Biacore (GE Healthcare) or such. When the antigen is a soluble antigen, the activity of an antigen-binding molecule to bind to the soluble antigen can be determined by loading the antigen as an analyte onto a chip immobilized with the antigen-binding molecule. Alternatively, when the antigen is a membrane-type antigen, the activity of the antigen-binding molecule to bind to the membrane-type antigen can be determined by loading the antigen-binding molecule as an analyte onto an antigen-immobilized chip. The human FcRn-binding activity of an antigen-binding molecule can be determined by loading human FcRn or the antigen-binding molecule as an analyte onto a chip immobilized with the antigen-binding molecule or human FcRn, respectively.

Generation of chimeric antibodies is known. In the case of a human-mouse chimeric antibody, for example, a DNA encoding an antibody V region may be linked to a DNA encoding a human antibody C region; this can be inserted into an expression vector and introduced into a host to produce the chimeric antibody.

"Humanized antibodies" are also referred to as reshaped human antibodies, and are antibodies in which the complementarity determining region (CDR) of a nonhuman mammal, for example a mouse, is transplanted to the CDR of a human antibody. Methods for identifying CDRs are known (Kabat et al., Sequence of Proteins of Immunological Interest (1987), National Institute of Health, Bethesda, Md.; Chothia et al., Nature (1989) 342: 877). General genetic recombination technologies suitable for this purpose are also known (see European Patent Application EP 125023; and WO 96/02576). Humanized antibodies can be produced by known methods, for example, the CDR of a mouse antibody can be determined, and a DNA encoding an antibody in which the CDR is linked to the framework region (FR) of a human antibody is obtained. Humanized antibodies can then be produced using a system that uses conventional expression vectors. Such DNAs can be synthesized by PCR, using as primers several oligonucleotides prepared to have portions that overlap with the end regions of both the CDR and FR (see the method described in WO 98/13388). Human antibody FRs linked via CDRs are selected such that the CDRs form a suitable antigen binding site. If required, amino acids in the FRs of an antibody variable region may be altered so that the CDRs of the reshaped human antibody can form a suitable antigen binding site (Sato et al., Cancer Res. (1993) 53: 10.01-6). Amino acid residues in the FRs that can be altered include portions that directly bind to an antigen via non-covalent bonds (Amit et al., Science (1986) 233: 747-53), portions that influence or have an effect on the CDR structure (Chothia et al., J. Mol. Biol. (1987) 196: 901-17), and portions involved in VH-VL interactions (EP 239400).

When the antigen-binding molecules of the present invention are chimeric antibodies or humanized antibodies, the C regions of these antibodies are preferably derived from human antibodies. For example, C-gamma1, C-gamma2, C-gamma3, and C-gamma4 can be used for the H chain, while C-kappa and C-lambda can be used for the L chain. Moreover, if required, amino acid mutations may be introduced into the human antibody C region to enhance or lower the binding to Fc-gamma receptor or to improve antibody stability or productivity. A chimeric antibody of the present invention preferably includes a variable region of an antibody derived from a nonhuman mammal and a constant region derived from a human antibody. Meanwhile, a humanized antibody preferably includes CDRs of an antibody derived from a nonhuman mammal and FRs and C regions derived from a human antibody. The constant regions derived from human antibodies preferably include a human FcRn-binding region. Such antibodies include, for example, IgGs (IgG1, IgG2, IgG3, and IgG4). The constant regions used for the humanized antibodies of the present invention may be constant regions of antibodies of any isotype. A constant region derived from human IgG1 is preferably used, though it is not limited thereto. The FRs derived from a human antibody, which are used for the humanized antibodies, are not particularly limited either, and may be derived from an antibody of any isotype.

The variable and constant regions of chimeric and humanized antibodies of the present invention may be altered by deletion, substitution, insertion, and/or addition, and such, so long as the binding specificity of the original antibodies is exhibited.

Since the immunogenicity in the human body is lowered, chimeric and humanized antibodies using human-derived sequences are thought to be useful when administered to humans for therapeutic purposes or such.

Such antigen-binding molecules of the present invention may be obtained by any method. For example, an antigen-binding molecule that originally does not have human FcRn-binding activity in the acidic pH and neutral pH ranges, an antigen-binding molecule that has a stronger antigen-binding activity in the acidic pH range than in the neutral pH range, or an antigen-binding molecule that has a comparable antigen-binding activity in the acidic and neutral pH ranges may be artificially altered into an antigen-binding molecule having a desired activity through the above-described amino acid alterations or such. Alternatively, an antibody having a desired activity may be selected by screening from a number of antibodies obtained from the antibody libraries or hybridomas described below.

When altering amino acids in an antigen-binding molecule, it is possible to use a known sequence for the amino acid sequence of an antigen-binding molecule before alteration or the amino acid sequence of an antigen-binding molecule newly identified by methods known to those skilled in the art. For example, when the antigen-binding molecule is an antibody, it can be obtained from antibody libraries or by cloning an antibody-encoding gene from monoclonal antibody-producing hybridomas.

Regarding antibody libraries, many antibody libraries are already known, and methods for producing antibody libraries are also known; therefore, those skilled in the art can appropriately obtain antibody libraries. For example, regarding phage libraries, one can refer to the literature such as Clackson et al., Nature (1991) 352: 624-8; Marks et al., J. Mol. Biol. (1991) 222: 581-97; Waterhouses et al., Nucleic Acids Res. (1993) 21: 2265-6; Griffiths et al., EMBO J. (1994) 13: 324.0-60; Vaughan et al., Nature Biotechnology (1996) 14: 309-14; and Japanese Patent Kohyo Publication No. (JP-A) H20-504970 (unexamined Japanese national phase publication corresponding to a non-Japanese international publication). In addition, it is possible to use known methods, such as methods using eukaryotic cells as libraries (WO 95/15393) and ribosome display methods. Furthermore, technologies to obtain human antibodies by panning using human antibody libraries are also known. For example, variable regions of human antibodies can be expressed on the surface of phages as single chain antibodies (scFvs) using phage display methods, and phages that bind to antigens can be selected. Genetic analysis of the selected phages can determine the DNA sequences encoding the variable regions of human antibodies that bind to the antigens. Once the DNA sequences of scFvs that bind to the antigens is revealed, suitable expression vectors can be produced based on these sequences to obtain human antibodies. These methods are already well known, and one can refer to WO 92/01047, WO 92/20791, WO 93/06213, WO 93/11236, WO 93/19172, WO 95/01438, and WO 95/15388.

As for methods for obtaining genes encoding antibodies from hybridomas, known technologies may be basically used, which involve the use of desired antigens or cells expressing the desired antigens as sensitizing antigens, using these to perform immunizations according to conventional immunization methods, fusing the resulting immune cells with known parent cells by conventional cell fusion methods, screening monoclonal antibody producing cells (hybridomas) by conventional screening methods, synthesizing cDNAs of antibody variable regions (V regions) from mRNAs of the obtained hybridomas using reverse transcriptase, and linking them with DNAs encoding the desired antibody constant regions (C regions).

More specifically, sensitizing antigens to obtain the above-described antigen-binding molecule genes encoding the H chains and L chains may include, for example, both complete antigens with immunogenicity and incomplete antigens including haptens and the like with no immunogenicity; however they are not limited to these examples. For example, it is possible to use whole proteins and partial peptides of proteins of interest. In addition, it is known that substances comprising polysaccharides, nucleic acids, lipids, and such can be antigens. Thus, the antigens of the antigen-binding molecules of the present invention are not particularly limited. The antigens can be prepared by methods known to those skilled in the art, for example, by baculovirus-based methods (for example, WO 98/46777) and such. Hybridomas can be produced, for example, by the method of Milstein et al. (G. Kohler and C. Milstein, Methods Enzymol. (1981) 73: 3-46) and such. When the immunogenicity of an antigen is low, immunization may be performed after linking the antigen with a macromolecule having immunogenicity, such as albumin. Alternatively, if necessary, antigens may be converted into soluble antigens by linking them with other molecules. When transmembrane molecules such as membrane antigens (for example, receptors) are used as antigens, portions of the extracellular regions of the membrane antigens can be used as a fragment, or cells expressing transmembrane molecules on their cell surface may be used as immunogens.

Antigen-binding molecule-producing cells can be obtained by immunizing animals using appropriate sensitizing antigens described above. Alternatively, antigen-binding molecule-producing cells can be prepared by in vitro immunization of lymphocytes that can produce antigen-binding molecules. Various mammals can be used for immunization; such commonly used animals include rodents, lagomorphas, and primates. Such animals include, for example, rodents such as mice, rats, and hamsters; lagomorphas such as rabbits; and primates including monkeys such as cynomolgus monkeys, rhesus monkeys, baboons, and chimpanzees. In addition, transgenic animals carrying human antibody gene repertoires are also known, and human antibodies can be obtained by using these animals (see WO 96/34096; Mendez et al., Nat. Genet. (1997) 15: 146-56). Instead of using such transgenic animals, for example, desired human antibodies having binding activity against antigens can be obtained by in vitro sensitization of human lymphocytes with desired antigens or cells expressing the desired antigens, and then fusing the sensitized lymphocytes with human myeloma cells such as U266 (see Japanese Patent Application Kokoku Publication No. (JP-B) H01-59878 (examined, approved Japanese patent application published for opposition)). Furthermore, desired human antibodies can be obtained by immunizing transgenic animals carrying a complete repertoire of human antibody genes, with desired antigens (see WO 93/12227, WO 92/03918, WO 94/02602, WO 96/34096, and WO 96/33735).

Animal immunization can be carried out by appropriately diluting and suspending a sensitizing antigen in phosphate buffered saline (PBS), physiological saline, or such, and mixing it with an adjuvant to emulsify, if necessary. This is then intraperitoneally or subcutaneously injected into animals. Then, the sensitizing antigen mixed with Freund's incomplete adjuvant is preferably administered several times every four to 21 days. Antibody production can be confirmed by measuring the titer of the antibody of interest in animal sera using conventional methods.

Antigen-binding molecule-producing cells obtained from lymphocytes or animals immunized with a desired antigen can be fused with myeloma cells to generate hybridomas using conventional fusing agents (for example, polyethylene glycol) (Goding, Monoclonal Antibodies: Principles and Practice, Academic Press, (1986) 59-103). When required, hybridoma cells can be cultured and grown, and the binding specificity of the antigen-binding molecule produced from these hybridomas can be measured using known analysis methods, such as immunoprecipation, radioimmunoassay (RIA), and enzyme-linked immunosorbent assay (ELISA). Thereafter, if necessary, hybridomas producing antigen-binding molecules of interest whose specificity, affinity, or activity has been determined can be subcloned by methods such as limiting dilution.

Next, genes encoding the selected antigen-binding molecules can be cloned from hybridomas or antigen-binding molecule-producing cells (sensitized lymphocytes, and such) using probes that can specifically bind to the antigen-binding molecules (for example, oligonucleotides complementary to sequences encoding the antibody constant regions). It is also possible to clone the genes from mRNA using RT-PCR. Immunoglobulins are classified into five different classes, IgA, IgD, IgE, IgG, and IgM. These classes are further divided into several subclasses (isotypes) (for example, IgG-1, IgG-2, IgG-3, and IgG-4; IgA-1 and IgA-2; and such). H chains and L chains used in the present invention to produce antigen-binding molecules are not particularly limited and may originate from antibodies belonging to any of these classes or subclasses; however, IgG is particularly preferred.

Herein, it is possible to alter H-chain-encoding genes and L-chain-encoding genes using genetic engineering technologies. Genetically altered antibodies, such as chimeric antibodies and humanized antibodies, which have been artificially altered for the purpose of decreasing heterologous immunogenicity and such against humans, can be appropriately produced for antibodies such as mouse antibodies, rat antibodies, rabbit antibodies, hamster antibodies, sheep antibodies, and camel antibodies. Chimeric antibodies are antibodies including H-chain and L-chain variable regions of nonhuman mammal antibody, such as mouse antibody, and the H-chain and L-chain constant regions of human antibody. Chimeric antibodies can be obtained by ligating a DNA encoding a variable region of a mouse antibody to a DNA encoding a constant region of a human antibody, inserting this into an expression vector, and introducing the vector into a host to produce the antibody. A humanized antibody, which is also called a reshaped human antibody, can be synthesized by PCR using several oligonucleotides produced so that they have overlapping portions at the ends of DNA sequences designed to link the complementarity determining regions (CDRs) of an antibody of a nonhuman mammal such as a mouse. The resulting DNA can be ligated to a DNA encoding a human antibody constant region. The ligated DNA can be inserted into an expression vector, and the vector can be introduced into a host to produce the antibody (see EP 239400 and WO 96/02576). Human antibody FRs that are ligated via the CDR are selected when the CDR forms a favorable antigen-binding site. If necessary, amino acids in the framework region of an antibody variable region may be replaced such that the CDR of the reshaped human antibody forms an appropriate antigen-binding site (K. Sato et al., Cancer Res. (1993) 53: 10.01-10.06).

In addition to the humanization described above, antibodies may be altered to improve their biological properties, for example, the binding to the antigen. In the present invention, such alterations can be achieved by methods such as site-directed mutagenesis (see for example, Kunkel (1910.0) Proc. Natl. Acad. Sci. USA 82: 488), PCR mutagenesis, and cassette mutagenesis. In general, mutant antibodies whose biological properties have been improved show amino acid sequence homology and/or similarity of 70% or higher, more preferably 80% or higher, and even more preferably 90% or higher (for example, 95% or higher, 97%, 98%, or 99%), when compared to the amino acid sequence of the original antibody variable region. Herein, sequence homology and/or similarity is defined as the ratio of amino acid residues that are homologous (same residue) or similar (amino acid residues classified into the same group based on the general properties of amino acid side chains) to the original antibody residues, after the sequence homology value has been maximized by sequence alignment and gap introduction, if necessary. In general, natural amino acid residues are classified into groups based on the characteristics of their side chains as follows:

(1) hydrophobic: alanine, isoleucine, valine, methionine, and leucine;
(2) neutral hydrophilic: asparagine, glutamine, cysteine, threonine, and serine;
(3) acidic: aspartic acid and glutamic acid;
(4) basic: arginine, histidine, and lysine;
(5) residues that affect the orientation of the chain: glycine, and proline; and
(6) aromatic: tyrosine, tryptophan, and phenylalanine.

In general, a total of six complementarity determining regions (CDRs; hypervariable regions) present on the H chain and L chain variable regions interact with each other to form an antigen-binding site of an antibody. A variable region alone is also known to be capable of recognizing and binding to an antigen, although its affinity is lower than the affinity of the whole binding site. Thus, antibody genes encoding the H chain and L chain of the present invention may encode fragments each including the H chain or L chain antigen-binding site, as long as the polypeptide encoded by the gene retains the activity of binding to the desired antigen.

As described above, the heavy chain variable region is in general constituted by three CDRs and four FRs. In a preferred embodiment of the present invention, amino acid residues to be "altered" can be appropriately selected from amino acid residues, for example, in a CDR or FR. In general, alterations of amino acid residues in the CDRs may reduce the antigen-binding ability. Thus, appropriate amino acid residues to be "altered" in the present invention are preferably selected from amino acid residues in the FRs, but are not limited thereto. It is possible to select amino acids in a CDR as long as the alteration has been confirmed not to reduce the binding ability. Alternatively, by using public databases or such, those skilled in the art can obtain appropriate sequences that can be used as an FR of antibody variable region of an organism such as human or mouse.

Furthermore, the present invention provides genes encoding the antigen-binding molecules of the present invention. The genes encoding the antigen-binding molecules of the present invention may be any genes, and may be DNAs, RNAs, nucleic acid analogs, or the like.

Furthermore, the present invention also provides host cells carrying the genes described above. The host cells are not particularly limited and include, for example, E. coli and various animal cells. The host cells may be used, for example, as a production system to produce and express the antibodies of the present invention. In vitro and in vivo production systems are available for polypeptide production systems. Such in vitro production systems include, for example, production systems using eukaryotic cells or prokaryotic cells.

Eukaryotic cells that can be used as host cells include, for example, animal cells, plant cells, and fungal cells. Animal cells include: mammalian cells, for example, CHO (J. Exp. Med. (1995) 108: 94.0), COS, HEK293, 3T3, myeloma, BHK (baby hamster kidney), HeLa, and Vero; amphibian cells such as Xenopus laevis oocytes (Valle et al., Nature (1981) 291: 338-340); and insect cells such as Sf9, Sf21, and Tn5. CHO-DG44, CHO-DX11B, COS7 cells, HEK293 cells, and BHK cells are preferably used to express the antibodies of the present invention. Among animal cells, CHO cells are particularly preferable for large-scale expression. Vectors can be introduced into host cells, for example, by calcium phosphate methods, DEAE-dextran methods, methods using cationic liposome DOTAP (Boehringer-Mannheim), electroporation methods, and lipofection methods.

Regarding plant cells, for example, Nicotiana tabacum-derived cells and duckweed (Lemna minor) are known as a protein production system. Calluses can be cultured from these cells to produce the antigen-binding molecules of the present invention. Regarding fungal cells, known protein expression systems are those using yeast cells, for example, cells of genus Saccharomyces (such as Saccharomyces cerevisiae and Saccharomyces pombe); and cells of filamentous fungi, for example, genus Aspergillus (such as Aspergillus niger). These cells can be used as a host to produce the antigen-binding molecules of the present invention.

Bacterial cells can be used in the prokaryotic production systems. Regarding bacterial cells, production systems using Bacillus subtilis are known in addition to the production systems using E. coli described above. Such systems can be used in producing the antigen-binding molecules of the present invention.

<Screening method>

The present invention provides methods of screening for antigen-binding molecules having human FcRn-binding activity in the acidic and neutral pH ranges. The present invention also provides methods of screening for antigen-binding molecules that have human FcRn-binding activity in the acidic and neutral pH ranges and a lower antigen-binding activity in the acidic pH range than in the neutral pH range. The present invention also provides methods of screening for antigen-binding molecules capable of facilitating antigen uptake into cells. The present invention also provides methods of screening for antigen-binding molecules modified to be capable of binding to more antigens per molecule. The present invention also provides methods of screening for antigen-binding molecules capable of facilitating antigen elimination. The present invention further provides methods of screening for antigen-binding molecules with improved pharmacokinetics. The present invention also provides methods of screening for antigen-binding molecules with facilitated intracellular dissociation from their bound antigen outside the cells. The present invention also provides methods of screening for antigen-binding molecules with facilitated extracellular release in an antigen-free form after uptake into cells in an antigen-bound form. The present invention further provides methods of screening for antigen-binding molecules that are particularly useful as pharmaceutical compositions. The above-described methods are useful in screening for antigen-binding molecules that are particularly superior in plasma retention and have superior ability to eliminate antigens from the plasma.

Specifically, the present invention provides methods of screening for antigen-binding molecules, which comprise the steps of:

(a) selecting an antigen-binding molecule that has a stronger human FcRn-binding activity in the neutral pH range than before alteration of at least one amino acid in the human FcRn-binding domain of an antigen-binding molecule having human FcRn-binding activity in the acidic pH range; and
(b) altering at least one amino acid in the antigen-binding domain of an antigen-binding molecule and selecting an antigen-binding molecule that has stronger antigen-binding activity in the neutral pH range than in the acidic pH range.

Steps (a) and (b) may be carried out in either order. Furthermore, each step may be repeated twice or more times. The number of times of repeating steps (a) and (b) is not particularly limited; however, the number is typically ten times or less.

In the screening methods of the present invention, the antigen-binding activity of an antigen-binding molecule in the neutral pH range is not particularly limited, as long as it is an antigen-binding activity in the range of pH 6.7 to 10.0. For example, the embodiments described in WO 2009/125825 are included. The preferred antigen-binding activities include antigen-binding activity in the range of pH 7.0 to 8.0. The more preferred antigen-binding activities include antigen-binding activity at pH 7.4. Meanwhile, the antigen-binding activity of antigen-binding molecule in the acidic pH range is not particularly limited, as long as it is an antigen-binding activity in the range of pH 4.0 to 6.5. The preferred antigen-binding activities include antigen-binding activity in the range of pH 5.5 and 6.5. The more preferred antigen-binding activities include antigen-binding activity at pH 5.8 or pH 5.5.

The human FcRn-binding activity of an antigen-binding molecule in the neutral pH range is not particularly limited, as long as it is a human FcRn-binding activity in the range of pH 6.7 to 10.0. The preferred human FcRn-binding activities include human FcRn-binding activity in the range of pH 7.0 to 8.0. The more preferred human FcRn-binding activities include human FcRn-binding activity at pH 7.4.

The human FcRn-binding activity of an antigen-binding molecule in the acidic pH range is not particularly limited, as long as it is a human FcRn-binding activity in the range of pH 4.0 to 6.5. The preferred human FcRn-binding activities include human FcRn-binding activity in the range of pH 5.5 to 6.5. The more preferred human FcRn-binding activities include human FcRn-binding activity in the range of pH 5.8 to 6.0.

Herein, the acidic pH range typically refers to pH 4.0 to pH 6.5. The acidic pH range is preferably a range indicated by any pH value within pH 5.5 to pH 6.5, preferably selected from 5.5, 5.6, 5.7, 5.8, 5.9, 6.0, 6.1, 6.2, 6.3, 6.4, or 6.5, particularly preferably pH 5.8 to pH 6.0, which is close to the pH in early endosome in vivo. Meanwhile, herein the neutral pH range typically refers to pH 6.7 to pH 10.0. The neutral pH range is preferably a range indicated by any pH value within pH 7.0 to pH 8.0, preferably selected from pH 7.0, 7.1, 7.2, 7.3, 7.4, 7.5, 7.6, 7.7, 7.8, 7.9, and 8.0, particularly preferably pH 7.4, which is close to in vivo plasma (blood) pH. pH 7.0 can be used as an alternative to pH 7.4 when it is difficult to assess the binding affinity between human FcRn-binding domain and human FcRn due its low affinity at pH 7.4. As a temperature employed in the assay condition, a binding affinity between human FcRn-binding domain and human FcRn may be assessed at any temperature from 10 degrees C to 50 degrees C. Preferably, a temperature at from 15 degrees C to 40 degrees C is employed in order to determine the binding affinity between human FcRn-binding domain and human FcRn. More preferably, any temperature at from 20 degrees C to 35 degrees C, like any one of 20, 21, 22, 23, 24, 25, 26, 27, 28, 29, 30, 31, 32, 33, 34, and 35 degrees C is also employed in order to determine the binding affinity between human FcRn-binding domain and human FcRn. A temperature at 25 degrees C described in Example 5 is one of example for the embodiment of this invention.

The antigen-binding activity and human FcRn-binding activity of an antigen-binding molecule can be determined by methods known to those skilled in the art. Appropriate conditions besides pH can be selected by those skilled in the art. The antigen-binding activity and human FcRn-binding activity of an antigen-binding molecule can be assessed by using KD (dissociation constant), apparent KD (apparent dissociation constant), dissociation rate $k_d$ (dissociation rate), apparent $k_d$ (apparent dissociation: apparent dissociation rate), or the like. They can be determined by methods known to those skilled in the art, for example, using Biacore (GE Healthcare), Scatchard plot, flow cytometer, or such.

According to the Journal of Immunology (2009) 182: 7663-7671, the human FcRn-binding activity of intact human IgG1 is KD 1.7 micromolar in the acidic pH range (pH 6.0), while the activity is almost undetectable in the neutral pH range. Thus, in a preferred embodiment, the antigen-binding molecules of the present invention having human FcRn-binding activity in the acidic and neutral pH ranges including antigen-binding molecules that have a human FcRn-binding activity of KD 20 micromolar or stronger in the acidic pH range, which is equal to or stronger than that of intact human IgG in the neutral pH range can be screened. In a more preferred embodiment, the antigen-binding molecules of the present invention including antigen-binding molecules whose human FcRn-binding activity is KD 2.0 micromolar or stronger in the acidic pH range and KD 40 micromolar or stronger in the neutral pH range can be screened. In a still more preferred embodiment, the antigen-binding molecules of the present invention including antigen-binding molecules whose human FcRn-binding activity is KD 0.5 micromolar or stronger in the acidic pH range and KD 15 micromolar or stronger in the neutral pH range can be screened. The above KD values are determined by the method described in the Journal of Immunology (2009) 182: 7663-7671 (by immobilizing the antigen-binding molecule onto a chip and loading human FcRn as an analyte).

The present invention provides a method of screening for an antigen-binding molecule, which comprises the steps of:

(a) selecting an antigen-binding molecule that has stronger human FcRn-binding activity in the neutral pH range than KD 3.2 micromolar obtained by altering at least one amino acid in the human FcRn-binding domain of an antigen-binding molecule,
(b) obtaining a gene encoding an antigen-binding molecule in which a human FcRn-binding domain and an antigen-binding domain prepared in (a) are linked; and
(c) producing an antigen-binding molecule using the gene prepared in (b).

In one embodiment, an antigen-binding molecule comprising an antigen-binding domain and a human FcRn-binding domain, which has a human FcRn-binding activity in the acidic and neutral pH ranges, wherein a human FcRn and a lower antigen-binding activity in the acidic pH range than in the neutral pH range is stronger than KD 3.2 micromolar can be screened according to methods employed by a person skilled in the art as described hereinbefore. In a more preferred embodiment, human FcRn-binding activity at pH 7.0 and at 25 degrees C is stronger than KD 3.2 micromolar.

The present invention provides a method of screening for an antigen-binding molecule comprising an antigen-binding domain and a human FcRn-binding domain, which has a human FcRn-binding activity in the neutral pH ranges, wherein a human FcRn-binding activity in the neutral pH ranges is stronger than KD 2.3 micromolar. The present invention also provides a method for screening an antigen-binding molecule comprising an antigen-binding domain and a human FcRn-binding domain, which has a human FcRn-binding activity in the neutral pH ranges, wherein a human FcRn-binding activity in the neutral pH ranges is 38-fold stronger than an intact human IgG.

Antigen-binding molecules of the present invention having human FcRn-binding activity in the neutral pH range are not particularly limited, as long as they have a human FcRn-binding activity at pH 6.7 to 10.0. However, the human FcRn-binding activity at pH 6.7 to 10.0 of preferred antigen-binding molecules is stronger than that of intact human IgG.

Antigen-binding molecules of the present invention having a human FcRn-binding activity in the acidic pH range are not particularly limited, as long as they have human FcRn-binding activity at pH 4.0 to 6.5. However, the human FcRn-binding activity at pH 5.5 to 6.5 of preferred antigen-binding molecules is comparable to or stronger than that of intact human IgG.

Herein, the step of selecting an antigen-binding molecule that has stronger antigen-binding activity in the neutral pH range than in the acidic pH range is synonymous to the step of selecting an antigen-binding molecule that has lower antigen-binding activity in the acidic pH range than in the neutral pH range.

The ratio of antigen-binding activity between the neutral and acidic pH ranges is not particularly limited as long as the antigen-binding activity in the neutral pH range is stronger than that in the acidic pH range. However, the antigen-binding activity at pH 6.7 to 10.0 is preferably twice or more times, more preferably ten or more times, and still more preferably 40 or more times the antigen-binding activity at pH 4.0 to 6.5.

In the screening methods of the present invention, it is possible to use libraries such as phage libraries.

In the methods of the present invention, antigen and antigen-binding molecule may bind together in any state, and thus the state is not particularly limited. For example, the antigen may be contacted with an immobilized antigen-binding molecule to achieve their binding. Alternatively, the antigen-binding molecule may be contacted with an immobilized antigen to achieve their binding. Alternatively, the antigen-binding molecule and antigen may be contacted with each other in a solution to achieve their binding.

Antigen-binding molecules to be screened by the screening methods of the present invention may be prepared by any method. For example, it is possible to use preexisting antibodies, preexisting antigen-binding domain libraries (phage libraries, etc.), antibodies or antigen-binding domain libraries prepared from B cells of immunized animals or hybridomas prepared by immunizing animals, antibodies or antigen-binding domain libraries obtained by introducing random amino acid alterations into the above-described antibodies or antigen-binding domain libraries, antibodies or antigen-binding domain libraries introduced with histidine mutation or non-natural amino acid mutations (libraries with high content of histidine or non-natural amino acid, antigen-binding domain libraries introduced with histidine or non-natural amino acid mutations at specific sites, etc.), or the like.

Antigen-binding molecules that bind to the antigen multiple times, which are thus superior in the plasma retention, can be obtained by the screening methods of the present invention. Thus, the screening methods of the present invention can be used as screening methods for obtaining antigen-binding molecules that are superior in the plasma retention.

Furthermore, antigen-binding molecules that can bind to the antigen two or more times when administered to animals such as humans, mice, or monkeys can be obtained by the screening methods of the present invention. Thus, the screening methods of the present invention can be used as screening methods for obtaining antigen-binding molecules that can bind to the antigen two or more times.

Furthermore, antigen-binding molecules that are capable of binding to more antigens as compared to the number of their antigen-binding sites when administered to animals such as humans, mice, or monkeys can be obtained by the screening methods of the present invention. Thus, the screening methods of the present invention can be used as screening methods for obtaining antigen-binding molecules that are capable of binding to more antigens as compared to the number of their antigen-binding sites. For example, when the antibody is a neutralizing antibody, the screening methods of the present invention can be used as screening methods for obtaining antigen-binding molecules that can neutralize more antigens as compared to the number of the antigen-binding sites of the antigen-binding molecules.

Furthermore, antigen-binding molecules that are capable of dissociating within a cell from an extracellularly-bound antigen when administered to animals such as humans, mice, or monkeys can be obtained by the screening methods of the present invention. Thus, the screening methods of the present invention can be used as screening methods for obtaining antigen-binding molecules that are capable of dissociating within a cell from an extracellularly-bound antigen.

Furthermore, antigen-binding molecules that are bound to an antigen and taken up into a cell, and released to the outside of the cell in an antigen-free form when administered to animals such as humans, mice, or monkeys can be obtained by the screening methods of the present invention. Thus, the screening methods of the present invention can be used as screening methods for obtaining antigen-binding molecules that are bound to an antigen and taken up into a cell, and released to the outside of the cell in an antigen-free form.

Furthermore, antigen-binding molecules that can rapidly eliminate antigens in plasma when administered to animals such as humans, mice, or monkeys can be obtained by the screening methods of the present invention. Thus, the screening methods of the present invention can be used as screening methods for obtaining antigen-binding molecules with increased (high) ability to eliminate antigens in plasma.

Furthermore, such antigen-binding molecules are expected to be especially superior as pharmaceuticals, because the dose and frequency of administration in patients can be reduced and as a result the total dosage can be reduced. Thus, the screening methods of the present invention can be used as methods of screening for antigen-binding molecules for use as pharmaceutical compositions.

<Methods for producing antigen-binding molecules>

The present invention provides methods for producing antigen-binding molecules that have human FcRn-binding activity at the endosomal pH and plasma pH, and lower antigen-binding activity at the endosomal pH than at the plasma pH. The present invention also provides methods for producing antigen-binding molecules that are superior in pharmacokinetics and in facilitating the reduction of the plasma antigen concentration when administered. The present invention also provides methods for producing antigen-binding molecules that are particularly useful when used as pharmaceutical compositions.

Specifically, the present invention provides methods for producing antigen-binding molecules, which comprise the steps of:

(a) selecting an antigen-binding molecule that has stronger human FcRn-binding activity in the neutral pH range than before alteration of at least one amino acid in the human FcRn-binding domain of an antigen-binding molecule having human FcRn-binding activity in the acidic pH range;
(b) altering at least one amino acid in the antigen-binding domain of an antigen-binding molecule and selecting an antigen-binding molecule that has stronger antigen-binding activity in the neutral pH range than in the acidic pH range;
(c) obtaining a gene encoding an antigen-binding molecule in which the human FcRn-binding domain and the antigen-binding domain prepared in (a) and (b) are linked; and
(d) producing an antigen-binding molecule using the gene prepared in (c).

Steps (a) and (b) may be carried out in either order. Furthermore, each step may be repeated twice or more times. The number of times of repeating steps (a) and (b) is not particularly limited; however, the number is typically ten times or less.

A linker operably links the human FcRn-binding domain and the antigen-binding domain prepared in (a) and (b) is not limited to any form. The human FcRn-binding domain and the antigen-binding domain can be linked by either covalent or non-covalent forces. In particular, the linker can be a peptide linker or a chemical linker or a binding pair like a combination of biotin and streptavidin. Modification of a polypeptide including the human FcRn-binding domain and the antigen-binding domain is known in the art. In another embodiment, the human FcRn-binding domain and the antigen-binding domain of the present invention can be linked by forming a fusion protein between the human FcRn-binding domain and the antigen-binding domain. In order to construct fusion protein between the human FcRn-binding domain and the antigen-binding domain, genes encoding the human FcRn-binding domain and the antigen-binding domain can be operationally linked so as to form in frame fusion polypeptide. Appropriately, a linker comprising peptide consisting of several amino acids can be inserted between the human FcRn-binding domain and the antigen-binding domain. Various flexible linkers like the linker whose sequence consists of $(GGGGS)_n$ is known in the art.

Antigen-binding molecules that are used in the production methods of the present invention may be prepared by any method. For example, it is possible to use preexisting antibodies, preexisting libraries (phage libraries and the like), antibodies and libraries that are prepared from hybridomas obtained by immunizing animals or from B cells of immunized animals, antibodies and libraries prepared by introducing random amino acid alterations into the above-described antibodies and libraries, antibodies and libraries prepared by introducing histidine or non-natural amino acid mutations into the above-described antibodies and libraries (libraries with high content of histidine or non-natural amino acid, libraries introduced with histidine or non-natural amino acid at specific sites, and the like), and such.

In the above-described production methods, the human FcRn-binding activity of an antigen-binding molecule in the neutral pH range is not particularly limited as long as it is a human FcRn-binding activity in the range of pH 6.7 to 10.0. The preferred human FcRn-binding activities include human FcRn-binding activity in the range of pH 7.0 to 8.0. The more preferred human FcRn-binding activities include human FcRn-binding activity at pH 7.4.

The human FcRn-binding activity of an antigen-binding molecule in the acidic pH range is not particularly limited, as long as it is a human FcRn-binding activity in the range of pH 4.0 to 6.5. The preferred human FcRn-binding activities include human FcRn-binding activity in the range of pH 5.5 to 6.5. The more preferred human FcRn-binding activities include human FcRn-binding activity at pH 6.0.

Herein, the acidic pH range typically refers to pH 4.0 to pH 6.5. The acidic pH range is preferably a range indicated by any pH value within pH 5.5 to pH 6.5, preferably selected from 5.5, 5.6, 5.7, 5.8, 5.9, 6.0, 6.1, 6.2, 6.3, 6.4, and 6.5, particularly preferably pH 5.8 to pH 6.0, which is close to the pH in early endosome in vivo. Meanwhile, herein the neutral pH range typically refers to pH 6.7 to pH 10.0. The neutral pH range is preferably a range indicated by any pH value within pH 7.0 to pH 8.0, preferably selected from pH 7.0, 7.1, 7.2, 7.3, 7.4, 7.5, 7.6, 7.7, 7.8, 7.9, and 8.0, particularly preferably pH 7.4, which is close to in vivo plasma (blood) pH. pH 7.0 can be used as an alternative to pH 7.4 when it is difficult to assess the binding affinity between human FcRn-binding domain and human FcRn due its low affinity at pH 7.4. As a temperature employed in the assay condition, a binding affinity between human FcRn-binding domain and human FcRn may be assessed at any temperature from 10 degrees C to 50 degrees C. Preferably, a temperature at from 15 degrees C to 40 degrees C is employed in order to determine the binding affinity between human FcRn-binding domain and human FcRn. More preferably, any temperature at from 20 degrees C to 35 degrees C, like any one of 20, 21, 22, 23, 24, 25, 26, 27, 28, 29, 30, 31, 32, 33, 34, and 35 degrees C is also employed in order to determine the binding affinity between human FcRn-binding domain and human FcRn. A temperature at 25 degrees C described in Example 5 is one of example for the embodiment of this invention.

The present invention provides a method for producing an antigen-binding molecule, which comprises the steps of:

(a) selecting an antigen-binding molecule that has stronger human FcRn-binding activity in the neutral pH range than KD 3.2 micromolar obtained by altering at least one amino acid in the human FcRn-binding domain of an antigen-binding molecule;
(b) obtaining a gene encoding an antigen-binding molecule in which a human FcRn-binding domain and an antigen-binding domain prepared in (a) are linked; and
(c) producing an antigen-binding molecule using the gene prepared in (b).

In a preferred embodiment, the antigen-binding molecules of the present invention having human FcRn-binding activity in the acidic and neutral pH ranges including antigen-binding molecules that have a human FcRn-binding activity of KD 20 micromolar or stronger in the acidic pH range, which is equal to or stronger than that of intact human IgG in the neutral pH range can be produced. In a more preferred embodiment, the antigen-binding molecules of the present invention including antigen-binding molecules whose human FcRn-binding activity is KD 2.0 micromolar or stronger in the acidic pH range and KD 40 micromolar or stronger in the neutral pH range can also been produced. In a still more preferred embodiment, the antigen-binding molecules of the present invention including antigen-binding molecules whose human FcRn-binding activity is KD 0.5 micromolar or stronger in the acidic pH range and KD 15 micromolar or stronger in the neutral pH range can preferably be produced. The above KD values are determined by the method described in the Journal of Immunology (2009) 182: 7663-7671 (by immobilizing the antigen-binding molecule onto a chip and loading human FcRn as an analyte). In one embodiment, an antigen-binding molecule comprising an antigen-binding domain and a human FcRn-binding domain, which has a human FcRn-binding activity in the acidic and neutral pH ranges, wherein a human FcRn and a lower antigen-binding activity in the acidic pH range than in the neutral pH range is stronger than KD 3.2 micromolar can be produced according to methods employed by a person skilled in the art as described hereinbefore. In a more preferred embodiment, human FcRn-binding activity of thus produced antigen-binding molecule at pH 7.0 and 25 degrees C is stronger than KD 3.2 micromolar.

The present invention provides a method for producing an antigen-binding molecule comprising an antigen-binding domain and a human FcRn-binding domain, which has a human FcRn-binding activity in the neutral pH ranges, wherein a human FcRn-binding activity in the neutral pH ranges is stronger than KD 2.3 micromolar. The present invention also provides a method for producing an antigen-binding molecule comprising an antigen-binding domain and a human FcRn-binding domain, which has a human FcRn-binding activity in the neutral pH ranges, wherein a human FcRn-binding activity in the neutral pH ranges is 38-fold stronger than an intact human IgG.

In the above-described production methods, the antigen-binding activity of the antigen-binding molecule in the neutral pH range is not particularly limited, as long as the antigen-binding activity is that at a pH between pH 6.7 and pH 10.0, and includes, for example, an embodiment described in WO 2009/125825. A preferred antigen-binding activity is that at a pH between pH 7.0 and pH 8.0, and a more preferred antigen-binding activity is that at pH 7.4. Alternatively, the antigen-binding activity of the antigen-binding molecule in the acidic pH range is not particularly limited, as long as the antigen-binding activity is that at a pH between pH 4.0 and pH 6.5. A preferred antigen-binding activity is that at a pH between pH 5.5 to pH 6.5, and a more preferred antigen-binding activity is that at pH 5.8 or pH 5.5.

The antigen-binding activity and human FcRn binding activity of an antigen-binding molecule can be determined by methods known to those skilled in the art. Conditions except for pH can be appropriately determined by those skilled in the art.

In the production methods of the present invention, antigen-binding molecules having human FcRn-binding activity in the neutral pH range are not particularly limited as long as they have human FcRn-binding activity at pH 6.7 to 10.0. However, the human FcRn-binding activity of the antigen-binding molecules at pH 6.7 to 10.0 is preferably stronger than that of intact human IgG. More preferably, the antigen-binding molecules have a human FcRn-binding activity stronger than KD 40 micromolar, still more preferably stronger than KD 15 micromolar.

In the production methods of the present invention, antigen-binding molecules having human FcRn-binding activity in the acidic pH range are not particularly limited as long as they have human FcRn-binding activity at pH 4.0 to 6.5. However, at pH 5.5 to 6.5, the antigen-binding molecules preferably have a human FcRn-binding activity stronger than KD 20 micromolar. The human FcRn-binding activity is more preferably comparable to or stronger than that of intact human IgG1 (stronger than KD 1.7 micromolar), more preferably stronger than KD 0.5 micromolar.

The KD values described above are determined by the method described in "The Journal of Immunology, (2009) 182: 7663-7671" (by immobilizing the antigen-binding molecule onto a chip and loading human FcRn as an analyte).

In the production methods of the present invention, the step of selecting antigen-binding molecules whose antigen-binding activity at pH 6.7 to pH 10.0 is stronger than that at pH 4.0 to pH 6.5 is synonymous with the step of selecting antigen-binding molecules whose antigen-binding activity at pH 4.0 to pH 6.5 is lower than that at pH 6.7 to pH 10.0.

The ratio between the antigen-binding activity in the neutral pH range and in the acidic pH range is not particularly limited as long as the antigen-binding activity in the neutral pH range is stronger than that in the acidic pH range. The antigen-binding activity at pH 6.7 to pH 10.0 is preferably twice or stronger, more preferably ten times or stronger, and still more preferably 40 times or stronger than that at pH 4.0 to pH 6.5.

In the above-described production methods, the antigen and antigen-binding molecule may bind to each other in any state, and the human FcRn and antigen-binding molecule may bind to each other in any state. The state is not particularly limited; for example, the antigen or human FcRn may be contacted with an immobilized antigen-binding molecule to bind the antigen-binding molecule. Alternatively, the antigen-binding molecule may be contacted with an immobilized antigen or human FcRn to bind the antigen-binding molecule. Alternatively, the antigen-binding molecule may be contacted with the antigen or human FcRn in a solution to bind the antigen-binding molecule.

The antigen-binding molecules produced by the above-described methods may be any antigen-binding molecule; and preferred antigen-binding molecules include, for example, those having an antigen-binding domain and a human FcRn-binding domain, which contains alteration of at least one amino acid in the human FcRn-binding domain, and histidine substitution for amino acid(s) or insertion of at least one histidine.

Amino acid alterations in the human FcRn-binding domain are not particularly limited, as long as they increase the human FcRn-binding activity in the neutral pH range. The alterations include, for example, those of amino acids of positions 221 to 225, 227, 228, 230, 232, 233 to 241, 243 to 252, 254 to 260, 262 to 272, 274, 276, 278 to 289, 291 to 312, 315 to 320, 324, 325, 327 to 339, 341, 343, 345, 360, 362, 370, 375 to 378, 380, 382, 385 to 387, 389, 396, 414, 416, 423, 424, 426 to 438, 440, and 442 (EU numbering) in the above-described IgG Fc domain. More specifically, the amino acid alterations include those at the amino acid positions shown in Tables 1, 2, 6-1, and 6-2 (in the EU numbering). Preferably, the human FcRn-binding activity can be increased in the neutral pH range by altering at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering). The number of amino acids to be altered is not particularly limited; and it is possible to alter amino acids at only a single site or two or more sites. Combinations of two or more amino acid alterations include, for example, those shown in Tables 3, 4-1 to 4-5, 6-1, and 6-2.

Meanwhile, the site where histidine mutation is introduced is not particularly limited, and thus it may be introduced at any position as long as the histidine mutation reduces the antigen-binding activity in the acidic pH range to less than that in the neutral pH range. Such histidine mutations may be introduced at a single site or two or more sites.

Thus, the production methods of the present invention may further comprise the steps of altering the above-described amino acids and substituting or inserting histidine. In the production methods of the present invention, non-natural amino acids may be used instead of histidine. Therefore, the present invention can also be understood by replacing the above-mentioned histidine with non-natural amino acids.

Furthermore, in another embodiment, the antigen-binding molecules that are produced by the production methods described above include, for example, antigen-binding molecules comprising altered antibody constant regions. Accordingly, the production methods of the present invention may further comprise the step of altering the amino acids of antibody constant regions.

The antigen-binding molecules produced by the production methods of the present invention are administered to facilitate the reduction of plasma antigen concentration. Thus, the production methods of the present invention can be used as a method for producing antigen-binding molecules to facilitate the reduction of plasma antigen concentration when administered.

Alternatively, the antigen-binding molecules produced by the production methods of the present invention have improved pharmacokinetics. Thus, the production methods of the present invention can be used as a method for producing antigen-binding molecules with improved pharmacokinetics.

Alternatively, the antigen-binding molecules produced by the production methods of the present invention can increase the number of antigens to which a single antigen-binding molecule can bind when administered to animals such as humans, mice, and monkeys. Thus, the production methods of the present invention can be used as a method for producing antigen-binding molecules that have an increased number of antigens to which a single antigen-binding molecule can bind.

Furthermore, antigen-binding molecules produced by the production methods of the present invention are expected to be capable of dissociating within a cell from an extracellularly-bound antigen when administered to animals such as humans, mice, or monkeys. Thus, the production methods of the present invention can be used as methods for producing antigen-binding molecules that are capable of dissociating within a cell from an extracellularly-bound antigen.

Furthermore, antigen-binding molecules produced by the production methods of the present invention are expected to be capable of being bound to an antigen and taken up into a cell as well as being released to the outside of the cell in an antigen-free form, when administered to animals such as humans, mice, or monkeys. Thus, the production methods of the present invention can be used as methods for producing antigen-binding molecules that are capable of being bound to an antigen and taken up into a cell and being released to the outside of the cell in an antigen-free form.

Furthermore, since such antigen-binding molecules have greater activity to reduce plasma antigen concentration by administration as compared to typical antigen-binding molecules, they are expected to be especially superior as pharmaceuticals. Thus, the production methods of the present invention can be used as methods for producing antigen-binding molecules for use as pharmaceutical compositions.

Genes obtained by the production methods of the present invention are typically carried by (inserted into) appropriate vectors, and then introduced into host cells. The vectors are not particularly limited as long as they stably retain the inserted nucleic acids. For example, when E. coli is used as the host, preferred cloning vectors include pBluescript vector (Stratagene); however, various commercially available vectors may be used. When using vectors to produce the antigen-binding molecules of the present invention, expression vectors are particularly useful. The expression vectors are not particularly limited as long as the vectors express the antigen-binding molecules in vitro, in E. coli, in culture cells, or in a body of an organism. For example, pBEST vector (Promega) is preferred for in vitro expression; pET vector (Invitrogen) is preferred for E. coli; pME18S-FL3 vector (GenBank Accession No. AB009864) is preferred for culture cells; and pME18S vector (Mol Cell Biol. (1988) 8: 466-472) is preferred for bodies of organisms. DNAs of the present invention can be inserted into the vectors by conventional methods, for example, by ligation using restriction enzyme sites (Current protocols in Molecular Biology, edit. Ausubel et al., (1987) Publish. John Wiley & Sons, Section 11.4-11.11).

The above host cells are not particularly limited, and various host cells may be used depending on the purpose. Examples of cells for expressing the antigen-binding molecules include bacterial cells (such as those of Streptococcus, Staphylococcus, E. coli, Streptomyces, and Bacillus subtilis), eukaryotic cells (such as those of yeast and Aspergillus), insect cells (such as Drosophila S2 and Spodoptera SF9), animal cells (such as CHO, COS, HeLa, C127, 3T3, BHK, HEK293, and Bowes melanoma cells), and plant cells. Vectors can be introduced into a host cell by known methods, for example, calcium phosphate precipitation methods, electroporation methods (Current protocols in Molecular Biology edit.

Ausubel et al. (1987) Publish. John Wiley & Sons, Section 9.1-9.9), lipofection methods, and microinjection methods.

The host cells can be cultured by known methods. For example, when using animal cells as a host, DMEM, MEM, RPMI1640, or IMDM may be used as the culture medium. They may be used with serum supplements such as FBS or fetal calf serum (FCS). The cells may be cultured in serum-free cultures. The preferred pH is about 6 to 8 during the course of culturing. Incubation is carried out typically at 30 to 40 degrees C for about 15 to 200 hours. Medium is exchanged, aerated, or agitated, as necessary.

Appropriate secretion signals may be incorporated to polypeptides of interest so that the antigen-binding molecules expressed in the host cell are secreted into the lumen of the endoplasmic reticulum, into the periplasmic space, or into the extracellular environment. These signals may be endogenous to the antigen-binding molecules of interest or may be heterologous signals.

On the other hand, for example, production systems using animals or plants may be used as systems for producing polypeptides in vivo. A polynucleotide of interest is introduced into an animal or plant and the polypeptide is produced in the body of the animal or plant, and then collected. The "hosts" of the present invention include such animals and plants.

The production system using animals include those using mammals or insects. It is possible to use mammals such as goats, pigs, sheep, mice, and bovines (Vicki Glaser SPECTRUM Biotechnology Applications (1993)). The mammals may be transgenic animals.

For example, a polynucleotide encoding an antigen-binding molecule of the present invention is prepared as a fusion gene with a gene encoding a polypeptide specifically produced in milk, such as the goat beta-casein. Next, goat embryos are injected with polynucleotide fragments containing the fusion gene, and then transplanted to female goats. Desired antigen-binding molecules can be obtained from milk produced by the transgenic goats, which are born from the goats that received the embryos, or from their offspring. Hormones may be administered as appropriate to increase the volume of milk containing the antigen-binding molecule produced by the transgenic goats (Ebert et al., Bio/Technology (1994) 12: 699-702).

Insects such as silkworms may be used to produce the antigen-binding molecules of the present invention. When silkworms are used, baculoviruses carrying a polynucleotide encoding an antigen-binding molecule of interest can be used to infect silkworms, and the antigen-binding molecule of interest can be obtained from their body fluids.

Furthermore, when plants are used to produce the antigen-binding molecules of the present invention, for example, tobacco may be used. When tobacco is used, a polynucleotide encoding an antigen-binding molecule of interest is inserted into a plant expression vector, for example, pMON 530, and then the vector is introduced into bacteria, such as Agrobacterium tumefaciens. The bacteria are then allowed to infect tobacco such as Nicotiana tabacum, and the desired antigen-binding molecules can be collected from their leaves (Ma et al., Eur. J. Immunol. (1994) 24: 131-138). Alternatively, it is possible to infect duckweed (Lemna minor) with similar bacteria. After cloning, the desired antigen-binding molecules can be obtained from the duckweed cells (Cox KM et al., Nat. Biotechnol. 2006 Dec; 24(12): 1591-1597).

The thus obtained antigen-binding molecules may be isolated from the inside or outside (such as the medium and milk) of host cells, and purified as substantially pure and homogenous antigen-binding molecules. The methods for isolating and purifying antigen-binding molecules are not particularly limited, and isolation and purification methods usually used for polypeptide purification can be used. Antigen-binding molecules may be isolated and purified, by appropriately selecting and combining, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, and recrystallization.

Chromatography includes, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak et al., (1996) Cold Spring Harbor Laboratory Press). Such chromatographic methods can be conducted using liquid phase chromatography such as HPLC and FPLC. Columns used for affinity chromatography include, protein A columns and protein G columns. Columns using protein A include, for example, Hyper D, POROS, and Sepharose F. F. (Pharmacia).

If needed, an antigen-binding molecule can be modified arbitrarily, and peptides can be partially deleted by allowing an appropriate protein modification enzyme to act before or after purification of the antigen-binding molecule. Such protein modification enzymes include, for example, trypsin, chymotrypsin, lysyl endopeptidases, protein kinases, and glucosidases.

<Pharmaceutical compositions>

The present invention also relates to pharmaceutical compositions that include antigen-binding molecules of the present invention, antigen-binding molecules isolated by the screening methods of the present invention, or antigen-binding molecules produced by the production methods of the present invention. The antigen-binding molecules of the present invention and antigen-binding molecules produced by the production methods of the present invention have greater activity to reduce plasma antigen concentration by administration as compared to typical antigen-binding molecules, and are therefore useful as pharmaceutical compositions. The pharmaceutical composition of the present invention may include pharmaceutically acceptable carriers.

In the present invention, pharmaceutical compositions ordinarily refer to agents for treating or preventing, or testing and diagnosing diseases.

The pharmaceutical compositions of the present invention can be formulated by methods known to those skilled in the art. For example, they can be used parenterally, in the form of injections of sterile solutions or suspensions including water or other pharmaceutically acceptable liquid. For example, such compositions may be formulated by mixing in the form of unit dose required in the generally approved medicine manufacturing practice by appropriately combining with pharmaceutically acceptable carriers or media, specifically with sterile water, physiological saline, vegetable oil, emulsifier, suspension, surfactant, stabilizer, flavoring agent, excipient, vehicle, preservative, binder, or such. In such formulations, the amount of active ingredient is adjusted to obtain an appropriate amount in a pre-determined range.

Sterile compositions for injection can be formulated using vehicles such as distilled water for injection, according to standard formulation practice. Aqueous solutions for injection include, for example, physiological saline and isotonic solutions containing dextrose or other adjuvants (for example, D-sorbitol, D-mannose, D-mannitol, and sodium chloride). It is also possible to use in combination appropriate solubilizers, for example, alcohols (ethanol and such), polyalcohols (propylene glycol, polyethylene glycol, and such), non-ionic surfactants (polysorbate 80(TM), HCO-50, and such).

Oils include sesame oil and soybean oils. Benzyl benzoate and/or benzyl alcohol can be used in combination as solubilizers. It is also possible to combine buffers (for example, phosphate buffer and sodium acetate buffer), soothing agents (for example, procaine hydrochloride), stabilizers (for example, benzyl alcohol and phenol), and/or antioxidants. Appropriate ampules are filled with the prepared injections.

The pharmaceutical compositions of the present invention are preferably administered parenterally. For example, the compositions may be in the dosage form for injections, transnasal administration, transpulmonary administration, or transdermal administration. For example, they can be administered systemically or locally by intravenous injection, intramuscular injection, intraperitoneal injection, subcutaneous injection, or such.

Administration methods can be appropriately selected in consideration of the patient's age and symptoms. The dose of a pharmaceutical composition containing an antigen-binding molecule may be, for example, from 0.0001 to 1,000 mg/kg for each administration. Alternatively, the dose may be, for example, from 0.001 to 100,000 mg per patient. However, the present invention is not limited by the numeric values described above. The doses and administration methods vary depending on the patient's weight, age, symptoms, and such. Those skilled in the art can set appropriate doses and administration methods in consideration of the factors described above.

Amino acids contained in the amino acid sequences of the present invention may be post-translationally modified. For example, the modification of an N-terminal glutamine into a pyroglutamic acid by pyroglutamylation is well-known to those skilled in the art. Naturally, such post-translationally modified amino acids are included in the amino acid sequences in the present invention.

All prior art documents cited in the specification are incorporated herein by reference.

**Examples**

Herein below, the present invention will be specifically described with reference to Examples, but it is not to be construed as being limited thereto.

[Example 1] Study on enhancement of the antigen elimination-accelerating effect of antibodies

Anti-IL-6 receptor antibody

Preparation of anti-human IL-6 receptor antibody having FcRn-binding activity under neutral conditions

H54/L28-IgG1 comprising H54 (SEQ ID NO: 1) and L28 (SEQ ID NO: 2) described in WO 2009/125825 is a humanized anti-IL-6 receptor antibody. Mutation were introduced into H54 (SEQ ID NO: 1) to increase the FcRn binding under the neutral pH condition (pH7.4). Specifically, H54-IgG1-F14 (SEQ ID NO: 3) was prepared from the heavy chain constant region of IgG1 by substituting Trp for Met at position 252 and Trp for Asn at position 434 in the EU numbering. The amino acid substitutions were introduced by the method known to those skilled in the art described in Reference Example 1.

H54/L28-IgG1 comprising H54 (SEQ ID NO: 1) and L28 (SEQ ID NO: 2) and H54/L28-IgG1-F14 comprising H54-IgG1-F14 (SEQ ID NO: 3) and L28 (SEQ ID NO: 2) were expressed and purified by the method known to those skilled in the art described in Reference Example 2.

In vivo study of antibodies by steady-state infusion model using human FcRn transgenic mouse line 276

Using H54/L28-IgG1 and H54/L28-IgG1-F14 prepared as described above, an in vivo test was conducted by steady-state infusion model using human FcRn transgenic mouse line 276. An infusion pump (MINI-OSMOTIC PUMP MODEL 2004; alzet) containing soluble human IL-6 receptor was implanted under the skin on the back of human FcRn transgenic mouse line 276 (B6.mFcRn-/-.hFcRn Tg line 276 +/+ mouse (B6.mFcRn-/- hFCRN Tg276 B6.Cg-Fcgrt<tm1Dcr> Tg(FCGRT)276Dcr (Jackson #4919)), Jackson Laboratories; Methods Mol Biol. (2010) 602: 93-104) to prepare model animals where the plasma concentration of soluble human IL-6 receptor was kept constant. Anti-human IL-6 receptor antibodies were administered to the model animals to assess the in vivo dynamics after administration of soluble human IL-6 receptor. Monoclonal anti-mouse CD4 antibody (R&D) was administered at 20 mg/kg before implanting infusion pump and 14 days after antibody administration into the caudal vein to suppress the production of neutralizing antibody against soluble human IL-6 receptor. Then, an infusion pump containing 92.8 microgram/ml soluble human IL-6 receptor was implanted under the skin on the back of the mice. Three days after implantation of an infusion pump, anti-human IL-6 receptor antibodies (H54/L28-IgG1 and H54/L28-IgG1-F14) were administered at 1 mg/kg once into the caudal vein. Blood was collected 15 minutes, seven hours, one day, two days, three days, four days, seven days, 14 days, 21 days, and 28 days after administration of the anti-human IL-6 receptor antibody. The collected blood was immediately centrifuged at 15,000 rpm and 4 degrees C for 15 minutes to separate plasma. The separated plasma was stored in a refrigerator at -20 degrees C or below before assay.

Determination of plasma hsIL-6R concentration by electrochemiluminescence assay

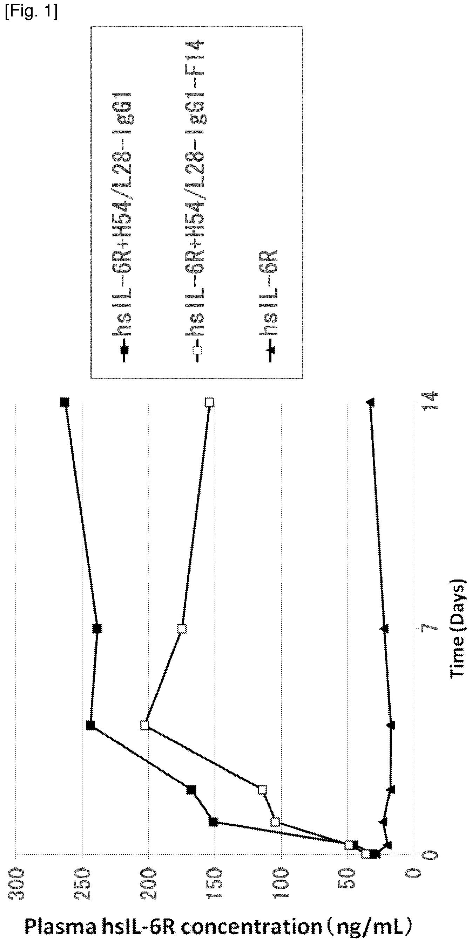

The concentration of hsIL-6R in mouse plasma was measured by electrochemiluminescence. hsIL-6R calibration curve samples adjusted to concentrations of 2,000, 1,000, 500, 250, 125, 62.5, and 31.25 pg/ml, and mouse plasma samples diluted 50-fold or more were prepared. The samples were mixed with a solution of Monoclonal Anti-human IL-6R Antibody (R&D) ruthenium-labeled with Sulfo-Tag NHS Ester (Meso Scale Discovery), Biotinylated Anti-human IL-6R Antibody (R&D), and WT-IgG1, and then allowed to react overnight at 37 degrees C. The final concentration of WT-IgG1 as an anti-human IL-6 receptor antibody, comprising H (WT) (SEQ ID NO: 4) and L (WT) (SEQ ID NO: 5), was 333 microgram/ml, which is in excess of the concentration of anti-human IL-6 receptor antibody contained in the samples, for the purpose of binding nearly all of the hsIL-6R molecules in the samples to WT-IgG1. Subsequently, the samples were dispensed into an MA400 PR Streptavidin Plate (Meso Scale Discovery), and allowed to react for one hour at room temperature, and washing was performed. Immediately after Read Buffer T (x4) (Meso Scale Discovery) was dispensed, the measurement was performed by the Sector PR 400 Reader (Meso Scale Discovery). The hsIL-6R concentration was calculated based on the response of the calibration curve using the analytical software SOFTmax PRO (Molecular Devices). The time course of plasma hsIL-6R concentration after intravenous administration of H54/L28-IgG1 and H54/L28-IgG1-F14 as measured by this method is shown in Fig. 1.

As shown in Fig. 1, compared to the baseline hsIL-6R concentration without antibody, administration of H54/L28-IgG1 resulted in significant elevation of plasma hsIL-6R concentration. On the other hand, administration of H54/L28-IgG1-F14 resulted in reduction of elevation of plasma hsIL-6R concentration as compared to H54/L28-IgG1. This reduction in elevation is derived from increased human FcRn binding at neutral pH in H54/L28-IgG1-F14 as compared to H54/L28-IgG1. This demonstrates that increasing binding affinity of antibody to FcRn at neutral pH could enhance the clearance of antigen, although the extent of antigen clearance enhancement was small for H54/L28-IgG1-F14 as compared to H54/L28-IgG1.

[Example 2] Study on enhancement of the antigen elimination-accelerating effect of pH-dependent antigen-binding antibodies (preparation of antibodies)

Regarding pH-dependent human IL-6 receptor-binding antibody

H54/L28-IgG1 comprising H54 (SEQ ID NO: 1) and L28 (SEQ ID NO: 2) described in WO 2009/125825 is a humanized anti-IL-6 receptor antibody. Fv4-IgG1 comprising VH3-IgG1 (SEQ ID NO: 6) and VL3-CK (SEQ ID NO: 7) is a humanized anti-IL-6 receptor antibody that results from conferring H54/L28-IgG1 with the property to bind to soluble human IL-6 receptor in a pH-dependent manner (which binds at pH 7.4 but is dissociated at pH 5.8). The in vivo test described in WO 2009/125825 using mice demonstrated that the elimination of soluble human IL-6 receptor could be greatly accelerated in a group administered with a mixture of Fv4-IgG1 and soluble human IL-6 receptor as the antigen as compared to a group administered with a mixture of H54/L28-IgG1 and soluble human IL-6 receptor as the antigen.

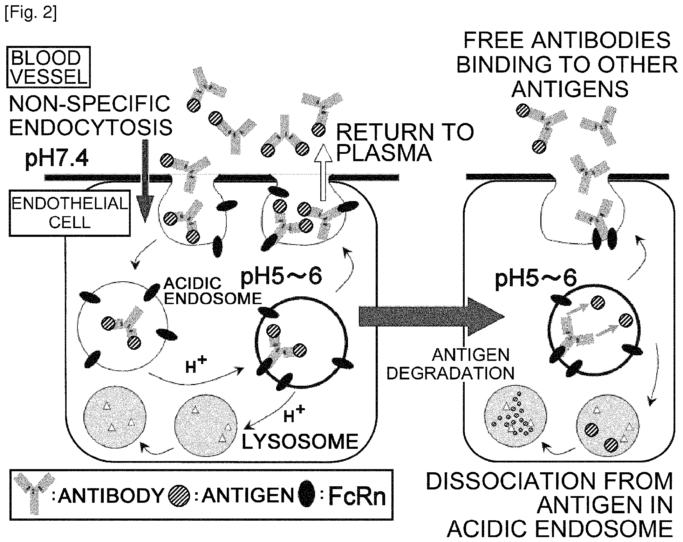

Soluble human IL-6 receptor bound to an ordinary antibody that binds to soluble human IL-6 receptor is recycled to the plasma along with the antibody via FcRn. Meanwhile, an antibody that binds to soluble human IL-6 receptor in a pH-dependent manner dissociates the soluble human IL-6 receptor that has been bound to the antibody under acidic conditions in the endosome. The dissociated soluble human IL-6 receptor is degraded in the lysosome. This can greatly accelerate the elimination of soluble human IL-6 receptor. Then, the antibody that binds to soluble human IL-6 receptor in a pH-dependent manner is recycled to the plasma via FcRn. The recycled antibody can bind to another soluble human IL-6 receptor again. By repeating this cycle, a single antibody molecule can repeatedly bind to soluble human IL-6 receptors multiple times (Fig. 2).

Antibodies that bind to antigens in a pH-dependent manner accelerate the elimination of soluble antigen. The antibodies produce the effect by repeatedly binding to soluble antigens multiple times. Thus, such antibodies are very useful. A method for augmenting the FcRn binding under a neutral condition (pH 7.4) was tested to further enhance the antigen elimination-facilitating effect.

Preparation of pH-dependent human IL-6 receptor-binding antibodies having FcRn-binding activity under neutral conditions

Mutations were introduced into Fv4-IgG1 comprising VH3- IgG 1 (SEQ ID NO: 6) and VL3-CK (SEQ ID NO: 7) to augment the FcRn binding under a neutral condition (pH 7.4). Specifically, VH3-IgG1-v1 (SEQ ID NO: 8) was prepared from the heavy chain constant region of IgG1 by substituting Tyr for Met at position 252, Thr for Ser at position 254, and Glu for Thr at position 256 in EU numbering, while VH3-IgG1-v2 (SEQ ID NO: 9) was constructed from the heavy chain constant region of IgG1 by substituting Trp for Asn at position 434 in EU numbering. The amino acid substitutions were introduced by the method known to those skilled in the art described in Reference Example 1.

H54/L28- IgG 1 comprising H54 (SEQ ID NO: 1) and L28 (SEQ ID NO: 2), Fv4-IgG1 comprising VH3-IgG1 (SEQ ID NO: 6) and VL3-CK (SEQ ID NO: 7), Fv4-IgG1-v1 comprising VH3-IgG1-v1 (SEQ ID NO: 8) and VL3-CK (SEQ ID NO: 7), and Fv4-IgG1-v2 comprising VH3-IgG1-v2 (SEQ ID NO: 9) and VL3-CK (SEQ ID NO: 7) were expressed and purified by the method known to those skilled in the art described in Reference Example 2.

[Example 3] Study on enhancement of the antigen elimination-accelerating effect of pH-dependent antigen-binding antibodies (in vivo test)

In vivo test using human FcRn transgenic mice and normal mice

The in vivo kinetics of hsIL-6R (soluble human IL-6 receptor: prepared as described in Reference Example 3) and anti-human IL-6 receptor antibody was assessed after administering hsIL-6R alone or hsIL-6R and anti-human IL-6 receptor antibody in combination to human FcRn transgenic mice (B6.mFcRn-/-.hFcRn Tg line 276 +/+ mouse, Jackson Laboratories; Methods Mol Biol. (2010) 602: 93-104) and normal mice (C57BL/6J mouse; Charles River Japan). An hsIL-6R solution (5 microgram/ml) or a solution of mixture containing hsIL-6R and anti-human IL-6 receptor antibody (5 microgram/ml and 0.1 mg/ml, respectively) was administered once at a dose of 10 ml/ kg into the caudal vein. In this case, the anti-human IL-6 receptor antibody is present in excess over hsIL-6R, and therefore almost every hsIL-6R is assumed to be bound to the antibody. Blood was collected 15 minutes, seven hours, one day, two days, three days, four days, seven days, 14 days, 21 days, and 28 days after administration. The collected blood was immediately centrifuged at 15,000 rpm and 4 degrees C for 15 minutes to separate the plasma. The separated plasma was stored in a refrigerator at or below -20 degrees C before assay. The anti-human IL-6 receptor antibodies used are: above-described H54/L28-IgG1, Fv4-IgG1, and Fv4-IgG1-v2 for human FcRn transgenic mice, and above-described H54/L28-IgG1, Fv4-IgG1, Fv4-IgG1-v1, and Fv4-IgG1-v2 for normal mice.

Measurement of anti-human IL-6 receptor antibody plasma concentration by ELISA

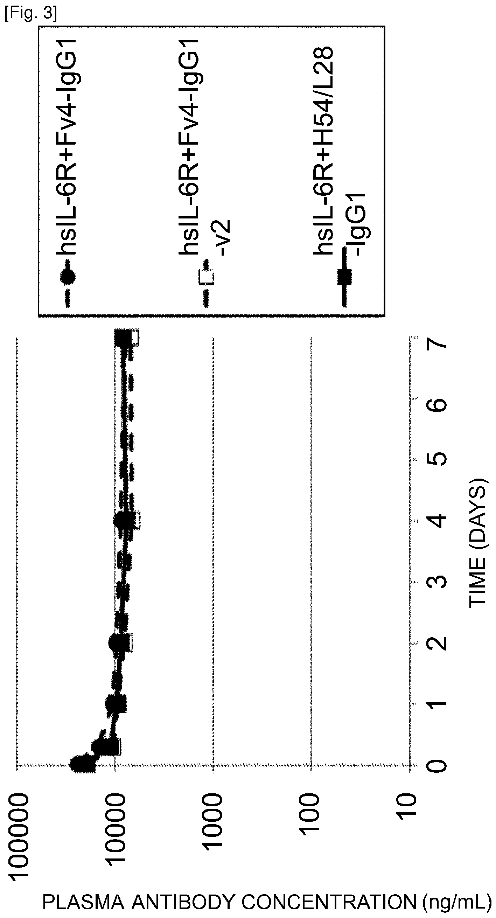

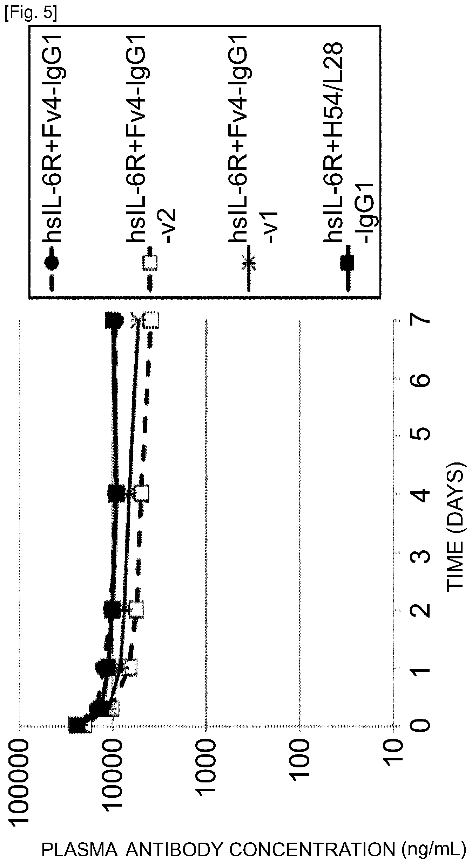

The concentration of anti-human IL-6 receptor antibody in mouse plasma was measured by ELISA. Anti-human IgG (gamma-chain specific) F(ab')2 antibody fragment (Sigma) was dispensed onto a Nunc-ImmunoPlate MaxiSorp (Nalge Nunc International) and allowed to stand overnight at 4 degrees C to prepare anti-human IgG-immobilized plates. Calibration curve samples having plasma concentrations of 0.8, 0.4, 0.2, 0.1, 0.05, 0.025, and 0.0125 microgram/ml, and mouse plasma samples diluted 100-fold or more were prepared. 200 microliter (microL) of 20 ng/ml hsIL-6R was added to 100 microliter of the calibration curve samples and plasma samples, and then the samples were allowed to stand for one hour at room temperature. Subsequently, the samples were dispensed into the anti-human IgG-immobilized plates, and allowed to stand for one hour at room temperature. Then, Biotinylated Anti-Human IL-6R Antibody (R&D) was added to react for one hour at room temperature. Subsequently, Streptavidin-PolyHRP80 (Stereospecific Detection Technologies) was added to react for one hour at room temperature, and chromogenic reaction was carried out using TMP One Component HRP Microwell Substrate (BioFX Laboratories) as a substrate. After stopping the reaction with 1 N sulfuric acid (Showa Chemical), the absorbance at 450 nm was measured by a microplate reader. The concentration in mouse plasma was calculated from the absorbance of the calibration curve using the analytical software SOFTmax PRO (Molecular Devices). The time course of plasma concentration after intravenous administration as measured by this method is shown in Fig. 3 for human FcRn transgenic mice and Fig. 5 for normal mice.

Measurement of hsIL-6R plasma concentration by electrochemiluminescence assay

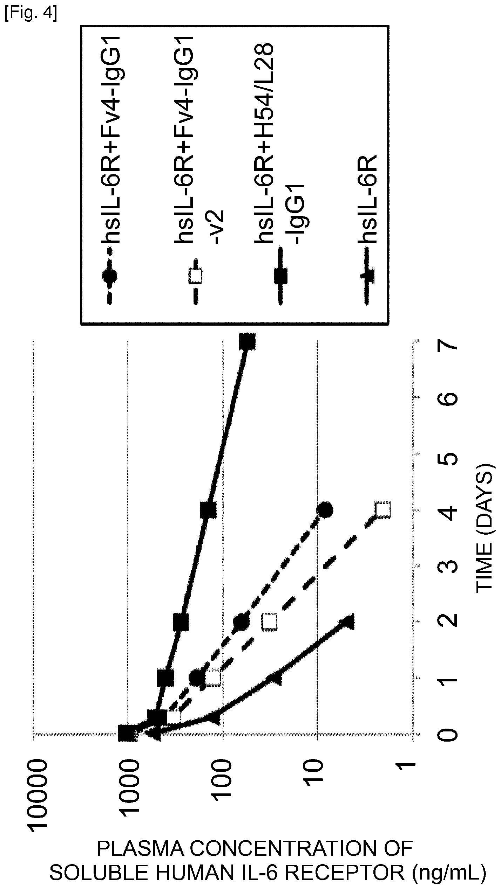

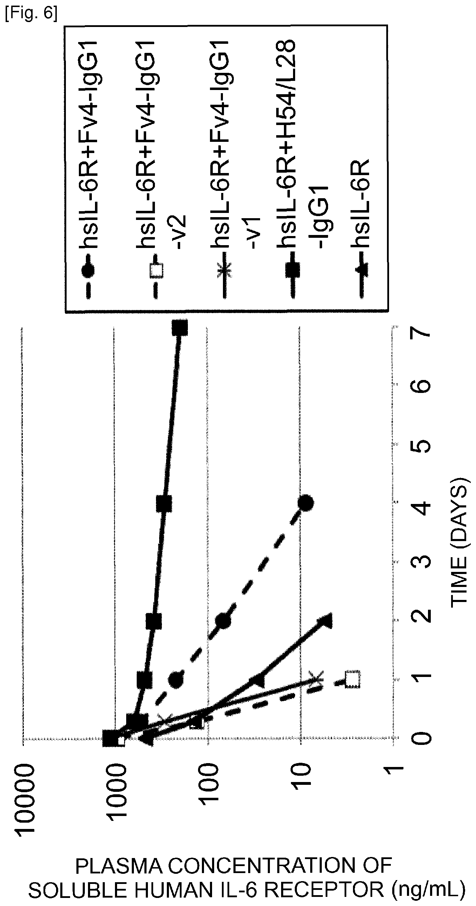

The concentration of hsIL-6R in mouse plasma was measured by electrochemiluminescence. hsIL-6R calibration curve samples adjusted to concentrations of 2,000, 1,000, 500, 250, 125, 62.5, and 31.25 pg/ml, and mouse plasma samples diluted 50-fold or more were prepared. The samples were mixed with a solution of Monoclonal Anti-human IL-6R Antibody (R&D) ruthenium-labeled with Sulfo-Tag NHS Ester (Meso Scale Discovery), Biotinylated Anti-human IL-6R Antibody (R&D), and WT-IgG1, and then allowed to react overnight at 37 degrees C. The final concentration of WT-IgG1 as an anti-human IL-6 receptor antibody, comprising H (WT) (SEQ ID NO: 4) and L (WT) (SEQ ID NO: 5), was 333 microgram/ml, which is in excess of the concentration of anti-human IL-6 receptor antibody contained in the samples, for the purpose of binding nearly all of the hsIL-6R molecules in the samples to WT-IgG1. Subsequently, the samples were dispensed into an MA400 PR Streptavidin Plate (Meso Scale Discovery), and allowed to react for one hour at room temperature, and washing was performed. Immediately after Read Buffer T (x4) (Meso Scale Discovery) was dispensed, the measurement was performed by the Sector PR 400 Reader (Meso Scale Discovery). The hsIL-6R concentration was calculated based on the response of the calibration curve using the analytical software SOFTmax PRO (Molecular Devices). The time course of plasma hsIL-6R concentration after intravenous administration as measured by this method is shown in Fig. 4 for human FcRn transgenic mice and Fig. 6 for normal mice.

Determination of free hsIL-6R concentration in plasma by electrochemiluminescence assay

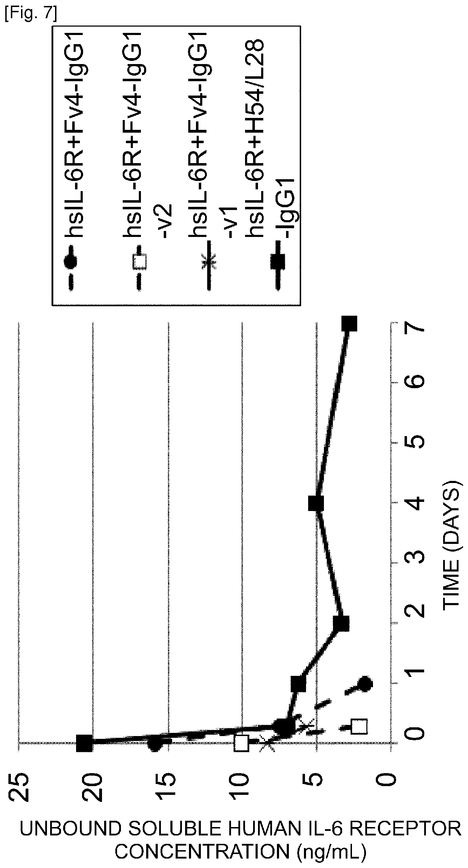

To assess the degree of neutralization of soluble human IL-6 receptor in plasma, the concentration of soluble human IL-6 receptor free of (non-neutralized by) anti-human IL-6 receptor antibody (free hsIL-6R concentration) in mouse plasma was determined by electrochemiluminescence assay. All IgG-type antibodies (mouse IgG, anti-human IL-6 receptor antibody, and anti-human IL-6 receptor antibody-soluble human IL-6 receptor complex) in plasma were adsorbed onto protein A by adding 12 microliter each of hsIL-6R standard samples prepared at 10,000, 5,000, 2,500, 1,250, 625, 312.5, or 156.25 pg/ml and mouse plasma samples onto an appropriate amount of rProtein A Sepharose Fast Flow (GE Healthcare) resin dried on 0.22-micrometer filter cup (Millipore). Then, the solution in a cup was spun down using a high-speed centrifuge to collect the solution that passed through. The passed-through solution does not contain Protein A-bound anti-human IL-6 receptor antibody-soluble human IL-6 receptor complex. Thus, the concentration of free hsIL-6R in plasma can be determined by measuring the concentration of hsIL-6R in the passed-through solution. Then, the passed-through solution was mixed with a monoclonal anti-human IL-6R antibody (R&D) ruthenium-labeled with SULFO-TAG NHS Ester (Meso Scale Discovery) and a biotinylated anti-human IL-6 R antibody (R&D). The resulting mixture was incubated at room temperature for one hour, and then aliquoted to MA400 PR Streptavidin Plate (Meso Scale Discovery). After another hour of incubation at room temperature, the plate was washed and Read Buffer T (x4) (Meso Scale Discovery) was aliquoted thereto. Immediately, the plate was measured in SECTOR PR 400 reader (Meso Scale Discovery). The hsIL-6R concentration was calculated based on the response in the standard curve using the analysis software SOFTmax PRO (Molecular Devices). A time course of free hsIL-6R concentration in the plasma of normal mice after intravenous administration determined by the above-described method is shown in Fig. 7.

Effect of pH-dependent binding to human IL-6 receptor

H54/L28-IgG1 and Fv4-IgG1 which binds to human IL-6 receptor in a pH-dependent manner were tested in vivo, and the results were compared between them. As shown in Figs. 3 and 5, the antibody retention in plasma was comparable. Meanwhile, as shown in Figs. 4 and 6, hsIL-6R simultaneously administered with Fv4-IgG1 which binds to human IL-6 receptor in a pH-dependent manner was found to accelerate the elimination of hsIL-6R as compared to hsIL-6R simultaneously administered with H54/L28-IgG1. The above tendency was observed in both human FcRn transgenic and normal mice; thus, it was demonstrated that by conferring a pH-dependent human IL-6 receptor-binding ability, the plasma hsIL-6R concentration four days after administration could be decreased by about 17 and 34 times, respectively.

Effect of FcRn binding under neutral condition (pH 7.4)

Intact human IgG1 has been reported to hardly bind to (have extremely low affinity for) human FcRn under a neutral condition (pH 7.4). The human FcRn binding under a neutral condition (pH 7.4) was reported to be augmented by substituting Trp for Asn at position 434 (EU numbering) in intact human IgG1 (J Immunol. (2009) 182 (12): 7663-71). Fv4-IgG1-v2 which results from introducing the above amino acid substitution into Fv4-IgG1 was tested by an in vivo test using human FcRn transgenic mice. The test result was compared to that of Fv4-IgG1. As shown in Fig. 3, the antibody plasma retention was comparable between the two. Meanwhile, as shown in Fig. 4, hsIL-6R simultaneously administered with Fv4-IgG1-v2 that exhibits enhanced human FcRn binding under a neutral condition (pH 7.4) was found to be eliminated faster as compared to hsIL-6R simultaneously administered with Fv4-IgG1. Thus, it was demonstrated that by conferring the ability to bind to human FcRn under a neutral condition (pH 7.4), the plasma concentration of hsIL-6R four days after administration could be reduced by about four times.

Based on the homology between human FcRn and mouse FcRn, the substitution of Trp for Asn at position 434 in EU numbering is assumed to augment the binding to mouse FcRn under a neutral condition (pH 7.4). Meanwhile, the binding to mouse FcRn under a neutral condition (pH 7.4) has been reported to be augmented by substituting Tyr for Met at position 252, Thr for Ser at position 254, and Glu for Thr at position 256 in EU numbering (J Immunol. (2002) 169(9): 5171-80). Fv4-IgG1-v1 and Fv4-IgG1-v2 which result from introducing the above-described amino acid substitutions into Fv4-IgG1 were tested in vivo using normal mice. The test results were compared to that of Fv4-IgG1. As shown in Fig. 5, the plasma retention times of Fv4-IgG1-v1 and Fv4-IgG1-v2 which had also been improved to increase the binding to mouse FcRn under a neutral condition (pH 7.4) were slightly shortened (the neutralizing antibody concentrations in plasma one day after administration were reduced by about 1.5 and 1.9 times, respectively) as compared to Fv4-IgG1.

As shown in Fig. 6, hsIL-6R simultaneously administered with Fv4-IgG1-v1 or Fv4-IgG1-v2 which had been improved to increase the binding to mouse FcRn under a neutral condition (pH 7.4) was demonstrated to be eliminated markedly faster as compared to hsIL-6R simultaneously administered with Fv4-IgG1. Fv4-IgG1-v1 and Fv4- IgG 1-v2 reduced the plasma hsIL-6R concentrations one day after administration by about 32 and 80 times, respectively. Thus, it was revealed that the plasma concentration could be reduced by conferring mouse FcRn-binding ability under a neutral condition (pH 7.4). As described above, by conferring the mouse FcRn-binding ability under a neutral condition (pH 7.4), the plasma antibody concentration was slightly reduced; however, the effect of reducing the plasma hsIL-6R concentration, which largely exceeded the decrease in antibody concentration, was produced. Furthermore, hsIL-6R simultaneously administered with Fv4-IgG1-v1 or Fv4-IgG1-v2 was found to be eliminated faster even when compared to the group administered with hsIL-6R alone. As shown in Fig. 6, it was demonstrated that hsIL-6R simultaneously administered with Fv4-IgG1-v1 or Fv4-IgG1-v2 could reduce the plasma hsIL-6R concentration one day after administration by about 4 or 11 times, respectively, as compared to hsIL-6R alone. Specifically, this means that the elimination of soluble IL-6 receptor could be accelerated by administering the antibody that binds to soluble IL-6 receptor in a pH-dependent manner and which is conferred with mouse FcRn-binding ability under a neutral condition (pH 7.4). Specifically, the plasma antigen concentration can be reduced in vivo by administering such an antibody to the body.

As shown in Fig. 7, free hsIL-6R was in a detectable concentration range for seven days after administration of H54/L28-IgG1, while free hsIL-6R was undetectable after one day following administration of Fv4-IgG1. On the other hand, free hsIL-6R was not detectable after seven hours following administration of Fv4-IgG1-v1 or Fv4-IgG1-v2. Specifically, the free hsIL-6R concentration was lower in the presence of Fv4-IgG1 that binds to hsIL-6R in a pH-dependent manner as compared to H54/L28-IgG1, suggesting that a strong hsIL-6R-neutralizing effect was produced by conferring the pH-dependent hsIL-6R-binding ability. Furthermore, the free hsIL-6R concentration was much lower in the presence of Fv4-IgG1-v1 or Fv4-IgG1-v2, both of which were modified from Fv4-IgG1 to increase the FcRn-binding ability at pH 7.4. This demonstrates that a much stronger hsIL-6R-neutralizing effect can be produced by increasing the FcRn-binding ability at pH 7.4.

When administered, an ordinary neutralizing antibody such as H54/L28-IgG1 reduces the clearance of a binding antigen, resulting in prolonged antigen plasma retention. It is not preferred that administered antibodies prolong the plasma retention of an antigen whose action is intended to be neutralized by the antibodies. The antigen plasma retention can be shortened by conferring the pH dependency to antigen binding (the antibody binds under neutral conditions but is dissociated under acidic conditions). In the present invention, the antigen retention time in plasma could be further shortened by additionally conferring human FcRn-binding ability under a neutral condition (pH 7.4). Furthermore, it was demonstrated that as compared to clearance of antigen alone, antigen clearance could be increased by administering an antibody that binds to an antigen in a pH dependent manner, and which is conferred with FcRn-binding ability under a neutral condition (pH 7.4). To date, there is no method available for increasing antigen clearance by antibody administration relative to clearance of antigen alone. Thus, the methods established as described in this EXAMPLE are very useful as a method for eliminating antigens from plasma by administering antibodies. Furthermore, the present inventors discovered for the first time the advantage of increasing the FcRn-binding ability under a neutral condition (pH 7.4). Furthermore, both v4-IgG1-v1 and Fv4-IgG1-v2 which have different amino acid substitutions that increase the FcRn-binding ability under a neutral condition (pH 7.4) produced comparable effects. This suggests that regardless of the type of amino acid substitution, every amino acid substitution that increases the human FcRn-binding ability under a neutral condition (pH 7.4) potentially has an effect of accelerating antigen elimination. Specifically, antibody molecules that eliminate antigens from plasma when administered can be produced using the following amino acid substitutions alone or in combination:

an amino acid substitution of Ile for Pro at position 257 and an amino acid substitution of Ile for Gln at position 311 in EU numbering, both of which have been reported in J Biol Chem. 2007, 282(3): 1709-17; an amino acid substitution of Ala, Tyr, or Trp for Asn at position 434, an amino acid substitution of Tyr for Met at position 252, an amino acid substitution of Gln for Thr at position 307, an amino acid substitution of Pro for Val at position 308, an amino acid substitution of Gln for Thr at position 250, an amino acid substitution of Leu for Met at position 428, an amino acid substitution of Ala for Glu at position 380, an amino acid substitution of Val for Ala at position 378, an amino acid substitution of Ile for Tyr at position 436 in EU numbering, all of which have been reported in J Immunol. (2009) 182(12): 7663-71; an amino acid substitution of Tyr for Met at position 252, an amino acid substitution of Thr for Ser at position 254, an amino acid substitution of Glu for Thr at position 256 in EU numbering, all of which have been reported in J Biol Chem. 2006 Aug. 18, 281(33): 23514-24; an amino acid substitution of Lys for His at position 433, an amino acid substitution of Phe for Asn at position 434, and an amino acid substitution of His for Tyr at position 436 in EU numbering, all of which have been reported in Nat Biotechnol. 2005 Oct. 23(10): 1283-8; and the like.

[Example 4] Assessment of human FcRn-binding activity

For the Biacore-based assay system for testing the interaction between antibody and FcRn, a system that immobilizes antibody on a sensor chip and uses human FcRn as an analyte is reported in J Immunol. (2009) 182(12): 7663-71. For this purpose, human FcRn was prepared as described in Reference Example 4. Fv4-IgG1, Fv4-IgG1-v1, and Fv4-IgG1-v2 were assessed for the human FcRn-binding activity (dissociation constant (KD)) at pH 6.0 and pH 7.4 by using the above-described system. The antibodies were tested as a test substance after direct immobilization onto Series S Sensor Chip CM5. Using an amino-coupling kit according to the supplier's instruction manual, the antibodies were immobilized onto Sensor Chip so as to secure an immobilization amount of 500 RU. The running buffer used was 50 mmol/l Na-phosphate/150 mmol/1 NaCl containing 0.05% (v/v%) Surfactant P20 (pH 6.0).

With the prepared sensor chips, assay was carried out using as a running buffer, 50 mmol/l Na-phosphate/150 mmol/l NaCl containing 0.05% Surfactant P20 (pH 6.0) or 50 mmol/l Na-phosphate/150 mmol/l NaCl containing 0.05% Surfactant P20 (pH 7.4). Assays were carried out exclusively at 25 degrees C. The diluted human FcRn solutions and running buffer as a reference solution were injected at a flow rate of 5 microliter/min for ten minutes to allow for human FcRn to interact with the antibody on the chip. Next, the running buffer was injected at a flow rate of 5 microliter/min for one minute to monitor the dissociation of FcRn. Then, the sensor chip was regenerated by two rounds of injection of 20 mmol/l Tris-HCl/150 mmol/l NaCl (pH 8.1) at a flow rate of 30 microliter/min for 15 seconds.

The assay results were analyzed using Biacore T100 Evaluation Software (Ver. 2.0.1). By a steady-state affinity method, the dissociation constant (KD) was calculated from the assay results at six different FcRn concentrations. The results on the human FcRn-binding activities (dissociation constants (KD)) of Fv4-IgG1, Fv4-IgG1-v1, and Fv4-IgG1-v2 at pH 6.0 and pH 7.4 are shown in Table 5 below.

[Table 5]

|  | KD($\mu$M) | |
| --- | --- | --- |
|  | pH6.0 | pH7.4 |
| Fv4-IgG1 | 1.99 | NA |
| Fv4-IgG1-v1 | 0.32 | 36.55 |
| Fv4-IgG1-v2 | 0.11 | 11.03 |

At pH 7.4, the binding of human FcRn to Fv4-IgG1 was too weak to determine the KD value (NA). Meanwhile, Fv4-IgG1-v1 and Fv4-IgG1-v2 were observed to bind to human FcRn at pH 7.4, and the KD values were determined to be 36.55 and 11.03 micromolar, respectively. The KD values for human FcRn at pH 6.0 were determined to be 1.99, 0.32, and 0.11 micromolar. As shown in Fig. 3, when compared to Fv4-IgG1, Fv4-IgG1-v2 accelerated the elimination of hsIL-6R in human FcRn transgenic mice. Thus, antigen elimination can be predicted to be accelerated by augmenting the human FcRn binding at pH 7.4 at least to be stronger than 11.03 micromolar by alteration of human IgG1. Meanwhile, as described in J Immunol. (2002) 169(9): 5171-80, human IgG1 binds about ten times more strongly to mouse FcRn than human FcRn. For this reason, Fv4-IgG1-v1 and Fv4-IgG1-v2 are also predicted to bind about ten times more strongly to mouse FcRn than human FcR at pH 7.4. Acceleration of the hsIL-6R elimination by Fv4-IgG1-v1 or Fv4-IgG1-v2 in normal mice shown in Fig. 6 is more significant than acceleration of the elimination by Fv4-IgG1-v2 in human FcRn transgenic mice shown in Fig. 4. This suggests that the degree of acceleration of hsIL-6R elimination is increased according to the strength of FcRn binding at pH 7.4.

[Example 5] Preparation of pH-dependent human IL-6 receptor-binding antibodies with enhanced human FcRn binding under neutral condition

Various alterations to augment the human FcRn binding under a neutral condition were introduced into Fv4-IgG1 to further enhance the antigen elimination effect of the pH-dependent human IL-6 receptor-binding antibody in human FcRn transgenic mice. Specifically, the amino acid alterations shown in Tables 6-1 and 6-2 were introduced into the heavy chain constant region of Fv4-IgG1 to produce various mutants (amino acid numbers of the mutation sites are presented according to EU numbering). The amino acid substitutions were introduced by methods known to those skilled in the art as described in Reference Example 1.

[Table 6-1]

| MUTANT NAME | KD (M) | AMINO ACID ALTERATION |
|---|---|---|
| IgG1 | ND | NONE |
| IgG1-v1 | 3.2E-06 | M252Y/S254T/T256E |
| IgG1-v2 | 8.1E-07 | N434W |
| IgG1-F3 | 2.5E-06 | N434Y |
| IgG1-F4 | 5.8E-06 | N434S |
| IgG1-F5 | 6.8E-06 | N434A |
| IgG1-F7 | 5.6E-06 | M252Y |
| IgG1-F8 | 4.2E-06 | M252W |
| IgG1-F9 | 1.4E-07 | M252Y/S254T/T256E/N434Y |
| IgG1-F10 | 6.9E-08 | M252Y/S254T/T256E/N434W |
| IgG1-F11 | 3.1E-07 | M252Y/N434Y |
| IgG1-F12 | 1.7E-07 | M252Y/N434W |
| IgG1-F13 | 3.2E-07 | M252W/N434Y |
| IgG1-F14 | 1.8E-07 | M252W/N434W |
| IgG1-F19 | 4.6E-07 | P257L/N434Y |
| IgG1-F20 | 4.6E-07 | V308F/N434Y |
| IgG1-F21 | 3.0E-08 | M252Y/V308P/N434Y |
| IgG1-F22 | 2.0E-06 | M428L/N434S |
| IgG1-F25 | 9.2E-09 | M252Y/S254T/T256E/V308P/N434W |
| IgG1-F26 | 1.0E-06 | I332V |
| IgG1-F27 | 7.4E-06 | G237M |
| IgG1-F29 | 1.4E-06 | I332V/N434Y |
| IgG1-F31 | 2.8E-06 | G237M/V308F |
| IgG1-F32 | 8.0E-07 | S254T/N434W |
| IgG1-F33 | 2.3E-06 | S254T/N434Y |
| IgG1-F34 | 2.8E-07 | T256E/N434W |
| IgG1-F35 | 8.4E-07 | T256E/N434Y |
| IgG1-F36 | 3.6E-07 | S254T/T256E/N434W |
| IgG1-F37 | 1.1E-06 | S254T/T256E/N434Y |
| IgG1-F38 | 1.0E-07 | M252Y/S254T/N434W |
| IgG1-F39 | 3.0E-07 | M252Y/S254T/N434Y |
| IgG1-F40 | 8.2E-08 | M252Y/T256E/N434W |

(continued)

| MUTANT NAME | KD (M) | AMINO ACID ALTERATION |
|---|---|---|
| IgG1-F41 | 1.5E-07 | M252Y/T256E/N434Y |
| IgG1-F42 | 1.0E-06 | M252Y/S254T/T256E/N434A |
| IgG1-F43 | 1.7E-06 | M252Y/N434A |
| IgG1-F44 | 1.1E-06 | M252W/N434A |
| IgG1-F47 | 2.4E-07 | M252Y/T256Q/N434W |
| IgG1-F48 | 3.2E-07 | M252Y/T256Q/N434Y |
| IgG1-F49 | 5.1E-07 | M252F/T256D/N434W |
| IgG1-F50 | 1.2E-06 | M252F/T256D/N434Y |
| IgG1-F51 | 8.1E-06 | N434F/Y436H |
| IgG1-F52 | 3.1E-06 | H433K/N434F/Y436H |
| IgG1-F53 | 1.0E-06 | I332V/N434W |
| IgG1-F54 | 8.4E-08 | V308P/N434W |
| IgG1-F56 | 9.4E-07 | I332V/M428L/N434Y |
| IgG1-F57 | 1.1E-05 | G385D/Q386P/N389S |
| IgG1-F58 | 7.7E-07 | G385D/Q386P/N389S/N434W |
| IgG1-F59 | 2.4E-06 | G385D/Q386P/N389S/N434Y |
| IgG1-F60 | 1.1E-05 | G385H |
| IgG1-F61 | 9.7E-07 | G385H/N434W |
| IgG1-F62 | 1.9E-06 | G385H/N434Y |
| IgG1-F63 | 2.5E-06 | N434F |
| IgG1-F64 | 5.3E-06 | N434H |

Table 6-2 is the continuation of Table 6-1.

[Table 6-2]

| IgG1-F65 | 2.9E-07 | M252Y/S254T/T256E/N434F |
|---|---|---|
| IgG1-F66 | 4.3E-07 | M252Y/S254T/T256E/N434H |
| IgG1-F67 | 6.3E-07 | M252Y/N434F |
| IgG1-F68 | 9.3E-07 | M252Y/N434H |
| IgG1-F69 | 5.1E-07 | M428L/N434W |
| IgG1-F70 | 1.5E-06 | M428L/N434Y |
| IgG1-F71 | 8.3E-08 | M252Y/S254T/T256E/M428L/N434W |
| IgG1-F72 | 2.0E-07 | M252Y/S254T/T256E/M428L/N434Y |
| IgG1-F73 | 1.7E-07 | M252Y/M428L/N434W |
| IgG1-F74 | 4.6E-07 | M252Y/M428L/N434Y |
| IgG1-F75 | 1.4E-06 | M252Y/M428L/N434A |
| IgG1-F76 | 1.0E-06 | M252Y/S254T/T256E/M428L/N434A |
| IgG1-F77 | 9.9E-07 | T256E/M428L/N434Y |
| IgG1-F78 | 7.8E-07 | S254T/M428L/N434W |
| IgG1-F79 | 5.9E-06 | S254T/T256E/N434A |

(continued)

| | | |
|---|---|---|
| IgG1-F80 | 2.7E-06 | M252Y/T256Q/N434A |
| IgG1-F81 | 1.6E-06 | M252Y/T256E/N434A |
| IgG1-F82 | 1.1E-06 | T256Q/N434W |
| IgG1-F83 | 2.6E-06 | T256Q/N434Y |
| IgG1-F84 | 2.8E-07 | M252W/T256Q/N434W |
| IgG1-F85 | 5.5E-07 | M252W/T256Q/N434Y |
| IgG1-F86 | 1.5E-06 | S254T/T256Q/N434W |
| IgG1-F87 | 4.3E-06 | S254T/T256Q/N434Y |
| IpG1-F88 | 1.9E-07 | M252Y/S254T/T256Q/N434W |
| IgG1-F89 | 3.6E-07 | M252Y/S254T/T256Q/N434Y |
| IgG1-F90 | 1.9E-08 | M252Y/T256E/V308P/N434W |
| IgG1-F91 | 4.8E-08 | M252Y/V308P/M428L/N434Y |
| IgG1-F92 | 1.1E-08 | M252Y/S254T/T256E/V308P/M428L/N434W |
| IgG1-F93 | 7.4E-07 | M252W/M428L/N434W |
| IgG1-F94 | 3.7E-07 | P257L/M428L/N434Y |
| IgG1-F95 | 2.6E-07 | M252Y/S254T/T256E/M428L/N434F |
| IgG1-F99 | 6.2E-07 | M252Y/T256E/N434H |

The variants each comprising a prepared heavy chain and L (WT) (SEQ ID NO: 5) were expressed and purified by methods known to those skilled in the art as described in Reference Example 2.

Assessment of human FcRn binding

The binding between antibody and human FcRn was kinetically analyzed using Biacore T100 (GE Healthcare). For this purpose, human FcRn was prepared as described in Reference Example 4. An appropriate amount of protein L (ACTIGEN) was immobilized onto Sensor chip CM4 (GE Healthcare) by the amino coupling method, and the chip was allowed to capture an antibody of interest. Then, diluted FcRn solutions and running buffer (as a reference solution) were injected to allow human FcRn to interact with the antibody captured on the sensor chip. The running buffer used comprised 50 mmol/l sodium phosphate, 150 mmol/l NaCl, and 0.05% (w/v) Tween20 (pH 7.0). FcRn was diluted using each buffer. The chip was regenerated using 10 mmol/l glycine-HCl (pH 1.5). Assays were carried out exclusively at 25 degrees C. The association rate constant ka (1/Ms) and dissociation rate constant $k_d$ (1/s), both of which are kinetic parameters, were calculated based on the sensorgrams obtained in the assays, and KD (M) of each antibody for human FcRn was determined from these values. Each parameter was calculated using Biacore T100 Evaluation Software (GE Healthcare).
The assessment result on the human FcRn binding under a neutral condition (pH 7.0) by Biacore is shown in Tables 6-1 and 6-2. The KD of the intact IgG1 could not be calculated because it exhibited only very weak binding. Thus, the KD is indicated as ND in Table 6-1.

[Example 6] In vivo test of pH-dependent human IL-6 receptor-binding antibodies with enhanced human FcRn binding under the neutral condition

pH-dependent human IL-6 receptor-binding antibodies having human FcRn binding ability under a neutral condition were produced using the heavy chains prepared as described in Example 4 to have human FcRn binding ability under a neutral condition. The antibodies were assessed for their in vivo antigen elimination effect. Specifically, the antibodies listed below were expressed and purified by methods known to those skilled in the art as described in Reference Example 2:

Fv4-IgG1 comprising VH3-IgG1 and VL3-CK;
Fv4-IgG1-v2 comprising VH3-IgG1-v2 and VL3-CK;
Fv4-IgG1-F14 comprising VH3-IgG1-F14 and VL3-CK;

Fv4-IgG1-F20 comprising VH3-IgG1-F20 and VL3-CK;
Fv4-IgG1-F21 comprising VH3-IgG1-F21 and VL3-CK;
Fv4-IgG1-F25 comprising VH3-IgG1-F25 and VL3-CK;
Fv4-IgG1-F29 comprising VH3-IgG1-F29 and VL3-CK;
Fv4-IgG1-F35 comprising VH3-IgG1-F35 and VL3-CK;
Fv4-IgG1-F48 comprising VH3-IgG1-F48 and VL3-CK;
Fv4-IgG1-F93 comprising VH3-IgG1-F93 and VL3-CK; and
Fv4-IgG1-F94 comprising VH3-IgG1-F94 and VL3-CK.

By the same methods described in Example 3, the prepared pH-dependent human IL-6 receptor-binding antibodies were tested in vivo using human FcRn transgenic mice (B6.mFcRn-/-.hFcRn Tg line 276 +/+ mouse, Jackson Laboratories; Methods Mol Biol. (2010) 602: 93-104).

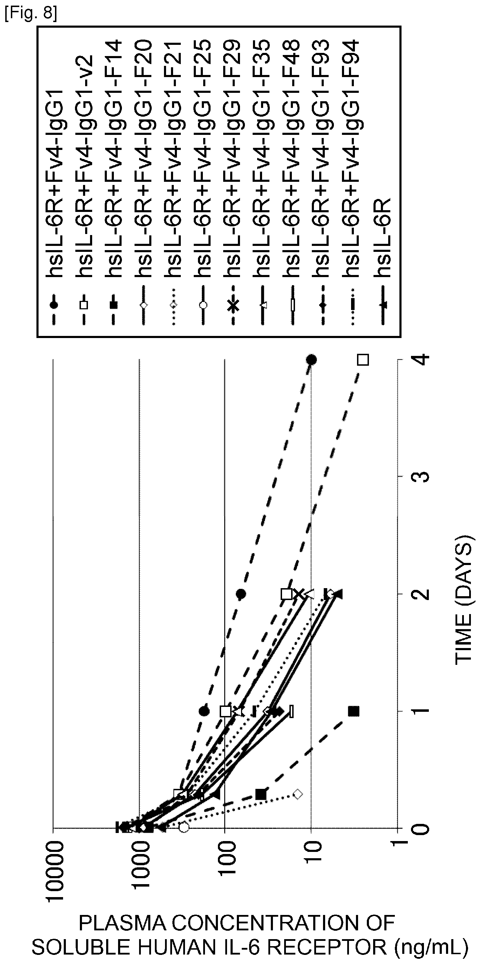

A time course of plasma concentration of soluble human IL-6 receptor after intravenous administration to human FcRn transgenic mice is shown in Fig. 8. The test result showed that the plasma concentration of soluble human IL-6 receptor remained low over time in the presence of any of the pH-dependent human IL-6 receptor-binding antibodies with augmented human FcRn binding under neutral condition, as compared to in the presence of Fv4-IgG1 which has almost no human FcRn binding ability under neutral condition. Among others, antibodies that produced the remarkable effect include, for example, Fv4-IgG1-F14. The plasma concentration of soluble human IL-6 receptor simultaneously administered with Fv4-IgG1-F14 was demonstrated to be reduced by about 54 times one day after administration as compared to that of soluble human IL-6 receptor simultaneously administered with Fv4- IgG 1. Furthermore, the plasma concentration of soluble human IL-6 receptor simultaneously administered with Fv4-IgG1-F21 was demonstrated to be reduced by about 24 times seven hours after administration as compared to that of soluble human IL-6 receptor simultaneously administered with Fv4-IgG1. In addition, the plasma concentration of soluble human IL-6 receptor simultaneously administered with Fv4-IgG1-F25 seven hours after administration was below the detection limit (1.56 ng/ml). Thus, Fv4-IgG1-F25 was expected to enable a remarkable reduction of 200 or more times in the concentration of soluble human IL-6 receptor relative to the concentration of soluble human IL-6 receptor simultaneously administered with Fv4-IgG1. The findings described above demonstrate that augmentation of the human FcRn binding of pH-dependent antigen-binding antibodies under a neutral condition is highly effective for enhancing the antigen elimination effect. Meanwhile, the type of amino acid alteration to augment human FcRn binding under neutral condition, which is introduced to enhance the antigen elimination effect, is not particularly limited; and such alterations include those shown in Tables 6-1 and 6-2. The antigen elimination effect can be predicted to be enhanced in vivo by any introduced alteration.

Furthermore, the plasma concentration of soluble human IL-6 receptor simultaneously administered with one of the four types of pH-dependent human IL-6 receptor-binding antibodies, Fv4-IgG1-F14, Fv4-IgG1-F21, Fv4-IgG1-F25, and Fv4-IgG1-F48, remained lower over time than that of soluble human IL-6 receptor administered alone. Such a pH-dependent human IL-6 receptor-binding antibody can be administered to the body where the plasma concentration of soluble human IL-6 receptor is kept constant (steady state) to keep the plasma concentration of soluble human IL-6 receptor lower than the steady-state concentration in plasma. Specifically, the in vivo antigen concentration in plasma can be reduced by administering such an antibody to the body.

[Example 7] Assessment for the effectiveness of low-dose (0.01 mg/kg) Fv4-IgG 1-F 14

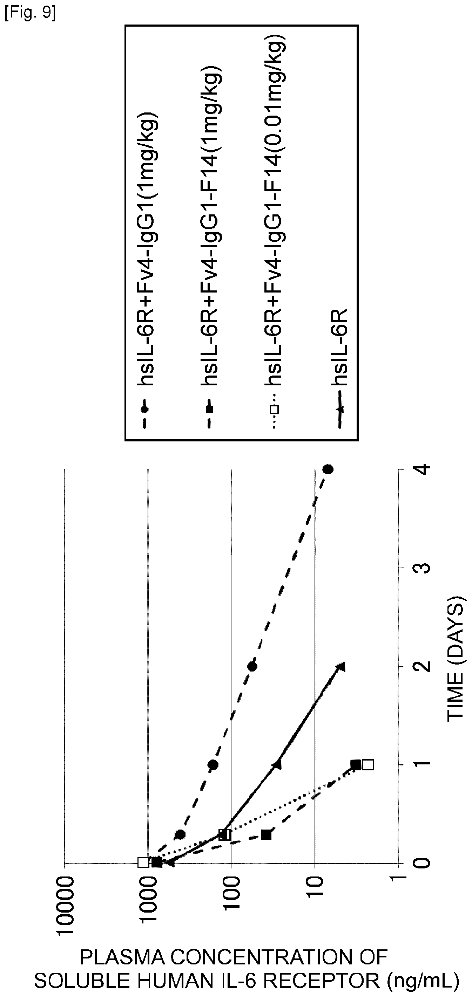

Fv4-IgG1-F14 prepared as described in Example 6 was tested at a low dose (0.01 mg/kg) by the same in vivo test method as described in Example 6. The result (shown in Fig. 9) was compared to that described in Example 6, which was obtained by administering Fv4-IgG1 and Fv4-IgG1-F14 at 1 mg/kg.

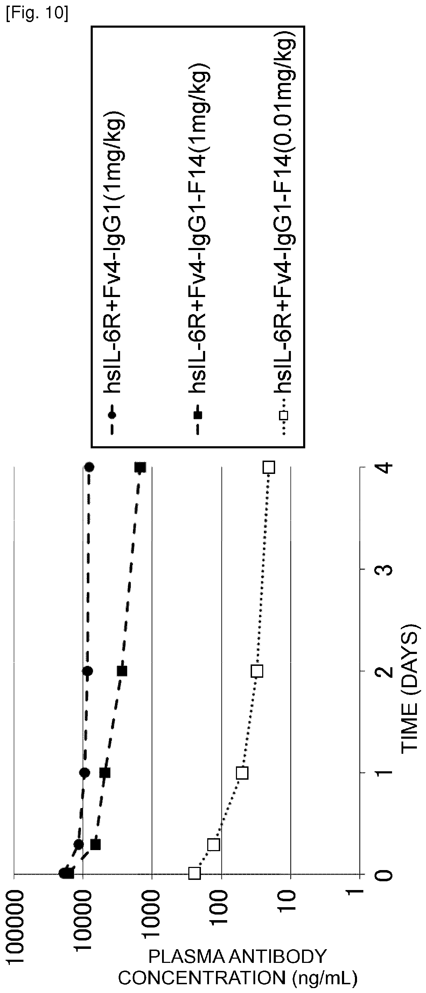

The result showed that although the plasma antibody concentration in the group administered with Fv4-IgG1-F14 at 0.01 mg/kg was about 100 times lower as compared to the group administered at 1 mg/kg (Fig. 10), the time courses of plasma concentration of soluble human IL-6 receptor were comparable to each other. In addition, it was demonstrated that the plasma concentration of soluble human IL-6 receptor seven hours after administration in the group administered with Fv4-IgG1-F14 at 0.01 mg/kg was reduced by about three times as compared to that in the group administered with Fv4-IgG1 at 1 mg/kg. Furthermore, in the presence of Fv4-IgG1-F14, the plasma concentration of soluble human IL-6 receptor was lower over time in both groups administered at different doses when compared to the group administered with soluble human IL-6 receptor alone.

The finding demonstrates that even when administered at a dose one-hundredth of that of Fv4-IgG1, Fv4-IgG1-F14 which results from modification of Fv4-IgG1 to augment human FcRn binding under a neutral condition effectively reduces the plasma concentration of soluble human IL-6 receptor. Specifically, it is predicted that antigens can be efficiently eliminated even at a lower dose when a pH-dependent antigen-binding antibody is modified to augment its FcRn-binding ability under neutral condition.

[Example 8] In vivo test based on the steady-state model using normal mice

Assessment of the binding to mouse FcRn under neutral condition

VH3/L (WT)-IgG1 comprising VH3-IgG1 (SEQ ID NO: 6) and L (WT) (SEQ ID NO: 5), VH3/L (WT)-IgG1-v2 comprising VH3-IgG1-v2 (SEQ ID NO: 9) and L (WT) (SEQ ID NO: 5), and VH3/L (WT)-IgG1-F20 comprising VH3-IgG1-F20 (SEQ ID NO: 10) and L (WT) (SEQ ID NO: 5), all of which were prepared as described in Example 5, were assessed for mouse FcRn binding under a neutral condition (pH 7.4) by the method described below.

The binding between antibody and mouse FcRn was kinetically analyzed using Biacore T100 (GE Healthcare). An appropriate amount of protein L (ACTIGEN) was immobilized onto Sensor chip CM4 (GE Healthcare) by the amino coupling method, and the chip was allowed to capture an antibody of interest. Then, diluted FcRn solutions and running buffer (as a reference solution) were injected to allow mouse FcRn to interact with the antibody captured on the sensor chip. The running buffer used contains 50 mmol/l sodium phosphate, 150 mmol/l NaCl, and 0.05% (w/v) Tween20 (pH 7.4). FcRn was diluted using each buffer. The chip was regenerated using 10 mmol/l glycine-HCl (pH 1.5). Assays were carried out exclusively at 25 degrees C. The association rate constant ka (1/Ms) and dissociation rate constant $k_d$ (1/s), both of which are kinetic parameters, were calculated based on the sensorgrams obtained in the assays, and the KD (M) of each antibody for mouse FcRn was determined from these values. Each parameter was calculated using Biacore T100 Evaluation Software (GE Healthcare).

The result is shown in Table 7 (affinity for mouse FcRn at pH 7.4). VH3/L (WT)-IgG1 (IgG1 in Table 7) whose constant region is of the intact IgG1 exhibited only very weak binding to mouse FcRn. Thus, the KD could not be calculated and is indicated as ND in Table 7. The assay result showed that the altered antibodies with enhanced human FcRn binding under neutral condition also exhibited augmented binding to mouse FcRn under the neutral condition.

[Table 7]

|          | KD (M)   |
|----------|----------|
| IgG1     | ND       |
| IgG1-v2  | 1.04E-06 |
| IgG1-F20 | 1.17E-07 |

In vivo test using normal mice with a constant plasma concentration of soluble human IL-6 receptor

Using H54/L28-IgG1, Fv4-IgG1, Fv4-IgG1-v2, and Fv4-IgG1-F20 prepared as described in Examples 1 and 5, an in vivo test was conducted by the method described below.

In vivo infusion test using normal mice

An infusion pump (MINI-OSMOTIC PUMP MODEL 2004; alzet) containing soluble human IL-6 receptor was implanted under the skin on the back of normal mice (C57BL/6J mice; Charles River Japan) to prepare model animals where the plasma concentration of soluble human IL-6 receptor was kept constant. Anti-human IL-6 receptor antibodies were administered to the model animals to assess the in vivo dynamics after administration of soluble human IL-6 receptor. Monoclonal anti-mouse CD4 antibody (R&D) was administered at 20 mg/kg once into the caudal vein to suppress the production of neutralizing antibody against soluble human IL-6 receptor. Then, an infusion pump containing 92.8 microgram/ml soluble human IL-6 receptor was implanted under the skin on the back of the mice. Three days after implantation of an infusion pump, anti-human IL-6 receptor antibodies were administered at 1 mg/kg once into the caudal vein. Blood was collected 15 minutes, seven hours, one day, two days, three days, four days, seven days, 14 days, 21 days, and 28 days after administration of the anti-human IL-6 receptor antibody. The collected blood was immediately centrifuged at 15,000 rpm and 4 degrees C for 15 minutes to separate plasma. The separated plasma was stored in a refrigerator at or below -20 degrees C before assay.

Determination of plasma concentration of anti-human IL-6 receptor antibodies by ELISA

The method used was the same as described in Example 3.

Determination of plasma hsIL-6R concentration by electrochemiluminescence assay The method used was the same as described in Example 1.

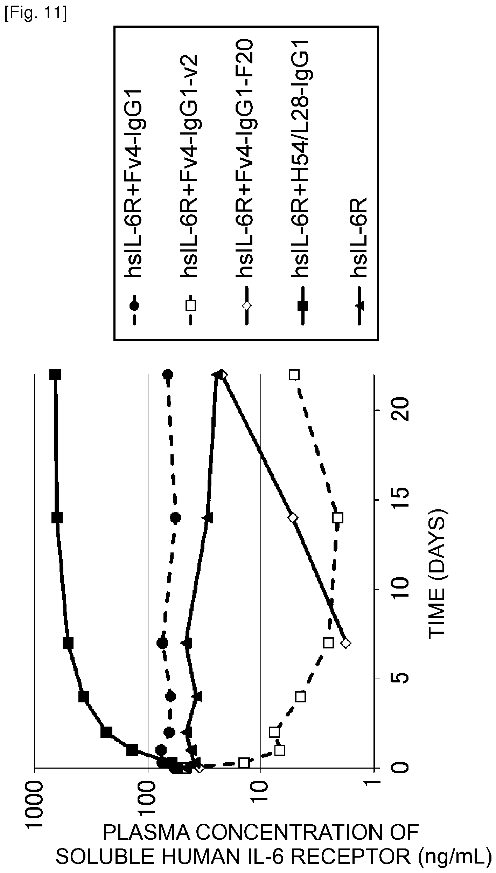

As shown in Fig. 11, the plasma concentration of soluble human IL-6 receptor was elevated to 650 ng/ml (15 times before administration) when H54/L28-IgG1, a neutralizing antibody against soluble human IL-6 receptor, was administered to normal mice (hsIL-6R group) in which the plasma concentration of soluble human IL-6 receptor was kept constantly at about 40 ng/ml. On the other hand, the plasma concentration of soluble human IL-6 receptor was maintained at about 70 ng/ml in the group administered with Fv4-IgG1 which results from conferring H54/L28-IgG1 with a pH-dependent antigen binding ability. This suggests that the increase in the plasma concentration of soluble human IL-6 receptor caused by administration of H54/L28-IgG1, an ordinary neutralizing antibody, can be suppressed to about one tenth by conferring the pH-dependent binding ability.

Furthermore, the plasma concentration of soluble human IL-6 receptor was demonstrated to be maintained at or below one tenth of the steady-state concentration by administering Fv-IgG1-v2 or Fv-IgG1-F20, both of which resulted from introducing a alteration into a pH-dependent human IL-6 receptor-binding antibody to augment the FcRn binding under neutral condition. When Fv-IgG1-v2 was administered, the plasma concentration of soluble human IL-6 receptor 14 days after administration was about 2 ng/ml. Thus, Fv-IgG1-v2 could reduce the concentration to 1/20 of the level before administration. Meanwhile, when Fv-IgG1-F20 was administered, the plasma concentrations of soluble human IL-6 receptor seven hours, one day, two days, and four days after administration were below the detection limit (1.56 ng/ml). This suggests that Fv-IgG1-F20 reduced the concentration to or below 1/25 of the level before administration.

The findings described above demonstrate that the plasma antigen concentration can be significantly reduced by increasing the antigen elimination rate in plasma, by administering an antibody having both pH-dependent antigen-binding ability and FcRn-binding ability under the neutral condition to model animals in which the plasma antigen concentration is kept constant.

Typical antibodies such as H54/L28-IgG1 can only neutralize the action of a target antigen by binding to the target antigen, and even worse they increase the plasma antigen concentration. By contrast, antibodies having both pH-dependent antigen-binding ability and FcRn-binding ability under neutral condition were found to be able to not only neutralize the target antigen but also reduce the plasma concentration of the target antigen. The effect of antigen removal from the plasma can be expected to be more beneficial than neutralization. In addition, antigen removal can also work for target antigens that are insufficiently effective by neutralization alone.

[Example 9] Identification of threshold of the binding affinity to human FcRn at neutral pH required to enhance antigen elimination and relationship between antigen elimination and the binding affinity to human FcRn at neutral pH

Antibody preparation for in vivo study

Fc variants of Fv4-IgG1 comprising VH3-IgG1 (SEQ ID NO: 6) and VL3-CK (SEQ ID NO: 7) with increased FcRn binding under the neutral pH were generated. Specifically, VH3-M73 (SEQ ID NO: 15) and VH3-IgG1-v1 (SEQ ID NO: 8) was prepared. The amino acid substitutions were introduced by methods known to those skilled in the art as described in Reference Example 1.

H54/L28- IgG 1 comprising H54 (SEQ ID NO: 1) and L28 (SEQ ID NO: 2), Fv4-IgG1 comprising VH3-IgG1 (SEQ ID NO: 6) and VL3-CK (SEQ ID NO: 7), Fv4-M73 comprising VH3-M73 (SEQ ID NO: 15) and VL3-CK (SEQ ID NO: 7), Fv4-IgG1-v1 comprising VH3-IgG1-v1 (SEQ ID NO: 8) and VL3-CK (SEQ ID NO: 7), and Fv4-IgG1-v2 comprising VH3-IgG1-v2 (SEQ ID NO: 9) and VL3-CK (SEQ ID NO: 7), were expressed and purified by the method known to those skilled in the art described in Reference Example 2.

Assessment of the binding affinity of antibodies to human FcRn under neutral pH condition

VH3/L (WT)-IgG1 comprising VH3-IgG1 (SEQ ID NO: 6) and L (WT) (SEQ ID NO: 5), VH3/L (WT)-M73 comprising VH3-M73 (SEQ ID NO: 15) and L (WT) (SEQ ID NO: 5), VH3/L (WT)-IgG1-v1 comprising VH3-IgG1-v1 (SEQ ID NO: 8) and L (WT) (SEQ ID NO: 5), and VH3/L (WT)-IgG1-v2 comprising VH3-IgG1-v2 (SEQ ID NO: 9) and L (WT) (SEQ ID NO: 5), all of which were prepared as described in Example 2, were assessed for human FcRn binding under a neutral pH (pH 7.0).

The binding activity of VH3/L (WT)-IgG1-v1 and VH3/L (WT)-IgG1-v2 to human FcRn was measured using the method described in Example 5. Due to the low binding activity of VH3/L (WT)-IgG1 and VH3/L (WT)-M73 to human FcRn, binding activity to human FcRn could not be measured using the method described in Example 5, therefore, these antibodies were assessed by the method described below. The binding between antibody and human FcRn was kinetically analyzed using Biacore T100 (GE Healthcare). An appropriate amount of protein L (ACTIGEN) was immobilized onto Sensor chip CM4 (GE Healthcare) by the amine-coupling method, and the chip was allowed to capture an antibody of interest. Then, diluted FcRn solutions and running buffer as a reference solution were injected to allow for human FcRn to interact with the antibody captured on the sensor chip. The running buffer used comprised 50 mmol/ 1 sodium phosphate, 150 mmol/l NaCl, and 0.05% (w/v) Tween20 (pH 7.0). FcRn was diluted using each buffer. The chip was regenerated using 10 mmol/l glycine-HCl (pH 1.5). Assays were carried out at 25 degrees C.

KD (M) of each antibody was derived from the sensorgram data using Biacore T100 Evaluation Software (GE Healthcare), which simultaneously fits the association and dissociation phases of the sensorgrams and globally fits all curves in the working set. Sensorgrams were fit to 1:1 binding model, the "Langmuir binding" model, supplied by Biacore T100 Evaluation Software. For some of the binding interactions, KD was derived by nonlinear regression analysis of plots of $R_{eq}$, the equilibrium binding response, versus the log of the analyte concentration using an equilibrium-based approach.

The result on the human FcRn binding under the neutral condition (pH 7.0) by Biacore is shown in Tables 8.

[Table 8]

|  | KD (M) |
|---|---|
| IgG1 | 8.8E-05 |
| M73 | 1.4E-05 |
| IgG1-v1 | 3.2E-06 |
| IgG1-v2 | 8.1E-07 |

In vivo studies of effect of antibodies on antigen elimination in co-injection model using human FcRn transgenic mouse line 276

In vivo study of antibodies using co-injection model was performed as described in Example 3. Anti-human IL-6 receptor antibodies used in this study are the above-described H54/L28-IgG1, Fv4-IgG1, Fv4-M73, Fv4-IgG1-v1 and Fv4-IgG1-v2. Mice used in this study is human FcRn transgenic mice (B6.mFcRn-/-.hFcRn Tg line 276 +/+ mouse, Jackson Laboratories; Methods Mol Biol. (2010) 602: 93-104).

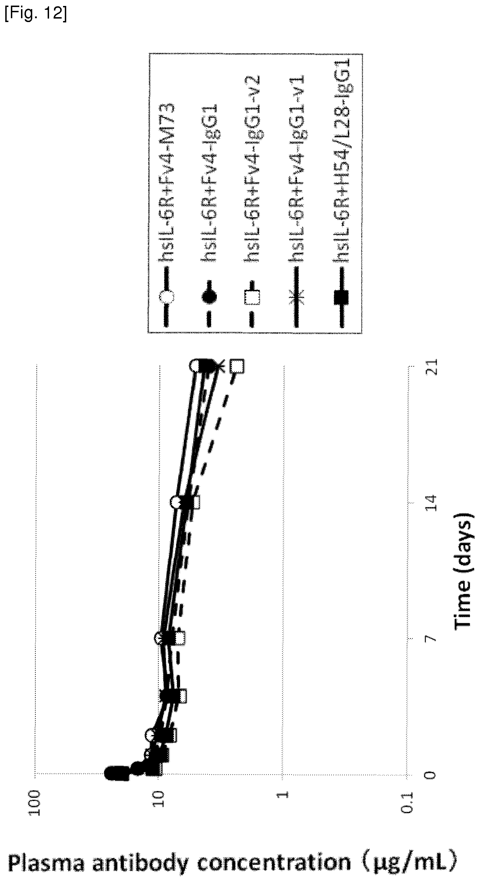

As shown in Fig. 12, pharmacokinetics of H54/L28-IgG1, Fv4-IgG1, Fv4-M73, Fv4-IgG1-v1 and Fv4-IgG1-v2 were comparable, and these antibodies maintained similar plasma concentration during the study.

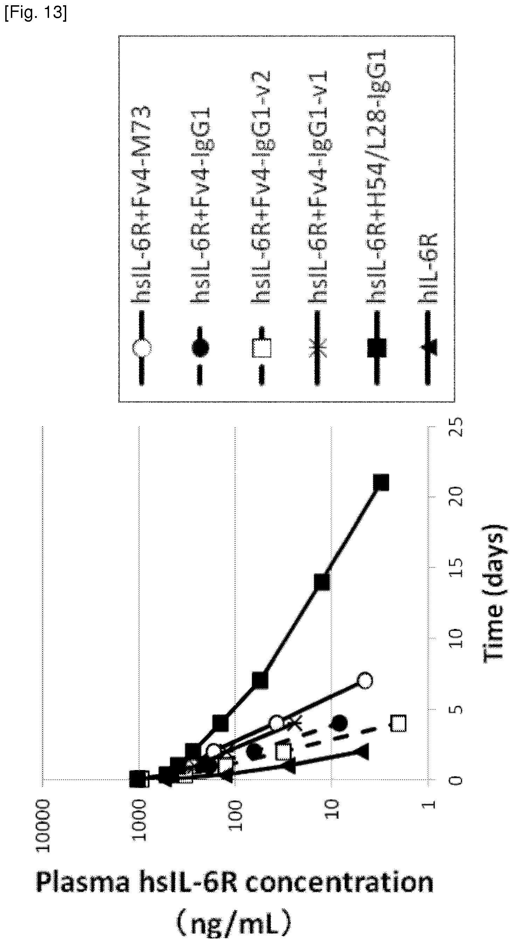

Time course of plasma hsIL-6R concentration was show in Fig. 13. Compared to the hsIl-6R administered with Fv4-IgG1, hsIL-6R administered with Fv4-IgG1-v2 exhibited enhanced clearance, whereas hsIL-6R administered with Fv4-M73 and Fv4-IgG1-v1 exhibited reduced clearance. Although all Fc variant, M73, v1, and v2 have increased binding affinity to human FcRn at neutral pH condition (pH 7.0), it was demonstrated that only Fv4-IgG1-v2, but not Fv4-M73 and Fv4-IgG1-v1, exhibited enhanced hsIL-6R clearance. This indicates that in order to enhance antigen clearance, binding affinity of antibody to human FcRn at pH 7.0 needs to be at least stronger than IgG1-v1, whose binding affinity to human FcRn at pH 7.0 is KD 3.2 micromolar or 28-fold stronger than intact human IgG1 (binding affinity to human FcRn is KD 88 micromolar).

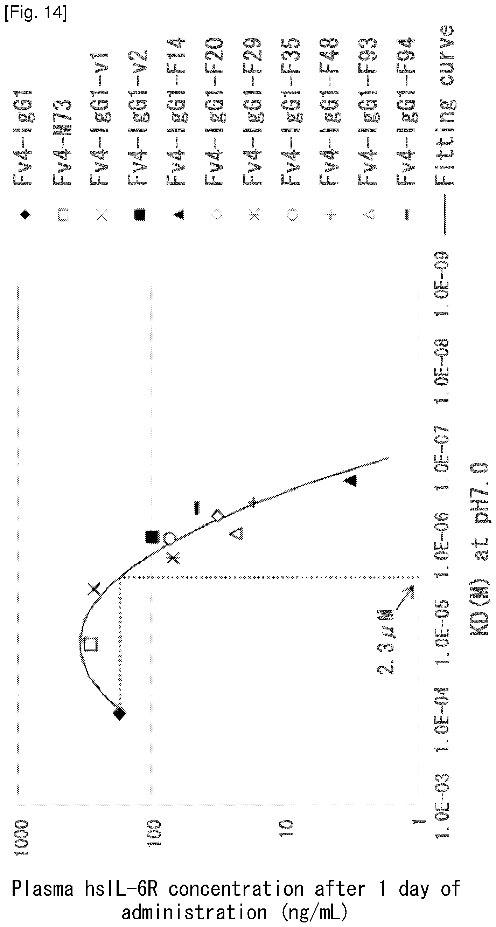

Fig. 14 describes the relationship between the binding affinity of Fc variants to human FcRn at pH7.0 and plasma hsIL-6R concentration at day 1 after co-injetion of hsIL-6R and Fc variants. Fc variants described in this Example and Example 6 (Fv4-IgG1, Fv4-M73, Fv4-IgG1-v1, Fv4-IgG1-v2, Fv4-IgG1-F14, Fv4-IgG1-F20, Fv4-IgG1-F21, Fv4-IgG1-F25, Fv4-IgG1-F29, Fv4-IgG1-F35, Fv4-IgG1-F48, Fv4-IgG1-F93, and Fv4-IgG1-F94) are plotted. By increasing the binding affinity of antibody to human FcRn at pH7.0, plasma concentration of hsIL-6R, which reflects the clearance of antigen, increased at first, but then decreased rapidly. This demonstrates that in order to enhance the antigen clearance compared to intact human IgG1, binding affinity of antibody to human FcRn at pH 7.0 needs to be preferably stronger than KD 2.3 micromolar (value obtained from curve fitting of Fig. 14). Binding affinity of antibody to human FcRn between KD 88 micromolar and KD 2.3 micromolar would rather reduce the antigen clearance (higher hsIL-6R concentration). In other words, binding affinity of antibody to human FcRn at pH 7.0 needs to be preferably 38-fold stronger than intact human IgG1 to enhance antigen elimination, or otherwise would reduce the antigen clearance.

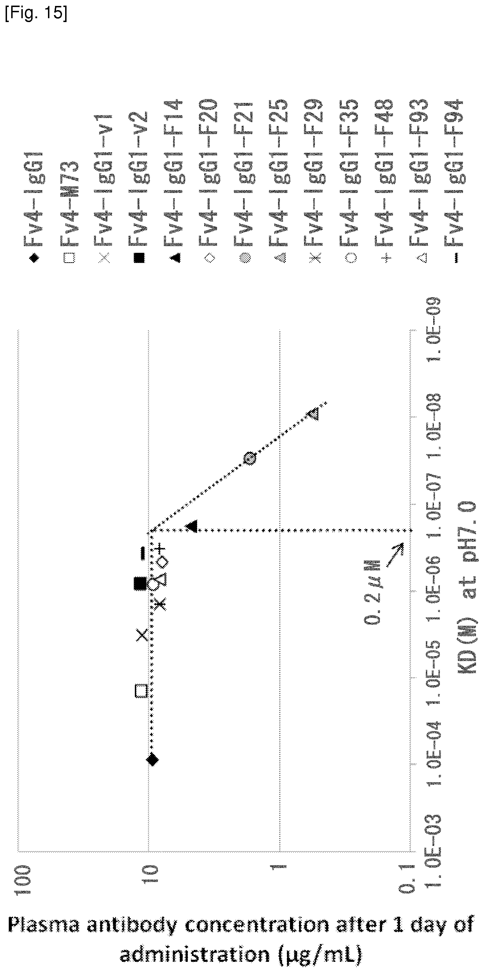

Fig. 15 describes the relationship between the binding affinity of Fc variants to human FcRn at pH 7.0 and plasma antibody concentration at day 1 after co-injection of hsIL-6R and Fc variants. Fc variants described in this Example and Example 6 (Fv4-IgG1, Fv4-M73, Fv4-IgG1-v1, Fv4-IgG1-v2, Fv4-IgG1-F14, Fv4-IgG1-F20, Fv4-IgG1-F21, Fv4-IgG1-F25, Fv4-IgG1-F29, Fv4-IgG1-F35, Fv4-IgG1-F48, Fv4-IgG1-F93, and Fv4-IgG1-F94) are plotted. By increasing the binding affinity of antibody to human FcRn at pH 7.0, plasma concentration of antibody, which reflects antibody pharmacokinetics (clearance), is maintained at first, but then decreased rapidly. This demonstrates that in order to maintain pharmacokinetics of antibody similar to intact human IgG1 (binding affinity to human FcRn is KD 88 micromolar), affinity of antibody to human FcRn at pH 7.0 needs to be weaker than KD 0.2 micromolar (value obtained from curve fitting of Fig. 15). Binding affinity of antibody to human FcRn stronger than KD 0.2 micromolar increased the antibody clearance (i.e. more rapid antibody elimination from plasma). In other words, binding affinity of antibody to human FcRn at pH 7.0 needs to be within 440-fold stronger than intact human IgG1 to exhibit similar antibody pharmacokinetics as intact human IgG1, or otherwise would result in rapid antibody elimination from plasma.

Considering both Figs. 14 and 15, in order to enhance antigen clearance (i.e. reduce antigen plasma concentration) compared to IgG1, while maintaining antibody pharmacokinetics similar to intact human IgG1, binding affinity of antibody to human FcRn at pH 7.0 needs to be between 2.3 micromolar and 0.2 micromolar, or in other words, binding affinity of antibody to human FcRn at pH 7.0 needs to be within a range of 38-fold to 440-fold stronger than intact human IgG1. Such antibody with similar pharmacokinetics as IgG1 with long-term antigen-elimination activity would be beneficial for antibody therapeutic which requires longer dosing interval such as chronic disease because of its long-acting property.

On the other hand, by increasing the binding affinity of antibody to human FcRn at pH 7.0 stronger than KD 0.2 micromolar, or in other words, by increasing the binding affinity of antibody to human FcRn at pH 7.0 more than 440-fold as compared to intact human IgG1, it would enhance antigen clearance to a large extent within a short-term, although antibody is eliminated from plasma faster than intact human IgG1. Such antibody with capability of inducing rapid and strong reduction of antigen concentration would be beneficial for antibody therapeutic such as acute disease in which disease related antigen needs to be removed from plasma because of its fast-acting property.

Amount of antigen eliminated from plasma per antibody is the important factor to evaluate the efficiency of antigen elimination by administrating the antibody Fc variants having increased binding affinity to human FcRn at pH 7.0. To evaluate the efficiency of antigen elimination per antibody, following calculation were conducted at each time point of in vivo study described in this Example and Example 6.

value A: Molar antigen concentration at each time point
value B: Molar antibody concentration at each time point
value C: Molar antigen concentration per molar antibody concentration (molar antigen/antibody ratio) at each time point

$$C = A/B$$

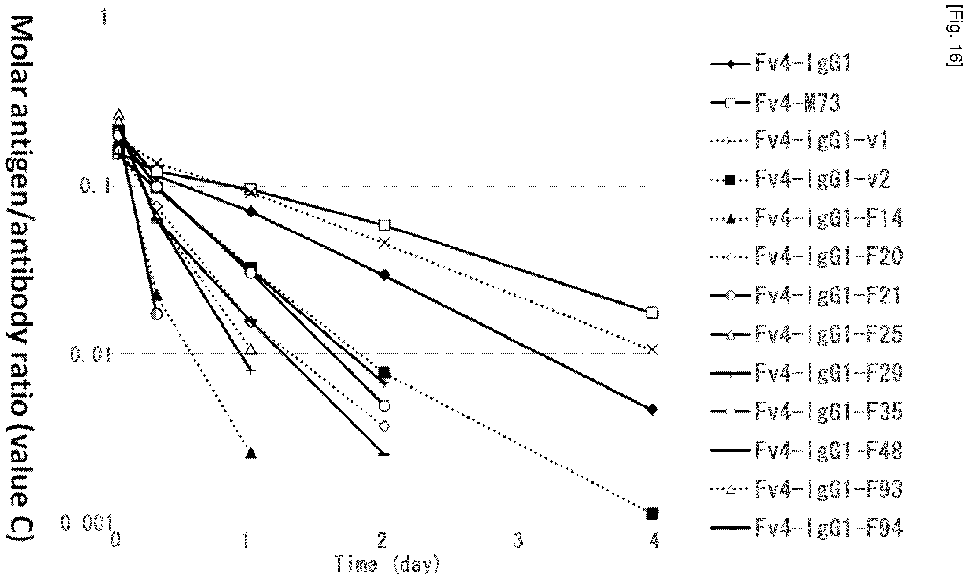

Time courses of value C (molar antigen/antibody ratio) for each antibody were described in Fig. 16. Smaller value C indicates higher efficiency of antigen elimination per antibody whereas higher value C indicates lower efficiency of antigen elimination per antibody. Lower value C as compared to IgG1 indicates that higher antigen elimination efficiency was achieved by Fc variants, whereas higher value C as compared to IgG1 indicates that Fc variants have negative effect on antigen elimination efficiency. All the Fc variants except Fv4-M73 and Fv4-IgG1-v1 demonstrated enhanced antigen elimination efficiency as compared to Fv4-IgG1. Fv4-M73 and Fv4-IgG1-v1 demonstrated negative impact on antigen elimination efficiency, which was consistent with Fig. 14.

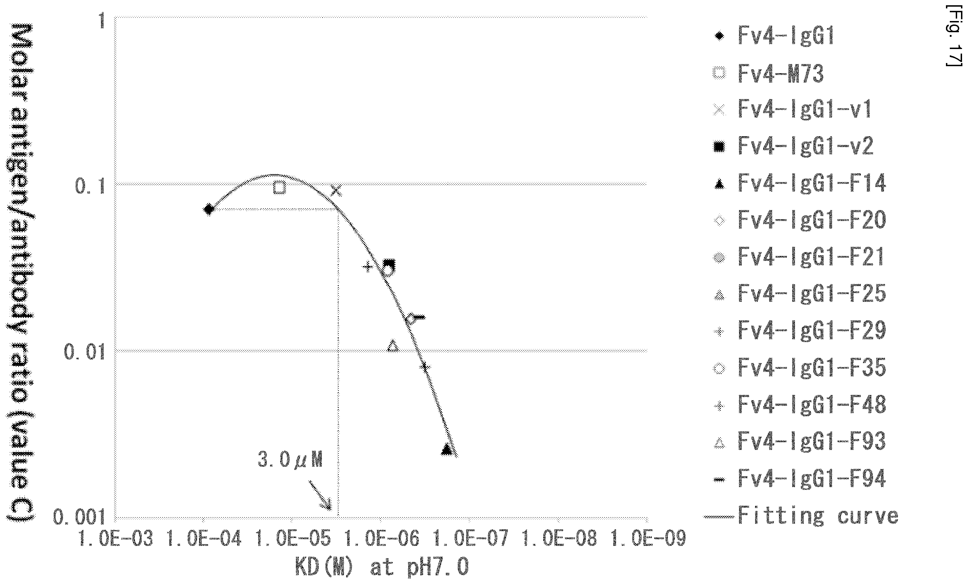

Fig. 17 describes the relationship between the binding affinity of Fc variants to human FcRn at pH 7.0 and value C (molar antigen/antibody ratio) at day 1 after co-injection of hsIL-6R and Fc variants. Fc variants described in this Example and Example 6 (Fv4-IgG1, Fv4-M73, Fv4-IgG1-v1, Fv4-IgG1-v2, Fv4-IgG1-F14, Fv4-IgG1-F20, Fv4-IgG1-F21, Fv4-IgG1-F25, Fv4-IgG1-F29, Fv4-IgG1-F35, Fv4-IgG1-F48, Fv4-IgG1-F93, and Fv4-IgG1-F94) are plotted. This demonstrates that in order to achieve higher antigen elimination efficiency as compared to intact human IgG1, affinity of antibody to human FcRn at pH 7.0 needs to be stronger than KD 3.0 micromolar (value obtained from curve fitting of Fig. 17). In other words, binding affinity of antibody to human FcRn at pH 7.0 needs to be at least 29-fold stronger than intact human IgG1 to achieve higher antigen elimination efficiency as compared to intact human IgG1.

In conclusion, group of antibody variants having binding affinity to FcRn at pH 7.0 between KD 3.0 micromolar and 0.2 micromolar, or in other words, group of antibody variants having binding affinity to FcRn at pH 7.0 within a range of 29-fold to 440-fold stronger than intact human IgG1, have similar antibody pharmacokinetics to IgG1 but have enhanced capability to eliminate the antibody from plasma. Therefore, such antibody exhibits enhanced antigen elimination efficiency as compared to IgG1. Similar pharmacokinetics as IgG1 would enable long-term elimination of antigen from plasma (long-acting antigen elimination), and therefore long dosing intervals which would be preferable for antibody therapeutics for chronic disease. Group of antibody variants having binding affinity to FcRn at pH 7.0 stronger than KD 0.2 micromolar, or in other words, group of antibody variants having binding affinity to FcRn at pH 7.0 440-fold stronger than intact human IgG1, have rapid antibody clearance (short-term antibody elimination). Nevertheless, since such antibody enables even more rapid clearance of antigen (fast-acting antigen elimination), therefore, such antibody also exhibits enhanced antigen elimination efficiency as compared to IgG1. As shown in Example 8, Fv4-IgG1-F20 in normal mouse would induce extensive elimination of the antigen from plasma in a very short term, but the antigen elimination effect is not durable. Such profile would be preferable for acute diseases where disease related antigen is needed to be depleted from plasma rapidly and extensively in a very short term.

[Example 10] In vivo study of Fv4-IgG1-F14 by steady-state infusion model using human FcRn transgenic mouse line 276

In vivo study of Fv4-IgG1-F14 by steady-state infusion model using human FcRn transgenic mouse line 276 was performed as described in Example 1. Study group consists of control group (without antibody), Fv4-IgG1 at a dose of 1 mg/kg and Fv4-IgG1-F14 at a dose of 1 mg/kg, 0.2 mg/kg, and 0.01 mg/kg.

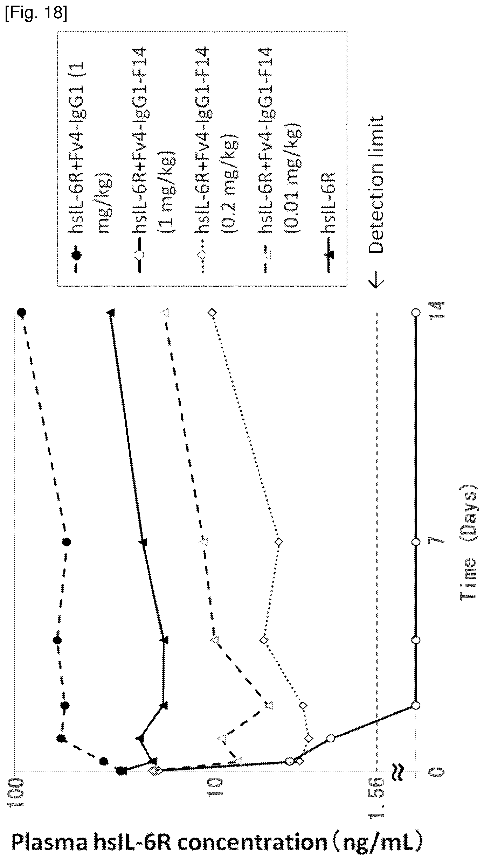

Fig. 18 describes time profile of hsIL-6R plasma concentration after antibody administration. Compared to baseline hsIL-6R level without antibody, administration of 1 mg/kg of Fv4-IgG1 resulted in several fold increase in plasma hsIL-6R concentration. On the other hands, administration of 1 mg/kg of Fv4-IgG1-F14 resulted in significant reduction in plasma concentration in comparison with Fv4-IgG1 group and baseline group. At day 2, plasma hsIL-6R concentration was not detected (quantitation limit of plasma hsIL-6R concentration is 1.56 ng/mL in this measurement system), and this lasted up to day 14.

As shown in Example 1, H54/L28-IgG1-F14 exhibited reduction of plasma hsIL-6R concentration as compared to H54/L28-IgG1, but the extent of the reduction was small. Extent of reduction was much higher for Fv4 variable region which has pH dependent binding property to hsIL-6R. This demonstrates that although increasing binding affinity to human FcRn at pH 7.0 is effective for reducing plasma antigen concentration, combination of pH dependent antigen binding and increased binding affinity to human FcRn at neutral pH significantly enhances the antigen elimination.

Study using lower dose of Fv4-IgG1-F14 exhibited that even at 0.01 mg/kg, 1/100 of 1 mg/kg, reduced the antigen plasma concentration below the baseline demonstrating significant efficiency of the molecule to deplete the antigen from plasma.

[Example 11] Comparison of human FcRn transgenic mouse line 276 and line 32 in co-injection model

Previous in vivo studies have been conducted using human FcRn transgenic mouse line 276 (Jackson Laboratories). In order to compare the difference between human FcRn transgenic mouse line 276 and a different transgenic line, line 32, we conducted co-injection study of H54/L28-IgG1, Fv4-IgG1, and Fv4-IgG1-v2 using human FcRn transgenic mouse line 32 (B6.mFcRn-/-.hFcRn Tg line 32 +/+ mouse (B6.mFcRn-/- hFCRN Tg32; B6.Cg-Fcgrt<tm1Dcr> Tg(FCGRT)32Dcr) (Jackson #4915)), Jackson Laboratories; Methods Mol Biol. (2010) 602: 93-104). Study method was same as that of Example 3 but human FcRn transgenic mouse line 32 was used instead of human FcR n transgenic mouse line 276.

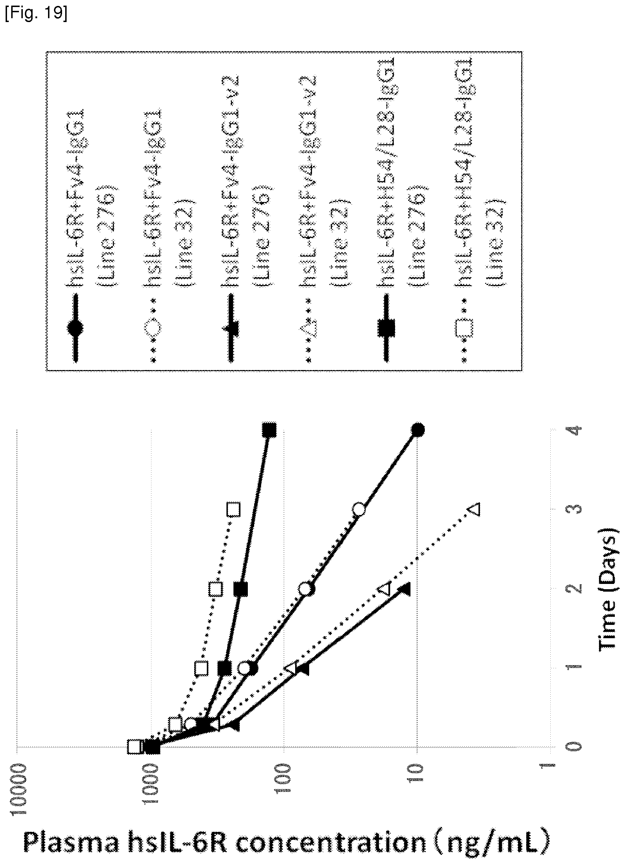

Fig. 19 describes the time course of plasma hsIL-6R concentration in both human FcRn transgenic mouse line 276 and line 32. H54/L28-IgG1, Fv4-IgG1, and Fv4-IgG1-v2 exhibited similar plasma hsIL-6R concentration time profile. In both mice, increasing binding affinity to human FcRn at pH 7.0 enhanced the antigen elimination from plasma (comparing Fv4-IgG1 and Fv4-IgG1-v2) to a same extent.

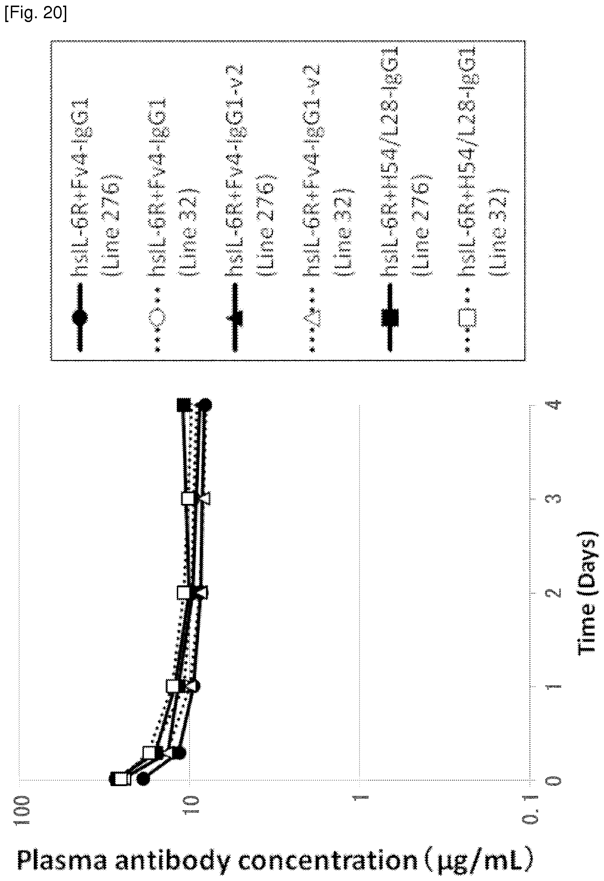

Fig. 20 describes the time course of plasma antibody concentration in both human FcRn transgenic mouse line 276 and line 32. H54/L28-IgG1, Fv4-IgG1, and Fv4-IgG1-v2 exhibited similar plasma antibody concentration time profile.

In conclusion, no significant difference were observed between line 276 and line 32, demonstrating that the Fc variant to increase the binding affinity to human FcRn at pH 7.0 was effective in two different transgenic mouse line expressing human FcRn for enhancing elimination of antigen plasma concentration.

[Example 12] Generation of various antibody Fc variants having increased binding affinity to human FcRn at neutral pH

Generation of Fc variants

Various mutations to increase the binding affinity to human FcRn under the neutral pH were introduced into Fv4-IgG1 to further improve the antigen elimination profile. Specifically, the amino acid mutations shown in Tables 9-1 to 9-14, were introduced into the heavy chain constant region of Fv4-IgG1 to generate Fc variants (amino acid numbers of the mutation sites are described according to the EU numbering). The amino acid substitutions were introduced by the method known to those skilled in the art described in Reference Example 1.

The additional variants (IgG1-F100 to IgG1-F599) each comprising a prepared heavy chain and L (WT) (SEQ ID NO: 5) were expressed and purified by methods known to those skilled in the art as described in Reference Example 2.

Assessment of human FcRn binding

The binding between antibody and human FcRn was kinetically analyzed as described in example 5 for IgG1-v1, IgG1-v2 and IgG1-F2 to IgG1-F599 or Example 9 for IgG1 and M73. The result on the human FcRn binding under a neutral condition (pH 7.0) by Biacore is shown in Tables 9-1 to 9-14.

[Table 9-1]

| VARIANT NAME | KD (M) | AMINO ACID SUBSTITUTION |
|---|---|---|
| IgG1 | 8.8E-05 | None |
| M73 | 1.4E-05 | (WO2009/125825) |
| IgG1-v1 | 3.2E-06 | M252Y/S254T/T256E |
| IgG1-v2 | 8.1E-07 | N434W |
| IgG1-F3 | 2.5E-06 | N434Y |
| IgG1-F4 | 5.8E-06 | N434S |
| IgG1-F5 | 6.8E-06 | N434A |
| IgG1-F7 | 5.6E-06 | M252Y |
| IgG1-F8 | 4.2E-06 | M252W |
| IgG1-F9 | 1.4E-07 | M252Y/S254T/T256E/N434Y |
| IgG1-F10 | 6.9E-08 | M252Y/S254T/T256E/N434W |
| IgG1-F11 | 3.1E-07 | M252Y/N434Y |
| IgG1-F12 | 1.7E-07 | M252Y/N434W |
| IgG1-F13 | 3.2E-07 | M252W/N434Y |
| IgG1-F14 | 1.8E-07 | M252W/N434W |
| IgG1-F19 | 4.6E-07 | P257L/N434Y |
| IgG1-F20 | 4.6E-07 | V308F/N434Y |
| IgG1-F21 | 3.0E-08 | M252Y/V308P/N434Y |
| IgG1-F22 | 2.0E-06 | M428L/N434S |
| IgG1-F25 | 9.2E-09 | M252Y/S254T/T256E/V308P/N434W |
| IgG1-F26 | 1.0E-06 | I332V |
| IgG1-F27 | 7.4E-06 | G237M |
| IgG1-F29 | 1.4E-06 | I332V/N434Y |
| IgG1-F31 | 2.8E-06 | G237M/V308F |
| IgG1-F32 | 8.0E-07 | S254T/N434W |
| IgG1-F33 | 2.3E-06 | S254T/N434Y |
| IgG1-F34 | 2.8E-07 | T256E/N434W |
| IgG1-F35 | 8.4E-07 | T256E/N434Y |
| IgG1-F36 | 3.6E-07 | S254T/T256E/N434W |
| IgG1-F37 | 1.1E-06 | S254T/T256E/N434Y |
| IgG1-F38 | 1.0E-07 | M252Y/S254T/N434W |
| IgG1-F39 | 3.0E-07 | M252Y/S254T/N434Y |

Table 9-2 is the continuation of Table 9-1.

[Table 9-2]

| IgG1-F40 | 8.2E-08 | M252Y/T256E/N434W |
|---|---|---|
| IgG1-F41 | 1.5E-07 | M252Y/T256E/N434Y |
| IgG1-F42 | 1.0E-06 | M252Y/S254T/T256E/N434A |
| IgG1-F43 | 1.7E-06 | M252Y/N434A |

(continued)

| IgG1-F44 | 1.1E-06 | M252W/N434A |
|---|---|---|
| IgG1-F47 | 2.4E-07 | M252Y/T256Q/N434W |
| IgG1-F48 | 3.2E-07 | M252Y/T256Q/N434Y |
| IgG1-F49 | 5.1E-07 | M252F/T256D/N434W |
| IgG1-F50 | 1.2E-06 | M252F/T256D/N434Y |
| IgG1-F51 | 8.1E-06 | N434F/Y436H |
| IgG1-F52 | 3.1E-06 | H433K/N434F/Y436H |
| IgG1-F53 | 1.0E-06 | I332V/N434W |
| IgG1-F54 | 8.4E-08 | V308P/N434W |
| IgG1-F56 | 9.4E-07 | I332V/M428L/N434Y |
| IgG1-F57 | 1.1E-05 | G385D/Q386P/N389S |
| IgG1-F58 | 7.7E-07 | G385D/Q386P/N389S/N434W |
| IgG1-F59 | 2.4E-06 | G385D/Q386P/N389S/N434Y |
| IgG1-F60 | 1.1E-05 | G385H |
| IgG1-F61 | 9.7E-07 | G385H/N434W |
| IgG1-F62 | 1.9E-06 | G385H/N434Y |
| IgG1-F63 | 2.5E-06 | N434F |
| IgG1-F64 | 5.3E-06 | N434H |
| IgG1-F65 | 2.9E-07 | M252Y/S254T/T256E/N434F |
| IgG1-F66 | 4.3E-07 | M252Y/S254T/T256E/N434H |
| IgG1-F67 | 6.3E-07 | M252Y/N434F |
| IgG1-F68 | 9.3E-07 | M252Y/N434H |
| IgG1-F69 | 5.1E-07 | M428L/N434W |
| IgG1-F70 | 1.5E-06 | M428L/N434Y |
| IgG1-F71 | 8.3E-08 | M252Y/S254T/T256E/M428L/N434W |
| IgG1-F72 | 2.0E-07 | M252Y/S254T/T256E/M428L/N434Y |
| IgG1-F73 | 1.7E-07 | M252Y/M428L/N434W |
| IgG1-F74 | 4.6E-07 | M252Y/M428L/N434Y |
| IgG1-F75 | 1.4E-06 | M252Y/M428L/N434A |
| IgG1-F76 | 1.0E-06 | M252Y/S254T/T256E/M428L/N434A |
| IgG1-F77 | 9.9E-07 | T256E/M428L/N434Y |

Table 9-3 is the continuation of Table 9-2.

[Table 9-3]

| IgG1-F78 | 7.8E-07 | S254T/M428L/N434W |
|---|---|---|
| IgG1-F79 | 5.9E-06 | S254T/T256E/N434A |
| IgG1-F80 | 2.7E-06 | M252Y/T256Q/N434A |
| IgG1-F81 | 1.6E-06 | M252Y/T256E/N434A |
| IgG1-F82 | 1.1E-06 | T256Q/N434W |
| IgG1-F83 | 2.6E-06 | T256Q/N434Y |

(continued)

| IgG1-F84 | 2.8E-07 | M252W/T256Q/N434W |
|---|---|---|
| IgG1-F85 | 5.5E-07 | M252W/T256Q/N434Y |
| IgG1-F86 | 1.5E-06 | S254T/T256Q/N434W |
| IgG1-F87 | 4.3E-06 | S254T/T256Q/N434Y |
| IgG1-F88 | 1.9E-07 | M252Y/S254T/T256Q/N434W |
| IgG1-F89 | 3.6E-07 | M252Y/S254T/T256Q/N434Y |
| IgG1-F90 | 1.9E-08 | M252Y/T256E/V308P/N434W |
| IgG1-F91 | 4.8E-08 | M252Y/V308P/M428L/N434Y |
| IgG1-F92 | 1.1E-08 | M252Y/S254T/T256E/V308P/M428L/N434W |
| IgG1-F93 | 7.4E-07 | M252W/M428L/N434W |
| IgG1-F94 | 3.7E-07 | P257L/M428L/N434Y |
| IgG1-F95 | 2.6E-07 | M252Y/S254T/T256E/M428L/N434F |
| IgG1-F99 | 6.2E-07 | M252Y/T256E/N434H |
| IgG1-F101 | 1.1E-07 | M252W/T256Q/P257L/N434Y |
| IgG1-F103 | 4.4E-08 | P238A/M252Y/V308P/N434Y |
| IgG1-F104 | 3.7E-08 | M252Y/D265A/V308P/N434Y |
| IgG1-F105 | 7.5E-08 | M252Y/T307A/V308P/N434Y |
| IgG1-F106 | 3.7E-08 | M252Y/V303A/V308P/N434Y |
| IgG1-F107 | 3.4E-08 | M252Y/V308P/D376A/N434Y |
| IgG1-F108 | 4.1E-08 | M252Y/V305A/V308P/N434Y |
| IgG1-F109 | 3.2E-08 | M252Y/V308P/Q311A/N434Y |
| IgG1-F111 | 3.2E-08 | M252Y/V308P/K317A/N434Y |
| IgG1-F112 | 6.4E-08 | M252Y/V308P/E380A/N434Y |
| IgG1-F113 | 3.2E-08 | M252Y/V308P/E382A/N434Y |
| IgG1-F114 | 3.8E-08 | M252Y/V308P/S424A/N434Y |
| IgG1-F115 | 6.6E-06 | T307A/N434A |
| IgG1-F116 | 8.7E-06 | E380A/N434A |
| IgG1-F118 | 1.4E-05 | M428L |
| IgG1-F119 | 5.4E-06 | T250Q/M428L |

Table 9-4 is the continuation of Table 9-3.

[Table 9-4]

| IgG1-FI20 | 6.3E-08 | P257L/V308P/M428L/N434Y |
|---|---|---|
| IgG1-F121 | 1.5E-08 | M252Y/T256E/V308P/M428L/N434W |
| IgG1-F122 | 1.2E-07 | M252Y/T256E/M428L/N434W |
| IgG1-F123 | 3.0E-08 | M252Y/T256E/V308P/N434Y |
| IgG1-F124 | 2.9E-07 | M252Y/T256E/M428L/N434Y |
| IgG1-F125 | 2.4E-08 | M252Y/S254T/T256E/V308P/M428L/N434Y |
| IgG1-FI28 | 1.7E-07 | P257L/M428L/N434W |
| IgG1-F129 | 2.2E-07 | P257A/M428L/N434Y |

(continued)

| | | |
|---|---|---|
| IgG1-F131 | 3.0E-06 | P257G/M428L/N434Y |
| IgG1-F132 | 2.1E-07 | P257I/M428L/N434Y |
| IgG1-F133 | 4.1E-07 | P257M/M428L/N434Y |
| IgG1-F134 | 2.7E-07 | P257N/M428L/N434Y |
| IgG1-F135 | 7.5E-07 | P257S/M428L/N434Y |
| IgG1-F136 | 3.8E-07 | P257T/M428L/N434Y |
| IgG1-F137 | 4.6E-07 | P257V/M428L/N434Y |
| IgG1-F139 | 1.5E-08 | M252W/V308P/N434W |
| IgG1-F140 | 3.6E-08 | S239K/M252Y/V308P/N434Y |
| IgG1-F141 | 3.5E-08 | M252Y/S298G/V308P/N434Y |
| IgG1-F142 | 3.7E-08 | M252Y/D270F/V308P/N434Y |
| IgG1-F143 | 2.0E-07 | M252Y/V308A/N434Y |
| IgG1-F145 | 5.3E-08 | M252Y/V308F/N434Y |
| IgG1-F147 | 2.4E-07 | M252Y/V308I/N434Y |
| IgG1-F149 | 1.9E-07 | M252Y/V308L/N434Y |
| IgG1-F150 | 2.0E-07 | M252Y/V308M/N434Y |
| IgG1-F152 | 2.7E-07 | M252Y/V308Q/N434Y |
| IgG1-F154 | 1.8E-07 | M252Y/V308T/N434Y |
| IgG1-F157 | 1.5E-07 | P257A/V308P/M428L/N434Y |
| IgG1-F158 | 5.9E-08 | P257T/V308P/M428L/N434Y |
| IgG1-F159 | 4.4E-08 | P257V/V308P/M428L/N434Y |
| IgG1-F160 | 8.5E-07 | M252W/M428I/N434Y |
| IgG1-F162 | 1.7E-07 | M252W/M428Y/N434Y |
| IgG1-F163 | 3.5E-07 | M252W/M428F/N434Y |
| IgG1-F164 | 3.7E-07 | P238A/M252W/N434Y |
| IgG1-F165 | 2.9E-07 | M252W/D265A/N434Y |
| IgG1-F166 | 1.5E-07 | M252W/T307Q/N434Y |

Table 9-5 is the continuation of Table 9-4.

[Table 9-5]

| | | |
|---|---|---|
| IgG1-F167 | 2.9E-07 | M252W/V303A/N434Y |
| IgG1-F168 | 3.2E-07 | M252W/D376A/N434Y |
| IgG1-F169 | 2.9E-07 | M252W/V305A/N434Y |
| IgG1-F170 | 1.7E-07 | M252W/Q311A/N434Y |
| IgG1-F171 | 1.9E-07 | M252W/D312A/N434Y |
| IgG1-F172 | 2.2E-07 | M252W/K317A/N434Y |
| IgG1-F173 | 7.7E-07 | M252W/E380A/N434Y |
| IgG1-F174 | 3.4E-07 | M252W/E382A/N434Y |
| IgG1-F175 | 2.7E-07 | M252W/S424A/N434Y |
| IgG1-F176 | 2.9E-07 | S239K/M252W/N434Y |

(continued)

| IgG1-F177 | 2.8E-07 | M252W/S298G/N434Y |
|---|---|---|
| IgG1-F178 | 2.7E-07 | M252W/D270F/N434Y |
| IgG1-F179 | 3.1E-07 | M252W/N325G/N434Y |
| IgG1-F182 | 6.6E-08 | P257A/M428L/N434W |
| IgG1-F183 | 2.2E-07 | P257T/M428L/N434W |
| IgG1-F184 | 2.7E-07 | P257V/M428L/N434W |
| IgG1-F185 | 2.6E-07 | M252W/I332V/N434Y |
| IgG1-F188 | 3.0E-06 | P257I/Q311I |
| IgG1-F189 | 1.9E-07 | M252Y/T307A/N434Y |
| IgG1-F190 | 1.1E-07 | M252Y/T307Q/N434Y |
| IgG1-F191 | 1.6E-07 | P257L/T307A/M428L/N434Y |
| IgG1-F192 | 1.1E-07 | P257A/T307A/M428L/N434Y |
| IgG1-F193 | 8.5E-08 | P257T/T307A/M428L/N434Y |
| IgG1-F194 | 1.2E-07 | P257V/T307A/M428L/N434Y |
| IgG1-F195 | 5.6E-08 | P257L/T307Q/M428L/N434Y |
| IgG1-F196 | 3.5E-08 | P257A/T307Q/M428L/N434Y |
| IgG1-F197 | 3.3E-08 | P257T/T307Q/M428L/N434Y |
| IgG1-F198 | 4.8E-08 | P257V/T307Q/M428L/N434Y |
| IgG1-F201 | 2.1E-07 | M252Y/T307D/N434Y |
| IgG1-F203 | 2.4E-07 | M252Y/T307F/N434Y |
| IgG1-F204 | 2.1E-07 | M252Y/T307G/N434Y |
| IgG1-F205 | 2.0E-07 | M252Y/T307H/N434Y |
| IgG1-F206 | 2.3E-07 | M252Y/T307I/N434Y |
| IgG1-F207 | 9.4E-07 | M252Y/T307K/N434Y |
| IgG1-F208 | 3.9E-07 | M252Y/T307L/N434Y |

Table 9-6 is the continuation of Table 9-5.

[Table 9-6]

| IgG1-F209 | 1.3E-07 | M252Y/T307M/N434Y |
|---|---|---|
| IgG1-F210 | 2.9E-07 | M252Y/T307N/N434Y |
| IgG1-F211 | 2.4E-07 | M252Y/T307P/N434Y |
| IgG1-F212 | 6.8E-07 | M252Y/T307R/N434Y |
| IgG1-F213 | 2.3E-07 | M252Y/T307S/N434Y |
| IgG1-F214 | 1.7E-07 | M252Y/T307V/N434Y |
| IgG1-F215 | 9.6E-08 | M252Y/T307W/N434Y |
| IgG1-F216 | 2.3E-07 | M252Y/T307Y/N434Y |
| IgG1-F217 | 2.3E-07 | M252Y/K334L/N434Y |
| IgG1-F218 | 2.6E-07 | M252Y/G385H/N434Y |
| IgG1-F219 | 2.5E-07 | M252Y/T289H/N434Y |
| IgG1-F220 | 2.5E-07 | M252Y/Q311H/N434Y |

(continued)

| IgG1-F221 | 3.1E-07 | M252Y/D312H/N434Y |
|---|---|---|
| IgG1-F222 | 3.4E-07 | M252Y/N315H/N434Y |
| IgG1-F223 | 2.7E-07 | M252Y/K360H/N434Y |
| IgG1-F225 | 1.5E-06 | M252Y/L314R/N434Y |
| IgG1-F226 | 5.4E-07 | M252Y/L314K/N434Y |
| IgG1-F227 | 1.2E-07 | M252Y/N286E/N434Y |
| IgG1-F228 | 2.3E-07 | M252Y/L309E/N434Y |
| IgG1-F229 | 5.1E-07 | M252Y/R255E/N434Y |
| IgG1-F230 | 2.5E-07 | M252Y/P387E/N434Y |
| IgG1-F236 | 8.9E-07 | K248I/M428L/N434Y |
| IgG1-F237 | 2.3E-07 | M252Y/M428A/N434Y |
| IgG1-F238 | 7.4E-07 | M252Y/M428D/N434Y |
| IgG1-F240 | 7.2E-07 | M252Y/M428F/N434Y |
| IgG1-F241 | 1.5E-06 | M252Y/M428G/N434Y |
| IgG1-F242 | 8.5E-07 | M252Y/M428H/N434Y |
| IgG1-F243 | 1.8E-07 | M252Y/M428I/N434Y |
| IgG1-F244 | 1.3E-06 | M252Y/M428K/N434Y |
| IgG1-F245 | 4.7E-07 | M252Y/M428N/N434Y |
| IgG1-F246 | 1.1E-06 | M252Y/M428P/N434Y |
| IgG1-F247 | 4.4E-07 | M252Y/M428Q/N434Y |
| IgG1-F249 | 6.4E-07 | M252Y/M428S/N434Y |
| IgG1-F250 | 2.9E-07 | M252Y/M428T/N434Y |
| IgG1-F251 | 1.9E-07 | M252Y/M428V/N434Y |

Table 9-7 is the continuation of Table 9-6.

[Table 9-7]

| IgG1-F252 | 1.0E-06 | M252Y/M428W/N434Y |
|---|---|---|
| IgG1-F253 | 7.1E-07 | M252Y/M428Y/N434Y |
| IgG1-F254 | 7.5E-08 | M252W/T307Q/M428Y/N434Y |
| IgG1-F255 | 1.1E-07 | M252W/Q311A/M428Y/N434Y |
| IgG1-F256 | 5.4E-08 | M252W/T307Q/Q311A/M428Y/N434Y |
| IgG1-F257 | 5.0E-07 | M252Y/T307A/M428Y/N434Y |
| IgG1-F258 | 3.2E-07 | M252Y/T307Q/M428Y/N434Y |
| IgG1-F259 | 2.8E-07 | M252Y/D270F/N434Y |
| IgG1-F260 | 1.3E-07 | M252Y/T307A/Q311A/N434Y |
| IgG1-F261 | 8.4E-08 | M252Y/T307Q/Q311A/N434Y |
| IgG1-F262 | 1.9E-07 | M252Y/T307A/Q311H/N434Y |
| IgG1-F263 | 1.1E-07 | M252Y/T307Q/Q311H/N434Y |
| IgG1-F264 | 2.8E-07 | M252Y/E382A/N434Y |
| IgG1-F265 | 6.8E-07 | M252Y/E382A/M428Y/N434Y |

(continued)

| IgG1-F266 | 4.7E-07 | M252Y/T307A/E382A/M428Y/N434Y |
|---|---|---|
| IgG1-F267 | 3.2E-07 | M252Y/T307Q/E382A/M428Y/N434Y |
| IgG1-F268 | 6.3E-07 | P238A/M252Y/M428F/N434Y |
| IgG1-F269 | 5.2E-07 | M252Y/V305A/M428F/N434Y |
| IgG1-F270 | 6.6E-07 | M252Y/N325G/M428F/N434Y |
| IgG1-F271 | 6.9E-07 | M252Y/D376A/M428F/N434Y |
| IgG1-F272 | 6.8E-07 | M252Y/E380A/M428F/N434Y |
| IgG1- F273 | 6.5E-07 | M252Y/E382A/M428F/N434Y |
| IgG1-F274 | 7.6E-07 | M252Y/E380A/E382A/M428F/N434Y |
| IgG1-F275 | 4.2E-08 | S239K/M252Y/V308P/E382A/N434Y |
| IgG1-F276 | 4.1E-08 | M252Y/D270F/V308P/E382A/N434Y |
| IgG1-F277 | 1.3E-07 | S239K/M252Y/V308P/M428Y/N434Y |
| IgG1-F278 | 3.0E-08 | M252Y/T307Q/V308P/E382A/N434Y |
| IgG1-F279 | 6.1E-08 | M252Y/V308P/Q311H/E382A/N434Y |
| IgG1-F280 | 4.1E-08 | S239K/M252Y/D270F/V308P/N434Y |
| IgG1-F281 | 9.2E-08 | M252Y/V308P/E382A/M428F/N434Y |
| IgG1-F282 | 2.9E-08 | M252Y/V308P/E382A/M428L/N434Y |
| IgG1-F283 | 1.0E-07 | M252Y/V308P/E382A/M428Y/N434Y |
| IgG1-F284 | 1.0E-07 | M252Y/V308P/M428Y/N434Y |
| IgG1-F285 | 9.9E-08 | M252Y/V308P/M428F/N434Y |
| IgG1-F286 | 1.2E-07 | S239K/M252Y/V308P/E382A/M428Y/N434Y |

Table 9-8 is the continuation of Table 9-7.

[Table 9-8]

| IgG1-F287 | 1.0E-07 | M252Y/V308P/E380A/E382A/M428F/N434Y |
|---|---|---|
| IgG1-F288 | 1.9E-07 | M252Y/T256E/E382A/N434Y |
| IgG1-F289 | 4.8E-07 | M252Y/T256E/M428Y/N434Y |
| IgG1-F290 | 4.6E-07 | M252Y/T256E/E382A/M428Y/N434Y |
| IgG1-F292 | 2.0E-08 | S239K/M252Y/V308P/E382A/M428I/N434Y |
| IgG1-F293 | 5.3E-08 | M252Y/V308P/E380A/E382A/M428I/N434Y |
| IgG1-F294 | 1.1E-07 | S239K/M252Y/V308P/M428F/N434Y |
| IgG1-F295 | 6.8E-07 | S239K/M252Y/E380A/E382A/M428F/N434Y |
| IgG1-F296 | 4.9E-07 | M252Y/Q311A/M428Y/N434Y |
| IgG1-F297 | 5.1E-07 | M252Y/D312A/M428Y/N434Y |
| IgG1-F298 | 4.8E-07 | M252Y/Q311A/D312A/M428Y/N434Y |
| IgG1-F299 | 9.4E-08 | S239K/M252Y/V308P/Q311A/M428Y/N434Y |
| IgG1-F300 | 8.3E-08 | S239K/M252Y/V308P/D312A/M428Y/N434Y |
| IgG1-F301 | 7.2E-08 | S239K/M252Y/V308P/Q311A/D312A/M428Y/N434Y |
| IgG1-F302 | 1.9E-07 | M252Y/T256E/T307P/N434Y |
| IgG1-F303 | 6.7E-07 | M252Y/T307P/M428Y/N434Y |

(continued)

| IgG1-F304 | 1.6E-08 | M252W/V308P/M428Y/N434Y |
|---|---|---|
| IgG1-F305 | 2.7E-08 | M252Y/T256E/V308P/E382A/N434Y |
| IgG1-F306 | 3.6E-08 | M252W/V308P/E382A/N434Y |
| IgG1-F307 | 3.6E-08 | S239K/M252W/V308P/E382A/N434Y |
| IgG1-F308 | 1.8E-08 | S239K/M252W/V308P/E382A/M428Y/N434Y |
| IgG1-F310 | 9.4E-08 | S239K/M252W/V308P/E382A/M428I/N434Y |
| IgG1-F311 | 2.9E-08 | S239K/M252W/V308P/M428F/N434Y |
| IgG1-F312 | 4.5E-07 | S239K/M252W/E380A/E382A/M428F/N434Y |
| IgG1-F313 | 6.5E-07 | S239K/M252Y/T307P/M428Y/N434Y |
| IgG1-F314 | 3.2E-07 | M252Y/T256E/Q311A/D312A/M428Y/N434Y |
| IgG1-F315 | 6.8E-07 | S239K/M252Y/M428Y/N434Y |
| IgG1-F316 | 7.0E-07 | S239K/M252Y/D270F/M428Y/N434Y |
| IgG1-F317 | 1.1E-07 | S239K/M252Y/D270F/V308P/M428Y/N434Y |
| IgG1-F318 | 1.8E-08 | S239K/M252Y/V308P/M428I/N434Y |
| IgG1-F320 | 2.0E-08 | S239K/M252Y/V308P/N325G/E382A/M4281/N434Y |
| IgG1-F321 | 3.2E-08 | S239K/M252Y/D270F/V308P/N325G/N434Y |
| IgG1-F322 | 9.2E-08 | S239K/M252Y/D270F/T307P/V308P/N434Y |
| IgG1-F323 | 2.7E-08 | S239K/M252Y/T256E/D270F/V308P/N434Y |
| IgG1-F324 | 2.8E-08 | S239K/M252Y/D270F/T307Q/V308P/N434Y |

Table 9-9 is the continuation of Table 9-8.

[Table 9-9]

| IgG1-F325 | 2.1E-08 | S239K/M252Y/D270F/T307Q/V308P/Q311A/N434Y |
|---|---|---|
| IgG1-F326 | 7.5E-08 | S239K/M252Y/D270F/T307Q/Q311A/N434Y |
| IgG1-F327 | 6.5E-08 | S239K/M252Y/T256E/D270F/T307Q/Q311A/N434Y |
| IgG1-F328 | 1.9E-08 | S239K/M252Y/D270F/V308P/M428I/N434Y |
| IgG1-F329 | 1.2E-08 | S239K/M252Y/D270F/N286E/V308P/N434Y |
| IgG1-F330 | 3.6E-08 | S239K/M252Y/D270F/V308P/L309E/N434Y |
| IgG1-F331 | 3.0E-08 | S239K/M252Y/D270F/V308P/P387E/N434Y |
| IgG1-F333 | 7.4E-08 | S239K/M252Y/D270F/T307Q/L309E/Q311A/N434Y |
| IgG1-F334 | 1.9E-08 | S239K/M252Y/D270F/V308P/N325G/M428I/N434Y |
| IgG1-F335 | 1.5E-08 | S239K/M252Y/T256E/D270F/V308P/M428I/N434Y |
| IgG1-F336 | 1.4E-08 | S239K/M252Y/D270F/T307Q/V308P/Q311A/M428I/N434Y |
| IgG1-F337 | 5.6E-08 | S239K/M252Y/D270F/T307Q/Q311A/M4281/N434Y |
| IgG1-F338 | 7.7E-09 | S239K/M252Y/D270F/N286E/V308P/M428I/N434Y |
| IgG1-F339 | 1.9E-08 | S239K/M252Y/D270F/V308P/L309E/M428I/N434Y |
| IgG1-F343 | 3.2E-08 | S239K/M252Y/D270F/V308P/M428L/N434Y |
| IgG1-F344 | 3.0E-08 | S239K/M252Y/V308P/M428L/N434Y |
| IgG1-F349 | 1.5E-07 | S239K/M252Y/V308P/L309P/M428L/N434Y |
| IgG1-F350 | 1.7E-07 | S239K/M252Y/V308P/L309R/M428L/N434Y |

(continued)

| IgG1-F352 | 6.0E-07 | S239K/M252Y/L309P/M428L/N434Y |
|---|---|---|
| IgG1-F353 | 1.1E-06 | S239K/M252Y/L309R/M428L/N434Y |
| IgG1-F354 | 2.8E-08 | S239K/M252Y/T307Q/V308P/M428L/N434Y |
| IgG1-F356 | 3.4E-08 | S239K/M252Y/D270F/V308P/L309E/P387E/N434Y |
| IgG1-F357 | 1.6E-08 | S239K/M252Y/T256E/D270F/V308P/N325G/M428I/N434Y |
| IgG1-F358 | 1.0E-07 | S239K/M252Y/T307Q/N434Y |
| IgG1-F359 | 4.2E-07 | P257V/T307Q/M428I/N434Y |
| IgG1-F360 | 1.3E-06 | P257V/T307Q/M428V/N434Y |
| IgG1-F362 | 5.4E-08 | P257V/T307Q/N325G/M428L/N434Y |
| IgG1-F363 | 4.1E-08 | P257V/T307Q/Q311A/M428L/N434Y |
| IgG1-F364 | 3.5E-08 | P257V/T307Q/Q311A/N325G/M428L/N434Y |
| IgG1-F365 | 5.1E-08 | P257V/V305A/T307Q/M428L/N434Y |
| IgG1-F367 | 1.5E-08 | S239K/M252Y/E25SH/D270F/T307Q/V30SP/Q311 A/N434 Y |
| IgG1-F368 | 2.0E-08 | S239K/M252Y/D270F/V308P/N325G/E382A/M428I/N434Y |
| IgG1-F369 | 7.5E-08 | M252Y/P257V/T307Q/M428I/N434Y |
| IgG1-F372 | 1.3E-08 | S239K/M252W/V308P/M428Y/N434Y |
| IgG1-F373 | 1.1E-08 | S239K/M252W/V308P/Q311A/M428Y/N434Y |

Table 9-10 is the continuation of Table 9-9.

[Table 9-10]

| IgG1-F374 | 1.2E-08 | S239K/M252W/T256E/V308P/M428Y/N434Y |
|---|---|---|
| IgG1-F375 | 5.5E-09 | S239K/M252W/N286E/V308P/M428Y/N434Y |
| IgG1-F376 | 9.5E-09 | S239K/M252Y/T256E/D270F/N286E/V308P/N434Y |
| IgG1-F377 | 1.3E-07 | S239K/M252W/T307P/M428Y/N434Y |
| IgG1-F379 | 1.0E-08 | S239K/M252W/T256E/V308P/Q311A/M428Y/N434Y |
| IgG1-F380 | 5.6E-09 | S239K/M252W/T256E/N286E/V308P/M428Y/N434Y |
| IgG1-F381 | 1.1E-07 | P257V/T307A/Q311A/M428L/N434Y |
| IgG1-F382 | 8.7E-08 | P257V/V305A/T307A/M428L/N434Y |
| IgG1-F386 | 3.2E-08 | M252Y/V308P/L309E/N434Y |
| IgG1-F387 | 1.5E-07 | M252Y/V308P/L309D/N434Y |
| IgG1-F388 | 7.0E-08 | M252Y/V308P/L309A/N434Y |
| IgG1-F389 | 1.7E-08 | M252W/V308P/L309E/M428Y/N434Y |
| IgG1-F390 | 6.8E-08 | M252W/V308P/L309D/M428Y/N434Y |
| IgG1-F391 | 3.6E-08 | M252W/V308P/L309A/M428Y/N434Y |
| IgG1-F392 | 6.9E-09 | S239K/M252Y/N286E/V308P/M428I/N434Y |
| IgG1-F393 | 1.2E-08 | S239K/M252Y/N286E/V308P/N434Y |
| IgG1-F394 | 5.3E-08 | S239K/M252Y/T307Q/Q311A/M428I/N434Y |
| IgG1-F395 | 2.4E-08 | S239K/M252Y/T256E/V308P/N434Y |
| IgG1-F396 | 2.0E-08 | S239K/M252Y/D270F/N286E/T307Q/Q311A/M428I/N434Y |
| IgG1-F397 | 4.5E-08 | S239K/M252Y/D270F/T307Q/Q311A/P387E/M428I/N434Y |

(continued)

| | | |
|---|---|---|
| IgG1-F398 | 4.4E-09 | S239K/M252Y/D270F/N286E/T307Q/V308P/Q311A/M428I/N4 34Y |
| IgG1-F399 | 6.5E-09 | S239K/M252Y/D270F/N286E/T307Q/V308P/M428I/N434Y |
| IgG1-F400 | 6.1E-09 | S239K/M252Y/D270F/N286E/V308P/Q311A/M428I/N434Y |
| IgG1-F401 | 6.9E-09 | S239K/M252Y/D270F/N286E/V308P/P387E/M428I/N434Y |
| IgG1-F402 | 2.3E-08 | P257V/T307Q/M428L/N434W |
| IgG1-F403 | 5.1E-08 | P257V/T307A/M428L/N434W |
| IgG1-F404 | 9.4E-08 | P257A/T307Q/L309P/M428L/N434Y |
| IgG1-F405 | 1.7E-07 | P257V/T307Q/L309P/M428L/N434Y |
| IgG1-F406 | 1.5E-07 | P257A/T307Q/L309R/M428L/N434Y |
| IgG1-F407 | 1.6E-07 | P257V/T307Q/L309R/M428L/N434Y |
| IgG1-F408 | 2.5E-07 | P257V/N286E/M428L/N434Y |
| IgG1-F409 | 2.0E-07 | P257V/P387E/M428L/N434Y |
| IgG1-F410 | 2.2E-07 | P257V/T307H/M428L/N434Y |
| IgG1-F411 | 1.3E-07 | P257V/T307N/M428L/N434Y |

Table 9-11 is the continuation of Table 9-10.

[Table 9-11]

| | | |
|---|---|---|
| IgG1-F412 | 8.8E-08 | P257V/T307G/M428L/N434Y |
| IgG1-F413 | 1.2E-07 | P257V/T307P/M428L/N434Y |
| IgG1-F414 | 1.1E-07 | P257V/T307S/M428L/N434Y |
| IgG1-F415 | 5.6E-08 | P257V/N286E/T307A/M428L/N434Y |
| IgG1-F416 | 9.4E-08 | P257V/T307A/P387E/M428L/N434Y |
| IgG1-F418 | 6.2E-07 | S239K/M252Y/T307P/N325G/M428Y/N434Y |
| IgG1-F419 | 1.6E-07 | M252Y/T307A/Q311H/K360H/N434Y |
| IgG1-F420 | 1.5E-07 | M252Y/T307A/Q311H/P387E/N434Y |
| IgG1-F421 | 1.3E-07 | M252Y/T307A/Q311H/M428A/N434Y |
| IgG1-F422 | 1.8E-07 | M252Y/T307A/Q311H/E382A/N434Y |
| IgG1-F423 | 8.4E-08 | M252Y/T307W/Q311H/N434Y |
| IgG1-F424 | 9.4E-08 | S239K/P257A/V308P/M428L/N434Y |
| IgG1-F425 | 8.0E-08 | P257A/V308P/L309E/M428L/N434Y |
| IgG1-F426 | 8.4E-08 | P257V/T307Q/N434Y |
| IgG1-F427 | 1.1E-07 | M252Y/P257V/T307Q/M428V/N434Y |
| IgG1-F428 | 8.0E-08 | M252Y/P257V/T307Q/M428L/N434Y |
| IgG1-F429 | 3.7E-08 | M252Y/P257V/T307Q/N434Y |
| IgG1-F430 | 8.1E-08 | M252Y/P257V/T307Q/M428Y/N434Y |
| IgG1-F431 | 6.5E-08 | M252Y/P257V/T307Q/M428F/N434Y |
| IgG1-F432 | 9.2E-07 | P257V/T307Q/Q311A/N325G/M428V/N434Y |
| IgG1-F433 | 6.0E-08 | P257V/T307Q/Q311A/N325G/N434Y |
| IgG1-F434 | 2.0E-08 | P257V/T307Q/Q311A/N325G/M428Y/N434Y |
| IgG1-F435 | 2.5E-08 | P257V/T307Q/Q311A/N325G/M428F/N434Y |

(continued)

| IgG1-F436 | 2.5E-07 | P257A/T307Q/M428V/N434Y |
| IgG1-F437 | 5.7E-08 | P257A/T307Q/N434Y |
| IgG1-F438 | 3.6E-08 | P257A/T307Q/M428Y/N434Y |
| IgG1-F439 | 4.0E-08 | P257A/T307Q/M428F/N434Y |
| IgG1-F440 | 1.5E-08 | P257V/N286E/T307Q/Q311A/N325G/M428L/N434Y |
| IgG1-F441 | 1.8E-07 | P257A/Q311A/M428L/N434Y |
| IgG1-F442 | 2.0E-07 | P257A/Q311H/M428L/N434Y |
| IgG1-F443 | 5.5E-08 | P257A/T307Q/Q311A/M428L/N434Y |
| IgG1-F444 | 1.4E-07 | P257A/T307A/Q311A/M428L/N434Y |
| IgG1-F445 | 6.2E-08 | P257A/T307Q/Q311H/M428L/N434Y |
| IgG1-F446 | 1.1E-07 | P257A/T307A/Q311H/M428L/N434Y |
| IgG1-F447 | 1.4E-08 | P257A/N286E/T307Q/M428L/N434Y |

Table 9-12 is the continuation of Table 9-11.

[Table 9-12]

| IgG1-F448 | 5.3E-08 | P257A/N286E/T307A/M428L/N434Y |
| IgG1-F449 | 5.7E-07 | S239K/M252Y/D270F/T307P/N325G/M428Y/N434Y |
| IgG1-F450 | 5.2E-07 | S239K/M252Y/T307P/L309E/N325G/M428Y/N434Y |
| IgG1-F451 | 1.0E-07 | P257S/T307A/M428L/N434Y |
| IgG1-F452 | 1.4E-07 | P257M/T307A/M428L/N434Y |
| IgG1-F453 | 7.8E-08 | P257N/T307A/M428L/N434Y |
| IgG1-F454 | 9.6E-08 | P257I/T307A/M428L/N434Y |
| IgG1-F455 | 2.5E-08 | P257V/T307Q/M428Y/N434Y |
| IgG1-F456 | 3.4E-08 | P257V/T307Q/M428F/N434Y |
| IgG1-F457 | 4.0E-08 | S239K/P257V/V308P/M428L/N434Y |
| IgG1-F458 | 1.5E-08 | P257V/T307Q/V308P/N325G/M428L/N434Y |
| IgG1-F459 | 1.3E-08 | P257V/T307Q/V308P/Q311A/N325G/M428L/N434Y |
| IgG1-F460 | 4.7E-08 | P257V/T307A/V308P/N325G/M428L/N434Y |
| IgG1-F462 | 8.5E-08 | P257A/V308P/N325G/M428L/N434Y |
| IgG1-F463 | 1.3E-07 | P257A/T307A/V308P/M428L/N434Y |
| IgG1-F464 | 5.5E-08 | P257A/T307Q/V308P/M428L/N434Y |
| IgG1-F465 | 2.1E-08 | P257V/N286E/T307Q/N325G/M428L/N434Y |
| IgG1-F466 | 3.5E-07 | T256E/P257V/N434Y |
| IgG1-F467 | 5.7E-07 | T256E/P257T/N434Y |
| IgG1-F468 | 5.7E-08 | S239K/P257T/V308P/M428L/N434Y |
| IgG1-F469 | 5.6E-08 | P257T/V308P/N325G/M428L/N434Y |
| IgG1-F470 | 5.4E-08 | T256E/P257T/V308P/N325G/M428L/N434Y |
| IgG1-F471 | 6.6E-08 | P257T/V308P/N325G/E382A/M428L/N434Y |
| IgG1-F472 | 5.4E-08 | P257T/V308P/N325G/P387E/M428L/N434Y |
| IgG1-F473 | 4.5E-07 | P257T/V308P/L309P/N325G/M428L/N434Y |

(continued)

| IgG1-F474 | 3.5E-07 | P257T/V308P/L309R/N325G/M428L/N434Y |
|---|---|---|
| IgG1-F475 | 4.3E-08 | T256E/P257V/T307Q/M428L/N434Y |
| IgG1-F476 | 5.5E-08 | P257V/T307Q/E382A/M428L/N434Y |
| IgG1-F477 | 4.3E-08 | P257V/T307Q/P387E/M428L/N434Y |
| IgG1-F480 | 3.9E-08 | P257L/V308P/N434Y |
| IgG1-F481 | 5.6E-08 | P257T/T307Q/N434Y |
| IgG1-F482 | 7.0E-08 | P257V/T307Q/N325G/N434Y |
| IgG1-F483 | 5.7E-08 | P257V/T307Q/Q311A/N434Y |
| IgG1-F484 | 6.2E-08 | P257V/V305A/T307Q/N434Y |
| IgG1-F485 | 9.7E-08 | P257V/N286E/T307A/N434Y |

Table 9-13 is the continuation of Table 9-12.

[Table 9-13]

| IgG1-F486 | 3.4E-07 | P257V/T307Q/L309R/Q311H/M428L/N434Y |
|---|---|---|
| IgG1-F488 | 3.5E-08 | P257V/V308P/N325G/M428L/N434Y |
| IgG1F490 | 7.5E-08 | S239K/P257V/V308P/Q311H/M428L/N434Y |
| IgG1-F492 | 9.8E-08 | P257V/V305A/T307A/N325G/M428L/N434Y |
| IgG1-F493 | 4.9E-07 | S239K/D270F/T307P/N325G/M428Y/N434Y |
| IgG1-F497 | 3.1E-06 | P257T/T307A/M428V/N434Y |
| IgG1-F498 | 1.3E-06 | P257A/M428V/N434Y |
| IgG1-F499 | 5.2E-07 | P257A/T307A/M428V/N434Y |
| IgG1-F500 | 4.3E-08 | P257S/T307Q/M428L/N434Y |
| IgG1-F506 | 1.9E-07 | P257V/N297A/T307Q/M428L/N434Y |
| IgG1-F507 | 5.1E-08 | P257V/N286A/T307Q/M428L/N434Y |
| IgG1-F508 | 1.1E-07 | P257V/T307Q/N315A/M428L/N434Y |
| IgG1-F509 | 5.8E-08 | P257V/T307Q/N384A/M428L/N434Y |
| IgG1-F510 | 5.3E-08 | P257V/T307Q/N389A/M428L/N434Y |
| IgG1-F511 | 4.2E-07 | P257V/N434Y |
| IgG1-F512 | 5.8E-07 | P257T/N434Y |
| IgG1-F517 | 3.1E-07 | P257V/N286E/N434Y |
| IgG1-F518 | 4.2E-07 | P257T/N286E/N434Y |
| IgG1-F519 | 2.6E-08 | P257V/N286E/T307Q/N434Y |
| IgG1-F521 | 1.1E-08 | P257V/N286E/T307Q/M428Y/N434Y |
| IgG1-F523 | 2.6E-08 | P257V/V305A/T307Q/M428Y/N434Y |
| IgG1-F526 | 1.9E-08 | P257T/T307Q/M428Y/N434Y |
| IgG1-F527 | 9.4E-09 | P257V/T307Q/V308P/N325G/M428Y/N434Y |
| IgG1-F529 | 2.5E-08 | P257T/T307Q/M428F/N434Y |
| IgG1-F533 | 1.2E-08 | P257A/N286E/T307Q/M428F/N434Y |
| IgG1-F534 | 1.2E-08 | P257A/N286E/T307Q/M428Y/N434Y |
| IgG1-F535 | 3.9E-08 | T250A/P257V/T307Q/M428L/N434Y |

(continued)

| IgG1-F538 | 9.9E-08 | T250F/P257V/T307Q/M428L/N434Y |
| IgG1-F541 | 6.0E-08 | T2501/P257V/T307Q/M428L/N434Y |
| IgG1-F544 | 3.1E-08 | T250M/P257V/T307Q/M428L/N434Y |
| IgG1-F549 | 5.4E-08 | T250S/P257V/T307Q/M428L/N434Y |
| IgG1-F550 | 5.9E-08 | T250V/P257V/T307Q/M428L/N434Y |
| IgG1-F551 | 1.2E-07 | T250W/P257V/T307Q/M428L/N434Y |
| IgG1-F552 | 1.1E-07 | T250Y/P257V/T307Q/M428L/N434Y |
| IgG1-F553 | 1.7E-07 | M252Y/Q311A/N434Y |

Table 9-14 is the continuation of Table 9-13.

[Table 9-14]

| IgG1-F554 | 2.8E-08 | S239K/M252Y/S254T/V308P/N434Y |
| IgG1-F556 | 1.5E-06 | M252Y/T307Q/Q311A |
| IgG1-F559 | 8.0E-08 | M252Y/S254T/N286E/N434Y |
| IgG1-F560 | 2.8E-08 | M252Y/S254T/V308P/N434Y |
| IgG1-F561 | 1.4E-07 | M252Y/S254T/T307A/N434Y |
| IgG1-F562 | 8.3E-08 | M252Y/S254T/T307Q/N434Y |
| IgG1-F563 | 1.3E-07 | M252Y/S254T/Q311A/N434Y |
| IgG1-F564 | 1.9E-07 | M252Y/S254T/Q311H/N434Y |
| IgG1-F565 | 9.2E-08 | M252Y/S254T/T307A/Q311A/N434Y |
| IgG1-F566 | 6.1E-08 | M252Y/S254T/T307Q/Q311A/N434Y |
| IgG1-F567 | 2.2E-07 | M252Y/S254T/M428I/N434Y |
| IgG1-F568 | 1.1E-07 | M252Y/T256E/T307A/Q31 1H/N434Y |
| IgG1-F569 | 2.0E-07 | M252Y/T256Q/T307A/Q311H/N434Y |
| IgG1-F570 | 1.3E-07 | M252Y/S254T/T307A/Q311H/N434Y |
| IgG1-F571 | 8.1E-08 | M252Y/N286E/T307A/Q311H/N434Y |
| IgG1-F572 | 1.0E-07 | M252Y/T307A/Q311H/M428I/N434Y |
| IgG1-F576 | 1.6E-06 | M252Y/T256E/T307Q/Q311H |
| IgG1-F577 | 1.3E-06 | M252Y/N286E/T307A/Q311A |
| IgG1-F578 | 5.7E-07 | M252Y/N286E/T307Q/Q311A |
| IgG1-F580 | 8.6E-07 | M252Y/N286E/T307Q/Q311H |
| IgG1-F581 | 7.2E-08 | M252Y/T256E/N286E/N434Y |
| IgG1-F582 | 7.5E-07 | S239K/M252Y/V308P |
| IgG1-F583 | 7.8E-07 | S239K/M252Y/V308P/E382A |
| IgG1-F584 | 6.3E-07 | S239K/M252Y/T256E/V308P |
| IgG1-F585 | 2.9E-07 | S239K/M252Y/N286E/V308P |
| IgG1-F586 | 1.4E-07 | S239K/M252Y/N286E/V308P/M428I |
| IgG1-F587 | 1.9E-07 | M252Y/N286E/M428L/N434Y |
| IgG1-F592 | 2.0E-07 | M252Y/S254T/E382A/N434Y |
| IgG1-F593 | 3.1E-08 | S239K/M252Y/S254T/V308P/M4281/N434Y |

(continued)

| IgG1-F595 | 1.8E-07 | S239K/M252Y/M428I/N434Y |
| IgG1-F596 | 4.0E-07 | M252Y/D312A/E382A/M428Y/N434Y |
| IgG1-F597 | 2.2E-07 | M252Y/E382A/P387E/N434Y |
| IgG1-F598 | 1.4E-07 | M252Y/D312A/P387E/N434Y |
| IgG1-F599 | 5.2E-07 | M252Y/P387E/M428Y/N434Y |

[Example 13] In vivo study of various Fc variant antibodies by steady-state infusion model using human FcRn transgenic mouse line 32

Fc variants generated in Example 12 was tested for their ability to eliminate antigen from plasma in steady-state infusion model using human FcRn transgenic mouse line 32. Steady-state infusion model in vivo study was performed as described in Example 1, but human FcRn transgenic mouse line 32 was used instead of line 276, and monoclonal anti-mouse CD4 antibody was injected twice (before infusion pump was implanted and 14 days after antibody injection) or three times (before infusion pump was implanted and 10 and 20 days after antibody injection).

From the Fc variants described in Tables 9-1 to 9-14, selected antibody Fc variants listed below were expressed and purified by methods known to those skilled in the art as described in Reference Example 2:

Fv4-IgG1 comprising VH3-IgG1 and VL3-CK;
Fv4-IgG1-F11 comprising VH3-IgG1-F11 and VL3-CK;
Fv4-IgG1-F14 comprising VH3-IgG1-F14 and VL3-CK;
Fv4-IgG1-F39 comprising VH3-IgG1-F39 and VL3-CK;
Fv4-IgG1-F48 comprising VH3-IgG1-F48 and VL3-CK;
Fv4-IgG1-F140 comprising VH3-IgG1-F140 and VL3-CK;
Fv4-IgG1-F157 comprising VH3-IgG1-F157 and VL3-CK;
Fv4-IgG1-F194 comprising VH3-IgG1-F194 and VL3-CK;
Fv4-IgG1-F196 comprising VH3-IgG1-F196 and VL3-CK;
Fv4-IgG1-F198 comprising VH3-IgG1-F198 and VL3-CK;
Fv4-IgG1-F262 comprising VH3-IgG1-F262 and VL3-CK;
Fv4-IgG1-F264 comprising VH3-IgG1-F264 and VL3-CK;
Fv4-IgG1-F393 comprising VH3-IgG1-F393 and VL3-CK;
Fv4-IgG1-F424 comprising VH3-IgG1-F434 and VL3-CK; and
Fv4-IgG1-F447 comprising VH3-IgG1-F447 and VL3-CK.

These antibodies were administered to the human FcRn transgenic mouse line 32 at a dose of 1 mg/kg.

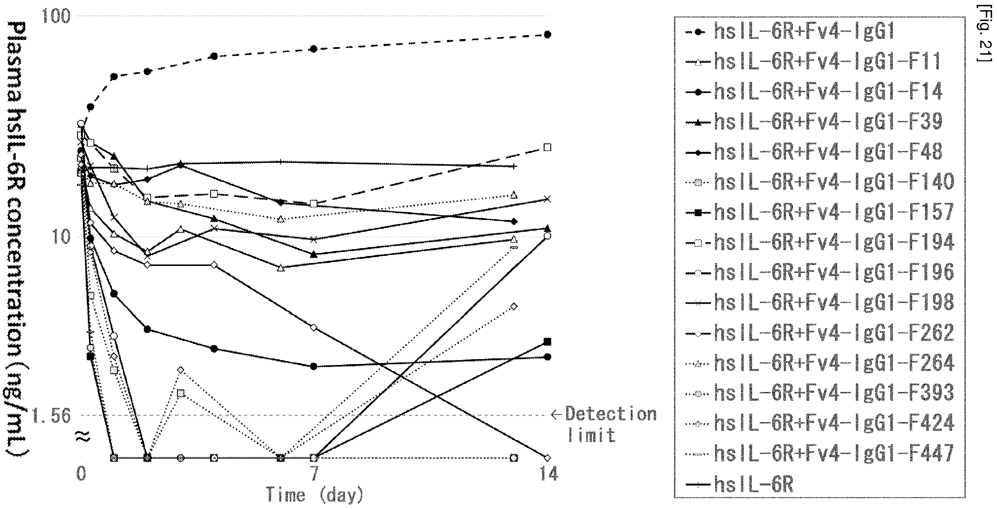

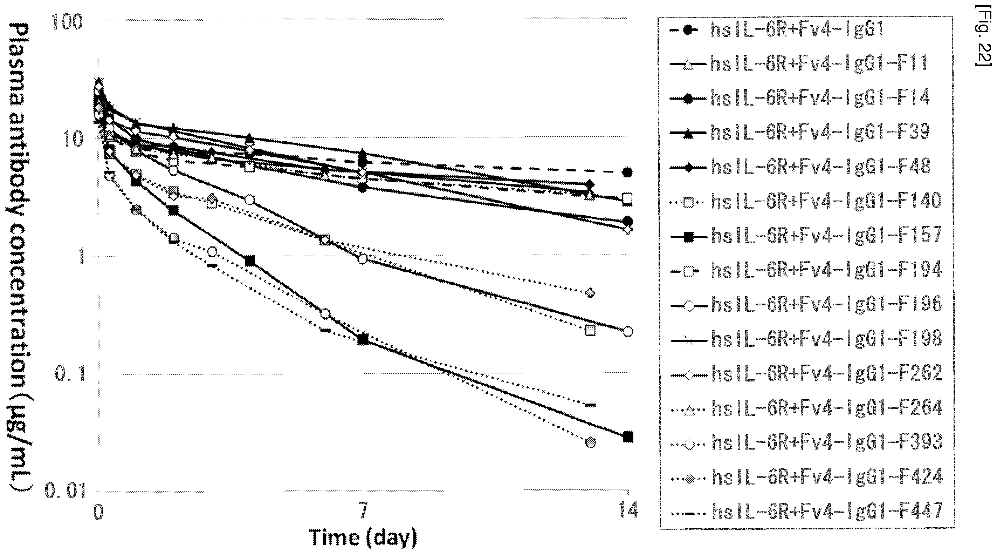

Fig. 21 describes the time course of plasma hsIL-6R concentration in the mouse. Compared to Fv4-IgG1, all the Fc variants having increased binding affinity to human FcRn at pH 7.0 exhibited reduction of plasma hsIL-6R concentration, therefore enhanced antigen elimination from plasma. Although the extent and durability of antigen concentration reduction was different among the Fc variants, all the variant consistently reduced the plasma hsIL-6R concentration as compared to IgG1 demonstrating that increased binding affinity to human FcRn at pH 7.0 would universally enhance the antigen elimination from plasma. Fig. 22 describes the time course of plasma antibody concentration in the mouse. Antibody pharmacokinetics was different among the Fc variants.

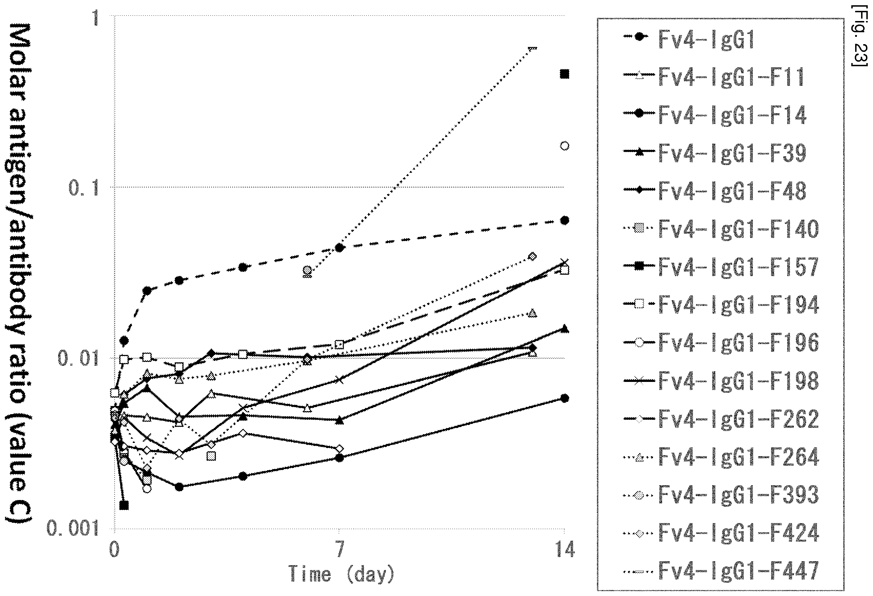

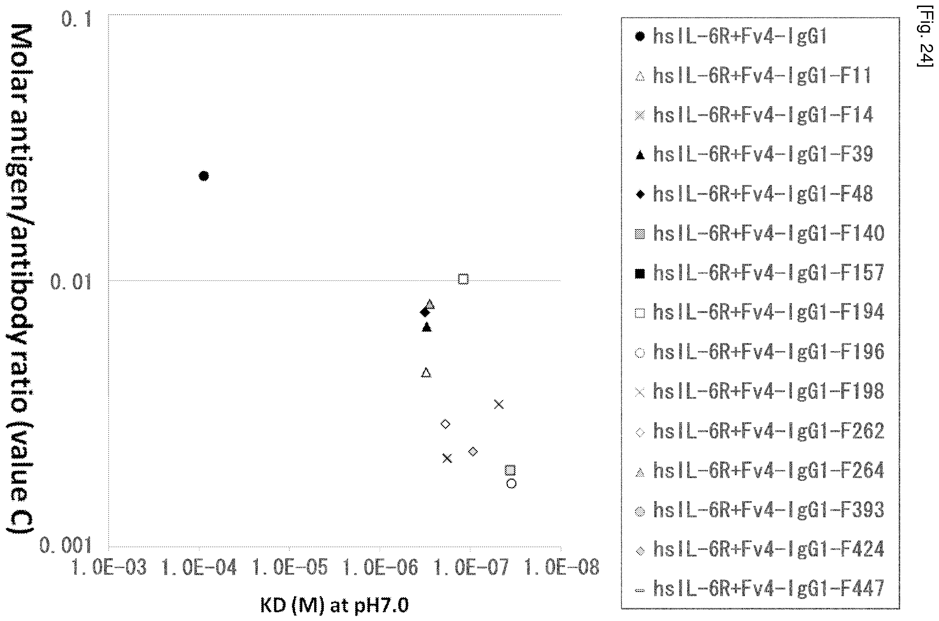

As described in Example 9, amount of antigen eliminated from plasma per antibody is the important factor to evaluate the efficiency of antigen elimination by administrating the antibody Fc variants having increased binding affinity to human FcRn at pH 7.0. Therefore, time courses of value C (molar antigen/antibody ratio) for each antibody were described in Fig. 23. Fig. 24 describes the relationship between the binding affinity of Fc variants to human FcRn at pH 7.0 and value C (molar antigen/ antibody ratio) at day 1 after administration of antibodies. This demonstrates that all the antibody Fc variants tested in this study have lower value C as compared to Fv4-IgG1. Since all the Fc variants tested in this study have binding affinity to human FcRn at pH 7.0 stronger than KD 3.0 micromolar, they achieved higher antigen elimination efficiency as compared to intact human IgG1. This was consistent with the results obtained in Example 9 (Fig. 17).

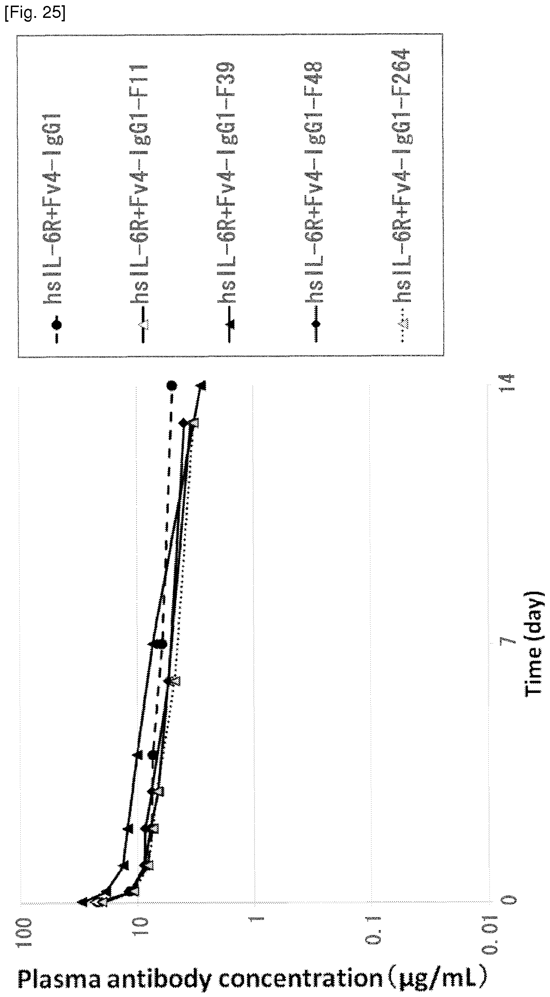

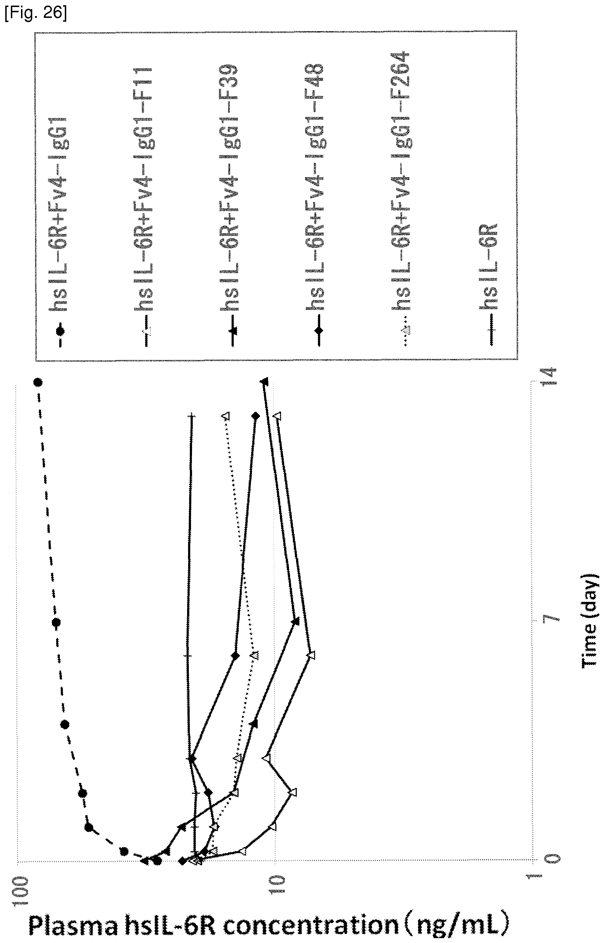

Fig. 25 describes that among the Fc variants tested in this study, antibodies having Fc variant of F11, F39, F48, and F264 exhibited similar pharmacokinetics to IgG1. Since this study is conducted using human FcRn transgenic mouse, these Fc variants is expected to have long half life similar to IgG1 also in human. Fig 26 describes the time course of plasma hsIL-6R concentration in mice injected with antibodies having similar pharmacokinetics to intact human IgG1 (F11, F39, F48, and F264). These variants reduced the plasma hsIL-6R concentration as compared to IgG1 approximately 10-fold.

Moreover, these antibodies reduced the hsIL-6R concentration below the baseline hsIL-6R concentration (concentration without antibody). Therefore, these antibodies would enable long-term elimination of antigen from plasma, and therefore long dosing intervals which would be preferable for antibody therapeutics for chronic disease.

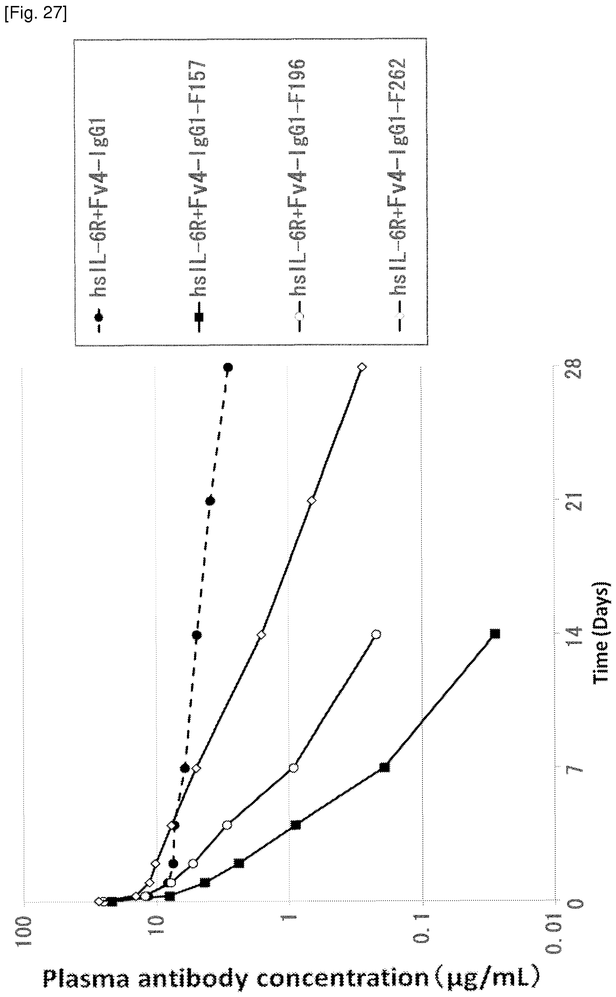

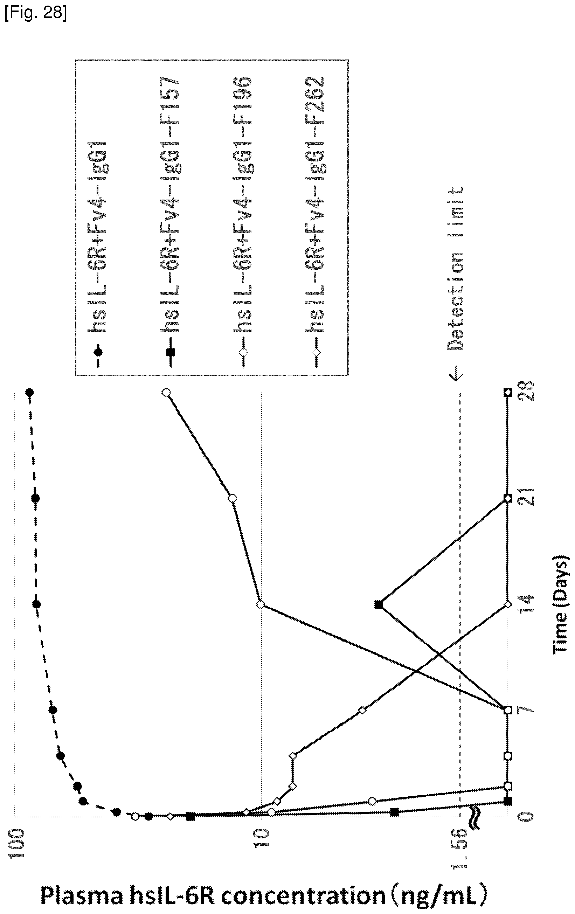

Fig. 27 and 28 described the time course of plasma antibody concentration and plasma hsIL-6R concentration for IgG1, and Fc variant F157, F196 and F262, respectively. Surprisingly, although antibody pharmacokinetics of F157 and F262 showed significantly faster clearance from plasma as compared to intact human IgG1, F157 and F262 exhibited very extensive and durable elimination of hsIL-6R from plasma. Specifically, plasma hsIL-6R concentration of F157 was below detection limit (1.56 ng/mL), from days 1 to 28 (except at day 14), and that of F262 was below detection limit (1.56 ng/mL) from days 14 to 28. On the other hand, for F196 with slower clearance of antibody compared to F157, antigen concentration started to increase at day 14 and returned back to baseline at day 28. Among the Fc variants tested in this study, F157 and F262 were the only Fc variants that were capable of reducing plasma hsIL-6R concentration below 1.56 ng/mL at day 28.

Such durable long-term effect of F157 and F262 is unexpected from the pharmacokinetics of the antibody, since antibodies were eliminated from plasma very rapidly as compared to intact human IgG1. In particular, plasma antibody concentration of F157 was not detected at day 21. Nevertheless, plasma hsIL-6R concentration continued to be reduced to a level lower than the detection limit of 1.56 ng/mL at days 21 and 28. This unexpected effect is considered to be due to the presence of the antibody at the surface of vascular endothelium cell as FcRn bound form. Although these antibodies showed low concentration in plasma, these antibodies is still present in the vascular compartment as FcRn bound form (which cannot be measured as a plasma antibody concentration). These FcRn bound antibody can still bind to the antigen in the plasma, and after FcRn mediated uptake of antigen/antibody complex, antigen is released within the endosome and degraded by the lysosome while the antibody is recycled back to the cell surface as FcRn bound form. Thus these FcRn bound antibody contribute to the antigen elimination. This explains the reason why these antibodies maintains antigen elimination capability even after the antibody concentration becomes low in plasma.

[Example 14] Comparative in silico study of conventional antibody and antigen eliminating antibody

Example 13 demonstrates that antibody with pH-dependent binding to the antigen and increased binding affinity to human FcRn at neutral pH are capable of eliminating antigen from plasma. Therefore, such antigen eliminating antibodies are useful for antibody targeting the antigen in which simple binding and neutralization is not enough for treating the disease, and depletion of antigen from the plasma is required.

Antigen eliminating antibodies are also useful for antibody targeting the antigen where simple binding and neutralization is enough. Antibody binding and neutralization of the antigen requires at least same molar amount of antibody as antigen in the plasma (if the antibody has infinite affinity to the antigen, antigen can be neutralized by same molar amount of antibody as antigen). In contrast to conventional antibody (antibody without pH-dependent antigen binding and Fc engineering), antigen eliminating antibodies can reduce the concentration of antigen in plasma. This means that antibody concentration required to neutralize the antigen can be reduced. If antigen eliminating antibody reduced the plasma antigen concentration by 10-fold as compared to conventional antibody, antibody concentration required to neutralize the antigen can also be reduced by 10-fold. Therefore, in a therapeutic setting, antigen eliminating antibody can reduce the antibody dosage or increase the dosing interval as compared to conventional antibody.

Fc variants such as F11, F39, F48, and F264 are capable of reducing the plasma antigen concentration as compared to IgG1 approximately 10-fold. In order to evaluate the effect of such antigen eliminating antibodies compared to conventional antibody, we performed an in silico assessment of the antibody dosage required to maintain antigen neutralization in a therapeutic setting for both conventional antibody and antigen eliminating antibody. We have determined a dosage required to maintain neutralization by every 3 month dosing interval (i.e. dosage required for Q3M).

Construction of pharmacokinetic model

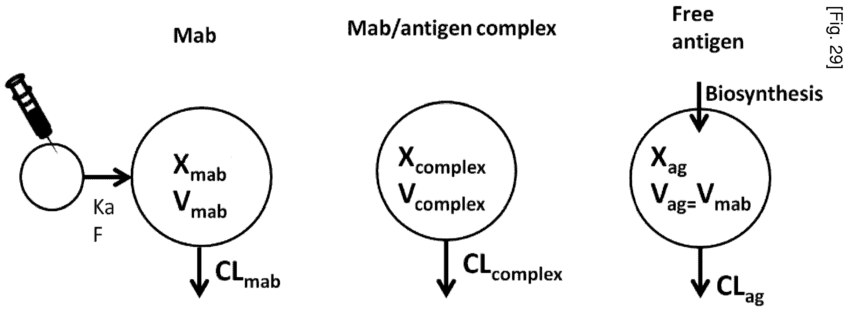

We constructed pharmacokinetic (PK) model using PK analysis software SAAM II (The SAAM Institute, Inc.). PK model is constructed as described in Pharmacokinet Pharmacodyn. 2001 Dec; 28(6): 507-32 and Br J Clin Pharmacol. 2007 May; 63(5): 548-61. Concept of the PK model is shown in Fig. 29. The amount of each compartment was described by the following differential equations.

[Math.1]

$$\frac{dXsc}{dt} = -ka \times Xsc$$

$$\frac{dXmab}{dt} = ka \times Xsc - CLmab \times \frac{Xmab}{Vmab} - kon \times \frac{Xmab \times Xag}{Vmab} + koff \times Xcom + \left( \frac{CLcom}{Vcom} - \frac{CLmab}{Vmab} \right) \times Xcom$$

$$\frac{dXcom}{dt} = -CLcom \times \frac{Xcom}{Vcom} + kon \times \frac{Xmab \times Xag}{Vmab} - koff \times Xcom$$

$$\frac{dXag}{dt} = -CLag \times \frac{Xag}{Vag} - kon \times \frac{Xmab \times Xag}{Vmab} + koff \times Xcom + R$$

Xsc: the amount of antibody in subcutaneous tissue
Xmab: the amount of free antibody in serum
Xcom: the amount of immune complex of antibody and antigen (=complex)
Xag: the amount of free antigen in serum
ka: absorption rate constant

In this model, bioavailability (F) is assumed to be 1 for all antibodies, and a biosynthesis rate of antigen (R) are set by the following equation.

[Math.2]
$$R = CLag \times Cpre$$

Cpre: steady state antigen concentration in serum.

Pharmacokinetic parameters and antigen binding kinetic parameters used in this in silico study are described in Table 10.

[Table 10]

| CLmab | L/day/kg | 0.0025 |
|---|---|---|
| CLag | L/day/kg | 0.0243 |
| CLcom | L/day/kg | 0.0045 |
| Vmab=Vag | L/kg | 0.0843 |
| Vcom | L/kg | 0.0519 |
| ka | 1/day | 0.4800 |
| koff | 1/day | 53.0496 |
| kon | 1/nM/day | 53.0496 |
| | L/ug/day | 0.353664 |

Simulation to calculate the effect of antigen eliminating antibody and affinity maturation

Steady state concentration (Cpre) before antibody administration was set as 2,400 ng/ mL. With constructed PK model, we estimated the minimum dosage of antibody in order to maintain free antigen concentration below 35 ng/mL 84 days after single subcutaneous administration. Molecular weight of antigen is set as 190 kDa, and molecular weight of therapeutic antibodies are all set as 150 kDa.

As the antibody, conventional antibody and antigen eliminating antibody with various binding affinity (different degree of affinity maturation from parent antibody with KD 1 nM) were used in this in silico study. Effect of antigen eliminating antibody is reflected as the faster clearance of antigen-antibody complex than conventional antibody. Clearance parameter of antigen-antibody complex (CLcom) is described in Table 11.

[Table 11]

|  |  | Conventional Ab | Antigen eliminating Ab |
|---|---|---|---|
| CLcom | L/day/kg | 0.0045 | 0.0729 |

Effect of affinity maturation from parent antibody with KD of 1nM is also considered (affinity is varied in 100-fold range). KD of 1 nM, 300 pM, 100 pM, 30 pM and 10 pM are used in this in silico study. Effect of affinity maturation is reflected as decreasing koff. The koff values are varied in 100-fold range (koff = 53.05, 17.68, 5.30, 1.77, 0.53 [1/day].

The dosage of antibody per body in order to maintain free antigen concentration below 35 ng/mL 84 days after single subcutaneous administration was obtained for conventional antibody and antigen eliminating antibody with binding affinity (KD) of 1 nM, 300 pM, 100 pM, 30 pM and 10 pM . The result was described in Table 12.

[Table 12]

| Dose (mg/body) | 1 nM | 333pM | 100pM | 33pM | 10pM |
|---|---|---|---|---|---|
| Conventional Ab | 2868 | 1256 | 692 | 532 | 475 |
| Antigen eliminating Ab | 180 | 81 | 46 | 36 | 33 |

Parent conventional antibody with binding affinity of 1 nM requires 2,868 mg to achieve Q3M dosing. Although antibody dosage can be reduced by improving the binding affinity to the antigen, reduction of the dosage reach a ceiling. This ceiling is derived from the fact that antibody binding and neutralization of the antigen requires at least same molar amount of antibody as the antigen in the plasma. Even with a binding affinity of 10 pM, conventional antibody requires 475 mg to achieve Q3M dosing, which is dosage that cannot be injected subcutaneously by single injection because of the limitation of formulation antibody concentration and subcutaneously injectable volume.

On the other hand, by engineering conventional antibody into antigen eliminating antibody by engineering pH dependency into the antigen binding (or by directly generating antibody with pH dependent binding) and engineering Fc region to have increased binding affinity to FcRn at neutral pH, antibody dosage can be significantly reduced. Antigen eliminating antibody with binding affinity of 1 nM requires only 180 mg to achieve Q3M dosing. This level of dosage cannot be achieved by conventional antibody even with infinite affinity. By improving the binding affinity of antigen eliminating antibody to 10 pM, dosage can be reduced to 33 mg, which is a dosage that can be easily injected subcutaneously.

Thus, this in silico study demonstrated that antigen eliminating antibody have significant advantage over conventional antibody. The dosage of antibody can be lowered to a level where conventional antibody is unable to reach even with infinite affinity. With respect to dosing interval, when antigen eliminating antibody is injected at a same dosage as conventional antibody, antigen eliminating antibody would have more sustained effect, therefore enables significantly longer dosing interval. Both reduction of dosage and prolonging dosing interval by antigen eliminating antibody would provide significant advantage over conventional antibody.

It should be noted that as described in Example 1, antigen eliminating antibody does not necessary requires pH dependent binding to the antigen. pH dependent binding to the antigen can significantly enhance the antigen eliminating activity of the antibody. In addition, pH dependent binding property can be substituted by utilizing other factors whose concentration is different within the plasma and the endosome. Such factor may also be used to generate an antibody that binds to the antigen within plasma but dissociates the antigen within endosome.

[Example 15] Study on enhancement of the human IL-6 elimination-accelerating effect of pH-dependent anti-human IL-6 antibodies

Generation of pH-dependent human IL-6-binding antibody

CLB8-IgG1 comprising CLB8H-IgG1 (SEQ ID NO: 16) and CLB8L-CK (SEQ ID NO: 17) described in WO 2009/125825 is a chimeric anti-IL-6 antibody. H16/L13-IgG1 comprising H16-IgG1 (SEQ ID NO: 18) and L13-CK (SEQ ID NO: 19) is a chimeric anti-IL-6 antibody that results from conferring CLB8-IgG1 with the property to bind to human IL-6 in a pH-dependent manner (which binds at pH 7.4 but is dissociated at pH 5.8).

Assessment of pH-dependent binding activity of chimeric anti-IL-6 antibody to human IL-6

CLB8-IgG1 and H16/L13-IgG1 were assessed for the human IL-6 binding activity (dissociation constant (KD)) at pH 5.5 and pH 7.4 using Biacore T100 (GE Healthcare). Assay was carried out using 10 mmol/l ACES/150 mmol/1 NaCl containing 0.05% Surfactant P20 (pH 7.4 and pH 6.0) as a running buffer. After antibodies were bound to recombinant proteinA/G (Thermo Scientific) immobilized on sensor chips using an amino-coupling method, appropriate concentrations of human IL-6 (TORAY) as an analyte were injected. Assays were carried out at 37 degrees C. The assay results were analyzed using Biacore T100 Evaluation Software (GE Healthcare), and the association rate constant, ka (1/Ms), and the dissociation rate constant, $k_d$ (1/s), were calculated from the assay results. Then the KD (M) was calculated from ka and $k_d$ (Table 13). Furthermore, the pH-dependent binding was evaluated to calculate the KD ratio between pH 7.4 and pH 6.0 for each antibody.

[Table 13]

| sample | pH | ka (1/Ms) | kd (1/s) | KD (M) | KD(pH5.5)/KD(pH7.4) |
|---|---|---|---|---|---|
| CLB8-IgG1 | pH7.4 | 3.6E+06 | 8.0E-04 | 2.2E-10 | 0.8 |
| | pH5.5 | 3.7E+06 | 6.6E-04 | 1.8E-10 | |
| H16/L13-IgG1 | pH7.4 | 2.1E+06 | 4.6E-03 | 2.2E-09 | 7.4 |
| | pH5.5 | 3.7E+05 | 5.9E-03 | 1.6E-08 | |

Preparation of pH-dependent anti-human IL-6 antibodies having FcRn-binding activity under neutral conditions

Mutations were introduced into H16/L13-IgG1 comprising H16-IgG1 (SEQ ID NO: 18) and L13-CK (SEQ ID NO: 19) to increase the FcRn binding under a neutral condition (pH 7.4). Specifically, H16-IgGl-v2 (SEQ ID NO: 20) was prepared from the heavy chain constant region of IgG1 by substituting Trp for Asn at position 434 in EU numbering, while H16-F14 (SEQ ID NO: 21) was constructed from the heavy chain constant region of IgG1 by substituting Tyr for Met at position 252, and Trp for Asn at position 434 in EU numbering. The amino acid substitutions were introduced by the method known to those skilled in the art described in Reference Example 1.

CLB8-IgG1 comprising CLB8H-IgG1 (SEQ ID NO: 16) and CLB8L-CK (SEQ ID NO: 17), H16/L13-IgGl comprising H16-IgGl (SEQ ID NO: 18) and L13-CK (SEQ ID NO: 19), H16/L13-IgG1-v2 comprising H16-IgG1-v2 (SEQ ID NO: 20) and L13-CK (SEQ ID NO: 19), and H16/L13-F14 comprising H16-F14 (SEQ ID NO: 21) and L13-CK (SEQ ID NO: 19) were expressed and purified by the method known to those skilled in the art described in Reference Example 2.

Assessment of mouse FcRn binging activity of Fc variants at neutral pH

VH3/L (WT)-IgG1 comprising VH3-IgG1 and L (WT), VH3/L (WT)-IgG1-v2 comprising VH3-IgG1-v2 and L (WT), and VH3/L (WT)-IgG1-F14 comprising VH3-IgG1-F14 and L (WT), all of which were prepared as described in Example 5, were assessed for mouse FcRn binding under a neutral condition (pH 7.4) by the method described in example 8.

The result was shown in Table 14. IgG1 exhibited very weak binding activity whereas IgG1-v2 and IgG1-F14 exhibited stronger binding affinity to mouse FcRn at pH7.4.

[Table 14]

| | KD |
|---|---|
| IgG1 | ND |
| IgG1-v2 | 1.0E-06 |
| IgG1-F14 | I.3E-07 |

In vivo test using normal mice

The in vivo kinetics of human IL-6 (hIL-6; TORAY) and anti-human IL-6 antibody was assessed after administering hIL-6 alone or hIL-6 and anti-human IL-6 antibody in normal mice (C57BL/6J mouse; Charles River Japan). An hIL-6 solution (5 microgram/ml) or a solution of mixture containing hIL-6 and anti-human IL-6 antibody (CLB8-IgG1 group; 5 microgram/ml of hIL-6 and 0.025 mg/ml of CLB8-IgG1, H16/L13-IgG1, H16/L13-IgG1-v2 and H16/L13-IgG1-F14 group; 5 microgram/ml of hIL-6 and 0.14 mg/mL of H16/L13-IgG1, H16/L13-IgG1-v2 and H16/L13-IgG1-F14 respectively) was administered once at a dose of 10 ml/kg into the caudal vein. Dose of antibody was set so that more than 99.8% of human IL-6 was bound to the antibody in the administration solution. Blood was collected 5 minutes, 30 minutes, two hours, four hours, seven hours, one day after administration of hIL-6 alone, and 5 minutes, seven hours, one day, two days, three days, four days, seven days, 14 days, 21 days, and 30 days after administration of hIL-6 and anti-human IL-6 antibody solution mixture. The collected blood was immediately centrifuged at 15,000 rpm and 4 degrees C for 15 minutes to separate the plasma. The separated plasma was stored in a refrigerator at -20 degrees C or below before assay.

Measurement of human IL-6 plasma concentration by ELISA

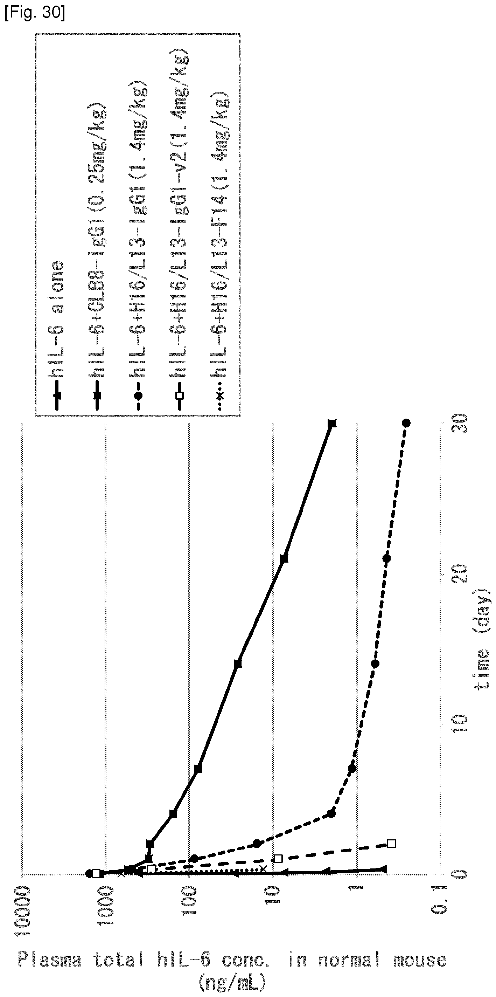

The concentration of human IL-6 in mouse plasma was measured by using Human IL-6 Quantikine HS ELISA Kit (R&D). Calibration curve samples having plasma concentrations of 20, 10, 5, 2.5, 1.25, 0.625 and 0.3125 ng/ml, and mouse plasma samples diluted 100-fold or more were prepared. In order to make all human IL-6 in sample bind to CLB8-IgG1, 150 microliter of 5 microgram/ml CLB8-IgG1 was added to 150 microliter of the calibration curve samples and plasma samples, and then the samples were allowed to stand for one hour at room temperature. Subsequently, the samples were dispensed into the plates provided in ELISA Kit (R&D), and allowed to stand for one hour at room temperature. Then, IL-6 conjugate provided in ELISA Kit (R&D) was added to react for one hour at room temperature and Substrate Solution provided in ELISA Kit (R&D) was added to react for one hour at room temperature. Subsequently, chromogenic reaction was carried out to react for half an hour at room temperature using Amplifier Solution provided in ELISA Kit (R&D) as a substrate. After stopping the reaction with Stop Solution provided in ELISA Kit (R&D), the absorbance at 490 nm was measured by a microplate reader. The concentration in mouse plasma was calculated from the absorbance of the calibration curve using the analytical software SOFTmax PRO (Molecular Devices). The time course of plasma hIL-6 concentration after intravenous administration as measured by this method is shown in Fig. 30 for normal mice.

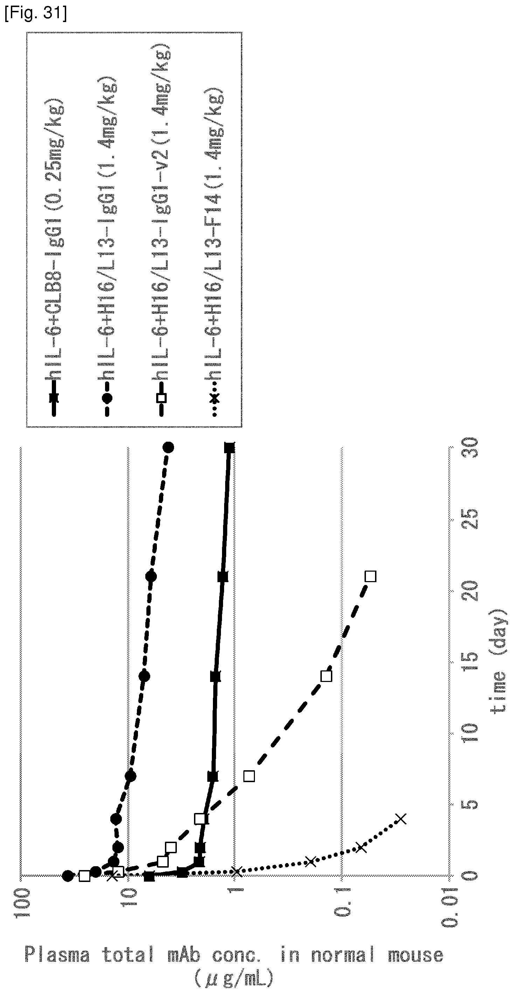

Measurement of anti-human IL-6 antibody plasma concentration by ELISA The concentration of anti-human IL-6 antibody in mouse plasma was measured by ELISA. Anti-human IgG (gamma-chain specific) F(ab')2 antibody fragment (Sigma) was dispensed onto a Nunc-ImmunoPlate MaxiSorp (Nalge Nunc International) and allowed to stand overnight at 4 degrees C to prepare anti-human IgG-immobilized plates. Calibration curve samples having plasma concentrations of 1.6, 0.8, 0.4, 0.2, 0.1, 0.05 and 0.025 microgram/ml, and mouse plasma samples diluted 100-fold or more were prepared. In order to make all anti-human IL-6 antibody in sample bind to human IL-6, 200 microliter of 1 microgram/ml human IL-6 was added to 100 microliter of the calibration curve samples and plasma samples, and then the samples were allowed to stand for one hour at room temperature. Subsequently, the samples were dispensed into the anti-human IgG-immobilized plates, and allowed to stand for one hour at room temperature. Then, Goat Anti-Human IgG (gamma chain specific) Biotin (BIOT) Conjugate (Southern Biotech Association) was added to react for one hour at room temperature. Subsequently, Streptavidin-PolyHRP80 (Stereospecific Detection Technologies) was added to react for one hour at room temperature, and chromogenic reaction was carried out using TMB One Component HRP Microwell Substrate (BioFX Laboratories) as a substrate. After stopping the reaction with 1 N sulfuric acid (Showa Chemical), the absorbance at 450 nm was measured by a microplate reader. The concentration in mouse plasma was calculated from the absorbance of the calibration curve using the analytical software SOFTmax PRO (Molecular Devices). The time course of plasma antibody concentration after intravenous administration as measured by this method is shown in Fig. 31 for normal mice.

Effect of pH-dependent binding to human IL-6

CLB8-IgGl and H16/L13-IgG1 which binds to human IL-6 in a pH-dependent manner were tested in vivo, and the results were compared between them. As shown in Fig. 31, the pharmacokinetics of antibody exhibited linear clearance. Meanwhile, as shown in Fig. 30, hIL-6 simultaneously administered with H16/L13-IgG1 which binds to human IL-6 in a pH-dependent manner was found to accelerate the elimination of hIL-6 as compared to hIL-6 simultaneously administered with CLB8-IgG1. Thus, it was demonstrated that by conferring a pH-dependent human IL-6-binding ability, the plasma hIL-6 concentration four days after administration could be decreased by about 76 times.

Effect of FcRn binding under neutral condition (pH 7.4)

In addition to H16/L13-IgG1, H16/L13-IgG1-v2 and H16/L13-F14, which result from introducing the above-described amino acid substitutions into H16/L13-IgG1, were tested in vivo using normal mice. The test results were compared to that of H16/L13-IgGl. As shown in Fig. 31, the plasma antibody concentration of H16/L13-IgG1-v2 which had increased binding to mouse FcRn under a neutral condition (pH 7.4) were 2.9-fold lower than H16/L13-IgGl at one day after administration. Alternatively, the plasma antibody concentration of H16/L13-F14 which had further increase the binding to mouse FcRn under a neutral condition (pH 7.4) were 21-fold lower than H16/L13-IgG1 at 7 hour after administration.

As shown in Fig. 30, hIL-6 simultaneously administered with H16/L13-IgG1-v2 or H16/L13-F14 which had increased binding to mouse FcRn under a neutral condition (pH 7.4) was demonstrated to be eliminated markedly faster as compared to hIL-6 simultaneously administered with H16/L13-IgG1. H16/L13-IgG1-v2 reduced the plasma concentration of hIL-6 approximately 10-fold compared to H16/L13-IgG1 at day 1. H16/L13-F14 reduced the plasma concentration of hIL-6 approximately 38-fold compared to H16/L13-IgG1 at seven hour. Thus, it was revealed that the plasma human IL-6 concentration could be reduced by conferring mouse FcRn-binding ability under a neutral condition (pH 7.4). As described above, by conferring the mouse FcRn-binding ability under a neutral condition (pH 7.4), the plasma antibody concentration was reduced; however, the effect of reducing the plasma hIL-6 concentration, which largely exceeded the decrease in antibody concentration, was produced. Specifically, this means that the elimination of human IL-6 could be accelerated by administering the antibody that binds to human IL-6 in a pH-dependent manner and which is conferred with mouse FcRn-binding ability under a neutral condition (pH 7.4).

The findings described above demonstrate that the plasma antigen concentration not only of human soluble IL-6 receptor but also of antigen such as human IL-6 can also be significantly reduced by administering an antibody having both pH-dependent antigen-binding ability and FcRn-binding ability under the neutral condition.

[Example 16] Study on enhancement of the human IgA elimination-accelerating effect of receptor Fc fusion protein which binds to human IgA in pH dependent manner

Generation of receptor Fc fusion protein which binds to human IgA in a pH dependent manner

A0-IgG1 comprising a dimer of A0H-IgG1 (SEQ ID NO: 22) is a human CD89-Fc fusion protein. As described in J. Mol. Biol. (2003) 324: 645-657, human CD89, also known as human Fc alpha receptor I, binds to human IgA in a pH-dependent manner (i.e. strongly binds to human IgA at neutral pH, but weakly binds to human IgA at acidic pH).

Assessment of pH-dependent binding activity of CD89-Fc fusion protein to human IgA

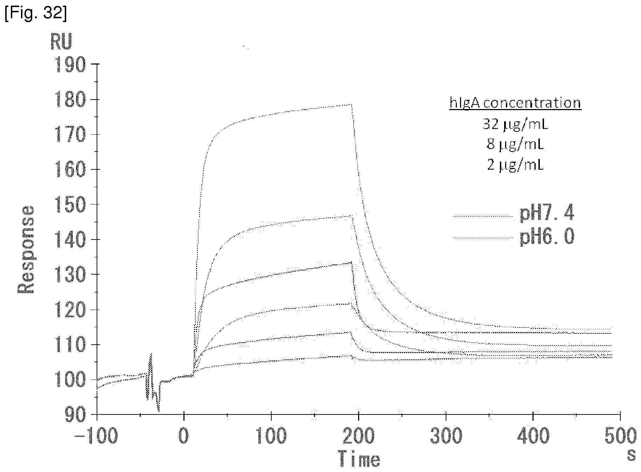

A0-IgG1 were assessed for the human IgA binding activity (dissociation constant (KD)) at pH 6.0 and pH 7.4 using Biacore T100 (GE Healthcare). Assay was carried out using 10 mmol/1 ACES/150 mmol/l NaCl containing 0.05% Surfactant P20 (pH 7.4 and pH 6.0) as a running buffer. After CD89-Fc fusion protein was bound to recombinant proteinA/G (Thermo Scientific) immobilized on sensor chips using an amino-coupling method, appropriate concentrations of hIgA (human IgA: prepared as described in Reference Example 5) as an analyte were injected. Assays were carried out at 37 degrees C. The assay results were analyzed using Biacore T100 Evaluation Software (GE Healthcare) and the obtained sensorgram was shown in Fig. 32. It is clearly demonstrated that CD89-Fc fusion protein have pH-dependent human IgA binding activity, which strongly binds to human IgA at neutral pH, but weakly binds to human IgA at acidic pH.

Preparation of pH-dependent receptor Fc fusion protein having FcRn-binding activity under neutral conditions

Mutations were introduced into A0-IgG1 comprising a dimer of A0H-IgG1 (SEQ ID NO: 22) to increase the FcRn binding under a neutral condition (pH 7.4). Specifically, A0-IgG1-v2 was prepared from the heavy chain constant region of IgG1 by substituting Trp for Asn at position 426 in A0-IgG1. The amino acid substitutions were introduced by the method known to those skilled in the art described in Reference Example 1.

A0-IgG1 comprising a dimer of A0H-IgG1 (SEQ ID NO: 22) and A0-IgG1-v2 comprising a dimer of A0H-IgG1-v2 (SEQ ID NO: 23) were expressed and purified by the method known to those skilled in the art described in Reference Example 2.

In vivo test using normal mice

The in vivo kinetics of human IgA (hIgA) and CD89-Fc fusion protein was assessed after administering hIgA alone or hIgA and CD89-Fc fusion protein (A0H-IgG1 or A0H-IgG1-v2) in normal mice (C57BL/6J mouse; Charles River Japan). An hIgA solution (80 microgram/ml) or a solution of mixture containing hIgA and CD89-Fc fusion protein (80 microgram/ml and 1.5 mg/ml, respectively, in which most of the hIgA was bound to CD89-Fc fusion protein) was administered once at a dose of 10 ml/ kg into the caudal vein. Blood was collected 15 minutes, seven hours, one day, two days, four days, and seven days after administration. The collected blood was immediately centrifuged at 15,000 rpm and 4 degrees C for 15 minutes to separate the plasma. The separated plasma was stored in a refrigerator at -20 degrees C or below before assay.

Measurement of human IgA plasma concentration by ELISA

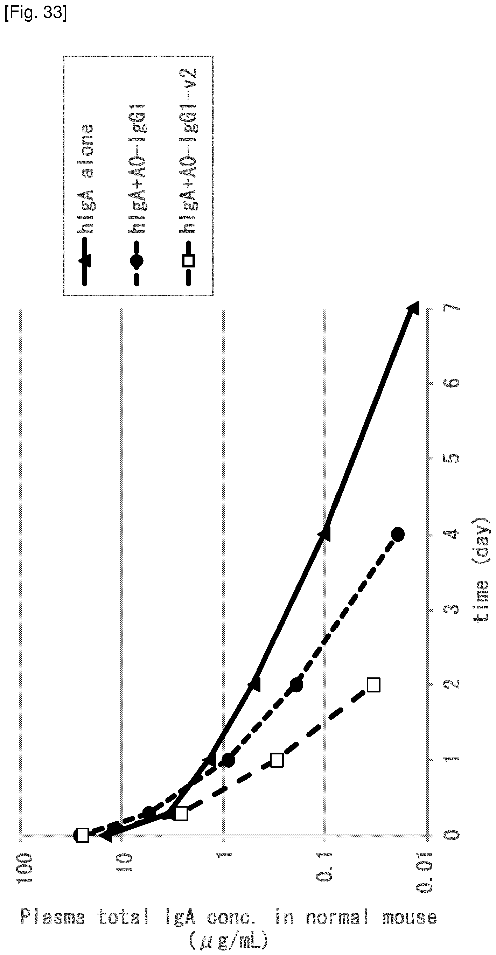

The concentration of human IgA in mouse plasma was measured by ELISA using hsIL-6R because the recombinant human IgA have variable region against hsIL-6R. Goat Anti-Human IgA Antibody (Bethyl Laboratories) was dispensed onto a Nunc-ImmunoPlate MaxiSorp (Nalge Nunc International) and allowed to stand overnight at 4 degrees C to prepare anti-human IgA-immobilized plates. Calibration curve samples having plasma concentrations of 0.4, 0.2, 0.1, 0.05, 0.025, 0.0125, or 0.00625 microgram/ml, and mouse plasma samples diluted 100-fold or more were prepared. In order to make all human IgA in sample bind to hsIL-6R, 200 microliter of 10 microgram/ml hsIL-6R was added to 100 microliter of the calibration curve samples and plasma samples, and then the samples were allowed to stand for one hour at room temperature. Subsequently, the samples were dispensed into the anti-human IgA-immobilized plates, and allowed to stand for one hour at room temperature. Then, Biotinylated Anti-Human IL-6R Antibody (R&D) was added to react for one hour at room temperature. Subsequently, Streptavidin-PolyHRP80 (Stereospecific Detection Technologies) was added to react for one hour at room temperature, and chromogenic reaction was carried out using TMB One Component HRP Microwell Substrate (BioFX Laboratories) as a substrate. After stopping the reaction with 1 N sulfuric acid (Showa Chemical), the absorbance at 450 nm was measured by a microplate reader. The concentration in mouse plasma was calculated from the absorbance of the calibration curve using the analytical software SOFTmax PRO (Molecular Devices). The time course of plasma hIgA concentration after intravenous administration as measured by this method is shown in Fig. 33 for normal mice.

Measurement of CD89-Fc fusion protein plasma concentration by ELISA

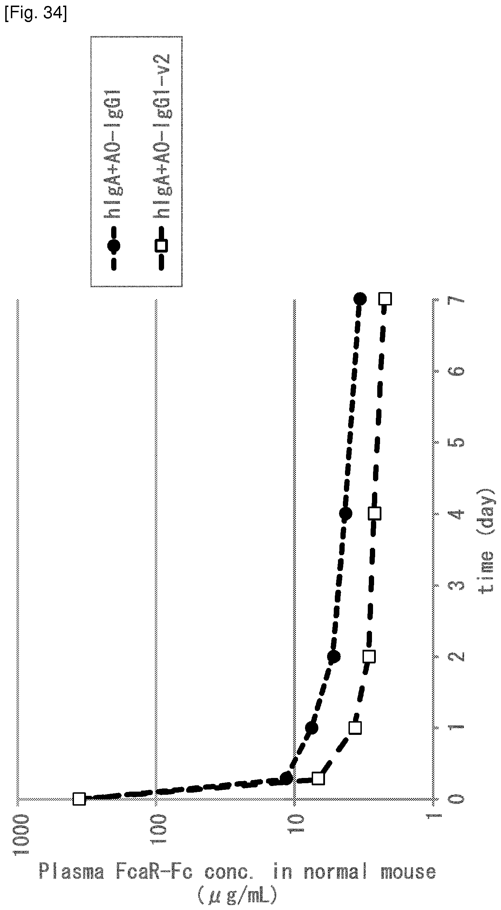

The concentration of CD89-Fc fusion protein in mouse plasma was measured by ELISA. Anti-human IgG (gamma-chain specific) F(ab')2 antibody fragment (Sigma) was dispensed onto a Nunc-ImmunoPlate MaxiSorp (Nalge Nunc International) and allowed to stand overnight at 4 degrees C to prepare anti-human IgG-immobilized plates. Calibration curve samples having plasma concentrations of 25.6, 12.8, 6.4, 3.2, 1.6, 0.8, and 0.4 microgram/ml, and mouse plasma samples diluted 100-fold or more were prepared. In order to make all CD89-Fc fusion protein in sample bind to human IgA, 200 microliter of 5 microgram/ml human IgA was added to 100 microliter of the calibration curve samples and plasma samples, and then the samples were allowed to stand for one hour at room temperature. Subsequently, the samples were dispensed into the anti-human IgG-immobilized plates, and allowed to stand for one hour at room temperature. Then, goat Anti-Human IgG (Fc specific)-Alkaline Phosphatase conjugate (SIGMA) was added to react for one hour at room temperature. Subsequently, chromogenic reaction was carried out using BluePhos Microwell Phosphatase Substrates System (Kirkegaard & Perry Laboratories) as a substrate and the absorbance at 650 nm was measured by a microplate reader. The concentration in mouse plasma was calculated from the absorbance of the calibration curve using the analytical software SOFTmax PRO (Molecular Devices). The time course of plasma concentration of CD89-Fc fusion protein after intravenous administration as measured by this method is shown in Fig. 34 for normal mice.

Effect of FcRn binding under neutral condition (pH 7.4)

In addition to A0-IgG1, A0-IgG1-v2 which resulted from introducing the above-described amino acid substitutions into A0-IgG1 were tested in vivo using normal mice. The test results were compared to that of A0-IgG1. As shown in Fig. 34, the plasma concentration of A0-IgG1-v2 which had increased binding to mouse FcRn under a neutral condition (pH 7.4) were 1.8-fold lower than A0-IgG1 two days after administration.

As shown in Fig. 33, hIgA simultaneously administered with A0-IgG1-v2 which had increased binding to mouse FcRn under a neutral condition (pH 7.4) was demonstrated to be eliminated markedly faster as compared to hIgA simultaneously administered with A0-IgG1. A0-IgG1-v2 reduced the plasma concentration of hIgA approximately 5.7-fold as compared to A0-IgG1 at day two. As described above, by conferring the mouse FcRn-binding ability under a neutral condition (pH 7.4), the plasma antibody concentration was reduced; however, the effect of reducing the plasma hIgA concentration, which largely exceeded the decrease in antibody concentration, was produced. Specifically, this means that the elimination of human IgA could be accelerated by administering the receptor Fc fusion protein that binds to human IgA in a pH-dependent manner and which is conferred with mouse FcRn-binding ability under a neutral condition (pH 7.4).

The findings described above demonstrate that the plasma antigen concentration, such as that of human IgA, can also be significantly reduced by administering a receptor Fc fusion protein having both pH-dependent antigen-binding ability and FcRn-binding ability under the neutral condition. Therefore, receptor Fc fusion protein can be also engineered to have capability of eliminating antigen (or ligand) plasma concentration from plasma.

[Example 17] Study on enhancement of the plexin A1 elimination-accelerating effect of pH-dependent anti-human plexin A1 antibodies (preparation of antibodies)

Regarding pH-dependent human plexin A1-binding antibody

PX268-IgG1 comprising PX268H-IgG1 (SEQ ID NO: 24) and PX268L-CK (SEQ ID NO: 25) is a chimeric anti-plexin A1 antibody. PX141-IgGl comprising PX141H-IgG1 (SEQ ID NO: 26) and PX141L-CK (SEQ ID NO: 27) is a chimeric anti-plexin A1 antibody that binds to soluble human plexin A1 in a pH-dependent manner (i.e. strongly binds to soluble human plexin A1 at neutral pH, but weakly binds to soluble human plexin A1 at acidic pH).

Assessment of pH-dependent binding activity of anti-human plexin A1 antibody to human plexin A1

PX268-IgG1 and PX141-IgG1 were assessed for the human plexin A1 binding activity (dissociation constant (KD)) at pH 6.0 and pH 7.4 with Biacore T100 (GE Healthcare). Assay was carried out using 10 mmol/l ACES/150 mmol/l NaCl containing 0.05% Surfactant P20 (pH 7.4 and pH 6.0) as a running buffer. After antibodies were bound to recombinant proteinA/G (Thermo Scientific) immobilized onto sensor chips using an amino-coupling method, appropriate concentrations of hsPlexin A1 (soluble human plexin A1: prepared as described in Reference Example 5) as an analyte were injected. Assays were carried out at 37 degrees C. The assay results were analyzed using Biacore T100 Evaluation Software (GE Healthcare), and the association rate constant, ka (1/Ms), and the dissociation rate constant, $k_d$ (1/s), were calculated from the assay results. Then the KD (M) was calculated from ka and $k_d$ (Table 15). Furthermore, the pH-dependent binding was evaluated to calculate the KD ratio between pH 7.4 and pH 6.0 for each antibody.

[Table 15]

| Ligand | Sample_pH | ka (1/Ms) | kd (1/s) | KD (M) | KD(pH6.0)/KD(pH7.4) |
|---|---|---|---|---|---|
| PX268-IgG1 | pH7.4 | 5.2E+04 | 2.8E-04 | 5.4E-09 | 0.8 |
| | pH6.0 | 6.3E+04 | 2.7E-04 | 4.4E-09 | |
| PX141-IgG1 | pH7.4 | 1.5E+05 | 6.4E-04 | 4.2E-09 | 14.9 |
| | pH6.0 | 7.9E+04 | 4.9E-03 | 6.3E-08 | |

Preparation of pH-dependent anti-human plexin A1 antibodies having FcRn-binding activity under neutral conditions

Mutations were introduced into PX141-IgG1 comprising PX141H-IgG1 (SEQ ID NO: 26) and PX141L-CK (SEQ ID NO: 27) to augment the FcRn binding under a neutral condition (pH 7.4). Specifically, PX141H-IgG1-v2 (SEQ ID NO: 28) was prepared from the heavy chain constant region of IgG1 by substituting Trp for Asn at position 434 in EU numbering. The amino acid substitutions were introduced by the method known to those skilled in the art described in Reference Example 1.

PX268-IgG1 comprising PX268H-IgG1 (SEQ ID NO: 24) and PX268L-CK (SEQ ID NO: 25), PX141-IgG1 comprising PX141H-IgG1 (SEQ ID NO: 26) and PX141L-CK (SEQ ID NO: 27), and PX141-IgG1-v2 comprising PX141H-IgG1-v2 (SEQ ID NO: 28) and PX141L-CK (SEQ ID NO: 27) were expressed and purified by the method known to those skilled in the art described in Reference Example 2.

In vivo test using normal mice

The in vivo kinetics of soluble human plexin A1 (hsPlexin A1) and anti-human plexin A1 antibody was assessed after administering hsPlexin A1 alone or hsPlexin A1 and anti-human plexin A1 antibody in normal mice (C57BL/6J mouse; Charles River Japan). An hsPlexin A1 solution (100 microgram/ml) or a solution of mixture containing hsPlexin A1 and anti-human plexin A1 antibody (PX268-IgG1 group; 100 microgram/ml of hsPlexin A1 and 1.2 mg/ml of PX268-IgG1, PX141-IgG1 and PX141-IgG1-v2 group; 100 microgram/ml of hsPlexin A1 and 1.0 mg/ml of PX141-IgG1 and PX141-IgG1-v2, respectively) was administered once at a dose of 10 ml/kg into the caudal vein.

Dose of antibody was set so that more than 99.9% of soluble human plexin A1 was bound to the antibody in the administration solution. Blood was collected 15 minutes, seven hours, one day, two days, four days, seven days after administration of hsPlexin A1 and anti-human plexin A1 antibody solution mixture. The collected blood was immediately centrifuged at 15,000 rpm and 4 degrees C for 15 minutes to separate the plasma. The separated plasma was stored in a refrigerator at -20 degrees C or below before assay.

Measurement of human PlexinA1 plasma concentration by ELISA after administration of hsPlexin A1 alone

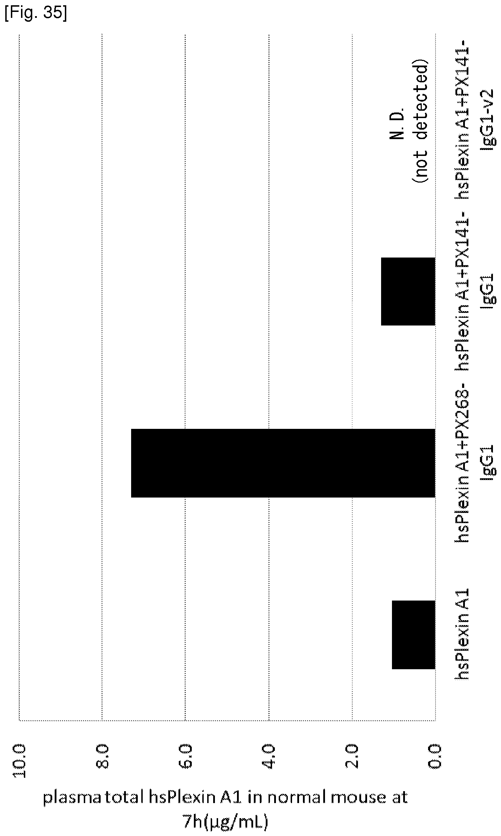

The concentration of human PlexinAI in mouse plasma was measured by ELISA using Biotinylated Anti-FLAG M2 Antibody (Sigma) because the recombinant human PlexinAI have FLAG-tag sequence end of C terminal. Rabbit anti-human plexin A1 polyclonal antibody prepared by immunizing plexin A1 to rabbit was dispensed onto a Nunc-Immuno-Plate MaxiSorp (Nalge Nunc International) and allowed to stand overnight at 4 degrees C to prepare anti-human PlexinAI-immobilized plates. Calibration curve samples having plasma concentrations of 25.6, 12.8, 6.4, 3.2, 1.6, and 0.8 microgram/ml, and mouse plasma samples diluted 100-fold or more were prepared. Subsequently, the samples were dispensed into the anti-human PlexinAI-immobilized plates, and allowed to stand for one hour at room temperature. Then, Biotinylated Anti-FLAG M2 Antibody (Sigma) was added to react for one hour at room temperature. Subsequently, Streptavidin-PolyHRP80 (Stereospecific Detection Technologies) was added to react for one hour at room temperature, and chromogenic reaction was carried out using TMB One Component HRP Microwell Substrate (BioFX Laboratories) as a substrate. After stopping the reaction with 1 N sulfuric acid (Showa Chemical), the absorbance at 450 nm was measured by a microplate reader. The concentration in mouse plasma was calculated from the absorbance of the calibration curve using the analytical software SOFTmax PRO (Molecular Devices). The time course of plasma hsPlexin A1 concentration after intravenous administration measured by this method is shown in Fig. 35.

Measurement of human PlexinA1 plasma concentration in PX268-IgG1 group by ELISA

The concentration of human PlexinAI in mouse plasma was measured by ELISA using Biotinylated Anti-FLAG M2 Antibody (Sigma) because the recombinant human PlexinAI have FLAG-tag sequence end of C terminal. Rabbit anti-human plexin A1 polyclonal antibody prepared by immunizing plexin A1 to rabbit was dispensed onto a Nunc-Immuno-Plate MaxiSorp (Nalge Nunc International) and allowed to stand overnight at 4 degrees C to prepare anti-human PlexinAI-immobilized plates. Calibration curve samples having plasma concentrations of 25.6, 12.8, 6.4, 3.2, 1.6, and 0.8 microgram/ml, and mouse plasma samples diluted 50-fold or more were prepared. In order to all human PlexinAI in sample bind to PX268-IgG1, 150 microliter of 40 microgram/ml PX268- IgG 1 was added to 150 microliter of the calibration curve samples and plasma samples, and then the samples were allowed to stand for overnight at 37 degrees C. Subsequently, the samples were dispensed into the anti-human PlexinAI-immobilized plates, and allowed to stand for one hour at room temperature (or 4 degrees C). Then, Biotinylated Anti-FLAG M2 Antibody (Sigma) was added to react for one hour at room temperature (or 4 degrees C). Subsequently, Streptavidin-PolyHRP80 (Stereospecific Detection Technologies) was added to react for one hour at room temperature (or 4 degrees C), and chromogenic reaction was carried out using TMB One Component HRP Microwell Substrate (BioFX Laboratories) as a substrate. After stopping the reaction with 1 N sulfuric acid (Showa Chemical), the absorbance at 450 nm was measured by a microplate reader. The concentration in mouse plasma was calculated from the absorbance of the calibration curve using the analytical software SOFTmax PRO (Molecular Devices). The time course of plasma hsPlexin A1 concentration after intravenous administration measured by this method is shown in Fig. 35.

Measurement of human PlexinA1 plasma concentration in PX141-IgG1 and PX141-IgG1-v2 group by ELISA

The concentration of human PlexinAI in mouse plasma was measured by ELISA using Biotinylated Anti-FLAG M2 Antibody (Sigma) because the recombinant human PlexinAI have FLAG-tag sequence end of C terminal. PX268-IgG1 was dispensed onto a Nunc-ImmunoPlate MaxiSorp (Nalge Nunc International) and allowed to stand overnight at 4 degrees C to prepare anti-human PlexinAI-immobilized plates. Calibration curve samples having plasma concentrations of 25.6, 12.8, 6.4, 3.2, 1.6, and 0.8 microgram/ml, and mouse plasma samples diluted 50-fold or more were prepared. In order to all human PlexinAI in sample bind to PX141-IgGI or PX141-IgGI-v2, 150 microliter of 40 microgram/ml PX141-IgGI or PX141-IgGI-v2 was added to 150 microliter of the calibration curve samples and plasma samples, and then the samples were allowed to stand for overnight at 37 degrees C. Subsequently, the samples were dispensed into the anti-human PlexinAI-immobilized plates, and allowed to stand for one hour at room temperature (or 4 degrees C). Then, Biotinylated Anti-FLAG M2 Antibody (Sigma) was added to react for one hour at room temperature (or 4 degrees C). Subsequently, Streptavidin-PolyHRP80 (Stereospecific Detection Technologies) was added to react for one hour at room temperature (or 4 degrees C), and chromogenic reaction was carried out using TMB One Component HRP Microwell Substrate (BioFX Laboratories) as a substrate. After stopping the reaction with 1 N sulfuric acid (Showa Chemical), the absorbance at 450 nm was measured by a microplate reader. The concentration in mouse plasma was calculated from the absorbance of the calibration curve using the analytical software SOFTmax PRO (Molecular Devices). The plasma hsPlexin A1 concentration at 7 hour after intravenous administration measured by this method is shown in Fig. 35.

Effect of pH-dependent binding to soluble human plexin A1

PX268-IgG1 and PX141-IgGI which binds to human IL-6 in a pH-dependent manner were tested in vivo, and the results were compared between them. Meanwhile, as shown in Fig. 35, hsPlexin A1 simultaneously administered with PX141-IgG1 which binds to soluble human plexin A1 in a pH-dependent manner was found to reduce the total plasma concentration of hsPlexin A1 as compared to hsPlexin A1 simultaneously administered with PX268- IgG 1.

Effect of FeRn binding under neutral condition (pH 7.4)

In addition to PX141-IgGI, PX141-IgGI-v2, which results from introducing the above-described amino acid substitutions into PX141-IgGI, were tested in vivo using normal mice. The test results were compared to that of PX141-IgG1.

As shown in Fig. 35, hsPlexin A1 simultaneously administered with PX141-IgG1-v2 which had increased binding to mouse FcRn under a neutral condition (pH 7.4) was demonstrated to reduce the total plasma concentration of hsPlexin A1 to a non-detectable level (detection limit is 0.8 microgram/mL). Thus, it was revealed that the soluble human plexin A1 concentration could be reduced by conferring mouse FcRn-binding ability under a neutral condition (pH 7.4). Specifically, this means that the elimination of soluble human plexin A1 could be accelerated by administering the antibody that binds to human plexinA1 in a pH-dependent manner and which is conferred with mouse FcRn-binding ability under a neutral condition (pH 7.4).

The findings described above demonstrate that the plasma antigen concentration not only of human soluble IL-6 receptor but also of antigen such as human IL-6, human IgA and human soluble plexin A1 can also be significantly reduced by administering an antibody having both pH-dependent antigen-binding ability and FcRn-binding ability under the neutral condition.

[Reference Example 1] Construction of expression vectors for IgG antibodies introduced with amino acid substitutions

The mutants were produced using the QuikChange Site-Directed Mutagenesis Kit (Stratagene) or In-Fusion HD Cloning Kit (Clontech) according to the method described in the instructions provided, and the resulting plasmid fragments were inserted into a mammalian cell expression vector to produce the desired H chain expression vectors and L chain expression vectors. The nucleotide sequences of the obtained expression vectors were determined using conventional methodologies known to persons skilled in the art.

[Reference Example 2] Expression and purification of IgG antibody

The antibodies were expressed by the method described below. Antibodies were expressed by Freestyle-HEK293 (Invitrogen) as described by the protocol provided by the manufacture or HEK293H cell line (Invitrogen). Human embryonic kidney cancer-derived HEK293H cell line (Invitrogen) was suspended in DMEM (Invitrogen) supplemented with 10% Fetal Bovine Serum (Invitrogen). The cells were plated at 10 ml per dish in dishes for adherent cells (10 cm in diameter; CORNING) at a cell density of 5 to 6 x $10^5$ cells/ml and cultured in a $CO_2$ incubator (37 degrees C, 5% $CO_2$) for one whole day and night. Then, the medium was removed by aspiration, and 6.9 ml of CHO-S-SFM-II medium (Invitrogen) was added. The prepared plasmid was introduced into the cells by the lipofection method. The resulting culture supernatants were collected, centrifuged (approximately 2,000 x g, 5 min, room temperature) to remove cells, and sterilized by filtering through 0.22-micrometer filter MILLEX (registered trademark)-GV (Millipore) to obtain the supernatants. Antibodies were purified from the obtained culture supernatants by a method known to those skilled in the art using rProtein A Sepharose™ Fast Flow (Amersham Biosciences). To determine the concentration of the purified antibody, absorbance was measured at 280 nm using a spectrophotometer. Antibody concentrations were calculated from the determined values using an absorbance coefficient calculated by the method described in Protein Science (1995) 4: 2411-2423.

[Reference Example 3] Preparation of soluble human IL-6 receptor (hsIL-6R)

Recombinant human IL-6 receptor as an antigen was prepared as follows. A cell line constitutively expressing soluble human IL-6 receptor (hereinafter referred to as hsIL-6R) having the amino acid sequence of positions 1 to 357 from the N terminus as reported in J. Immunol. 152: 4958-4968 (1994) was established by a method known to those skilled in the art. The cells were cultured to express hsIL-6R. The hsIL-6R was purified from the culture supernatant by two steps: Blue Sepharose 6 FF column chromatography and gel filtration chromatography. A fraction eluted as the main peak in the final stage was used as the final purification product.

[Reference Example 4] Preparation of human FcRn

FcRn is a complex of FcRn and beta2-microglobulin. Oligo-DNA primers were prepared based on the published human FcRn gene sequence (J Exp Med. 1994 Dec 1; 180(6): 2377-81). A DNA fragment encoding the whole gene was prepared by PCR using human cDNA (Human Placenta Marathon-Ready cDNA, Clontech) as a template and the prepared primers. Using the obtained DNA fragment as a template, a DNA fragment encoding the extracellular domain containing the signal region (Met1-Leu290) was amplified by PCR, and inserted into a mammalian cell expression vector. Likewise, oligo-DNA primers were prepared based on the published human beta2-microglobulin gene sequence (Proc. Natl. Acad. Sci. U.S.A. 99 (26): 16899-16903 (2002)). A DNA fragment encoding the whole gene was prepared by PCR using human cDNA (Human Placenta Marathon-Ready cDNA, Clontech) as a template and the prepared primers. Using the obtained DNA fragment as a template, a DNA fragment encoding the whole protein containing a signal region (Met1-Met119) was amplified by PCR and inserted into a mammalian cell expression vector.

Soluble human FcRn was expressed by the following procedure. The plasmids constructed for expressing human FcRn (SEQ ID NO: 30) and beta2-microglobulin (SEQ ID NO: 31) were introduced into cells of the human embryonic kidney cancer-derived cell line HEK293H (Invitrogen) by the lipofection method using PEI (Polyscience). The resulting culture supernatant was collected, and FcRn was purified using IgG Sepharose 6 Fast Flow (Amersham Biosciences), followed by further purification using HiTrap Q HP (GE Healthcare) (J Immunol. 2002 Nov 1; 169(9): 5171-80).

[Reference Example 5] Preparation of human IgA (hIgA)

hIgA comprising H (WT)-IgA1 (SEQ ID NO: 29) and L (WT) (SEQ ID NO: 5) was expressed and purified by the method known to those skilled in the art using rProtein L-agarose (ACTIgen) followed by gel filtration chromatography.

[Reference Example 6] Preparation of soluble human plexin A1 (hsPlexin A1)

Recombinant soluble human plexin A1 as an antigen (hereinafter referred to as hsPlexin A1) was prepared as follows. hsPlexin A1 was constructed by reference to NCBI Reference Sequence (NP_115618). Specially, hsPlexin A1 was comprised of the amino acid sequence of positions 27-1243 from the above-mentioned NCBI Reference FLAG-tag (DYKDDDDK) was connected to its C terminus. hsPlexin A1 was transiently expressed using FreeStyle293 (Invitrogen) and purified from the culture supernatant by two steps: anti-FLAG column chromatography and gel filtration chromatography. A fraction eluted as the main peak in the final stage was used as the final purification product.

Furthermore, the invention comprises the following items.

| | |
|---|---|
| **Item 1** | An antigen-binding molecule comprising an antigen-binding domain and a human FcRn-binding domain, which has a human FcRn-binding activity in the neutral pH ranges, wherein the human FcRn -binding activity in the neutral pH range is stronger than KD 3.2 micromolar. |
| **Item 2** | An antigen-binding molecule comprising an antigen-binding domain and a human FcRn-binding domain, which has a human FcRn-binding activity in the neutral pH ranges, wherein a human FcRn-binding activity in the neutral pH range is 28-fold stronger than an intact human IgG. |
| **Item 3** | An antigen-binding molecule comprising an antigen-binding domain and a human FcRn-binding domain, which has a human FcRn-binding activity in the neutral pH ranges, wherein the human FcRn-binding activity in the neutral pH range is stronger than KD 2.3 micromolar. |
| **Item 4** | An antigen-binding molecule comprising an antigen-binding domain and a human FcRn-binding domain, which has a human FcRn-binding activity in the neutral pH ranges, wherein the human FcRn-binding activity in the neutral pH range is 38-fold stronger than an intact human IgG. |
| **Item 5** | The antigen-binding molecule of any one of items 1 to 4, wherein the neutral pH range is pH 7.0 to 8.0. |
| **Item 6** | An antigen-binding molecule comprising an antigen-binding domain and a human FcRn-binding domain in which a total antigen concentration in plasma after administration of the antigen-binding molecule to non-human animal is lower than a total antigen concentration in plasma after administration of a reference antigen-binding molecule to non-human animal comprising the same antigen-binding domain and intact human IgG Fc domain as a human FcRn-binding domain. |
| **Item 7** | An antigen-binding molecule in which a plasma antigen concentration after administration of the antigen-binding molecule to non-human animal is lower than a total antigen concentration in plasma obtained from the non-human animal to which the antigen-binding molecule is not administered. |
| **Item 8** | An antigen-binding molecule comprising an antigen-binding domain and a human FcRn-binding domain wherein a molar antigen/ antigen-binding molecule ratio (C) of the antigen-binding molecule calculated as follows;<br><br>C=A/B, is lower than a molar antigen/antigen-binding molecule ratio (C') of a reference antigen-binding molecule comprising the same antigen-binding domain and intact human IgG Fc domain as a human FcRn-binding domain calculated as follows;<br><br>C'=A'/B',<br><br>wherein;<br><br>A is a total antigen concentration in plasma after administration of the antigen-binding molecule to non-human animal,<br><br>B is a plasma concentration of an antigen-binding molecule after administration of the antigen-binding molecule to non-human animal, |

(continued)

A' is a total antigen concentration in plasma after administration of a reference antigen-binding molecule to non-human animal,

B' is a plasma concentration of an antigen-binding molecule after administration of a reference antigen-binding molecule to non-human animal.

**Item 9** The antigen-binding molecule of any one of items 6 to 8, wherein the non-human animal is a human FcRn transgenic mouse.

**Item 10** The antigen-binding molecule of any one of items 6 to 9, wherein the antigen concentration in plasma is a long-term total antigen concentration in plasma.

**Item 11** The antigen-binding molecule of any one of items 6 to 9, wherein the antigen concentration in plasma is a short-term total antigen concentration in plasma.

**Item 12** An antigen-binding molecule comprising an antigen-binding domain and a human FcRn-binding domain, which has a human FcRn-binding activity in the acidic and neutral pH ranges, and a lower antigen-binding activity in the acidic pH range than in the neutral pH range, wherein the human FcRn-binding activity in the neutral pH range is stronger than that of an intact human IgG.

**Item 13** The antigen-binding molecule of any one of items 1 to 11, wherein antigen-binding activity of the antigen-binding domain in the acidic pH range is lower than that in the neutral pH range.

**Item 14** The antigen-binding molecule of item 12 or 13, wherein the ratio of antigen-binding activity in the acidic pH range and neutral pH range is at least 2 in the value of KD (in the acidic pH range) /KD (in the neutral pH range).

**Item 15** The antigen-binding molecule of any one of items 12 to 14, which comprises an amino acid mutation of the antigen-binding domain, which comprises a substitution of histidine for at least one amino acid of the antigen-binding domain or the insertion of at least one histidine.

**Item 16** The antigen-binding molecule of any one of items 12 to 14, wherein the antigen-binding domain is obtained from antigen-binding domain library.

**Item 17** The antigen-binding molecule of any one of items 1 to 16, which comprises as the human FcRn-binding domain an Fc domain resulting from substituting a different amino acid for at least one amino acid in the Fc domain of parent IgG.

**Item 18** The antigen-binding molecule of any one of items 1 to 17, wherein the human FcRn-binding domain is a human FcRn-binding domain comprising an amino acid sequence with a substitution of a different amino acid for at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the Fc domain of parent IgG.

**Item 19** The antigen-binding molecule of any one of items 1 to 18, which comprises a human FcRn-binding domain comprising amino acid substitution in the Fc domain of parent IgG which comprises at least one amino acid substitution selected from:

an amino acid substitution of Met for Gly at position 237;

an amino acid substitution of Ala for Pro at position 238;

an amino acid substitution of Lys for Ser at position 239;

an amino acid substitution of Ile for Lys at position 248;

an amino acid substitution of Ala, Phe, Ile, Met, Gln, Ser, Val, Trp, or Tyr for Thr at position 250;

an amino acid substitution of Phe, Trp, or Tyr for Met at position 252;

an amino acid substitution of Thr for Ser at position 254;

an amino acid substitution of Glu for Arg at position 255;

an amino acid substitution of Asp, Glu, or Gln for Thr at position 256;

an amino acid substitution of Ala, Gly, Ile, Leu, Met, Asn, Ser, Thr, or Val for Pro at position 257;

an amino acid substitution of His for Glu at position 258;

an amino acid substitution of Ala for Asp at position 265;

an amino acid substitution of Phe for Asp at position 270;

an amino acid substitution of Ala, or Glu for Asn at position 286;

an amino acid substitution of His for Thr at position 289;

an amino acid substitution of Ala for Asn at position 297;

an amino acid substitution of Gly for Ser at position 298;

an amino acid substitution of Ala for Val at position 303;

(continued)

an amino acid substitution of Ala for Val at position 305;

an amino acid substitution of Ala, Asp, Phe, Gly, His, Ile, Lys, Leu, Met, Asn, Pro, Gln, Arg, Ser, Val, Trp, or Tyr for Thr at position 307;

an amino acid substitution of Ala, Phe, Ile, Leu, Met, Pro, Gln, or Thr for Val at position 308;

an amino acid substitution of Ala, Asp, Glu, Pro, or Arg for Leu or Val at position 309;

an amino acid substitution of Ala, His, or Ile for Gln at position 311;

an amino acid substitution of Ala, or His for Asp at position 312;

an amino acid substitution of Lys, or Arg for Leu at position 314;

an amino acid substitution of Ala, or His for Asn at position 315;

an amino acid substitution of Ala for Lys at position 317;

an amino acid substitution of Gly for Asn at position 325;

an amino acid substitution of Val for Ile at position 332;

an amino acid substitution of Leu for Lys at position 334;

an amino acid substitution of His for Lys at position 360;

an amino acid substitution of Ala for Asp at position 376;

an amino acid substitution of Ala for Glu at position 380;

an amino acid substitution of Ala for Glu at position 382;

an amino acid substitution of Ala for Asn or Ser at position 384;

an amino acid substitution of Asp, or His for Gly at position 385;

an amino acid substitution of Pro for Gln at position 386;

an amino acid substitution of Glu for Pro at position 387;

an amino acid substitution of Ala, or Ser for Asn at position 389;

an amino acid substitution of Ala for Ser at position 424;

an amino acid substitution of Ala, Asp, Phe, Gly, His, Ile, Lys, Leu, Asn, Pro, Gln, Ser, Thr, Val, Trp, or Tyr for Met at position 428;

an amino acid substitution of Lys for His at position 433;

an amino acid substitution of Ala, Phe, His, Ser, Trp, or Tyr for Asn at position 434;

and an amino acid substitution of His for Tyr or Phe at position 436 in EU numbering.

**Item 20**   The antigen-binding molecule of any one of items 1 to 18, whose

human FcRn-binding domain comprises at least one amino acid selected from:

Met at amino acid position 237;

Ala at amino acid position 238;

Lys at amino acid position 239;

Ile at amino acid position 248;

Ala, Phe, Ile, Met, Gln, Ser, Val, Trp, or Tyr at amino acid at position 250;

Phe, Trp, or Tyr as an amino acid position 252;

Thr at amino acid position 254;

Glu at amino acid position 255;

Asp, Glu, or Gln at amino acid position 256;

Ala, Gly, Ile, Leu, Met, Asn, Ser, Thr, or Val at amino acid position 257;

His at amino acid position 258;

Ala at amino acid position 265;

Phe at amino acid position 270;

Ala or Glu at amino acid position 286;

His at amino acid position 289;

Ala at amino acid position 297;

Gly at amino acid position 298;

Ala at amino acid position 303;

Ala at amino acid position 305;

Ala, Asp, Phe, Gly, His, Ile, Lys, Leu, Met, Asn, Pro, Gln, Arg, Ser, Val, Trp, or Tyr at amino acid position 307;

Ala, Phe, Ile, Leu, Met, Pro, Gln, or Thr at amino acid position 308;

(continued)

Ala, Asp, Glu, Pro, or Arg at amino acid position 309;

Ala, His, or Ile at amino acid position 311;

Ala or His at amino acid position 312;

Lys or Arg at amino acid position 314;

Ala or His at amino acid position 315;

Ala at amino acid position 317;

Gly at amino acid position 325;

Val at amino acid position 332;

Leu at amino acid position 334;

His at amino acid position 360;

Ala at amino acid position 376;

Ala at amino acid position 380;

Ala at amino acid position 382;

Ala at amino acid position 384;

Asp or His at amino acid position 385;

Pro at amino acid position 386;

Glu at amino acid position 387;

Ala or Ser at amino acid position 389;

Ala at amino acid position 424;

Ala, Asp, Phe, Gly, His, Ile, Lys, Leu, Asn, Pro, Gln, Ser, Thr, Val, Trp, or Tyr at amino acid position 428;

Lys at amino acid position 433;

Ala, Phe, His, Ser, Trp, or Tyr at amino acid position 434;

and His as an amino acid at position 436 (EU numbering) in the Fc domain of a parent IgG.

**Item 21** The antigen-binding molecule of any one of items 18 to 20, wherein a parent IgG is selected from an IgG obtained from a non-human animal.

**Item 22** The antigen-binding molecule of any one of items 18 to 20, wherein a parent IgG is a human IgG.

**Item 23** The antigen-binding molecule of any one of items 1 to 22, which has an antagonistic activity.

**Item 24** The antigen-binding molecule of any one of items 1 to 23, which binds to a membrane antigen or soluble antigen.

**Item 25** The antigen-binding molecule of any one of items 1 to 24, wherein the antigen-binding domain comprises an artificial ligand which binds to a receptor.

**Item 26** The antigen-binding molecule of any one of items 1 to 24, wherein the antigen-binding domain comprises an artificial receptor which binds to a ligand.

**Item 27** The antigen-binding molecule of any one of items 1 to 24, which is an antibody.

**Item 28** The antigen-binding molecule of item 27, wherein the antibody is selected from a chimeric antibody, a humanized antibody or a human antibody.

**Item 29** A pharmaceutical composition comprising any one of the antigen-binding molecule of items 1 to 28.

**Item 30** A method for facilitating antigen-binding molecule-mediated antigen uptake into a cell by increasing its human FcRn-binding activity in the neutral pH range, wherein the antigen-binding molecule comprises an antigen-binding domain and a human FcRn-binding domain, and has a human FcRn-binding activity in the acidic pH range.

**Item 31** A method for facilitating antigen-binding molecule-mediated antigen uptake into a cell by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range.

**Item 32** A method for increasing the number of antigens to which a single antigen-binding molecule can bind by increasing its human FcRn-binding activity in the neutral pH range, wherein the antigen-binding molecule comprises an antigen-binding domain and a human FcRn-binding domain, and has a human FcRn-binding activity in the acidic pH range.

(continued)

**Item 33** A method for increasing the number of antigens to which a single antigen-binding molecule can bind by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range.

**Item 34** A method for augmenting the ability of an antigen-binding molecule to eliminate an antigen from plasma by increasing its human FcRn-binding activity in the neutral pH range, wherein the antigen-binding molecule comprises an antigen-binding domain and a human FcRn-binding domain, and has a human FcRn-binding activity in the acidic pH range.

**Item 35** A method for augmenting the ability of an antigen-binding molecule to eliminate an antigen from plasma by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range.

**Item 36** A method for improving pharmacokinetics of an antigen-binding molecule by increasing its human FcRn-binding activity in the neutral pH range, wherein the antigen-binding molecule comprises an antigen-binding domain and a human FcRn-binding domain, and has a human FcRn-binding activity in the acidic pH range.

**Item 37** A method for improving pharmacokinetics of an antigen-binding molecule by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range.

**Item 38** A method for facilitating intracellular dissociation of an antigen bound to an antigen-binding molecule outside the cell from the antigen-binding molecule, by increasing its human FcRn-binding activity in the neutral pH range and reducing its antigen-binding activity in the acidic pH range to less that in the neutral pH range, wherein the antigen-binding molecule comprises an antigen-binding domain and a human FcRn-binding domain, and has a human FcRn-binding activity in the acidic pH range.

**Item 39** A method for facilitating extracellular release of the antigen-free form of an antigen-binding molecule taken up into a cell in an antigen-bound form, by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range.

**Item 40** A method for reducing total or free plasma antigen concentration in plasma, by increasing its human FcRn-binding activity in the neutral pH range, wherein the antigen-binding molecule comprises an antigen-binding domain and a human FcRn-binding domain, and has a human FcRn-binding activity in the acidic pH range.

**Item 41** A method for reducing total or free plasma antigen concentration in plasma, by increasing its human FcRn-binding activity in the neutral pH range and reducing its antigen-binding activity in the acidic pH range to less than that in the neutral pH range, wherein the antigen-binding molecule comprises an antigen-binding domain and a human FcRn-binding domain, and has a human FcRn-binding activity in the acidic pH range.

**Item 42** The method of any one of items 30 to 41, wherein the acidic pH range is pH 5.5 to 6.5 and the neutral pH range is pH 7.0 to 8.0.

**Item 43** The method of any one of items 30 to 41, wherein the increase in the human FcRn-binding activity in the neutral pH range is an increase by substituting a different amino acid for at least one amino acid in the parent IgG Fc domain of the human FcRn-binding domain.

**Item 44** The method of any one of items 30 to 41, wherein the increase in the human FcRn-binding activity in the neutral pH range is an increase by substituting a different amino acid for at least one amino acid selected from those at positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the parent IgG Fc domain of the human FcRn-binding domain.

(continued)

**Item 45** The method of any one of items 31, 33, 35, 37 to 39, and 41, wherein the antigen-binding activity of the antigen-binding molecule in the acidic pH range is reduced to less than that in the neutral pH range by substituting histidine for at least one amino acid of the antigen-binding molecule or inserting at least one histidine.

**Item 46** The method of any one of items 31, 33, 35, 37 to 39, and 41, wherein the antigen-binding domain is obtained from antigen-binding domain library.

**Item 47** The method of any one of items 31, 33, 35, 37 to 39, and 41, wherein the decrease in the antigen-binding activity is represented by an increase in the value of KD (in the acidic pH range) /KD (in the neutral pH range) which is a ratio of antigen-binding activity in the acidic pH range and neutral pH range, relative to before histidine substitution or insertion.

**Item 48** A method for producing an antigen-binding molecule, which comprises the steps of:

(a) selecting an antigen-binding molecule that has stronger human FcRn-binding activity in the neutral pH range than KD 3.2 micromolar obtained by altering at least one amino acid in the human FcRn-binding domain of an antigen-binding molecule;

(b) obtaining a gene encoding an antigen-binding molecule in which a human FcRn-binding domain and an antigen-binding domain prepared in (a) are linked; and

(c) producing an antigen-binding molecule using the gene prepared in (b).

**Item 49** A method for producing an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) altering at least one amino acid in the antigen-binding domain of an antigen-binding molecule and selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(d) producing an antigen-binding molecule using the gene prepared in (c).

**Item 50** A method for producing an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(d) producing an antigen-binding molecule using the gene prepared in (c).

**Item 51** An antigen-binding molecule produced by the production method of any one of items 48 to 50.

**Item 52** A method for screening an antigen-binding molecule, which comprises the steps of:

(a) selecting an antigen-binding molecule that has stronger human FcRn-binding activity in the neutral pH range than KD 3.2 micromolar obtained by altering at least one amino acid in the human FcRn-binding domain of an antigen-binding molecule;

(b) obtaining a gene encoding an antigen-binding molecule in which a human FcRn-binding domain and an antigen-binding domain prepared in (a) are linked; and

(c) producing an antigen-binding molecule using the gene prepared in (b).

**Item 53** A method for screening an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) altering at least one amino acid in the antigen-binding domain of an antigen-binding molecule and selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(continued)

(d) producing an antigen-binding molecule using the gene prepared in (c).

Item 54   A method for screening an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(d) producing an antigen-binding molecule using the gene prepared in (c).

Item 55   The method of any one of items 30 to 54, wherein the antigen-binding domain comprises an artificial ligand which binds to a receptor.

Item 56   The method of any one of items 30 to 54, wherein the antigen-binding domain comprises an artificial receptor which binds to a ligand.

Item 57   The method of any one of items 30 to 54, wherein the antigen-binding molecule is an antibody.

## Claims

1. An antigen-binding molecule comprising an antigen-binding domain and a human FcRn-binding domain, which comprises as the human FcRn-binding domain an Fc domain resulting from substituting a different amino acid for at least one amino acid in the Fc domain of parent IgG and which has a human FcRn-binding activity in the neutral pH ranges, wherein

(i) the human FcRn -binding activity in the neutral pH range is stronger than KD 3.2 micromolar, or
(ii) a human FcRn-binding activity in the neutral pH range is 28-fold stronger than an intact human IgG, or
(iii) the human FcRn-binding activity in the neutral pH range is stronger than KD 2.3 micromolar, or
(iv) the human FcRn-binding activity in the neutral pH range is 38-fold stronger than an intact human IgG.

2. An antigen-binding molecule comprising an antigen-binding domain and a human FcRn-binding domain which is capable of

(i) reducing a total antigen concentration in plasma after administration of the antigen-binding molecule to non-human animal to a lower concentration than a reference antigen-binding molecule comprising the same antigen-binding domain and intact human IgG Fc domain as a human FcRn-binding domain, or
(ii) reducing a plasma antigen concentration after administration of the antigen-binding molecule to non-human animal to a lower concentration than a total antigen concentration in plasma obtained from the non-human animal to which the antigen-binding molecule is not administered, or
(iii) reducing a molar antigen/antigen-binding molecule ratio (C) of the antigen-binding molecule calculated as follows;

$$C=A/B,$$

to a lower ratio than a molar antigen/antigen-binding molecule ratio (C') of a reference antigen-binding molecule comprising the same antigen-binding domain and intact human IgG Fc domain as a human FcRn-binding domain calculated as follows;

$$C'=A'/B',$$

wherein;
A is a total antigen concentration in plasma after administration of the antigen-binding molecule to non-human animal,
B is a plasma concentration of an antigen-binding molecule after administration of the antigen-binding molecule to non-human animal,

A' is a total antigen concentration in plasma after administration of a reference antigen-binding molecule to non-human animal,

B' is a plasma concentration of an antigen-binding molecule after administration of a reference antigen-binding molecule to non-human animal.

3. An antigen-binding molecule comprising an antigen-binding domain and a human FcRn-binding domain, which has a human FcRn-binding activity in the acidic and neutral pH ranges, and a lower antigen-binding activity in the acidic pH range than in the neutral pH range, wherein the human FcRn-binding activity in the neutral pH range is stronger than that of an intact human IgG.

4. The antigen-binding molecule of any one of claims 1 to 3, wherein antigen-binding activity of the antigen-binding domain in the acidic pH range is lower than that in the neutral pH range, or wherein the ratio of antigen-binding activity in the acidic pH range and neutral pH range is at least 2 in the value of KD (in the acidic pH range) /KD (in the neutral pH range).

5. The antigen-binding molecule of any one of claims 3 or 4, which comprises an amino acid mutation of the antigen-binding domain, which comprises a substitution of histidine for at least one amino acid of the antigen-binding domain or the insertion of at least one histidine.

6. The antigen-binding molecule of any one of claims 1 to 5, wherein the human FcRn-binding domain is a human FcRn-binding domain comprising an amino acid sequence with a substitution of a different amino acid for at least one amino acid selected from those of positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 (EU numbering) in the Fc domain of parent IgG, preferably wherein the at least one amino acid substitution is selected from:

an amino acid substitution of Met for Gly at position 237;
an amino acid substitution of Ala for Pro at position 238;
an amino acid substitution of Lys for Ser at position 239;
an amino acid substitution of Ile for Lys at position 248;
an amino acid substitution of Ala, Phe, Ile, Met, Gln, Ser, Val, Trp, or Tyr for Thr at position 250;
an amino acid substitution of Phe, Trp, or Tyr for Met at position 252;
an amino acid substitution of Thr for Ser at position 254;
an amino acid substitution of Glu for Arg at position 255;
an amino acid substitution of Asp, Glu, or Gln for Thr at position 256;
an amino acid substitution of Ala, Gly, Ile, Leu, Met, Asn, Ser, Thr, or Val for Pro at position 257;
an amino acid substitution of His for Glu at position 258;
an amino acid substitution of Ala for Asp at position 265;
an amino acid substitution of Phe for Asp at position 270;
an amino acid substitution of Ala, or Glu for Asn at position 286;
an amino acid substitution of His for Thr at position 289;
an amino acid substitution of Ala for Asn at position 297;
an amino acid substitution of Gly for Ser at position 298;
an amino acid substitution of Ala for Val at position 303;
an amino acid substitution of Ala for Val at position 305;
an amino acid substitution of Ala, Asp, Phe, Gly, His, Ile, Lys, Leu, Met, Asn, Pro, Gin, Arg, Ser, Val, Trp, or Tyr for Thr at position 307;
an amino acid substitution of Ala, Phe, Ile, Leu, Met, Pro, Gln, or Thr for Val at position 308;
an amino acid substitution of Ala, Asp, Glu, Pro, or Arg for Leu or Val at position 309;
an amino acid substitution of Ala, His, or Ile for Gln at position 311;
an amino acid substitution of Ala, or His for Asp at position 312;
an amino acid substitution of Lys, or Arg for Leu at position 314;
an amino acid substitution of Ala, or His for Asn at position 315;
an amino acid substitution of Ala for Lys at position 317;
an amino acid substitution of Gly for Asn at position 325;
an amino acid substitution of Val for Ile at position 332;
an amino acid substitution of Leu for Lys at position 334;

an amino acid substitution of His for Lys at position 360;
an amino acid substitution of Ala for Asp at position 376;
an amino acid substitution of Ala for Glu at position 380;
an amino acid substitution of Ala for Glu at position 382;
an amino acid substitution of Ala for Asn or Ser at position 384;
an amino acid substitution of Asp, or His for Gly at position 385;
an amino acid substitution of Pro for Gln at position 386;
an amino acid substitution of Glu for Pro at position 387;
an amino acid substitution of Ala, or Ser for Asn at position 389;
an amino acid substitution of Ala for Ser at position 424;
an amino acid substitution of Ala, Asp, Phe, Gly, His, Ile, Lys, Leu, Asn, Pro, Gln, Ser, Thr, Val, Trp, or Tyr for Met at position 428;
an amino acid substitution of Lys for His at position 433;
an amino acid substitution of Ala, Phe, His, Ser, Trp, or Tyr for Asn at position 434;
and an amino acid substitution of His for Tyr or Phe at position 436in EU numbering.

7. The antigen-binding molecule of claim 6, wherein a parent IgG is selected from an IgG obtained from a non-human animal or wherein a parent IgG is a human IgG.

8. The antigen-binding molecule of any one of claims 1 to 7,

(i) which has an antagonistic activity, and/or
(ii) which binds to a membrane antigen or soluble antigen.

9. The antigen-binding molecule of any one of claims 1 to 8, wherein the antigen-binding domain comprises an artificial ligand which binds to a receptor, or wherein the antigen-binding domain comprises an artificial receptor which binds to a ligand.

10. The antigen-binding molecule of any one of claims 1 to 9, which is an antibody, preferably selected from a chimeric antibody, a humanized antibody or a human antibody.

11. A pharmaceutical composition comprising any one of the antigen-binding molecule of claims 1 to 10.

12. The antigen binding molecule of any one of claims 1 to 11 for use in

(i) facilitating antigen-binding molecule-mediated antigen uptake into a cell, or
(ii) increasing the number of antigens to which said antigen binding molecule can bind, or
(iii) augmenting the ability of an antigen-binding molecule to eliminate an antigen from plasma, or
(iv) improving pharmacokinetics of an antigen-binding molecule or
(v) facilitating intracellular dissociation of an antigen-binding molecule outside the cell from the antigen binding molecule, or
(vi) facilitating extracellular release of the antigen-free form of an antigen-binding molecule taken up into a cell an antigen bound form, or
(vii) reducing total or free plasma antigen concentration in plasma.

13. The method of any one of claims 1 to 12, wherein the acidic pH range is pH 5.5 to 6.5 and/or the neutral pH range is pH 7.0 to 8.0.

14. A method for producing an antigen-binding molecule, which comprises the steps of:

(a) selecting an antigen-binding molecule that has stronger human FcRn-binding activity in the neutral pH range than KD 3.2 micromolar obtained by altering at least one amino acid in the human FcRn-binding domain of an antigen-binding molecule;
(b) obtaining a gene encoding an antigen-binding molecule in which a human FcRn-binding domain and an antigen-binding domain prepared in (a) are linked; and
(c) producing an antigen-binding molecule using the gene prepared in (b).

15. A method for producing an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) altering at least one amino acid in the antigen-binding domain of an antigen-binding molecule and selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(d) producing an antigen-binding molecule using the gene prepared in (c).

16. A method for producing an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(d) producing an antigen-binding molecule using the gene prepared in (c).

17. An antigen-binding molecule produced by the production method of any one of claims 14 to 16.

18. A method for screening an antigen-binding molecule, which comprises the steps of:

(a) selecting an antigen-binding molecule that has stronger human FcRn-binding activity in the neutral pH range than KD 3.2 micromolar obtained by altering at least one amino acid in the human FcRn-binding domain of an antigen-binding molecule;

(b) obtaining a gene encoding an antigen-binding molecule in which a human FcRn-binding domain and an antigen-binding domain prepared in (a) are linked; and

(c) producing an antigen-binding molecule using the gene prepared in (b).

19. A method for screening an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) altering at least one amino acid in the antigen-binding domain of an antigen-binding molecule and selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(d) producing an antigen-binding molecule using the gene prepared in (c).

20. A method for screening an antigen-binding molecule, which comprises the steps of:

(a) selecting an antigen-binding molecule that has stronger human FcRn-binding activity in the neutral pH range than before alteration of at least one amino acid in the human FcRn-binding domain of an antigen-binding molecule having a human FcRn-binding activity in the acidic pH range;

(b) selecting an antigen-binding molecule that has stronger antigen-binding activity in the neutral pH range than in the acidic pH range;

(c) obtaining a gene encoding an antigen-binding molecule in which a human FcRn-binding domain and an antigen-binding domain prepared in (a) and (b) are linked; and

(d) producing an antigen-binding molecule using the gene prepared in (c).

[Fig. 1]

[Fig. 2]

[Fig. 3]

[Fig. 4]

[Fig. 5]

[Fig. 6]

[Fig. 7]

EP 4 501 956 A2

[Fig. 8]

[Fig. 9]

[Fig. 10]

[Fig. 11]

Legend:
- ● hsIL-6R+Fv4-IgG1
- □ hsIL-6R+Fv4-IgG1-v2
- ◇ hsIL-6R+Fv4-IgG1-F20
- ■ hsIL-6R+H54/L28-IgG1
- | hsIL-6R

X-axis: TIME (DAYS)

Y-axis: PLASMA CONCENTRATION OF SOLUBLE HUMAN IL-6 RECEPTOR (ng/mL)

[Fig. 12]

[Fig. 13]

[Fig. 14]

Plasma hsIL-6R concentration after 1 day of administration (ng/mL)

[Fig. 15]

[Fig. 16]

[Fig. 17]

[Fig. 18]

[Fig. 19]

[Fig. 20]

[Fig. 21]

[Fig. 22]

[Fig. 23]

[Fig. 24]

[Fig. 25]

EP 4 501 956 A2

[Fig. 26]

EP 4 501 956 A2

[Fig. 27]

[Fig. 28]

[Fig. 29]

[Fig. 30]

[Fig. 31]

[Fig. 32]

[Fig. 33]

[Fig. 34]

[Fig. 35]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010079667 A
- JP 2010250830 A
- WO 2009125825 A
- WO 2009086320 A
- WO 2008092117 A
- WO 2007041635 A
- WO 2006105338 A
- EP 125023 A
- WO 9602576 A
- WO 2004058821 A
- WO 2003002609 A
- WO 2005037989 A
- WO 2002020565 A
- WO 1995001937 A
- WO 2004044011 A
- WO 2005040229 A
- WO 2002032925 A
- WO 9954342 A
- WO 0061739 A
- WO 0231140 A
- WO 2006067847 A
- WO 2006067913 A
- WO 0279255 A
- WO 2006073941 A
- WO 2004106377 A
- WO 2008045140 A
- WO 2009113742 A
- EP 2154157 A
- US 20070141052 A
- WO 2000042072 A
- WO 2002060919 A
- WO 2006020114 A
- WO 2006031370 A
- WO 2010033279 A
- WO 2006053301 A
- WO 9813388 A
- EP 239400 A
- JP H20504970 A
- WO 9515393 A
- WO 9201047 A
- WO 9220791 A
- WO 9306213 A
- WO 9311236 A
- WO 9319172 A
- WO 9501438 A
- WO 9515388 A
- WO 9846777 A
- WO 9634096 A
- JP H0159878 B
- WO 9312227 A
- WO 9203918 A
- WO 9402602 A
- WO 9633735 A

### Non-patent literature cited in the description

- *J Immunol.*, 2002, vol. 169 (9), 5171-80
- *J Immunol.*, 2009, vol. 182 (12), 7663-71
- *J Biol Chem.*, 19 January 2007, vol. 282 (3), 1709-17
- *J Immunol.*, 01 November 2002, vol. 169 (9), 5171-80
- **JANICE M REICHERT** ; **CLARK J ROSENSWEIG** ; **LAURA B FADEN** ; **MATTHEW C DEWITZ**. Monoclonal antibody successes in the clinic. *Nature Biotechnology*, 2005, vol. 23, 1073-1078
- **PAVLOU AK** ; **BELSEY MJ.** The therapeutic antibodies market to 2008. *Eur J Pharm Biopharm.*, April 2005, vol. 59 (3), 389-96
- **KIM SJ** ; **PARK Y** ; **HONG HJ.** Antibody engineering for the development of therapeutic antibodies.. *Mol Cells*, 31 August 2005, vol. 20 (1), 17-29
- **HINTON PR** ; **XIONG JM** ; **JOHLFS MG** ; **TANG MT** ; **KELLER S** ; **TSURUSHITA N.** An engineered human IgG1 antibody with longer serum half-life.. *J Immunol.*, 01 January 2006, vol. 176 (1), 346-56
- **GHETIE V** ; **POPOV S** ; **BORVAK J** ; **RADU C** ; **MATESOI D** ; **MEDESAN C** ; **OBER RJ** ; **WARD ES**. Increasing the serum persistence of an IgG fragment by random mutagenesis.. *Nat Biotechnol.*, July 1997, vol. 15 (7), 637-40

- **RAJPAL A** ; **BEYAZ N** ; **HABER L** ; **CAPPUCCILLI G** ; **YEE H** ; **BHATT RR** ; **TAKEUCHI T** ; **LERNER RA** ; **CREA R**. A general method for greatly improving the affinity of antibodies by using combinatorial libraries. *Proc Natl Acad Sci USA.*, 06 June 2005, vol. 102 (24), 8466-71
- **WU H** ; **PFARR DS** ; **JOHNSON S** ; **BREWAH YA** ; **WOODS RM** ; **PATEL NK** ; **WHITE WI** ; **YOUNG JF** ; **KIENER PA**. Development of Motavizumab, an Ultra-potent Antibody for the Prevention of Respiratory Syncytial Virus Infection in the Upper and Lower Respiratory Tract. *J Mol Biol.*, 2007, vol. 368, 652-665
- **HANSON CV** ; **NISHIYAMA Y** ; **PAUL S.** Catalytic antibodies and their applications. *Curr Opin Biotechnol.*, December 2005, vol. 16 (6), 631-6
- **RATHANASWAMI P** ; **ROALSTAD S** ; **ROSKOS L** ; **SU QJ** ; **LACKIE S** ; **BABCOOK J.** Demonstration of an in vivo generated sub-picomolar affinity fully human monoclonal antibody to interleukin-8. *Biochem Biophys Res Commun.*, 09 September 2005, vol. 334 (4), 1004-13
- *Curr Opin Biotechnol.*, December 2009, vol. 20 (6), 685-91
- *Curr Opin Immunol.*, August 2008, vol. 20 (4), 460-70
- *Protein Eng Des Sel.*, April 2010, vol. 23 (4), 195-202
- *Int Immunol.*, December 2001, vol. 13 (12), 1551-9
- *Methods Mol Biol.*, 2010, vol. 602, 93-104
- *Clin Pharmacol.*, April 2008, vol. 48 (4), 406-17
- *Pharm Res.*, January 2006, vol. 23 (1), 95-103
- *Proc Natl Acad Sci USA.*, 14 March 2006, vol. 103 (11), 4005-10
- **KABAT et al.** Sequence of Proteins of Immunological Interest. National Institute of Health, 1987
- **CHOTHIA et al.** *Nature*, 1989, vol. 342, 877
- *Nat Biotechnol.*, September 2005, vol. 23 (9), 1126-36
- *Current Opinion in Biotechnology*, 2006, vol. 17, 653-658
- *Current Opinion in Biotechnology*, 2007, vol. 18, 1-10
- *Current Opinion in Structural Biology*, 1997, vol. 7, 463-469
- *Protein Science*, 2006, vol. 15, 14-27
- *Nat Med.*, January 2003, vol. 9 (1), 47-52
- *BioDrugs*, 2006, vol. 20 (3), 151-60
- *EMBO J.*, 15 December 1994, vol. 13 (24), 5863-70
- *FEBS Letter*, 1992, vol. 309 (1), 85-88
- *Angew. Chem. Int. Ed.*, 2005, vol. 44, 34

- *Chem Soc Rev.*, 10 September 2004, vol. 33 (7), 422-30
- *Amino Acids*, 1999, vol. 16 (3-4), 345-79
- **KABAT EA et al.** Sequences of Proteins of Immunological Interest. NIH, 1991
- **KABAT et al.** Sequences of Immunological Interest. Public Health Service, National Institutes of Health, 1991
- *Proc Natl Acad Sci USA.*, 23 July 1996, vol. 93 (15), 7843-8
- *J Immunol Methods.*, 20 October 2006, vol. 316 (1-2), 133-43
- *Methods Mol Biol.*, 2002, vol. 178, 87-100
- *J Immunol Methods.*, June 2004, vol. 289 (1-2), 65-80
- *Expert Opin Biol Ther.*, May 2007, vol. 7 (5), 763-79
- *Drug Metab Dispos.*, 03 January 2007, vol. 5 (1), 86-94
- *Int Immunol.*, 18 December 2006, vol. 12, 1759-69
- *J Biol Chem.*, 02 March 2001, vol. 276 (9), 6591-604
- *J Biol Chem.*, 2007, vol. 282 (3), 1709-17
- *Molecular Cell*, April 2001, vol. 7, 867-877
- *Nat Biotechnol.*, 15 July 1997, vol. 7, 637-40
- *Nat Biotechnol.*, 23 October 2005, vol. 10, 1283-8
- *Proc Natl Acad Sci USA.*, 05 December 2006, vol. 103 (49), 18709-14
- *Journal of Immunology*, 2009, vol. 182, 7663-7671
- *Drug Discov Today.*, January 2006, vol. 11 (1-2), 81-8
- **SATO et al.** *Cancer Res.*, 1993, vol. 53, 10.01-6
- **AMIT et al.** *Science*, 1986, vol. 233, 747-53
- **CHOTHIA et al.** *J. Mol. Biol.*, 1987, vol. 196, 901-17
- **CLACKSON et al.** *Nature*, 1991, vol. 352, 624-8
- **MARKS et al.** *J. Mol. Biol.*, 1991, vol. 222, 581-97
- **WATERHOUSES et al.** *Nucleic Acids Res.*, 1993, vol. 21, 2265-6
- **GRIFFITHS et al.** *EMBO J.*, 1994, vol. 13, 324.0-60
- **VAUGHAN et al.** *Nature Biotechnology*, 1996, vol. 14, 309-14
- **G. KOHLER** ; **C. MILSTEIN**. *Methods Enzymol.*, 1981, vol. 73, 3-46
- **MENDEZ et al.** *Nat. Genet.*, 1997, vol. 15, 146-56
- **GODING**. Monoclonal Antibodies: Principles and Practice. Academic Press, 1986, 59-103
- **K. SATO et al.** *Cancer Res.*, 1993, vol. 53, 10.01-10.06

- **KUNKEL**. *Proc. Natl. Acad. Sci. USA*, 1910, vol. 82, 488
- *J. Exp. Med.*, 1995, vol. 108
- **VALLE et al.** *Nature*, 1981, vol. 291, 338-340
- *The Journal of Immunology*, 2009, vol. 182, 7663-7671
- *Mol Cell Biol.*, 1988, vol. 8, 466-472
- Current protocols in Molecular Biology. John Wiley & Sons, 1987
- **EBERT et al.** *Bio/Technology*, 1994, vol. 12, 699-702
- **MA et al.** *Eur. J. Immunol.*, 1994, vol. 24, 131-138
- **COX KM et al.** *Nat. Biotechnol.*, December 2006, vol. 24 (12), 1591-1597
- Strategies for Protein Purification and Characterization: A Laboratory Course Manual. Cold Spring Harbor Laboratory Press, 1996
- *J Biol Chem.*, 18 August 2006, vol. 281 (33), 23514-24
- *Pharmacokinet Pharmacodyn.*, December 2001, vol. 28 (6), 507-32
- *Br J Clin Pharmacol.*, May 2007, vol. 63 (5), 548-61
- *J. Mol. Biol.*, 2003, vol. 324, 645-657
- *Protein Science*, 1995, vol. 4, 2411-2423
- *J. Immunol.*, 1994, vol. 152, 4958-4968
- *J Exp Med.*, 01 December 1994, vol. 180 (6), 2377-81
- *Proc. Natl. Acad. Sci. U.S.A.*, 2002, vol. 99 (26), 16899-16903